(19) Europäisches Patentamt
European Patent Office
Office européen des brevets

(11) EP 2 550 037 B1

(12) **EUROPÄISCHE PATENTSCHRIFT**

(45) Veröffentlichungstag und Bekanntmachung des Hinweises auf die Patenterteilung:
**16.05.2018 Patentblatt 2018/20**

(21) Anmeldenummer: **11709645.3**

(22) Anmeldetag: **18.03.2011**

(51) Int Cl.:
***A61M 1/28*** (2006.01) ***C08B 37/00*** (2006.01)
***B01D 61/24*** (2006.01) ***C08L 5/16*** (2006.01)
***A61M 1/16*** (2006.01) ***C08B 37/16*** (2006.01)

(86) Internationale Anmeldenummer:
**PCT/EP2011/001355**

(87) Internationale Veröffentlichungsnummer:
**WO 2011/116908 (29.09.2011 Gazette 2011/39)**

# (54) PHARMAZEUTISCHE ZUSAMMENSETZUNG ENTHALTEND CYCLODEXTRIN-COPOLYMER

PHARMACEUTICAL COMPOSITION CONTAINING CYCLODEXTRIN COPOLYMER

COMPOSITION PHARMACEUTIQUE RENFERMANT UN COPOLYMÈRE DE CYCLODEXTRINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.03.2010 DE 102010012282**

(43) Veröffentlichungstag der Anmeldung:
**30.01.2013 Patentblatt 2013/05**

(73) Patentinhaber: **Fresenius Medical Care Deutschland GmbH**
**61352 Bad Homburg (DE)**

(72) Erfinder:
- **FICHERT, Thomas**
  **48231 Warendorf (DE)**
- **BICHLMAIER, Ingo**
  **61206 Wöllstadt (DE)**

(74) Vertreter: **Breuninger, Marcus**
**Fresenius Medical Care AG & Co. KGaA**
**Global Patents & IP**
**Else-Kröner-Strasse 1**
**61352 Bad Homburg (DE)**

(56) Entgegenhaltungen:
**WO-A1-2009/048849 CN-A- 101 322 932**
**JP-A- 8 071 146 US-A- 4 535 152**
**US-A- 4 889 634 US-B1- 6 660 804**

- **CHIARI M ET AL: "Allylamine-beta-cyclodextrin copolymer - A novel chiral selector for capillary electrophoresis", JOURNAL OF CHROMATOGRAPHY, ELSEVIER SCIENCE PUBLISHERS B.V, NL, Bd. 894, Nr. 1-2, 13. Oktober 2000 (2000-10-13), Seiten 95-103, XP004218161, ISSN: 0021-9673, DOI: DOI:10.1016/S0021-9673(00)00740-8**
- **ZHAO X-B ET AL: "Sorption of unconjugated bilirubin by means of novel immobilized beta-cyclodextrin polymers", REACTIVE POLYMERS, ELSEVIER SCIENCE PUBLISHERS, AMSTERDAM, NL, Bd. 24, Nr. 1, 1. November 1994 (1994-11-01), Seiten 1-8, XP024184169, ISSN: 0923-1137, DOI: DOI:10.1016/0923-1137(94)90129-5 [gefunden am 1994-11-01]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft die Verwendung wasserlöslicher Cyclodextrin-Copolymere enthaltend wenigstens zwei Cyclodextrin-Monomere und wenigstens einen Linker in der Behandlung von Krankheiten des Urogenitalsystems.

Osmotisch aktive Verbindungen (Osmotika) finden in der Pharmazie und Medizin breite Anwendung. So werden Osmotika beispielsweise zur Einstellung der Tonizität von Arzneimitteln, insbesondere parenteralen Medikamenten verwendet. Hierbei wird der osmotische Druck eines Arzneimittels je nach Art der Anwendung hypotonisch, hypertonisch oder isotonisch eingestellt. Beispielsweise kann der osmotische Druck einer parenteralen Arzneilösung dem osmotischen Druck des menschlichen Bluts durch Zugabe eines Osmotikums angeglichen werden (isoosmotische Lösungen).

Ferner werden Osmotika bei der Dialysebehandlung, insbesondere bei der Peritonealdialyse eingesetzt, um dem Dialysepatienten überschüssiges Wasser zu entziehen.

Das Peritonealdialyseverfahren beruht darauf, dass eine Lösung, die osmotisch aktive Verbindungen enthält, über einen Katheter in die Bauchhöhle des Dialysepatienten eingebracht wird. Diese Lösung wird für eine bestimmte Zeit (üblicherweise einige Stunden) in der Bauchhöhle des Patienten belassen und entfaltet dort ihre osmotische Wirkung; d.h. dem Patienten wird körpereigenes Wasser in die Bauchhöhle entzogen. Nach einer bestimmten Verweildauer wird die nunmehr verdünnte Peritonealdialyselösung über einen Katheter abgelassen.

Dieses Prinzip findet in verschiedenen Verfahren der Peritonealdialysebehandlung Anwendung. Nach Bedarf können beispielsweise die Verfahren der intermittierenden (IPD), nächtlichen intermittierenden (NIPD), kontinuierlichen zyklischen (CCPD) oder kontinuierlichen ambulanten Peritonealdialyse (CAPD), angewendet werden. Bei IPD, NIPD und CAPD kommen Geräte zum Einsatz, die den Patienten bei der Durchführung des Peritonealdialyseverfahrens unterstützen. Die CAPD ist ein manuelles Verfahren.

Durch die Zugabe von osmotisch aktiven Verbindungen soll insbesondere gewährleistet werden, dass der osmotische Druck der Peritonealdialyselösung während der gesamten Verweildauer in der Bauchhöhle hoch genug ist, um dem Patienten Wasser zu entziehen; d.h. Wasser geht aus dem Kreislauf des Patienten in die Bauchhöhle über (Ultrafiltration).

Jedoch kommt es aufgrund des Wasserübertritts in die Bauchhöhle zwangsläufig zu einer Verdünnung der eingebrachten Peritonealdialyselösung. Diese Verdünnung hat zur Folge, dass die Konzentration der osmotisch aktiven Verbindung und somit auch der osmotische Druck dieser Lösung abnimmt.

Nimmt der osmotische Druck der Peritonealdialyselösung aufgrund dieser Verdünnung ab, so hat dies wiederum zur Folge, dass auch der pro Zeiteinheit stattfindende Wasserübertritt in die Bauchhöhle abnimmt oder möglicherweise gänzlich zum Erliegen kommt. In diesen Fällen findet also mit fortschreitender Verweildauer der Peritonealdialyselösung in der Bauchhöhle des Patienten kein effektiver Wasserentzug mehr statt.

Durch Absorption von osmotisch aktiven Verbindungen in den Blutkreislauf des Patienten, kann sich die Richtung des Wasserübertritts sogar umkehren, d.h. Wasser geht aus der Bauchhöhle in den Blutkreislauf des Patienten über (negative Ultrafiltration). Dies ist dann der Fall, wenn die verdünnte Peritonealdialyselösung in der Bauchhöhle einen geringeren osmotischen Druck als das körpereigene Wasser (z.B. das Blut) des Patienten aufweist.

Durch Zugabe geeigneter osmotisch aktiver Verbindungen zur Peritonealdialyselösung kann der osmotische Druck über eine für die Peritonealdialyse geeignete Behandlungsdauer aufrechterhalten werden, so dass es innerhalb der Verweildauer der Lösung in der Bauchhöhle zu keinem übermäßigen Rückgang der Ultrafiltration kommt. Somit wird auch eine negative Ultrafiltration weitestgehend verhindert.

Die in der Peritonealdialysebehandlung eingesetzten Lösungen enthalten in der Regel Zuckermonomere oder -polymere, wie beispielsweise Glucose oder Polyglucose (z.B. Stärkederivate), als osmotisch wirksame Verbindungen.

US 4889634 betrifft eine Peritonealdialyselösung, die Hydroxypropyl-β-cyclodextrin enthält.

US 4535152 offenbart Polymere von mit ionischen Gruppen substituierten Cyclodextrinen.

US 6660804 offenbart Cyclodextrin-Polymere, die durch Verestern von Polycarbonsäuren mit Cyclodextrinen erhalten werden.

CN 101322932 offenbart ein Adsorptionsmaterial auf Basis eines Cyclodextrin-Polymers.

Chiari et al. ("Allylamine-beta-cyclodextrin copolymer - A novel chiral selector for capillary electrophoresis", JOURNAL OF CHROMATOGRAPHY, ELSEVIER SCIENCE PUBLISHERS B.V, NL, (2000), 894 (1-2), 95-103) offenbaren ein Copolymer aus Allylamin und 2-Hydroxy-3-methacryloyl-β-Cyclodextrin als chiraler Selektor in der Kapillarelektrophorese.

JPH08071146 betrifft eine Peritonealdialyselösung, die α- oder γ-Cyclodextrin, 2-Hydroxyethylether-, 2-Hydroxypropylether-, 6-O-α-Glucosyl- oder 6-O-α-Maltosylderivate von α-, β- und γ-Cyclodextrin enthält.

Aufgabe der vorliegenden Erfindung ist es, neuartige Cyclodextrin-Copolymere zur Verfügung zu stellen, die als Osmotika, insbesondere in Dialyselösungen verwendet werden können.

Diese Aufgabe wird durch den Gegenstand der Patentansprüche gelöst.

Gegenstand dieser Erfindung sind Copolymere enthaltend wenigstens zwei Cyclodextrin-Monomere und wenigstens einen Linker; zur Verwendung in der Behandlung von Krankheiten des Urogenitalsystems.

Vorzugsweise enthalten die erfindungsgemäßen Copolymere wenigstens zwei gleiche oder verschiedene, unsubstituierte oder ein- oder mehrfach substituierte Cyclodextrin-Monomere und einen oder mehrere gleiche oder verschiedene, unsubstituierte oder ein- oder mehrfach substituierte Linker.

In einer bevorzugten Ausführungsform enthalten die erfindungsgemäßen Copolymere gleiche oder verschiedene Cyclodextrin-Monomere und gleiche oder verschiedene Linker, wobei der Stoffmengenanteil der Cyclodextrin-Monomere 5,0 bis 80 mol% und der Stoffmengenanteil der Linker 20 bis 95 mol% beträgt, bezogen auf die Gesamtstoffmenge der im Copolymer enthaltenen Monomere.

Der Ausdruck "Copolymer" steht im Sinne dieser Erfindung für makromolekulare Verbindungen mit einem Molekulargewicht von ≥ 2,0 kDa. Die erfindungsgemäßen Copolymere können statistische, alternierende, Block- oder vernetzte Copolymere sein (zur Definition der Begriffe wird verwiesen auf: A.D. Jenkins, P. Kratochvíl, R.F.T. Stepto, U.W. Suter. Glossary of basic terms in polymer science (IUPAC Recommendations 1996). Pure Appl. Chem., 1996, 68(12), 2287-2311).

Im Sinne dieser Erfindung steht der Ausdruck "Copolymer" sowohl für ein einzelnes erfindungsgemäßes Copolymer als auch für ein Gemisch, das zwei oder mehr verschiedene, z.B. 3, 4, 5, 6 oder 7, aber auch eine nicht bestimmbare Anzahl an verschiedenen erfindungsgemäßen Copolymeren enthält. Der Ausdruck "verschiedene Copolymere" umfasst für die Zwecke dieser Erfindung beispielsweise erfindungsgemäße Copolymere unterschiedlicher chemischer Struktur, die unterschiedliche Monomere enthalten oder Isomere voneinander sind und/ oder sich in ihrem Molekulargewicht, Polymerisationsgrad, Gesamtsubstitutionsgrad, Schmelzpunkt, $^{1}$H-NMR-, $^{13}$C-NMR-, IR- und/ oder MS-Spektrum voneinander unterscheiden.

Für die Zwecke dieser Erfindung umfasst der Ausdruck "Cyclodextrin-Verbindung" unsubstituiertes oder ein- oder mehrfach substituiertes α-, β- und γ-Cyclodextrin.

Cyclodextrine sind cyclische Oligosaccharide, wobei die Glucosyleinheiten der Ringstruktur 1→4-glycosidisch verknüpft sind. Die Cyclodextrine werden nach der Größe des Oligosaccharid-Rings, d.h. nach der Anzahl der in der Ringstruktur enthaltenen Glucosyleinheiten unterschieden: so enthält α-Cyclodextrin sechs, β-Cyclodextrin sieben, γ-Cyclodextrin acht und δ-Cyclodextrin neun Glucosyleinheiten in der Cyclodextrin-Ringstruktur.

Cyclodextrine (z.B. α-, β-, γ-, δ-Cyclodextrin) werden in der Regel durch biologische (z.B. enzymatische) Verfahren hergestellt. Die mittels dieser biologischen Herstellungsverfahren erhaltenen Produkte sind meistens Gemische, die Cyclodextrine unterschiedlicher Ringgröße enthalten. Die durch Aufreinigung dieser Gemische erhaltenen Produkte enthalten überwiegend Cyclodextrin einer einheitlichen Ringgröße, können jedoch noch immer Mengen an Cyclodextrinen anderer Ringgröße enthalten. Dies liegt insbesondere an den ähnlichen physiko-chemischen Eigenschaften der Cyclodextrine unterschiedlicher Ringgröße und der damit verbundenen schwierigen Isolierung eines Cyclodextrins einer bestimmten Ringgröße. Beispielsweise kann das Produkt β-Cyclodextrin mit einer vom Hersteller ausgewiesenen Reinheit von ≥ 98% noch immer kleine Mengen (Spuren) an anderen Cyclodextrinen (z.B. α-Cyclodextrin, γ-Cyclodextrin, δ-Cyclodextrin) enthalten. Aus diesem Grund können die erfindungsgemäßen Copolymere, die Cyclodextrin-Monomere bestimmter Ringgrößen enthalten, auch Spuren (geringe Mengen) an Cyclodextrin-Monomeren anderer Ringgrößen enthalten. Beispielsweise kann das erfindungsgemäße β-Cyclodextrin-Epichlorhydrin-Copolymer auch Spuren an α-, β-, γ- und/ oder δ-Cyclodextrin-Monomeren enthalten.

Der Ausdruck "Spuren an Cyclodextrin-Monomeren anderer Ringgrößen" bedeutet im Sinne dieser Erfindung, dass diese anderen Cyclodextrin-Monomere in Mengen von vorzugsweise ≤ 10 Gew.-%, bevorzugter ≤ 5,0 Gew.-%, noch bevorzugter ≤ 2,5 Gew.-%, am Bevorzugtesten ≤ 1,0 Gew.-% und insbesondere ≤ 0,50 Gew.-% im erfindungsgemäßen Cyclodextrin-Copolymer enthalten sein können, bezogen auf die Gesamtmasse der im erfindungsgemäßen Cyclodextrin-Copolymer enthaltenen Cyclodextrin-Monomere. Die Cyclodextrin-Monomere anderer Ringgrößen können auch in analytisch nicht-messbaren Mengen im erfindungsgemäßen Copolymer enthalten sein, wobei der Ausdruck "analytisch nicht messbar" im Sinne dieser Erfindung für Mengen steht, die unterhalb der Nachweisgrenze eines analytischen Nachweisverfahrens (z.B. NMR, LC/MS) liegen.

Der Ausdruck "Monomer" steht für die Zwecke dieser Erfindung für die in Copolymeren enthaltenen Strukturelemente, die sich von Cyclodextrin-Verbindungen und Vernetzungsmitteln ableiten; so umfasst der Ausdruck "Linker" (Vernetzungsmittel-Monomer) die von Vernetzungsmitteln und der Ausdruck "Cyclodextrin-Monomer" die von Cyclodextrin-Verbindungen abgeleiteten Strukturelemente.

Wird beispielsweise die unsubstituierte Cyclodextrin-Verbindung α-Cyclodextrin mit dem Vernetzungsmittel Epichlorhydrin umgesetzt, so kann u.a. das folgende α-Cyclodextrin-Epichlorhydrin-Copolymer erhalten werden:

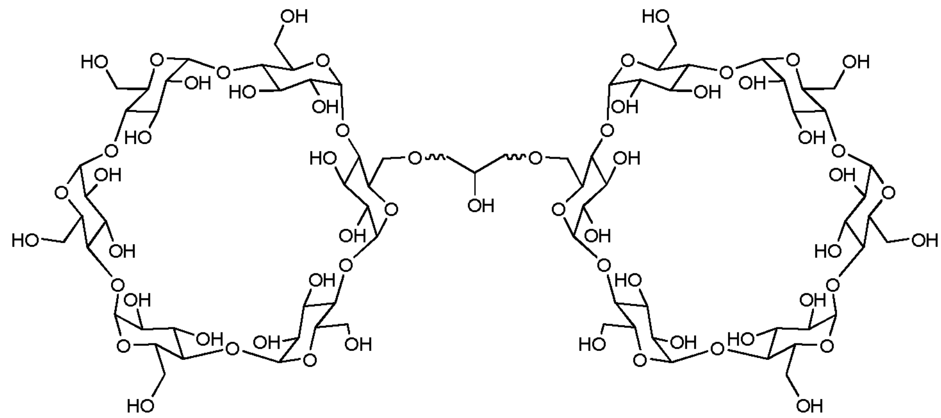

Im α-Cyclodextrin-Epichlorhydrin-Copolymer dieses Beispiels ist das

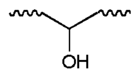

der Linker bzw. Epichlorhydrin-Linker und Strukturelement das

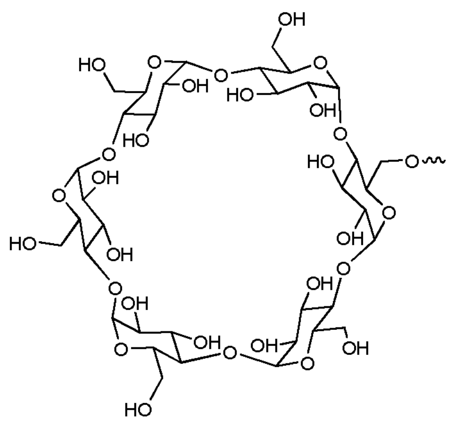

das Cyclodextrin-Monomer Strukturelement bzw. α-Cyclodextrin-Monomer, wobei das Symbol



für eine kovalente Bindung zwischen dem Linker und Cyclodextrin-Monomer steht.

Im Sinne dieser Erfindung steht der Ausdruck "vernetzt" für die kovalente Verbindung wenigstens zweier Cyclodextrin-Monomere durch wenigstens einen Linker. Die Vernetzung erfolgt in der Regel durch Ausbildung von kovalenten Bindungen zwischen einem bi- oder polyfunktionellen Vernetzungsmittel und nukleophilen Resten von wenigstens zwei Cyclodextrin-Verbindungen. Nukleophile Reste im Sinne dieser Erfindung sind beispielsweise Sauerstoffatome unsubstituierter Hydroxygruppen der Glucosyleinheiten von Cyclodextrin-Verbindungen oder Sauerstoffatome unsubstituierter Hydroxygruppen von Hydroxyalkyl-Substituenten, die an Cyclodextrin-Verbindungen gebunden sind. Die Vernetzung kann somit über Sauerstoffatome unsubstituierter Hydroxygruppen des Cyclodextrin-Ringsystems als auch über Sauerstoffatome unsubstituierter Hydroxygruppen entsprechender Substituenten, die an die Cyclodextrin-Verbindung gebunden sind, erfolgen. Beispielsweise sind jedoch auch Stickstoffatome von primären, sekundären und tertiären Aminogruppen sowie Schwefelatome von unsubstituierten Thiolgruppen nukleophile Reste im Sinne dieser Erfindung. Im Sinne dieser Erfindung werden diese nukleophilen Reste auch als "nukleophile Hydroxygruppen (-OH)", "nukleophile Thiolgruppe (-SH)" und "nukleophile Aminogruppen bezeichnet.

Für die Zwecke dieser Erfindung sind Cyclodextrin-Monomere unsubstituiert oder ein- oder mehrfach mit gleichen oder verschiedenen Resten substituiert.

In Bezug auf die Cyclodextrin-Monomere und Cyclodextrin-Verbindungen versteht man im Sinne dieser Erfindung unter "einfach substituiert" die einfache Substitution eines Wasserstoffrests von einer der unsubstituierten Hydroxygruppen, die in den Cyclodextrin-Monomeren und Cyclodextrin-Verbindungen enthaltenen sind.

In Bezug auf die Cyclodextrin-Monomere und Cyclodextrin-Verbindungen versteht man im Sinne dieser Erfindung unter "mehrfach substituiert" die mehrfache (z.B. zwei-, drei-, vier- oder fünffache) Substitution der Wasserstoffreste von mehreren unsubstituierten Hydroxygruppen (z.B. 2, 3, 4 oder 5), die in den Cyclodextrin-Monomeren und Cyclodextrin-Verbindungen enthalten sind, durch mehrere gleiche oder verschiedene Substituenten. Des Weiteren können jedoch auch die Substituenten (Reste) der Cyclodextrin-Monomere und Cyclodextrin-Verbindungen weiter ein- oder mehrfach substituiert sein.

In Bezug auf die Linker versteht man unter dem Ausdruck "ein- oder mehrfach substituiert" im Sinne dieser Erfindung die Substitution eines oder mehrerer Wasserstoffreste durch einen oder mehrere Substituenten; insbesondere die Substitution des Wasserstoffrests einer Hydroxygruppe oder die Substitution der Wasserstoffreste mehrerer Hydroxygruppen durch einen oder mehrere gleiche oder verschiedene Substituenten.

In Bezug auf die Substituenten versteht man unter dem Ausdruck "ein- oder mehrfach substituiert" im Sinne dieser Erfindung die Substitution des Wasserstoffrests einer Hydroxy- oder Thiolgruppe oder die Substitution der Wasserstoffreste mehrerer Hydroxy- oder Thiolgruppen durch einen oder mehrere gleiche oder verschiedene Substituenten. Des Weiteren können auch die in Substituenten und Resten enthaltenen primären, sekundären oder tertiären Aminogruppen substituiert sein.

Für die Zwecke dieser Erfindung stehen die Ausdrücke "Krankheiten des Urogenitalsystems" und "Niereninsuffizienz" für medizinische Indikationen, wie sie von der Weltgesundheitsorganisation in der "Internationalen statistischen Klassifikation der Krankheiten und verwandter Gesundheitsprobleme" definiert sind (ICD, 10. Revision, German Modification, Version 2009; im Folgenden als "ICD-10" bezeichnet). Hierbei umfasst die Indikation "Krankheiten des Urogenitalsystems" die Abschnitte N00 bis N99 und die Indikation "Niereninsuffizienz" die Abschnitte N17 bis N19 der ICD-10.

Die erfindungsgemäßen Copolymere eignen sich zur Verwendung in der Behandlung von Krankheiten des Urogenitalsystem und können vorzugsweise zur Verwendung in der Behandlung von Niereninsuffizienz eingesetzt werden. Die erfindungsgemäßen Copolymere sind insbesondere geeignet zur Verwendung in der Dialysebehandlung (Hämodialyse oder Peritonealdialyse) und können in der Peritonealdialysebehandlung eingesetzt werden.

Im Sinne dieser Erfindung steht das Zeichen "≥" für "größer oder gleich", das Zeichen "≤" für "kleiner oder gleich", das Zeichen "-" für eine negative Ladung und das Zeichen "+" für eine positive Ladung.

In einer bevorzugten Ausführungsform beträgt der Stoffmengenanteil der im erfindungsgemäßen Copolymer enthaltenen Linker 20 bis 95 mol%, bevorzugter 25 bis 80 mol%, noch bevorzugter 30 bis 70 mol%, am Bevorzugtesten 30 bis 60 mol% und insbesondere 30 bis 50 mol%, bezogen auf die Gesamtstoffmenge der Monomere, die im erfindungsgemäßen Copolymer enthalten sind.

Der Stoffmengenanteil der im erfindungsgemäßen Copolymer enthaltenen Cyclodextrin-Monomere beträgt vorzugsweise 5,0 bis 80 mol%, bevorzugter 10 bis 75 mol%, noch bevorzugter 20 bis 70 mol%, am Bevorzugtesten 30 bis 70 mol% und insbesondere 40 bis 70 mol%, bezogen auf die Gesamtstoffmenge der Monomere, die im erfindungsgemäßen Copolymer enthalten sind.

In einer weiteren bevorzugten Ausführungsform ist die Summe aus dem Stoffmengenanteil der Linker und dem Stoffmengenanteil der Cyclodextrin-Monomere ≥ 25 mol%, ≥ 30 mol% oder ≥ 40 mol%, bevorzugter ≥ 50 mol% oder ≥ 60 mol%, noch bevorzugter ≥ 70 mol% oder ≥ 80 mol%, am Bevorzugtesten ≥ 90 mol% oder ≥ 95 mol%, und insbesondere 100 mol%, bezogen auf die Gesamtstoffmenge der Monomere, die im erfindungsgemäßen Copolymer enthalten sind.

Aufgrund von Verunreinigungen in den Edukten (Ausgangsstoffen) kann in der Regel ein Wert von exakt 100 mol% in der Praxis nicht erreicht werden. Der Wert 100 mol% ist deshalb im Sinne dieser Erfindung so zu verstehen, dass außer Cyclodextrin-Verbindungen, Vernetzungsmitteln und ggf. Funktionalisierungsmitteln, keine weiteren Verbindungen zur Herstellung des erfindungsgemäßen Copolymers verwendet wurden.

Die Wasserlöslichkeit der erfindungsgemäßen Copolymere ist bei 23 °C vorzugsweise ≥ 5,0 g $l^{-1}$, ≥ 10 g $l^{-1}$, ≥ 25 g $l^{-1}$ oder ≥ 50 g $l^{-1}$, bevorzugter ≥ 75 g $l^{-1}$, ≥ 100 g $l^{-1}$, ≥ 125 g $l^{-1}$ oder ≥ 150 g $l^{-1}$, noch bevorzugter ≥ 175 g $l^{-1}$, ≥ 200 g $l^{-1}$, ≥ 225 g $l^{-1}$ oder ≥ 250 g $l^{-1}$, am Bevorzugtesten ≥ 300 g $l^{-1}$ oder ≥ 400 g $l^{-1}$ und insbesondere ≥ 500 g $l^{-1}$.

Die Osmolalität einer 50 mM wässrigen Lösung an erfindungsgemäßen Copolymer ist vorzugsweise ≥ 50 mosm $kg^{-1}$, ≥ 75 mosm $kg^{-1}$ oder ≥ 100 mosm $kg^{-1}$, bevorzugter ≥ 125 mosm $kg^{-1}$, ≥ 150 mosm $kg^{-1}$ oder ≥ 175 mosm $kg^{-1}$, noch bevorzugter ≥ 200 mosm $kg^{-1}$ oder ≥ 225 mosm $kg^{-1}$, am Bevorzugtesten ≥ 250 mosm $kg^{-1}$ oder ≥ 275 mosm $kg^{-1}$ und insbesondere ≥ 300 mosm $kg^{-1}$.

Der Polymerisationsgrad der erfindungsgemäßen Copolymere ist vorzugsweise ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

Für die Zwecke dieser Erfindung steht der Ausdruck "Polymerisationsgrad" für folgenden Quotienten:

$$\text{Polymerisationsgrad} = \frac{M(qCD)}{M(CDM)},$$

wobei M(qCD) für das mittlere Molekulargewicht des erfindungsgemäßen Copolymers steht und M(CDM) für das mittlere Molekulargewicht der Cyclodextrin-Monomere steht.

Das mittlere Molekulargewicht der erfindungsgemäßen Copolymere ist vorzugsweise ≥ 2,0 kDa, bevorzugter ≥ 2,5 kDa, noch bevorzugter ≥ 3,0 kDa, am Bevorzugtesten ≥ 4,0 kDa und insbesondere ≥ 5,0 kDa. Das Molekulargewicht wird vorzugsweise mit Hilfe eines Membranosmometers gemessen (destilliertes Wasser, 23 °C).

In einer weiteren bevorzugten Ausführungsform ist das mittleres Molekulargewicht (M) der erfindungsgemäßen Polymere vorzugsweise 2,0 ≤ M ≤ 100 kDa, bevorzugter 2,0 ≤ M ≤ 50 kDa, noch bevorzugter 2,0 ≤ M ≤ 25 kDa, am Bevorzugtesten 2,0 ≤ M ≤ 15 kDa und insbesondere 2,0 ≤ M ≤ 10 kDa.

In einer weiteren bevorzugten Ausführungsform ist der mittlere Partikeldurchmesser der erfindungsgemäßen Copolymere ≤ 1000 μm, ≤ 900 μm oder ≤ 800 μm, bevorzugter ≤ 700 μm, ≤ 600 μm oder ≤ 500 μm, noch bevorzugter ≤ 400 μm, ≤ 300 μm oder ≤ 200 μm, am Bevorzugtesten ≤ 100 μm, ≤ 75 μm oder ≤ 50 μm und insbesondere ≤ 25 μm.

In einer bevorzugten Ausführungsform sind die Cyclodextrin-Monomere, gleich oder verschieden, abgeleitet von α-, β- oder γ-Cyclodextrin, die jeweils unsubstituiert oder ein- oder mehrfach substituiert sind. Im Sinne dieser Erfindung werden unsubstituiertes, ein- oder mehrfach substituierte α-, β- und γ-Cyclodextrine als "Cyclodextrin-Verbindungen" bezeichnet.

Im Sinne dieser Erfindung steht der Ausdruck "von Cyclodextrin-Verbindungen abgeleitete Cyclodextrin-Monomere" für Strukturelemente, die in den erfindungsgemäßen Copolymeren enthalten sind und auf die strukturelle Abwandlung der Cyclodextrin-Verbindungen durch die Vernetzungsreaktion mit Vernetzungsmitteln zurückzuführen sind (vgl. hierzu auch die oben stehende Beispiel reaktion).

Die von Cyclodextrin-Verbindungen abgeleiteten Cyclodextrin-Monomere sind für die Zwecke dieser Erfindung vorzugsweise unsubstituierte, ein- oder mehrfach substituierte Monomere des erfindungsgemäßen Copolymers, die wenigstens eine kovalente Bindung zu einem Linker (Vernetzungsmittel-Monomer) enthalten; d.h. beispielsweise, dass wenigstens der Wasserstoffrest einer unsubstituierten Hydroxygruppe oder Thiolgruppe der Cyclodextrin-Verbindung durch eine kovalente Bindung zu einem Vernetzungsmittel-Monomer ersetzt ist. Des Weiteren kann die kovalente Bindung auch über das Stickstoffatom einer Aminogruppe substituierter Cyclodextrin-Verbindungen erfolgen.

Im Sinne dieser Erfindung steht der Ausdruck "unsubstituierte Hydroxygruppen der Cyclodextrin-Verbindungen" für OH-Reste in den Positionen 2, 4 und/ oder 6 jeder Glucosyleinheit der Cyclodextrin-Ringsysteme aber auch für Hydroxygruppen, die in den Substituenten (Resten) substituierter Cyclodextrin-Verbindungen enthalten sind.

Für die Zwecke dieser Erfindung steht der Ausdruck "unsubstituierte Thiolgruppen der Cyclodextrin-Verbindungen" für SH-Reste, die in den Substituenten (Resten) der substituierten Cyclodextrin-Verbindungen enthalten sind.

Der Ausdruck "Aminogruppen substituierter Cyclodextrin-Verbindungen" umfasst im Sinne dieser Erfindung primäre ($--NH_2$), sekundäre [z.B. $--NH(CH_3)$] und tertiäre Aminogruppen [z.B. $-N(CH_3)_2$], die in den Substituenten (Resten) von substituierten Cyclodextrin-Verbindungen enthalten sind, wobei das Symbol "--" für die Bindung zu einem Kohlenstoffatom des entsprechenden Substituenten (Rests) steht.

Die Substituenten (Reste) der Cyclodextrin-Verbindungen sind vorzugsweise abgeleitet von einem oder mehreren gleichen oder verschiedenen Funktionalisierungsmitteln; die wiederum ggf. weiter substituiert sein können.

Die Substituenten sind vorzugsweise abgeleitet von Funktionalisierungsmitteln ausgewählt aus der Gruppe bestehend aus $C_{1-6}$-Alkyl-Y, $C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-7}$-Cycloalkyl-C(=O)-OH, Y-$C_{1-6}$-Alkyl-C(=O)-O-$C_{1-6}$-Alkyl, Y-$C_{3-7}$-Cycloalkyl-C(=O)-O-$C_{3-7}$-Cycloalkyl, $[C_{1-6}$-Alkyl-$C(=O)]_2O$, $[C_{3-7}$-Cycloalkyl-$C(=O)]_2O$, Dihydrofuran-2,5-dion, Furan-2,5-dion, Dihydro-2H-pyran-2,6(3H)-dion, Oxiran, $C_{1-6}$-Alkyloxiran, $C_{3-7}$-Cycloalkyloxiran, Di-$C_{1-6}$-Alkyloxiran, Di-$C_{3-7}$-Cycloalkyloxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, ($C_{1-6}$-Alkyl)$_2$NH, ($C_{3-7}$-Cycloalkyl)$_2$NH, ($C_{1-6}$-Alkyl)$_3$N, ($C_{3-7}$-Cycloalkyl)$_3$N, [(Y-$C_{1-6}$-Alkyl)N($C_{1-6}$-Alkyl)$_3$]$^+$ Halogenid, 1,3-Propansulton und 1,4-Butansulton; wobei $C_{1-6}$-Alkyl und $C_{3-7}$-Cycloalkyl, unabhängig voneinander, unsubstituiert oder ein- oder mehrfach mit gleichen oder verschiedenen Resten Z substituiert sein können, wobei die weiteren Substituenten Z zu keiner oder zu keiner wesentlichen Vernetzung der Cyclodextrin-Monomere bzw. -Verbindungen führen; Halogenid vorzugsweise für Chlorid, Bromid oder Iodid, besonders bevorzugt für Chlorid oder Bromid und insbesondere für Chlorid steht; und die Reste Y für Abgangsgruppen stehen.

Die Abgangsgruppen Y sind, unabhängig voneinander, vorzugsweise ausgewählt aus der Gruppe bestehend aus -Cl, -Br, -I, -Mesyl, -Tosyl und Epoxy. Besonders vorteilhaft sind -Cl, -Br, -Mesyl und -Tosyl; insbesondere -Cl.

Die Substituenten Z sind, unabhängig voneinander, vorzugsweise ausgewählt aus der Gruppe bestehend aus -OH, =O, -C(=O)OH, $-C(=O)OCH_3$, $-C(=O)OCH_2CH_3$, $-NH_2$, $-NH(CH_3)$, $-N(CH_3)_2$, $-N(CH_3)_3{}^+A^-$, $-NH(CH_2CH_3)$, $-N(CH_2CH_3)_2$ und $-N(CH_2CH_3)_3{}^+A^-$; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht. Besonders vorteilhaft sind -OH, =O, -C(=O)OH, -C(=O)$OCH_3$ und -C(=O)$OCH_2CH_3$; insbesondere -OH, =O und -C(=O)OH.

In einer weiteren bevorzugten Ausführungsform sind die Substituenten abgeleitet von wenigstens einem, vorzugsweise nur einem Funktionalisierungsmittel ausgewählt aus der Gruppe bestehend aus $CH_3$-$Y^1$, $CH_3$-$CH_2$-$Y^1$, $CH_3$-$CH_2$-$CH_2$-$Y^1$, $(CH_3)_2$CH-$Y^1$, $CH_3$-$CH_2$-$CH_2$-$CH_2$-$Y^1$, $(CH_3)_2$CH-$CH_2$-$Y^1$, $CH_3$-$CH_2$-CH($CH_3$)-$Y^1$, $(CH_3)_3$C-$Y^1$, $CH_2$=CH-$Y^1$, $CH_2$=CH-$CH_2$-$Y^1$, Cyclopropyl-$Y^1$, $Y^1$-$CH_2$-C(=O)-OH, $Y^{1-}$$CH_2$-$CH_2$-C(=O)-OH, $Y^1$-CH($CH_3$)-C(=O)-OH, $Y^1$-$CH_2$-C(=O)-O-$CH_3$, $Y^1$-$CH_2$-$CH_2$-C(=O)-O-$CH_3$, $Y^1$-CH($CH_3$)-C(=O)-O-$CH_3$, $Y^1$-$CH_2$-C(=O)-O-$CH_2CH_3$, $Y^1$-$CH_2$-$CH_2$-C(=O)-O-$CH_2$-$CH_3$, $Y^1$-CH($CH_3$)-C(=O)-O-$CH_2$-$CH_3$, $CH_3$-C(=O)$Y^2$, $CH_3$-$CH_2$-C(=O)$Y^2$, $(CH_3)_2$CH-C(=O)$Y^2$, $CH_3$-$CH_2$-$CH_2$-C(=O)$Y^2$, $(CH_3)_3$C-C(=O)$Y^2$, $CH_3$-$CH_2$-CH($CH_3$)-C(=O)$Y^2$, $(CH_3)_2$CH-$CH_2$-C(=O)$Y^2$, $CH_2$=CH-C(=O)$Y^2$, $CH_2$=CH-$CH_2$-C(=O)$Y^2$, Cycloproyl-C(=O)$Y^2$, Dihydrofuran-2,5-dion, Furan-2,5-dion, Dihydro-2H-pyran-2,6(3H)-dion, [$CH_3$-C(=O)$]_2$O, [$CH_3$-$CH_2$-C(=O)$]_2$O, [$CH_3$-$CH_2$-$CH_2$-C(=O)$]_2$O, [$(CH_3)_2$CH-C(=O)$]_2$O, [$CH_3$-$CH_2$-$CH_2$-$CH_2$-C(=O)$]_2$O, [$CH_3$-$CH_2$-CH($CH_3$)-C(=O)$]_2$O, [$(CH_3)_2$CH-$CH_2$-C(=O)$]_2$O, [$(CH_3)_3$C-C(=O)$]_2$O, [$CH_2$=CH-C(=O)$]_2$O, [$CH_2$=CH-$CH_2$-C(=O)$]_2$O, [Cyclopropyl-C(=O)$]_2$O, $CH_2$=CH-C(=O)-OH, $CH_3$-CH=CH-C(=O)-OH, $(CH_3)_2$C=CH-C(=O)-OH, $CH_3$-$CH_2$-CH=CH-C(=O)-OH, $CH_3$-CH=C($CH_3$)-C(=O)-OH, $CH_2$=CH-C(=O)-O-$CH_3$, $CH_3$-CH=CH-C(=O)-O-$CH_3$, $(CH_3)_2$C=CH-C(=O)-O-$CH_3$, $CH_3$-$CH_2$-CH=CH-C(=O)-O-$CH_3$, $CH_3$-CH=C($CH_3$)-C(=O)-O-$CH_3$, $CH_2$=CH-C(=O)-O-$CH_2$-$CH_3$, $CH_3$-CH=CH-C(=O)-O-$CH_2$-$CH_3$, $(CH_3)_2$C=CH-C(=O)-O-$CH_2$-$CH_3$, $CH_3$-$CH_2$-CH=CH-C(=O)-O-$CH_2$-$CH_3$, $CH_3$-CH=C($CH_3$)-C(=O)O-$CH_2$-$CH_3$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, $(CH_3$-$O)_2$S(=O$)_2$, $(CH_3$-$CH_2$-$O)_2$S(=O$)_2$, $(CH_3$-$O)_2$P(=O)Cl, $(CH_3$-$CH_2$-$O)_2$P(=O)Cl, [$(CH_3)_2$CH-O$]_2$P(=O)Cl, $NH_3$, $CH_3$-$NH_2$, $(CH_3)_2$NH, $(CH_3)_3$N, $CH_3$-$CH_2$-$NH_2$, $(CH_3$-$CH_2)_2$NH, $(CH_3$-$CH_2)_3$N, [Cl-$CH_2$-$CH_2$-N$(CH_3)_3]^+Cl^-$, [Cl-$CH_2$-$CH_2$-N($CH_2$-$CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

In einer weiteren bevorzugten Ausführungsform sind die Substituenten abgeleitet von wenigstens einem, vorzugsweise nur einem Funktionalisierungsmittel ausgewählt aus der Gruppe bestehend aus $CH_3$-$Y^1$, $CH_3$-$CH_2$-$Y^1$, Cyclopropyl-$Y^1$, $Y^1$-$CH_2$-C(=O)-OH, $Y^1$-$CH_2$-C(=O)-O-$CH_3$, $Y^1$-$CH_2$-C(=O)-O-$CH_2$-$CH_3$, $CH_3$-C(=O)$Y^2$, Dihydrofuran-2,5-dion, Furan-2,5-dion, Dihydro-2H-pyran-2,6(3H)-dion, [$CH_3$-C(=O)$]_2$O, [Cyclopropyl-C(=O)$]_2$O, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, $(CH_3$-$O)_2$S(=O$)_2$, $(CH_3$-$CH_2$-$O)_2$S(=O$)_2$, $CH_3$-$NH_2$, $(CH_3)_2$NH, $(CH_3)_3$N, $CH_3$-$CH_2$-$NH_2$, $(CH_3$-$CH_2)_2$NH, $(CH_3$-$CH_2)_3$N, [Cl-$CH_2$-$CH_2$-N$(CH_3)_3]^+Cl^-$, [Cl-$CH_2$-$CH_2$-N($CH_2$-$CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

In einer weiteren bevorzugten Ausführungsform sind die Substituenten abgeleitet von wenigstens einem, vorzugsweise nur einem Funktionalisierungsmittel ausgewählt aus der Gruppe bestehend aus 1,3-Propansulton, 1,4-Butansulton, Chloressigsäure, Chloressigsäuremethylester und Chloressigsäureethylester.

Die von Funktionalisierungsmitteln abgeleitete Substituenten können mit Hybridsubstituenten weiter substituiert sein.

Die Cyclodextrin-Monomere sind vorzugsweise Strukturelemente der allgemeinen Ringstrukturen I, II oder III

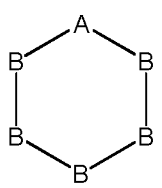
I

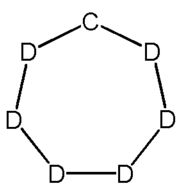
II

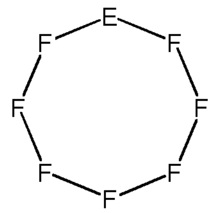
III

worin

die Glucosyleinheiten A, C und E, unabhängig voneinander, ausgewählt sind aus der Gruppe bestehend aus

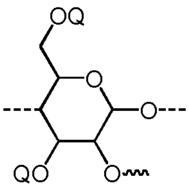, 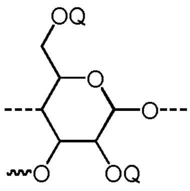, 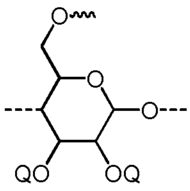, 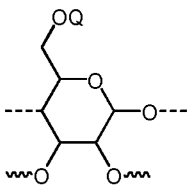, 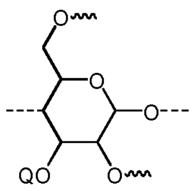,

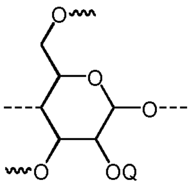 und 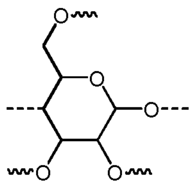 ;

und

jede einzelne der Glucosyleinheiten B, D und F, unabhängig voneinander, entweder für

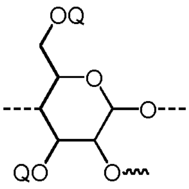, 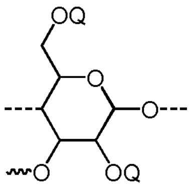, 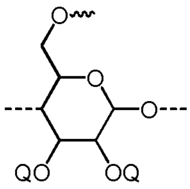, 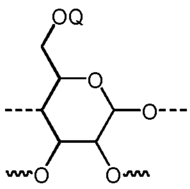, 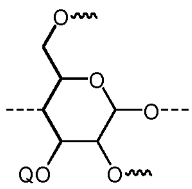,

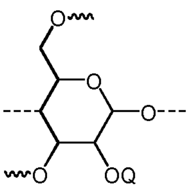, 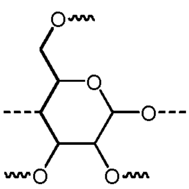 oder 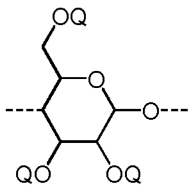

steht; und

---- für die 1→4-glycosidischen Bindungen in den Cyclodextrin-Monomeren steht;

~~~ für die Bindung zu einem Linker steht; wobei

jeder einzelne Rest Q entweder

(i) für Wasserstoff steht; oder

(ii) für einen Rest steht, der ausgewählt ist aus der Gruppe bestehend aus Glycosyl, -C(=O)$R^1$ und $R^2$; wobei $R^1$ und $R^2$, unabhängig voneinander, für $C_{1-6}$-Alkyl, $C_{3-7}$-Cycloalkyl, Phenyl oder 5- bis 7-gliedriges Heteroaryl stehen.

Im Sinne dieser Erfindung sind die α-Cyclodextrin-Monomere Strukturelemente der allgemeinen Ringstruktur I; die β-Cyclodextrin-Monomere Strukturelemente der allgemeinen Ringstruktur II; und die γ-Cyclodextrin-Monomere Strukturelemente der allgemeinen Ringstruktur III.

In einer weiteren bevorzugten Ausführungsform sind die Cyclodextrin-Monomere, unabhängig voneinander, ausgewählt aus der Gruppe bestehend aus
~~~

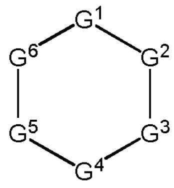, 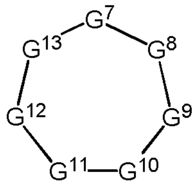 und 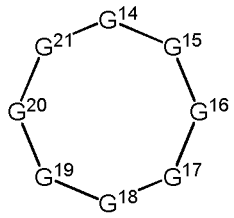;

worin
$G^1$, $G^7$ und $G^{14}$, unabhängig voneinander, ausgewählt sind aus der Gruppe bestehend aus

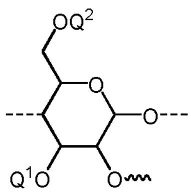, 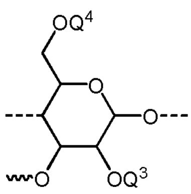, 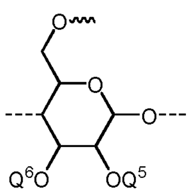, 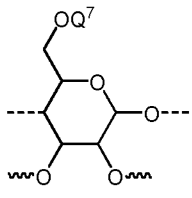, 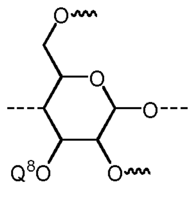,

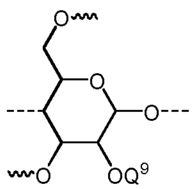 und 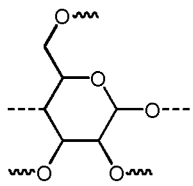;

und
$G^2$, $G^3$, $G^4$, $G^5$, $G^6$, $G^8$, $G^9$, $G^{10}$, $G^{11}$, $G^{12}$, $G^{13}$, $G^{15}$, $G^{16}$, $G^{17}$, $G^{18}$, $G^{19}$, $G^{20}$ und $G^{21}$, unabhängig voneinander, ausgewählt sind aus der Gruppe bestehend aus

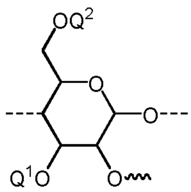, 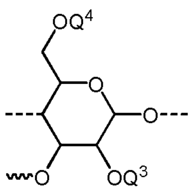, 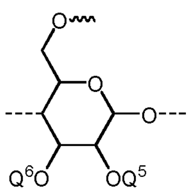, 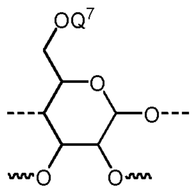, 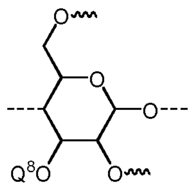,

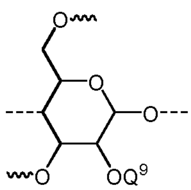, 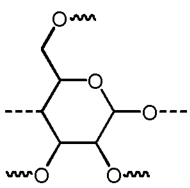 und 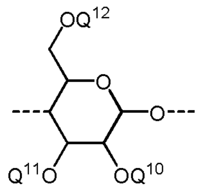;

wobei ---- für die 1→4-glycosidischen Bindungen in den Cyclodextrin-Monomeren steht;  für die Bindung zu einem Linker steht; und
$Q^1$, $Q^2$, $Q^3$, $Q^4$, $Q^5$, $Q^6$, $Q^7$, $Q^8$, $Q^9$ und $Q^{10}$, unabhängig voneinander, für Wasserstoff, -Glycosyl, -C(=O)$R^3$ oder -$R^4$ stehen; wobei
$R^3$ und $R^4$, unabhängig voneinander, für $C_{1-6}$-Alkyl, $C_{3-7}$-Cycloalkyl, Phenyl oder 5- bis 7-gliedriges Heteroaryl stehen.

Der Ausdruck "Glycosyl" umfasst für die Zwecke dieser Erfindung Saccharide, d.h. Zucker, Zuckersäuren und Zuckeralkohole, die Monomere (Monosaccharide), Dimere (Disaccharide), Trimere (Trisaccharide), Oligomere (Oligosaccharide) oder Polymere (Polysaccharide) sein können, wobei die Bindung an die Cyclodextrin-Monomere über jedes beliebige und mögliche Ringglied des Glycosyls erfolgen kann. Typische Monosaccharide, die in den erfindungsgemäßen

Copolymeren enthalten sein können, sind beispielsweise Mannitol, Isomalt, Lactit, Sorbitol, Glucitol, Xylitol, Threit, Erythrit, Arabit, Glyceraldeyhd, Erythrose, Threose, Ribose, Arabinose, Xylose, Lyxose, Allose, Altrose, Glucose, Mannose, Gulose, Idose, Galactose, Talose, Dihydroxyaceton, Erythrulose, Ribulose, Xylulose, Psicose, Fructose, Sorbose, Tagatose, Glucosamin, Fucosamin, N-Acetylmannosamin, Hamamelose, Apiose, Gluconsäure, Galacturonsäure, Erythrarsäure, Ascorbinsäure, Glucuronsäure, Abequose, Apiose, Cladinose, Desoxyribose, Desoxyglucose, Digitalose, Digitoxose, Fucose, Fucosamin, Galactosamin, Glucosamin, Glucosaminitol, Glycerin, Glyceron, Mannosamin, Mannozuckersäure, Neuraminsäure, Rhamnose, Schleimsäure, Sedoheptulose, Streptose, Trehalosamin, Trehalose, Weinsäure und Glucarsäure.

In einer bevorzugten Ausführungsform ist das Glycosyl ein Monosaccharid, das ausgewählt ist aus der Gruppe bestehend aus Mannitol, Isomalt, Lactit, Sorbitol, Glucitol, Xylitol, Threit, Erythrit, Arabit, Glyceraldeyhd, Erythrose, Threose, Ribose, Arabinose, Xylose, Lyxose, Allose, Altrose, Glucose, Mannose, Gulose, Idose, Galactose, Talose, Dihydroxyaceton, Erythrulose, Ribulose, Xylulose, Psicose, Fructose, Sorbose, Tagatose, Glucosamin, Fucosamin, N-Acetylmannosamin, Hamamelose, Apiose, Gluconsäure, Galacturonsäure, Erythrarsäure, Ascorbinsäure, Glucuronsäure, Abequose, Apiose, Cladinose, Desoxyribose, Desoxyglucose, Digitalose, Digitoxose, Fucose, Fucosamin, Galactosamin, Glucosamin, Glucosaminitol, Glycerin, Glyceron, Mannosamin, Mannozuckersäure, Neuraminsäure, Rhamnose, Schleimsäure, Sedoheptulose, Streptose, Trehalosamin, Trehalose, Weinsäure und Glucarsäure. Besonders vorteilhaft ist Glucose.

Das Glycosyl kann auch ein Disaccharid, Trisaccharid, Oligosaccharid oder Polysaccharid sein, die vorzugsweise gleiche oder verschiedene Monomere der oben stehenden Monosaccharide enthalten.

Im Sinne dieser Erfindung umfasst der Begriff "Disaccharid" Glycosyle, die vorzugsweise 2 gleiche oder verschiedene Monomere der oben stehenden Monosaccharide enthalten; der Begriff "Trisaccharid" umfasst Glycosyle, die vorzugsweise 3 gleiche oder verschiedene Monomere der oben stehenden Monosaccharide enthalten; der Begriff "Oligosaccharid" umfasst Glycosyle, die vorzugsweise 4, 5, 6, 7, 8 oder 9 gleiche oder verschiedene Monomere der oben stehenden Monosaccharide enthalten; und der Begriff "Polysaccharid" umfasst Glycosyle, die vorzugsweise $\geq 10$ (z.B. 10, 11, 12, 13, 14, $\geq 15$) gleiche oder verschiedene Monomere der oben stehenden Monosaccharide enthalten. Die in den Disacchariden, Trisacchariden, Oligosacchariden und Polysacchariden enthaltenen Monosaccharid-Monomere sind vorzugsweise über glycosidische Bindungen, vorzugsweise über $1\rightarrow2$-glycosidische, $1\rightarrow3$-glycosidische, $1\rightarrow4$-glycosidische und/ oder $1\rightarrow6$-glycosidische Bindungen miteinander verbunden. Als Glycosyle besonders vorteilhaft sind die Monosaccharide Glucose und Galactose, das Disaccharid Maltose und das Trisaccharid Maltotriose. Die Bindung des Glycosyls kann über jedes beliebige und mögliche Ringglied des Glycosyls an die Cyclodextrin-Monomere und -Verbindungen erfolgen.

In einer bevorzugten Ausführungsform ist Glycosyl ausgewählt aus der Gruppe bestehend aus Mannitol, Isomalt, Lactit, Sorbitol, Glucitol, Xylitol, Threit, Erythrit, Arabit, Glyceraldeyhd, Erythrose, Threose, Ribose, Arabinose, Xylose, Lyxose, Allose, Altrose, Glucose, Mannose, Gulose, Idose, Galactose, Talose, Dihydroxyaceton, Erythrulose, Ribulose, Xylulose, Psicose, Fructose, Sorbose, Tagatose, Glucosamin, Fucosamin, N-Acetylmannosamin, Hamamelose, Apiose, Gluconsäure, Galacturonsäure, Erythrarsäure, Ascorbinsäure, Glucuronsäure, Abequose, Apiose, Cladinose, Desoxyribose, Desoxyglucose, Digitalose, Digitoxose, Fucose, Fucosamin, Galactosamin, Glucosamin, Glucosaminitol, Glycerin, Glyceron, Mannosamin, Mannozuckersäure, Neuraminsäure, Rhamnose, Schleimsäure, Sedoheptulose, Streptose, Trehalosamin, Trehalose, Weinsäure, Glucarsäure, Maltose und Maltriose. Es ist besonders bevorzugt, dass Glycosyl ausgewählt ist aus der Gruppe bestehend aus Glucose, Galactose, Maltose und Maltriose.

Der Ausdruck "$C_{1-6}$-Alkyl" umfasst im Sinne dieser Erfindung acyclische, gesättigte oder ungesättigte Kohlenwasserstoffreste, die verzweigt- oder geradkettig sein können und 1, 2, 3, 4, 5 oder 6 Kohlenstoffatome enthalten sowie unsubstituiert oder ein- oder mehrfach substituiert sein können, wobei die Substituenten gleich oder verschieden und in jeder beliebigen und möglichen Position des $C_{1-6}$-Alkyls sein können. Vorzugsweise umfasst $C_{1-6}$-Alkyl die Reste $C_{1-6}$-Alkanyl, $C_{2-6}$-Alkenyl und $C_{2-6}$-Alkinyl. Dabei weisen $C_{2-6}$-Alkenyle mindestens eine C-C-Doppelbindung und $C_{2-6}$-Alkinyle mindestens eine C-C-Dreifachbindung auf, wohingegen $C_{1-6}$-Alkanyle vollständig gesättigt sind. Vorzugsweise ist $C_{1-6}$-Alkyl ausgewählt aus der Gruppe, die Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl, Prop-1-enyl, iso-Butenyl, n-Butenyl, cis-2-Butenyl, trans-2-Butenyl, 1,2-Butadienyl, 1,3-Butadienyl, Pent-1-enyl, cis-Pent-2-enyl, trans-Pent-2-enyl, 2-Methyl-but-1-enyl, 2-Methyl-but-2-enyl, 3-Methyl-but-1-enyl, Hex-1-enyl, Hex-2-enyl und Hex-3-enyl enthält. Die Bindung von $C_{1-6}$-Alkyl-Resten an Cyclodextrin-Monomere und Cyclodextrin-Verbindungen kann über jedes beliebige und mögliche Atom der $C_{1-6}$-Alkyle erfolgen kann.

Der Ausdruck "$C_{3-7}$-Cycloalkyl" umfasst für die Zwecke dieser Erfindung cyclische Kohlenwasserstoffe, die 3, 4, 5, 6 oder 7 Kohlenstoffatome enthalten, gesättigt oder ein- oder mehrfach ungesättigt (aber nicht aromatisch) sind, und unsubstituiert oder ein- oder mehrfach substituiert sein können, wobei die Substituenten gleich oder verschieden und in jeder beliebigen und möglichen Position des $C_{3-7}$-Cycloalkyls sein können. $C_{3-7}$-Cycloalkyl umfasst auch gesättigte oder ungesättigte (aber nicht aromatische) Cycloalkyle, in denen ein oder zwei Kohlenstoffatome durch ein oder zwei gleiche oder verschiedene Heteroatome, die aus der Gruppe bestehend aus N, O und S ausgewählt sind, ersetzt sind.

Das $C_{3-7}$-Cycloalkyl kann mit einem oder mehreren gesättigten, ungesättigten oder aromatischen Ringsystemen kondensiert sein. Vorzugsweise ist $C_{3-7}$-Cycloalkyl ausgewählt aus der Gruppe, die Cyclopropyl, Cyclobutyl, Cyclopentyl, Cyclohexyl, Cycloheptyl, Cyclopropenyl, Cyclobutenyl, Cyclopentenyl, Cyclopentadienyl, Cyclohexenyl, Cyclohexadienyl, Cycloheptenyl, Cycloheptadienyl, Cycloheptatrienyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, Tetrahydropyranyl, Piperazinyl, Dioxanyl, Morpholinyl, Chinuclidinyl, Pyrazolinyl, Pyrazolinonyl, Pyranyl, Indolinyl, Chinolizinyl, Chromanyl, Isochromanyl, Chromenyl, Isochromenyl und Benzodioxolanyl enthält. Die Bindungen an die Cyclodextrin-Monomere und Cyclodextrin-Verbindungen kann über jedes beliebige und mögliche Ringglied der $C_{3-7}$-Cycloalkyle erfolgen. Der $C_{3-7}$-Cycloalkyl-Rest kann auch über eine $C_{1-4}$-Alkylenbrücke, vorzugsweise $-CH_2-$, $-CH_2-CH_2-$, $-CH_2-CH_2-CH_2-$ oder $-CH_2-CH_2-CH_2-CH_2-$, an die Cyclodextrin-Monomere gebunden sein.

Im Sinne dieser Erfindung umfasst der Ausdruck "$C_{1-4}$-Alkylenbrücke" Strukturelemente, die die $C_{3-7}$-Cycloalkyl-, Phenyl- und 5- bis 7-gliedrige Heteroaryl-Reste mit den Cyclodextrin-Monomeren und Cyclodextrin-Verbindungen verbinden. Die $C_{1-4}$-Alkylenbrücke kann verzweigt- oder geradkettig, gesättigt ($C_{1-4}$-Alkanylenbrücke), oder ein- oder mehrfach ungesättigt sein ($C_{2-4}$-Alkenylenbrücke, $C_{2-4}$-Alkinylenbrücke). Die $C_{2-4}$-Alkylenbrücke enthält wenigstens eine C-C-Doppelbindung und die $C_{2-4}$-Alkinylenbrücke enthält wenigstens eine C-C-Dreifachbindung. Vorteilhaft sind $C_{1-4}$-Alkylenbrücken ausgewählt aus der Gruppe bestehend aus $-CH_2-$, $-CH_2-CH_2-$, $-CH_2-CH_2-CH_2-$ oder $-CH_2-CH_2-CH_2-CH_2-$, $-CH=CH-$, $-C=C-$, $-C=CH-CH_2-$, $-CH_2-CH=CH-CH_2-$ und $-CH=CH-CH=CH-$.

Der Ausdruck "Phenyl" steht im Sinne dieser Erfindung für einen aromatischen Benzen-Rest (Benzol), der mit weiteren gesättigten, ungesättigten oder aromatischen Ringsystemen kondensiert sein kann (z.B. Kondensation von Phenyl zu Naphthyl).

Das Phenyl kann unsubstituiert oder ein- oder mehrfach substituiert sein, wobei die Substituenten gleich oder verschieden und in jeder beliebigen und möglichen Position des Phenyls sein können. Das Phenyl kann an die Cyclodextrin-Monomere und Cyclodextrin-Verbindungen über jedes beliebige und mögliche Ringglied des Phenyls direkt oder über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, gebunden sein.

Der Ausdruck "5- bis 7-gliedriges Heteroaryl" umfasst für die Zwecke dieser Erfindung 5-, 6- oder 7-gliedrige cyclische aromatische Reste, die mindestens 1, ggf. auch 2, 3, 4 oder 5 Heteroatome enthalten, wobei die Heteroatome gleich oder verschieden sind, und das Heteroaryl unsubstituiert oder ein- oder mehrfach substituiert sein kann. im Falle der Substitution am 5- bis 7-gliedrigen Heteroaryl können die Substituenten gleich oder verschieden und in jeder beliebigen und möglichen Position des Heteroaryls sein. Bevorzugte Heteroatome sind N, O und S. Das Heteroaryl kann auch Teil eine bi- oder polycyclischen, carbo- oder heterocyclischen Systems sein. Es ist bevorzugt, dass das 5- bis 7-gliedrige Heteroaryl ausgewählt ist aus der Gruppe, die Pyrrolyl, Pyrazolyl, Imidazolyl, Thienyl, Furyl, Oxazolyl, Isoxazolyl, Thiazolyl, Isothiazolyl, Furazanyl, Oxadiazolyl, Pyridyl, Pyridazinyl, Pyrazinyl, Pyrimidinyl, Indolyl, Isoindolyl, Indolizinyl, Indazolyl, Benzotriazolyl, Chinolinyl, Isochinolinyl, Naphthyridinyl, Chinazolinyl, Phthalazinyl, Chinoxalinyl, Purinyl, Pteridinyl, Benzofuryl, Isobenzofuryl, Benzothienyl, Benzothiazolyl und Benzothiadiazolyl enthält. Die Bindung der 5- bis 7-gliedrigen Heteroaryl-Reste an die Cyclodextrin-Monomere und Cyclodextrin-Verbindungen kann über jedes beliebige und mögliche Ringglied des Heteroaryls erfolgen. Der 5- bis 7-gliedriges Heteroaryl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere und Cyclodextrin-Verbindungen gebunden sein.

In Bezug auf $C_{1-6}$-Alkyl versteht man unter dem Ausdruck "ein- oder mehrfach substituiert" im Sinne dieser Erfindung die Substitution eines oder mehrerer Wasserstoffreste durch einen oder mehrere Substituenten, wobei unter mehrfach substituierten Alkyl-Resten solche Reste zu verstehen sind, die entweder an gleichen oder verschiedenen Atomen mehrfach, z.B. zwei- oder dreifach, substituiert sind, beispielsweise zweifach am gleichen Kohlenstoffatom wie im Falle von $-CHF_2$, oder an verschiedenen Stellen wie im Falle von $-CH_2CH(OH)CH_2(OH)$. Die Mehrfachsubstitution kann mit dem gleichen oder mit verschiedenen Substituenten erfolgen. Ggf. kann ein Substituent auch seinerseits substituiert sein.

In Bezug auf $C_{3-7}$-Cycloalkyl, Phenyl und 5- bis 7-gliedriges Heteroaryl versteht man im Sinne dieser Erfindung unter "ein- oder mehrfach" substituiert die ein- oder mehrfache (z.B. zwei-, drei- vier- oder fünffache) Substitution eines oder mehrerer Wasserstoffatome des Ringsystems an einem oder verschiedenen Atomen. Die Mehrfachsubstitution erfolgt dabei mit dem gleichen oder verschiedenen Substituenten.

Im Fall einer Mehrfachsubstitution eines Cyclodextrin-Monomers, kann dieses zwei oder mehr (z.B. drei, vier oder fünf) gleiche oder verschiedene Reste enthalten, die aus der Gruppe bestehend aus Glycosyl, $-C(=O)R^1$ und $R^2$ oder der Gruppe bestehend aus Glycosyl, $-C(=O)R^3$ und $R^4$ ausgewählt sind. Im Fall einer Mehrfachsubstitution einer Cyclodextrin-Verbindung, kann diese zwei oder mehr (z.B. drei, vier oder fünf) gleiche oder verschiedene Reste enthalten, die aus der Gruppe bestehend aus Glycosyl, $-C(=O)R^6$ und $R^7$ ausgewählt sind.

In Bezug auf die Reste $R^1$, $R^2$, $R^3$ und $R^4$ ist $C_{1-6}$-Alkyl vorzugsweise ausgewählt aus der Gruppe bestehend aus Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl, Prop-1-enyl, iso-Butenyl, n-Butenyl, cis-2-Butenyl, trans-2-Butenyl, 1,2-Butadienyl, 1,3-Butadienyl, Pent-1-enyl, cis-Pent-2-enyl, trans-Pent-2-enyl, 2-Methyl-but-1-enyl, 2-Methyl-but-2-enyl, 3-Methyl-but-1-enyl, Hex-1-enyl, Hex-2-enyl und Hex-3-enyl. Besonders bevorzugt ist, dass $C_{1-6}$-Alkyl ausgewählt ist aus der Gruppe bestehend aus Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl und Prop-1-enyl. Die genannten $C_{1-6}$-Alkyl-Reste können unsubstituiert oder ein- oder mehrfach (z.B. zwei-, drei- vier- oder fünffach) mit Resten $R^5$ substituiert sein.

In Bezug auf die Reste $R^1$, $R^2$, $R^3$ und $R^4$ ist $C_{3-7}$-Cycloalkyl vorzugsweise ausgewählt aus der Gruppe bestehend aus Cyclopropyl, Cyclobutyl, Cyclopentyl, Cyclohexyl, Cycloheptyl, Cyclopropenyl, Cyclobutenyl, Cyclopentenyl, Cyclopentadienyl, Cyclohexenyl, Cyclohexadienyl, Cycloheptenyl, Cycloheptadienyl, Cycloheptatrienyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, Tetrahydropyranyl, Piperazinyl, Dioxanyl, Morpholinyl, Chinuclidinyl, Pyrazolinyl, Pyrazolinonyl, Pyranyl, Indolinyl, Chinolizinyl, Chromanyl, Isochromanyl, Chromenyl, Isochromenyl und Benzodioxolanyl. Es ist besonders bevorzugt, dass $C_{3-7}$-Cycloalkyl ausgewählt ist aus der Gruppe bestehend aus Cyclopropyl, Cyclobutyl, Cyclopentyl, Cyclohexyl, Cycloheptyl, Cyclopropenyl, Cyclobutenyl, Cyclopentenyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, Tetrahydropyranyl, Piperazinyl, Dioxanyl, Morpholinyl, Chinuclidinyl, Pyrazolinyl, Pyrazolinonyl und Pyranyl. Es ist weiterhin besonders bevorzugt, dass $C_{3-7}$-Cycloalkyl ausgewählt ist aus der Gruppe bestehend aus Cyclopropyl, Cyclobutyl, Cyclopentyl, Cyclohexyl, Tetrahydrofuranyl, Piperidinyl, Piperazinyl, Morpholinyl und Chinuclidinyl. Es ist insbesondere bevorzugt, dass $C_{3-7}$-Cycloalkyl ausgewählt ist aus der Gruppe bestehend aus Cyclopropyl, Cyclobutyl, Cyclopentyl, Cyclohexyl, Piperidinyl, Piperazinyl und Morpholinyl. Die genannten $C_{3-7}$-Cycloalkyl-Reste können unsubstituiert oder ein- oder mehrfach (z.B. zwei-, drei- vier- oder fünffach) mit Resten $R^5$ substituiert sein. Der $C_{3-7}$-Cycloalkyl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere gebunden sein.

In Bezug auf die Reste $R^1$, $R^2$, $R^3$ und $R^4$ kann der Phenyl-Rest unsubstituiert oder ein- oder mehrfach (z.B. zwei-, drei- vier- oder fünffach) mit Resten $R^5$ substituiert sein. Der Phenyl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere gebunden sein.

In Bezug auf die Reste $R^1$, $R^2$, $R^3$ und $R^4$ ist 5- bis 7-gliedriges Heteroaryl vorzugsweise ausgewählt aus der Gruppe bestehend aus Pyrrolyl, Pyrazolyl, Imidazolyl, Thienyl, Furyl, Oxazolyl, Isoxazolyl, Thiazolyl, Isothiazolyl, Furazanyl, Oxadiazolyl, Pyridyl, Pyridazinyl, Pyrazinyl, Pyrimidinyl, Indolyl, Isoindolyl, Indolizinyl, Indazolyl, Benzotriazolyl, Chinolinyl, Isochinolinyl, Naphthyridinyl, Chinazolinyl, Phthalazinyl, Chinoxalinyl, Purinyl, Pteridinyl, Benzofuryl, Isobenzofuryl, Benzothienyl, Benzothiazolyl und Benzothiadiazolyl. In einer bevorzugten Ausführungsform ist 5- bis 7-gliedriges Heteroaryl ausgewählt aus der Gruppe bestehend aus Pyrrolyl, Pyrazolyl, Imidazolyl, Thienyl, Furyl, Oxazolyl, Isoxazolyl, Thiazolyl, Isothiazolyl, Pyridyl, Indolyl, Chinolinyl, Purinyl, Pteridinyl und Benzofuryl. In einer besonders bevorzugten Ausführungsform ist 5- bis 7-gliedriges Heteroaryl ausgewählt aus der Gruppe bestehend aus Pyrrolyl, Pyrazolyl, Imidazolyl, Thienyl, Furyl, Oxazolyl, Isoxazolyl, Thiazolyl, Isothiazolyl und Pyridyl. Die genannten 5-7-gliedrigen Heteroaryl-Reste können unsubstituiert oder ein- oder mehrfach (z.B. zwei-, dreivier- oder fünffach) mit Resten $R^5$ substituiert sein. Der 5- bis 7-gliedrige Heteroaryl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere gebunden sein.

In einer bevorzugten Ausführungsform sind die Substituenten der Cyclodextrin-Monomere ausgewählt aus der Gruppe bestehend aus Glucose, Galactose, Maltose, Maltriose, $-C(=O)-C_{1-6}$-Alkyl, $-C(=O)-C_{3-7}$-Cycloalkyl, -C(=O)-Phenyl, -C(=O)-(5- bis 7-gliedriges Heteroaryl), $-C_{1-6}$-Alkyl, $-C_{3-7}$-Cycloalkyl, Phenyl und 5- bis 7-gliedriges Heteroaryl; wobei die Reste $-C_{1-6}$-Alkyl, $-C_{3-7}$-Cycloalkyl, Phenyl und 5- bis 7-gliedriges Heteroaryl unsubstituiert oder ein- oder mehrfach (z.B. zwei-, drei- vier- oder fünffach) mit gleichen oder verschiedenen Resten $R^5$ substituiert sein können; und die $C_{3-7}$-Cycloalkyl-, Phenyl- und 5- bis 7-gliedrigen Heteroaryl-Reste über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere gebunden sein können.

Die Cyclodextrin-Monomere sind vorzugsweise mit einem oder mehreren, gleichen oder verschiedenen, vorzugsweise gleichen Resten substituiert, die ausgewählt sind aus der Gruppe bestehend aus Glucose, Galactose, Maltose, Maltriose, -C(=O)-Methyl, -C(=O)-Ethyl, -C(=O)-iso-Propyl, -C(=O)-n-Propyl, -C(=O)-iso-Butyl, -C(=O)-n-Butyl, -C(=O)-sec-Butyl, -C(=O)-tert-Butyl, -C(=O)-n-Pentyl, -C(=O)-iso-Pentyl, -C(=O)-neo-Pentyl, -C(=O)-iso-Hexyl, -C(=O)-n-Hexyl, -C(=O)-neo-Hexyl, -C(=O)-(3-Methylpentyl), -C(=O)-(2,3-Dimethylbutyl), -C(=O)-Ethenyl, -C(=O)-Ethinyl, -C(=O)-Prop-1-enyl, -C(=O)-Cyclopropyl, -C(=O)-Cyclobutyl, -C(=O)-Cyclopentyl, -C(=O)-Cyclohexyl, -C(=O)-Piperidinyl, -C(=O)-Piperazinyl, -C(=O)-Morpholinyl, -C(=O)-Phenyl, -C(=O)-Imidazolyl, -C(=O)-Thienyl, -C(=O)-Furyl, -C(=O)-Oxazolyl, -C(=O)-Isoxazolyl, -C(=O)-Thiazolyl, -C(=O)-Isothiazolyl, -C(=O)-Pyridyl, Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl, Prop-1-enyl, Cyclopropyl, Cyclobutyl, Cyclopentyl, Cyclohexyl, Piperidinyl, Piperazinyl, Morpholinyl, Phenyl, Pyrrolyl, Pyrazolyl, Imidazolyl, Thienyl, Furyl, Oxazolyl, Isoxazolyl, Thiazolyl, Isothiazolyl und Pyridyl; wobei die Reste unsubstituiert oder ein- oder mehrfach (z.B. zwei-, drei- vier- oder fünffach) mit Resten $R^5$ substituiert sein können; und die $C_{3-7}$-Cycloalkyl-, Phenyl- und 5- bis 7-gliedrigen Heteroaryl-Reste über eine $C_{1-4}$-Al-

kylenbrücke, vorzugsweise -CH$_2$-, -CH$_2$-CH$_2$-, -CH$_2$-CH$_2$-CH$_2$-, oder -CH$_2$-CH$_2$-CH$_2$-CH$_2$-, an die Cyclodextrin-Monomere gebunden sein können.

R$^5$ ist vorzugsweise ausgewählt aus der Gruppe bestehend aus -C(=O)H, -C(=O)X, -C(=O)OH, -C(=O)OX, -C(=O)NH$_2$, -C(=O)NHX, -C(=O)NX$_2$, -C(=O)NHC(=O)X, -C(=O)NXC(=O)X, -OH, -OX, -OT, -OC(=O)H, -OC(=O)X, -OC(=O)OH, -OC(=O)OX, -OC(=O)NH$_2$, -OC(=O)NHX, -OC(=O)NX$_2$, -O-N=O, -OS(=O)H, -OS(=O)X, -OS(=O)OH, -OS(=O)OX, -OS(=O)$_2$H, -OS(=O)$_2$X, -OS(=O)$_2$OH, -OS(=O)$_2$OX, -OS(=O)NH$_2$, -OS(=O)NHX, -OS(=O)NX$_2$, -OS(=O)$_2$NH$_2$, -OS(=O)$_2$NHX, -OS(=O)$_2$NX$_2$, -OP(=O)(X)$_2$, -OP(=O)(OH)(X), -OP(=O)(OX)(X), -OP(=O)(OH)$_2$, -OP(=O)(OX)(OH), -OP(=O)(OX)$_2$, -O-P(=NH)(X)$_2$, -OP(=NX)(X)$_2$, -OP(=O)(NH$_2$)$_2$, -OP(=O)(NX)(NH$_2$), -OP(=S)(X)$_2$, -OP(=S)(OH)(X), -OP(=S)(OX)(X), -OP(=S)(OH)$_2$, -OP(=S)(OX)(OH), -OP(=S)(OX)$_2$, -SH, -SX, -ST, -S(=O)H, -S(=O)X, -S(=O)OH, -S(=O)OX, -S(=O)$_2$H, -S(=O)$_2$X, -S(=O)$_2$OH, -S(=O)$_2$OX, -S(=O)NH$_2$, -S(=O)NHX, -S(=O)NX$_2$, -S(=O)$_2$NH$_2$, -S(=O)$_2$NHX, -S(=O)$_2$NX$_2$, -NH$_2$, -NHX, -NHT, -NX$_2$, -NT$_2$, -N(X)$_3$$^+$A$^-$, -NHC(=O)H, -NHC(=O)X, -N[C(=O)X]$_2$, -NXC(=O)X, -NHC(=O)NH$_2$, -NHC(=O)NHX, -NXC(=O)NHX, -NXC(=O)NX$_2$, -NXC(=O)NX$_2$, -NHC(=O)OH, -NHC(=O)OX, -NXC(=O)OX, -NHS(=O)H, -NHS(=O)X, -NXS(=O)H, -NXS(=O)X, -NHS(=O)OH, -NHS(=O)OX, -NXS(=O)OH, -NXS(=O)OX, -NHS(=O)$_2$H, -NHS(=O)$_2$X, -NXS(=O)$_2$H, -NXS(=O)$_2$X, -NHS(=O)$_2$OH, -NHS(=O)$_2$OX, -NXS(=O)$_2$OH, -NXS(=O)$_2$OX, -NHS(=O)NH$_2$, -NXS(=O)NHX, -NHS(=O)NX$_2$, -NXS(=O)NH$_2$, -NXS(=O)NX$_2$, -NHS(=O)$_2$NH$_2$, -NHS(=O)$_2$NHX, -NHS(=O)$_2$NX$_2$, -NXS(=O)$_2$NH$_2$, -NXS(=O)$_2$NHX, -NXS(=O)$_2$NX$_2$, =N-OH, =N-NH$_2$, -N=N-X, -PH$_2$, -PHX, -PX$_2$, -P(=O)X$_2$, -P(=O)(OH)(X), -P(=O)(OX)(X), -P(=O)(OH)$_2$, -P(=O)(OX)(OH), -P(=O)(OX)$_2$, -P(=NH)(X)$_2$, -P(=NX)(X)$_2$, -P(=O)(NH$_2$)$_2$, -P(=O)(NX$_2$)$_2$, -P(=S)(X)$_2$, -P(=S)(OH)(X), -P(=S)(OX)X, -P(=S)(OH)$_2$, -P(=S)(OX)(OH), -P(=S)(OX)$_2$, =NH, =NX, =O, =S, Pyrrolidinyl, Pyrazolinyl, Pyrazolidinyl, Imidazolinyl, Imidazolidinyl, Dioxolanyl, Piperidinyl, Piperazinyl, 4-X-Piperazinyl, Dioxanyl, Morpholinyl, Chinuclidinyl, Pyrazolinonyl, Indolinyl, Isoindolinyl, Chinolizinyl, Benzodioxolanyl, Pyrrolyl, Pyrazolyl, Imidazolyl, Thienyl, Oxazolyl, Isoxazolyl, Thiazolyl, Isothiazolyl, Furazanyl, Oxadiazolyl, Pyridyl, Pyridazinyl, Pyrazinyl, Pyrimidinyl, Indolyl, Isoindolyl, Indolizinyl, Indazolyl, Benzotriazolyl, Chinolinyl, Isochinolinyl, Naphthyridinyl, Chinazolinyl, Phthalazinyl, Chinoxalinyl, Purinyl, Pteridinyl, Benzothienyl, Benzothiazolyl und Benzothiadiazolyl; wobei der Rest X vorzugsweise für Methyl, Ethyl, n-Propyl, iso-Propyl, iso-Butyl, n-Butyl, sec-Butyl, tert-Butyl, Cyclopropyl, Cyclobutyl, Cyclopentyl, Cyclohexyl, Allyl, Vinyl oder Ethinyl steht; der Rest T für einen Hybridrest steht; und A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform ist R$^5$ ausgewählt aus der Gruppe bestehend aus -C(=O)H, -C(=O)X, -C(=O)OH, -C(=O)OX, -C(=O)NH$_2$, -C(=O)NHX, -C(=O)NX$_2$, -C(=O)NHC(=O)X, -C(=O)NXC(=O)X, -OH, -OX, -OT, -OC(=O)H, -OC(=O)X, -OC(=O)OH, -OC(=O)OX, -OC(=O)NH$_2$, -OC(=O)NHX, -OC(=O)NX$_2$, -OS(=O)H, -OS(=O)X, -OS(=O)OH, -OS(=O)OX, -OS(=O)$_2$H, -OS(=O)$_2$X, -OS(=O)$_2$OH, -OS(=O)$_2$OX, -OS(=O)NH$_2$, -OS(=O)NHX, -OS(=O)NX$_2$, -OS(=O)$_2$NH$_2$, -OS(=O)$_2$NHX, -OS(=O)$_2$NX$_2$, -OP(=O)(OH)(X), -OP(=O)(OH)$_2$, -OP(=O)(OX)(OH), -OP(=O)(OX)$_2$, -SH, -ST, -S(=O)H, -S(=O)X, -S(=O)OH, -S(=O)OX, -S(=O)$_2$H, -S(=O)$_2$X, -S(=O)$_2$OH, -S(=O)$_2$OX, -S(=O)NH$_2$, -S(=O)NHX, -S(=O)NX$_2$, -S(=O)$_2$NH$_2$, -S(=O)$_2$NHX, -S(=O)$_2$NX$_2$, -NH$_2$, -NHX, -NHT, -NX$_2$, -NT$_2$, -N(X)$_3$$^+$A$^-$ , -NHC(=O)H, -NHC(=O)X, -N[C(=O)X]$_2$, -NXC(=O)X, -NHC(=O)NH$_2$, -NHC(=O)NHX, -NXC(=O)NX$_2$, -NXC(=O)NHX, -NXC(=O)NX$_2$, -NHC(=O)OH, -NHC(=O)OX, - NXC(=O)OX, -NHS(=O)H, -NXS(=O)H, -NHS(=O)OH, -NXS(=O)OH, -NHS(=O)$_2$OH, -NXS(=O)$_2$OH, =N-OH, =N-NH$_2$, -PH$_2$, -P(=O)X$_2$, -P(=O)(OH)(X), -P(=O)(OH)$_2$, -P(=O)(OX)(OH), -P(=O)(OX)$_2$, =NH, =NX, =O, =S, Pyrrolidinyl, Pyrazolinyl, Pyrazolidinyl, Imidazolinyl, Imidazolidinyl, Piperidinyl, Piperazinyl, 4-X-Piperazinyl, Morpholinyl, Chinuclidinyl, Pyrazolinonyl, Imidazolyl; wobei der Rest X vorzugsweise für Methyl, Ethyl, n-Propyl, iso-Propyl, Cyclopropyl, Allyl, Vinyl oder Ethinyl steht; der Rest T für einen Hybridrest steht; und A$^-$ für ein anorganisches oder organisches Anion steht, das vorzugsweise ausgewählt ist aus der Gruppe bestehend aus Cl$^-$ , Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht

In einer weiteren bevorzugten Ausführungsform steht R$^5$ für einen ionischen, ionisierbaren, Hydroxy- oder Polyol-Rest. In diesem Zusammenhang ist R$^5$ vorzugsweise ausgewählt aus der Gruppe bestehend aus -C(=O)OH, -OH, -OT, -OC(=O)OH, -OC(=O)NH$_2$, -OS(=O)OH, -OS(=O)$_2$OH, -OP(=O)(OH)(X), -OP(=O)(OH)$_2$, -OP(=O)(OX)(OH), -OP(=O)(OX)$_2$, -SH, -ST, -S(=O)OH, -S(=O)$_2$OH, -NH$_2$, -NHX, -NHT, -NX$_2$, -NT$_2$, -N(X)$_3$$^+$A$^-$, -NHC(=O)OH, -NXC(=O)OH, -NHS(=O)OH, -NXS(=O)OH, -NHS(=O)$_2$OH, -NXS(=O)$_2$OH, =N-OH, =N-NH$_2$, -P(=O)(OH)(X), -P(=O)(OH)$_2$, -P(=O)(OX)(OH), -P(=O)(OX)$_2$, =NH, =NX, Piperidinyl, Morpholinyl und Imidazolyl; wobei der Rest X ausgewählt ist aus der Gruppe bestehend aus Methyl, Ethyl, n-Propyl, iso-Propyl, Cyclopropyl, Allyl, Vinyl und Ethinyl; der Rest T für einen Hybridrest; wobei A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht

In einer weiteren bevorzugten Ausführungsform steht R$^5$ für einen ionischen, ionisierbaren, Hydroxy- oder Polyol-Rest. In diesem Zusammenhang ist R$^5$ vorzugsweise ausgewählt aus der Gruppe bestehend aus -C(=O)OH, -OH, -OT, -OS(=O)$_2$OH, -OP(=O)(OH)$_2$, -SH, -ST, -S(=O)$_2$OH, -NH$_2$, -NHX, -NHT, -NX$_2$, -NT$_2$, -N(X)$_3$$^+$A$^-$, -NHC(=O)OH, -NXC(=O)OH, -P(=O)(OH)(X), -P(=O)(OH)$_2$, -P(=O)(OX)(OH), wobei der Rest X ausgewählt ist aus der Gruppe bestehend aus Methyl, Ethyl, n-Propyl, iso-Propyl, Cyclopropyl, Allyl, Vinyl und Ethinyl; der Rest T für einen Hybridrest steht; wobei A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht

In einer weiteren bevorzugten Ausführungsform sind die Reste $R^1$, $R^2$, $R^3$ und $R^4$, unabhängig voneinander ausgewählt aus der Gruppe bestehend aus Methyl, Ethyl, iso-Propyl, n-Propyl, iso-Butyl, n-Butyl, sec-Butyl, tert-Butyl, Ethenyl, Ethinyl, Prop-1-enyl, Cyclopropyl, Cyclobutyl, Cyclopentyl, Cyclohexyl, Phenyl, Benzyl, Phenethyl, -CH$_2$-C(=O)-OH, -(CH$_2$)$_2$-C(=O)-OH, -(CH$_2$)$_3$-C(=O)-OH, -(CH$_2$)$_4$-C(=O)-OH, -(CH$_2$)$_5$-C(=O)-OH, -(CH$_2$)$_6$-C(=O)-OH, -CH$_2$-CH(OH)-C(=O)OH, -CH$_2$-CH(OH)-C(=O)O-CH$_3$, -CH$_2$-CH(OH)-C(=O)O-CH$_2$-CH$_3$, -CH(CH$_2$OH)-C(=O)OH, -CH(CH$_2$OH)-C(=O)O-CH$_3$, -CH(CH$_2$OH)-C(=O)O-CH$_2$-CH$_3$, -CH$_2$-CH(OH)-CH$_2$-C(=O)-OH, -CH$_2$-CH(OH)-CH$_2$-C(=O)-O-CH$_3$, -CH$_2$-CH(OH)-CH$_2$-C(=O)-OCH$_2$-CH$_3$, -CH(CH$_2$-OH)-CH$_2$-C(=O)-O-CH$_3$, -CH(CH$_2$-OH)-CH$_2$-C(=O)-O-CH$_2$-CH$_3$, -CH$_2$-OH, -CH(OH)-CH$_3$, -(CH$_2$)$_2$-OH, - CH(OH)-CH$_2$-CH$_3$, -CH$_2$-CH(OH)-CH$_3$, -(CH$_2$)$_3$-OH, -CH(CH$_3$)-CH$_2$-OH, -CH(OH)-(CH$_2$)$_2$-CH$_3$, -CH$_2$-CH(OH)-CH$_2$-CH$_3$, -(CH$_2$)$_2$-CH(OH)-CH$_3$, -(CH$_2$)$_4$-OH, -(CH$_2$)$_5$-OH, -(CH$_2$)$_6$-OH, -CH(OH)-CH$_2$(OH), -CH(OH)-CH(OH)-CH$_3$, -CH(OH)-CH$_2$-CH$_2$(OH), -CH$_2$-CH(OH)-CH$_2$(OH), -CH(OH)-CH(OH)-CH$_2$-CH$_3$, -CH(OH)-CH$_2$CH(OH)-CH$_3$, -CH(OH)-(CH$_2$)$_2$-CH$_2$(OH), -CH$_2$-CH(OH)-CH(OH)-CH$_3$, -CH$_2$-CH(OH)-CH$_2$-CH$_2$(OH), -(CH$_2$)$_2$-CH(OH)-CH$_2$(OH), -CH(OH)-CH(OH)-CH$_2$(OH), -CH(OH)-CH(OH)-CH(OH)-CH$_3$, -CH(OH)-CH(OH)-CH$_2$-CH$_2$(OH), -CH(OH)-CH$_2$-CH(OH)-CH$_2$(OH), -CH$_2$-CH(OH)-CH(OH)-CH$_2$(OH), -CH(OH)-CH(OH)-CH(OH)-CH$_2$(OH), -CH$_2$-O-S(=O)$_2$OH, -(CH$_2$)$_2$-O-S(=O)$_2$OH, -(CH$_2$)$_3$-O-S(=O)$_2$OH, -(CH$_2$)$_4$-O-S(=O)$_2$OH, -(CH$_2$)$_5$-O-S(=O)$_2$OH, -(CH$_2$)$_6$-O-S(=O)$_2$OH, -CH$_2$-O-P(=O)(OH)$_2$, -(CH$_2$)$_2$-O-P(=O)(OH)$_2$, - (CH$_2$)$_3$-O-P(=O)(OH)$_2$, -(CH$_2$)$_4$-O-P(=O)(OH)$_2$, -(CH$_2$)$_5$-O-P(=O)(OH)$_2$, -(CH$_2$)$_6$-O-P(=O)(OH)$_2$, -CH$_2$-SH, -CH(SH)-CH$_3$, -(CH$_2$)$_2$-SH, -CH(SH)-CH$_2$-CH$_3$, -CH$_2$-CH(SH)-CH$_3$, -(CH$_2$)$_3$-SH, -CH(SH)-(CH$_2$)$_2$-CH$_3$, -CH$_2$-CH(SH)-CH$_2$-CH$_3$, -(CH$_2$)$_2$-CH(SH)-CH$_3$, -(CH$_2$)$_4$-SH, -(CH$_2$)$_5$-SH, -(CH$_2$)$_6$-SH, -CH(SH)-CH$_2$(SH), -CH(SH)-CH(SH)-CH$_3$, -CH(SH)-CH$_2$-CH$_2$(SH), -CH$_2$-CH(SH)-CH$_2$(SH), -CH(SH)-CH(SH)-CH$_2$-CH$_3$, -CH(SH)-CH$_2$CH(SH)-CH$_3$, -CH(SH)-(CH$_2$)$_2$-CH$_2$(SH), -CH$_2$-CH(SH)-CH(SH)-CH$_3$, -CH$_2$-CH(SH)-CH$_2$-CH$_2$(SH), -(CH$_2$)$_2$-CH(SH)-CH$_2$(SH), -CH(SH)-CH(SH)-CH$_2$(SH), -CH(SH)-CH(SH)-C(SH)-CH$_3$, -CH(SH)-CH(SH)-CH$_2$-CH$_2$(SH), -CH(SH)-CH$_2$-CH(SH)-CH$_2$(SH), -CH$_2$-CH(SH)-CH(SH)-CH$_2$(SH), -CH(SH)-CH(SH)-CH(SH)-CH$_2$(SH), -CH$_2$-S(=O)$_2$OH, -(CH$_2$)$_2$-S(=O)$_2$OH, -(CH$_2$)$_3$-S(=O)$_2$OH, -(CH$_2$)$_4$-S(=O)$_2$OH, -(CH$_2$)$_5$-S(=O)$_2$OH, -(CH$_2$)$_6$-S(=O)$_2$OH, -CH$_2$-NH$_2$, -(CH$_2$)$_2$-NH$_2$, -(CH$_2$)$_3$-NH$_2$, -(CH$_2$)$_4$-NH$_2$, -(CH$_2$)$_5$-NH$_2$, -(CH$_2$)$_6$-NH$_2$, -CH$_2$-NH(CH$_3$), -(CH$_2$)$_2$-NH(CH$_3$), -(CH$_2$)$_3$-NH(CH$_3$), -(CH$_2$)$_4$-NH(CH$_3$), -(CH$_2$)$_5$-NH(CH$_3$), -(CH$_2$)$_6$-NH(CH$_3$), -CH$_2$-NH(CH$_2$CH$_3$), -(CH$_2$)$_2$-NH(CH$_2$CH$_3$), -(CH$_2$)$_3$-NH(CH$_2$CH$_3$), -(CH$_2$)$_4$-NH(CH$_2$CH$_3$), -(CH$_2$)$_5$-NH(CH$_2$CH$_3$), -(CH$_2$)$_6$-NH(CH$_2$CH$_3$), -CH$_2$-N(CH$_3$)$_2$, -(CH$_2$)$_2$-N(CH$_3$)$_2$, -(CH$_2$)$_3$-N(CH$_3$)$_2$, -(CH$_2$)$_4$-N(CH$_3$)$_2$, -(CH$_2$)$_5$-N(CH$_3$)$_2$, -(CH$_2$)$_6$-N(CH$_3$)$_2$, -CH$_2$-N(CH$_2$CH$_3$)$_2$, -(CH$_2$)$_2$-N(CH$_2$CH$_3$)$_2$, -(CH$_2$)$_3$-N(CH$_2$CH$_3$)$_2$, -(CH$_2$)$_4$-N(CH$_2$-CH$_3$)$_2$, -(CH$_2$)$_5$-N(CH$_2$CH$_3$)$_2$, -(CH$_2$)$_6$-N(CH$_2$CH$_3$)$_2$, -CH$_2$-N(CH$_3$)$_3$$^+$A$^-$, -(CH$_2$)$_2$-N(CH$_3$)$_3$$^+$A$^-$, -(CH$_2$)$_3$-N(CH$_3$)$_3$$^+$A$^-$, -(CH$_2$)$_4$-N(CH$_3$)$_3$$^+$A$^-$, -(CH$_2$)$_5$-N(CH$_3$)$_3$$^+$A$^-$, -(CH$_2$)$_6$-N(CH$_3$)$_3$$^+$A$^-$, -CH$_2$-N(CH$_2$-CH$_3$)$_3$$^+$A$^-$, -(CH$_2$)$_2$-N(CH$_2$CH$_3$)$_3$$^+$A$^-$, -(CH$_2$)$_3$-N(CH$_2$CH$_3$)$_3$$^+$A$^-$, -(CH$_2$)$_4$-N(CH$_2$CH$_3$)$_3$$^+$A$^-$, -(CH$_2$)$_5$-N(CH$_2$CH$_3$)$_3$$^+$A$^-$, -(CH$_2$)$_6$-N(CH$_2$CH$_3$)$_3$$^+$A$^-$, -CH$_2$-P(=O)(OH)$_2$, -(CH$_2$)$_2$-P(=O)(OH)$_2$, -(CH$_2$)$_3$-P(=O)(OH)$_2$, -(CH$_2$)$_4$-P(=O)(OH)$_2$, -(CH$_2$)$_5$-P(=O)(OH)$_2$, -(CH$_2$)$_6$-P(=O)(OH)$_2$, -CH$_2$-Pyrrolyl, -(CH$_2$)$_2$-Pyrrolyl, -(CH$_2$)$_3$-Pyrrolyl, -(CH$_2$)$_4$-Pyrrolyl, -CH$_2$-Pyrazolyl, -(CH$_2$)$_2$-Pyrazolyl, -(CH$_2$)$_3$-Pyrazolyl, -(CH$_2$)$_4$-Pyrazolyl, -CH$_2$-Imidazolyl, -(CH$_2$)$_2$-Imidazolyl, -(CH$_2$)$_3$-Imidazolyl, -(CH$_2$)$_4$-Imidazolyl, -CH$_2$-Oxazolyl, -(CH$_2$)$_2$-Oxazolyl, -(CH$_2$)$_3$-Oxazolyl, -(CH$_2$)$_4$-Oxazolyl, -CH$_2$-Isoxazolyl, -(CH$_2$)$_2$-Isoxazolyl, -(CH$_2$)$_3$-Isoxazolyl, -(CH$_2$)$_4$-Isoxazolyl, -CH$_2$-Isothiazolyl, -(CH$_2$)$_2$-Isothiazolyl, -(CH$_2$)$_3$-Isothiazolyl, -(CH$_2$)$_4$-Isothiazolyl, -CH$_2$-Pyridyl, -(CH$_2$)$_2$-Pyridyl, -(CH$_2$)$_3$-Pyridyl und -(CH$_2$)$_4$-Pyridyl; wobei A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht; wobei die Reste ggf. mit Hybridresten weiter substituiert sein können.

In einer weiteren bevorzugten Ausführungsform sind Q, $Q^1$, $Q^2$, $Q^3$, $Q^4$, $Q^5$, $Q^6$, $Q^7$, $Q^8$, $Q^9$ und $Q^{10}$, unabhängig voneinander, ausgewählt aus der Gruppe bestehend aus Wasserstoff, Glucose, Galactose, Maltose, Maltriose, -C(=O)-C$_{1-6}$-Alkyl, -C(=O)-C$_{3-7}$-Cycloalkyl, -C(=O)-Phenyl, -C(=O)-(5- bis 7-gliedriges Heteroaryl), -C$_{1-6}$-Alkyl,-C$_{3-7}$-Cycloalkyl, -Phenyl und -(5- bis 7-gliedriges Heteroaryl); wobei die C$_{3-7}$-Cycloalkyl-, Phenyl- und 5- bis 7-gliedrigen Heteroaryl-Reste über eine C$_{1-4}$-Alkylenbrücke, vorzugsweise -CH$_2$-, -CH$_2$-CH$_2$-, -CH$_2$-CH$_2$-CH$_2$-, oder -CH$_2$-CH$_2$-CH$_2$-CH$_2$-, an die Cyclodextrin-Monomere gebunden sein können; und C$_{1-6}$-Alkyl, C$_{3-7}$-Cycloalkyl, Phenyl und 5- bis 7-gliedriges Heteroaryl unsubstituiert oder ein- oder mehrfach (z.B. zwei-, drei- vier- oder fünffach) mit gleichen oder verschiedenen Resten $R^5$ substituiert sein können; wobei $R^5$ ausgewählt ist aus der Gruppe bestehend aus -C(=O)OH, -OH, -OT, -OC(=O)OH, -OC(=O)NH$_2$, -OS(=O)OH, -OS(=O)$_2$OH, -OP(=O)(OH)(X), -OP(=O)(OH)$_2$, -OP(=O)(OX)(OH), -OP(=O)(OX)$_2$, -SH, -ST, -S(=O)OH, -S(=O)$_2$OH, -NH$_2$, -NHX, -NHT, -NX$_2$, -NT$_2$, -N(X)$_3$$^+$A$^-$, -NHC(=O)OH, -NXC(=O)OH, -NHS(=O)OH, -NXS(=O)OH, -NHS(=O)$_2$OH, -NXS(=O)$_2$OH, =N-OH, =N-NH$_2$, -P(=O)(OH)(X), -P(=O)(OH)$_2$, -P(=O)(OX)(OH), -P(=O)(OX)$_2$, =NH, =NX, Pyrrolidinyl, Pyrazolinyl, Pyrazolidinyl, Imidazolinyl, Imidazolidinyl, Piperidinyl, Piperazinyl, 4-X-Piperazinyl, Morpholinyl, Chinuclidinyl, Pyrazolinonyl und Imidazolyl; wobei der Rest X ausgewählt ist aus der Gruppe bestehend aus Methyl, Ethyl, n-Propyl, iso-Propyl, Cyclopropyl, Allyl, Vinyl und Ethinyl; der Rest T

für einen Hybridrest steht; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht

In einer weiteren bevorzugten Ausführungsform sind Q, $Q^1$, $Q^2$, $Q^3$, $Q^4$, $Q^5$, $Q^6$, $Q^7$, $Q^8$, $Q^9$ und $Q^{10}$, unabhängig voneinander, ausgewählt aus der Gruppe bestehend aus Wasserstoff, Glucose, Galactose, Maltose, Maltriose, -C(=O)-Methyl, -C(=O)-Ethyl, -C(=O)-iso-Propyl, -C(=O)-n-Propyl, -C(=O)-iso-Butyl, -C(=O)-n-Butyl, -C(=O)-sec-Butyl, -C(=O)-tert-Butyl, -C(=O)-n-Pentyl, -C(=O)-iso-Pentyl, -C(=O)-neo-Pentyl, -C(=O)-iso-Hexyl, -C(=O)-n-Hexyl, -C(=O)-neo-Hexyl, -C(=O)-(3-Methylpentyl), -C(=O)-(2,3-Dimethylbutyl), -C(=O)-Ethenyl, -C(=O)-Ethinyl, -C(=O)-Prop-1-enyl, -C(=O)-Cyclopropyl, -C(=O)-Cyclobutyl, -C(=O)-Cyclopentyl, -C(=O)-Cyclohexyl, -C(=O)-Piperidinyl, -C(=O)-Piperazinyl, -C(=O)-Morpholinyl, -C(=O)-Phenyl, -C(=O)-Imidazolyl, -C(=O)-Thienyl, -C(=O)-Furyl, -C(=O)-Oxazolyl, -C(=O)-Isoxazolyl, -C(=O)-Thiazolyl, -C(=O)-Isothiazolyl, -C(=O)-Pyridyl, Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methyl-pentyl, 2,3-Dimethylbutyl, Ethenyl, Ethinyl, Prop-1-enyl, Cyclopropyl, Cyclobutyl, Cyclopentyl, Cyclohexyl, Piperidinyl, Piperazinyl, Morpholinyl, Phenyl, Pyrrolyl, Pyrazolyl, Imidazolyl, Thienyl, Furyl, Oxazolyl, Isoxazolyl, Thiazolyl, Isothiazolyl und Pyridyl; wobei die $C_{3-7}$-Cycloalkyl-, Phenyl- und 5- bis 7-gliedrigen Heteroaryl-Reste über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die entsprechenden Cyclodextrin-Monomere gebunden sein können; und die $C_{1-6}$-Alkyl-, $C_{3-7}$-Cycloalkyl-, Phenyl- und 5- bis 7-gliedrigen Heteroaryl-Reste unsubstituiert oder ein- oder mehrfach (z.B. zwei-, drei- vier- oder fünffach) mit Resten $R^5$ substituiert sein können; wobei $R^5$ vorzugsweise ausgewählt ist aus der Gruppe bestehend aus -C(=O)OH, -OH, -OT, $-OS(=O)_2OH$, $-OP(=O)(OH)_2$, -SH, -ST, $-S(=O)_2OH$, $-NH_2$, -NHX, -NHT, $-NX_2$, $-NT_2$, $-N(X)_3{}^+A^-$ -NHC(=O)OH, -NXC(=O)OH, -P(=O)(OH)(X), $-P(=O)(OH)_2$, -P(=O)(OX)(OH), Piperidinyl, Piperazinyl, Morpholinyl und Imidazolyl; wobei der Rest X ausgewählt ist aus der Gruppe bestehend aus Methyl, Ethyl, n-Propyl, iso-Propyl, Cyclopropyl, Allyl, Vinyl und Ethinyl; der Rest T für einen Hybridrest steht; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht

In einer weiteren bevorzugten Ausführungsform sind Q, $Q^1$, $Q^2$, $Q^3$, $Q^4$, $Q^5$, $Q^6$, $Q^7$, $Q^8$, $Q^9$ und $Q^{10}$, unabhängig voneinander, ausgewählt aus der Gruppe bestehend aus Wasserstoff, Glucose, Galactose, Maltose, Maltriose, -C(=O)-Methyl, -C(=O)-Ethyl, -C(=O)-iso-Propyl, -C(=O)-n-Propyl, -C(=O)-iso-Butyl, -C(=O)-n-Butyl, -C(=O)-sec-Butyl, -C(=O)-tert-Butyl, -C(=O)-Ethenyl, -C(=O)-Ethinyl, -C(=O)-Prop-1-enyl, -C(=O)-Cyclopropyl, -C(=O)-Cyclobutyl, -C(=O)-Cyclopentyl, -C(=O)-Cyclohexyl, -C(=O)-Phenyl, -C(=O)-Benzyl, -C(=O)-Phenethyl, $-C(=O)-CH_2-C(=O)-OH$, $-C(=O)-(CH_2)_2-C(=O)-OH$, $-C(=O)-(CH_2)_3-C(=O)-OH$, $-C(=O)-(CH_2)_4-C(=O)-OH$, $-C(=O)-(CH_2)_5-C(=O)-OH$, $-C(=O)-(CH_2)_6-C(=O)-OH$, $-C(=O)-CH_2-OH$, $-C(=O)-CH(OH)-CH_3$, $-C(=O)-(CH_2)_2-OH$, $-C(=O)-CH(OH)-CH_2-CH_3$, $-C(=O)-CH_2-CH(OH)-CH_3$, $-C(=O)-(CH_2)_3-OH$, $-C(=O)-CH(OH)-(CH_2)_2-CH_3$, $-C(=O)-CH_2-CH(OH)-CH_2-CH_3$, $-C(=O)-(CH_2)_2-CH(OH)-CH_3$, $-C(=O)-(CH_2)_4-OH$, $-C(=O)-(CH_2)_5-OH$, $-C(=O)-(CH_2)_6-OH$, $-C(=O)-CH(OH)-CH_2(OH)$, $-C(=O)-CH(OH)-CH(OH)-CH_3$, $-C(=O)-CH(OH)-CH_2-CH_2(OH)$, $-C(=O)-CH_2-CH(OH)-CH_2(OH)$, $-C(=O)-CH(OH)-CH(OH)-CH_2-CH_3$, $-C(=O)-CH(OH)-CH_2CH(OH)-CH_3$, $-C(=O)-CH(OH)-(CH_2)_2-CH_2(OH)$, $-C(=O)-CH_2-CH(OH)-CH(OH)-CH_3$, $-C(=O)-CH_2-CH(OH)-CH_2-CH_2(OH)$, $-C(=O)-(CH_2)_2-CH(OH)-CH_2(OH)$, $-C(=O)-CH(OH)-CH(OH)-CH_2(OH)$, $-C(=O)-CH(OH)-CH(OH)-CH(OH)-CH_3$, $-C(=O)-CH(OH)-CH(OH)-CH_2-CH_2(OH)$, $-C(=O)-CH(OH)-CH_2-CH(OH)-CH_2(OH)$, $-C(=O)-CH_2-CH(OH)-CH(OH)-CH_2(OH)$, $-C(=O)-CH(OH)-CH(OH)-CH(OH)-CH_2(OH)$, $-C(=O)-CH_2-NH_2$, $-C(=O)-(CH_2)_2-NH_2$, $-C(=O)-(CH_2)_3-NH_2$, $-C(=O)-(CH_2)_4-NH_2$, $-C(=O)-(CH_2)_5-NH_2$, $-C(=O)-(CH_2)_6-NH_2$, $-C(=O)-CH_2-NH(CH_3)$, $-C(=O)-(CH_2)_2-NH(CH_3)$, $-C(=O)-(CH_2)_3-NH(CH_3)$, $-C(=O)-(CH_2)_4-NH(CH_3)$, $-C(=O)-(CH_2)_5-NH(CH_3)$, $-C(=O)-(CH_2)_6-NH(CH_3)$, $-C(=O)-CH_2-NH(CH_2CH_3)$, $-C(=O)-(CH_2)_2-NH(CH_2CH_3)$, $-C(=O)-(CH_2)_3-NH(CH_2CH_3)$, $-C(=O)-(CH_2)_4-NH(CH_2CH_3)$, $-C(=O)-(CH_2)_5-NH(CH_2CH_3)$, $-C(=O)-(CH_2)_6-NH(CH_2CH_3)$, $-C(=O)-CH_2-N(CH_3)_2$, $-C(=O)-(CH_2)_2-N(CH_3)_2$, $-C(=O)-(CH_2)_3-N(CH_3)_2$, $-C(=O)-(CH_2)_4-N(CH_3)_2$, $-C(=O)-(CH_2)_5-N(CH_3)_2$, $-C(=O)-(CH_2)_6-N(CH_3)_2$, $-C(=O)-CH_2-N(CH_2-CH_3)_2$, $-C(=O)-(CH_2)_2-N(CH_2CH_3)_2$, $-C(=O)-(CH_2)_3-N(CH_2CH_3)_2$, $-C(=O)-(CH_2)_4-N(CH_2CH_3)_2$, $-C(=O)-(CH_2)_5-N(CH_2CH_3)_2$, $-C(=O)-(CH_2)_6-N(CH_2CH_3)_2$, Methyl, Ethyl, iso-Propyl, n-Propyl, iso-Butyl, n-Butyl, sec-Butyl, tert-Butyl, Ethenyl, Ethinyl, Prop-1-enyl, Cyclopropyl, Cyclobutyl, Cyclopentyl, Cyclohexyl, Phenyl, Benzyl, Phenethyl, $-CH_2-C(=O)-OH$, $-(CH_2)_2-C(=O)-OH$, $-(CH_2)_3-C(=O)-OH$, $-(CH_2)_4-C(=O)-OH$, $-(CH_2)_5-C(=O)-OH$, $-(CH_2)_6-C(=O)-OH$, $-CH_2-CH(OH)-C(=O)OH$, $-CH_2-CH(OH)-C(=O)O-CH_3$, $-CH_2-CH(OH)-C(=O)O-CH_2-CH_3$, $-CH(CH_2OH)-C(=O)OH$, $-CH(CH_2-OH)-C(=O)O-CH_3$, $-CH(CH_2OH)-C(=O)O-CH_2-CH_3$, $-CH_2-CH(OH)-CH_2-C(=O)-OH$, $-CH_2-CH(OH)-CH_2-C(=O)-O-CH_3$, $-CH_2-CH(OH)-CH_2-C(=O)-OCH_2-CH_3$, $-CH(CH_2-OH)-CH_2-C(=O)-O-CH_3$, $-CH(CH_2-OH)-CH_2-C(=O)-O-CH_2-CH_3$, $-CH_2-OH$, $-CH(OH)-CH_3$, $-(CH_2)_2-OH$, $-CH(OH)-CH_2-CH_3$, $-CH_2-CH(OH)-CH_3$, $-(CH_2)_3-OH$, $-CH(CH_3)-CH_2-OH$, $-CH(OH)-(CH_2)_2-CH_3$, $-CH_2-CH(OH)-CH_2-CH_3$, $-(CH_2)_2-CH(OH)-CH_3$, $-(CH_2)_4-OH$, $-(CH_2)_5-OH$, $-(CH_2)_6-OH$, $-CH(OH)-CH_2(OH)$, $-CH(OH)-CH(OH)-CH_3$, $-CH(OH)-CH_2-CH_2(OH)$, $-CH_2-CH(OH)-CH_2(OH)$, $-CH(OH)-CH(OH)-CH_2-CH_3$, $-CH(OH)-CH_2-CH(OH)-CH_3$, $-CH(OH)-(CH_2)_2-CH_2(OH)$, $-CH_2-CH(OH)-CH(OH)-CH_3$, $-CH_2-CH(OH)-CH_2-CH_2(OH)$, $-(CH_2)_2-CH(OH)-CH_2(OH)$, $-CH(OH)-CH(OH)-CH_2(OH)$, $-CH(OH)-CH(OH)-CH(OH)-CH_3$, $-CH(OH)-CH(OH)-CH_2-CH_2(OH)$, $-CH(OH)-CH_2-CH(OH)-CH_2(OH)$,

$-CH_2-CH(OH)-CH(OH)-CH_2(OH)$, $-CH(OH)-CH(OH)-CH(OH)-CH_2(OH)$, $-CH_2-O-S(=O)_2OH$, $-(CH_2)_2-O-S(=O)_2OH$, $-(CH_2)_3-O-S(=O)_2OH$, $-(CH_2)_4-O-S(=O)_2OH$, $-(CH_2)_5-O-S(=O)_2OH$, $-(CH_2)_6-O-S(=O)_2OH$, $-CH_2-O-P(=O)(OH)_2$, $-(CH_2)_2-O-P(=O)(OH)_2$, $-(CH_2)_3-O-P(=O)(OH)_2$, $-(CH_2)_4-O-P(=O)(OH)_2$, $-(CH_2)_5-O-P(=O)(OH)_2$, $-(CH_2)_6-O-P(=O)(OH)_2$, $-CH_2-SH$, $-CH(SH)-CH_3$, $-(CH_2)_2-SH$, $-CH(SH)-CH_2-CH_3$, $-CH_2-CH(SH)-CH_3$, $-(CH_2)_3-SH$, $-CH(SH)-(CH_2)_2-CH_3$, $-CH_2-CH(SH)-CH_2-CH_3$, $-(CH_2)_2-CH(SH)-CH_3$, $-(CH_2)_4-SH$, $-(CH_2)_5-SH$, $-(CH_2)_6-SH$, $-CH_2-S(=O)_2OH$, $-(CH_2)_2-S(=O)_2OH$, $-(CH_2)_3-S(=O)_2OH$, $-(CH_2)_4-S(=O)_2OH$, $-(CH_2)_5-S(=O)_2OH$, $-(CH_2)_6-S(=O)_2OH$, $-CH_2-NH_2$, $-(CH_2)_2-NH_2$, $-(CH_2)_3-NH_2$, $-(CH_2)_4-NH_2$, $-(CH_2)_5-NH_2$, $-(CH_2)_6-NH_2$, $-CH_2-NH(CH_3)$, $-(CH_2)_2-NH(CH_3)$, $-(CH_2)_3-NH(CH_3)$, $-(CH_2)_4-NH(CH_3)$, $-(CH_2)_5-NH(CH_3)$, $-(CH_2)_6-NH(CH_3)$, $-CH_2-NH(CH_2CH_3)$, $-(CH_2)_2-NH(CH_2CH_3)$, $-(CH_2)_3-NH(CH_2CH_3)$, $-(CH_2)_4-NH(CH_2CH_3)$, $-(CH_2)_5-NH(CH_2CH_3)$, $-(CH_2)_6-NH(CH_2CH_3)$, $-CH_2-N(CH_3)_2$, $-(CH_2)_2-N(CH_3)_2$, $-(CH_2)_3-N(CH_3)_2$, $-(CH_2)_4-N(CH_3)_2$, $-(CH_2)_5-N(CH_3)_2$, $-(CH_2)_6-N(CH_3)_2$, $-CH_2-N(CH_2CH_3)_2$, $-(CH_2)_2-N(CH_2CH_3)_2$, $-(CH_2)_3-N(CH_2CH_3)_2$, $-(CH_2)_4-N(CH_2-CH_3)_2$, $-(CH_2)_5-N(CH_2CH_3)_2$, $-(CH_2)_6-N(CH_2CH_3)_2$, $-CH_2-N(CH_3)_3^+A^-$, $-(CH_2)_2-N(CH_3)_3^+A^-$, $-(CH_2)_3-N(CH_3)_3^+A^-$, $-(CH_2)_4-N(CH_3)_3^+A^-$, $-(CH_2)_5-N(CH_3)_3^+A^-$, $-(CH_2)_6-N(CH_3)_3^+A^-$, $-CH_2-N(CH_2-CH_3)_3^+A^-$, $-(CH_2)_2-N(CH_2CH_3)_3^+A^-$, $-(CH_2)_3-N(CH_2CH_3)_3^+A^-$, $-(CH_2)_4-N(CH_2CH_3)_3^+A^-$ , $-(CH_2)_5-N(CH_2-CH_3)_3^+A^-$, $-(CH_2)_6-N(CH_2CH_3)_3^+A^-$, $-CH_2-P(=O)(OH)_2$, $-(CH_2)_2-P(=O)(OH)_2$, $-(CH_2)_3-P(=O)(OH)_2$, $-(CH_2)_4-P(=O)(OH)_2$, $-(CH_2)_5-P(=O)(OH)_2$, $-(CH_2)_6-P(=O)(OH)_2$; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht; und die Reste ggf. mit Hybridresten weiter substituiert sein können.

Für die Zwecke dieser Erfindung steht der Ausdruck "Linker" für die von Vernetzungsmitteln abgeleiteten Monomere, die in den erfindungsgemäßen Copolymeren enthalten sind und die wenigstens zwei kovalente Bindungen zu wenigstens zwei Cyclodextrin-Monomeren enthalten.

Im Sinne dieser Erfindung steht der Ausdruck "von Vernetzungsmitteln abgeleiteter Linker" für Strukturelemente, die in den erfindungsgemäßen Copolymeren enthalten sind und deren chemische Struktur auf die strukturelle Abwandlung der Vernetzungsmittel durch die Vernetzungsreaktion mit Cyclodextrin-Verbindungen zurückzuführen ist (vgl. hierzu auch die oben stehende Beispielreaktion).

Die Linker sind vorzugsweise abgeleitet von bi- und/ oder poylfunktionellen Vernetzungsmitteln, bevorzugter von homobifunktionellen, homopolyfunktionellen, heterobifunktionellen und/ oder heteropolyfunktionellen Vernetzungsmitteln und insbesondere von homo- und/ oder heterobifunktionellen Vernetzungsmitteln.

In einer bevorzugten Ausführungsform sind die Linker abgeleitet von Vernetzungsmitteln ausgewählt aus der Gruppe bestehend aus Glyoxal, 1,2-Dihalogenethan (z.B. 1,2-Dichlorethan), 1,3-Dihalogenpropan (z.B. 1,3-Dichlorpropan), Halogencarbonsäurehalogenid [beispielsweise Halogenessigsäurehalogenid (z.B. Chloressigsäurechlorid) oder Halogenpropionsäurehalogenid (z.B. Chlorpropionsäurechlorid)], Epichlorhydrin, 4-Chlor-1,2-epoxybutan, 1,2,3,4-Diepoxybutan, Tetramethylendiisocyanat und Hexamethylendiisocyanat; wobei Halogen und Halogenid, unabhängig voneinander, vorzugsweise für -Cl, -Br oder -I, besonders bevorzugt für -Cl oder -Br und insbesondere für -Cl stehen.

In einer weiteren bevorzugten Ausführungsform ist der Linker abgeleitet von einem Vernetzungsmittel ausgewählt aus der Gruppe bestehend aus Glyoxal, Epichlorhydrin, 4-Chlor-1,2-epoxybutan und 1,2, 3,4-Diepoxybutan. Besonders vorteilhaft sind Epichlorhydrin, 4-Chlor-1,2-epoxybutan und 1,2,3,4-Diepoxybutan. Insbesondere bevorzugt ist das Vernetzungsmittel Epichlorhydrin.

Die Linker sind vorzugsweise ausgewählt aus der Gruppe umfassend $\sim CH_2\sim$, $\sim CH_2-CH_2\sim$, $\sim CH(CH_3)\sim$, $\sim CH_2-CH_2-CH_2\sim$, $\sim CH(CH_2-CH_3)\sim$, $\sim CH(CH_3)-CH_2\sim$, $\sim C(CH_3)_2\sim$, $\sim CH_2-CH_2-CH_2-CH_2\sim$, $\sim CH(CH_2-CH_2-CH_3)\sim$, $\sim C(CH_3)(CH_2CH_3)\sim$, $\sim C(CH_3)_2-CH_2\sim$, $\sim CH(CH_2CH_3)-CH_2\sim$, $\sim CH(CH_3)-CH(CH_3)\sim$, $\sim CH_2-CH_2-CH_2-CH_2-CH_2\sim$, $\sim CH_2-CH_2-CH_2-CH_2-CH_2-CH_2\sim$, $\sim C(=O)-CH_2\sim$, $\sim C(=O)-CH_2-CH_2\sim$, $\sim C(=O)-CH(CH_3)\sim$, $\sim C(=O)-CH_2-CH_2-CH_2\sim$, $\sim C(=O)-CH(CH_2-CH_3)\sim$, $\sim C(=O)-CH(CH_3)-CH_2\sim$, $\sim C(=O)-C(CH_3)_2\sim$, $\sim C(=O)-CH_2-CH_2-CH_2-CH_2\sim$, $\sim C(=O)-CH(CH_2-CH_2-CH_3)\sim$, $\sim C(=O)-C(CH_3)(CH_2CH_3)\sim$, $\sim C(=O)-C(CH_3)_2-CH_2\sim$, $\sim C(=O)-CH(CH_2CH_3)-CH_2\sim$, $\sim C(=O)-CH(CH_3)-CH(CH_3)\sim$, $\sim C(=O)-CH_2-CH_2-CH_2-CH_2-CH_2\sim$, $\sim C(=O)-CH_2-CH_2-CH_2-CH_2-CH_2-CH_2\sim$, $\sim C(=O)-CH_2-C(=O)\sim$, $\sim C(=O)-CH_2-CH_2-C(=O)\sim$, $\sim C(=O)-CH(CH_3)-C(=O)\sim$, $\sim C(=O)-CH_2-CH_2-CH_2-C(=O)\sim$, $\sim C(=O)-CH(CH_2-CH_3)-C(=O)\sim$, $\sim C(=O)-CH(CH_3)-CH_2-C(=O)\sim$, $\sim C(=O)-C(CH_3)_2-C(=O)\sim$, $\sim C(=O)-CH_2-CH_2-CH_2-CH_2-C(=O)\sim$, $\sim C(=O)-CH(CH_2-CH_2-CH_3)-C(=O)\sim$, $\sim C(=O)-C(CH_3)(CH_2CH_3)-C(=O)\sim$, $\sim C(=O)-C(CH_3)_2-CH_2-C(=O)\sim$, $\sim C(=O)-CH(CH_2CH_3)-CH_2-C(=O)\sim$, $\sim C(=O)-CH(CH_3)-CH(CH_3)-C(=O)\sim$, $\sim C(=O)-CH_2-CH_2-CH_2-CH_2-CH_2-C(=O)\sim$, $\sim C(=O)-CH_2-CH_2-CH_2-CH_2-CH_2-CH_2-C(=O)\sim$, $\sim C(=O)-NH-CH_2-NH-C(=O)\sim$, $\sim C(=O)-NH-(CH_2)_2-NH-C(=O)\sim$, $\sim C(=O)-NH-CH(CH_3)-NH-C(=O)\sim$, $\sim C(=O)-NH-CH_2-CH_2-CH_2-NH-C(=O)\sim$, $\sim C(=O)-NH-CH(CH_2-CH_3)-NH-C(=O)\sim$, $\sim C(=O)-NH-CH(CH_3)-CH_2-NH-C(=O)\sim$, $\sim C(=O)-NH-C(CH_3)_2-NH-C(=O)\sim$, $\sim C(=O)-NH-CH_2-CH_2-CH_2-CH_2-NH-C(=O)\sim$, $\sim C(=O)-NH-CH(CH_2-CH_2-CH_3)-NH-C(=O)\sim$, $\sim C(=O)-NH-C(CH_3)(CH_2CH_3)-NH-C(=O)\sim$, $\sim C(=O)-NH-C(CH_3)_2-CH_2-NH-C(=O)\sim$, $\sim C(=O)-NH-CH(CH_2CH_3)-CH_2-NH-C(=O)\sim$, $\sim C(=O)-NH-CH(CH_3)-CH(CH_3)-NH-C(=O)\sim$, $\sim C(=O)-NH-(CH_2)_5-NH-C(=O)\sim$, $\sim C(=O)-NH-(CH_2)_6-NH-C(=O)\sim$, $\sim CH_2-CH(OH)-CH_2\sim$, $\sim CH(CH_2OH)-CH_2\sim$, $\sim CH_2-CH(OH)-CH_2-CH_2\sim$, $\sim CH(CH_2OH)-CH_2-CH_2\sim$, $\sim CH_2-CH(OH)-CH(OH)-CH_2\sim$, $\sim CH(CH_2OH)-CH(CH_2OH)\sim$, $\sim CH_2-CH(OH)-CH(CH_2OH)\sim$, $\sim CH(OH)-CH_2-CH_2-CH_2\sim$ und $\sim CH(CH_2OH)-CH_2-CH_2\sim$; wobei das Symbol "~" für eine kovalente Bindung zu einem Cyclodextrin-Monomer steht

und die Linker ggf. mit Hybridresten weiter substituiert sein können.

In einer weiteren bevorzugten Ausführungsform sind die Linker ausgewählt aus der Gruppe umfassend ~$CH_2$~, ~$CH_2$-$CH_2$~, ~$CH_2$-$CH_2$-$CH_2$~, ~$CH_2$-$CH_2$-$CH_2$-$CH_2$~, ~$CH_2$-$CH_2$-$CH_2$-$CH_2$-$CH_2$~, ~$CH_2$-$CH_2$-$CH_2$-$CH_2$-$CH_2$-$CH_2$~, ~C(=O)-$CH_2$~, ~C(=O)-$CH_2$-$CH_2$~, ~C(=O)-$CH_2$-$CH_2$-$CH_2$~, ~C(=O)-$CH_2$-$CH_2$-$CH_2$-$CH_2$~ ~C(=O)-$CH_2$-$CH_2$-$CH_2$-$CH_2$-$CH_2$~, ~C(=O)-$CH_2$-$CH_2$-$CH_2$-$CH_2$-$CH_2$-$CH_2$~, ~C(=O)-$CH_2$-C(=O)~, ~C(=O)-$CH_2$-$CH_2$-C(=O)~, ~C(=O)-$CH_2$-$CH_2$-$CH_2$-C(=O)~, ~C(=O)-$CH_2$-$CH_2$-$CH_2$-$CH_2$-C(=O)~, ~C(=O)-$CH_2$-$CH_2$-$CH_2$-$CH_2$-$CH_2$-C(=O)~, ~C(=O)-$CH_2$-$CH_2$-$CH_2$-$CH_2$-$CH_2$-$CH_2$-C(=O)~, ~C(=O)-NH-$CH_2$-NH-C(=O)~, ~C(=O)-NH-$CH_2$-$CH_2$-NH-C(=O)~, ~C(=O)-NH-$CH_2$-$CH_2$-$CH_2$-NH-C(=O)~, ~C(=O)-NH-$CH_2$-$CH_2$-$CH_2$-$CH_2$-NH-C(=O)~, ~C(=O)-NH-$CH_2$-$CH_2$-$CH_2$-$CH_2$-$CH_2$-NH-C(=O)~, ~C(=O)-NH-$CH_2$-$CH_2$-$CH_2$-$CH_2$-$CH_2$-$CH_2$-NH-C(=O)~, ~$CH_2$-CH(OH)-$CH_2$~, ~CH($CH_2$OH)-$CH_2$~, ~$CH_2$-CH(OH)-$CH_2$-$CH_2$~, ~CH($CH_2$OH)-$CH_2$-$CH_2$~, ~$CH_2$-CH(OH)-CH(OH)-$CH_2$~, ~CH($CH_2$OH)-CH($CH_2$OH)~ und ~$CH_2$-CH(OH)-CH($CH_2$OH)~; wobei das Symbol "~" für eine kovalente Bindung zu einem Cyclodextrin-Monomer steht und die Linker ggf. mit Hybridresten weiter substituiert sein können.

Besonders vorteilhaft ist, dass die erfindungsgemäßen Cyclodextrin-Copolymere einen oder mehrere Linker enthalten, die ausgewählt sind aus der Gruppe umfassend ~C(=O)-NH-$CH_2$-$CH_2$-$CH_2$-$CH_2$-NH-C(=O)~, ~C(=O)-NH-$CH_2$-$CH_2$-$CH_2$-$CH_2$-$CH_2$-NH-C(=O)~, ~C(=O)-NH-$CH_2$-$CH_2$-$CH_2$-$CH_2$-$CH_2$-$CH_2$-NH-C(=O)~~$CH_2$-CH(OH)-$CH_2$~, ~CH($CH_2$OH)-$CH_2$~, ~$CH_2$-CH(OH)-$CH_2$-$CH_2$~, ~CH($CH_2$OH)-$CH_2$-$CH_2$~, ~$CH_2$-CH(OH)-CH(OH)-$CH_2$~, ~CH($CH_2$OH)-CH($CH_2$OH)~, ~$CH_2$-CH(OH)-CH($CH_2$OH)~; wobei das Symbol "~" für eine kovalente Bindung zu einem Cyclodextrin-Monomer steht und die Linker ggf. mit Hybridresten weiter substituiert sein können.

Linker und Substituenten, die wenigstens einen nukleophilen Rest enthalten (z.B. - OH, -SH, -$NH_2$), können während der Synthese der erfindungsgemäßen Copolymere mit den eingesetzten Vernetzungs- und/ oder Funktionalisierungsmitteln reagieren.

Durch diese Reaktionen können strukturell verschiedene und strukturell komplexe Linker und Substituenten entstehen, die aufgrund ihrer strukturellen Komplexität nicht eindeutig als Strukturformel dargestellt werden können. Beispielsweise können bei der Reaktion des Funktionalisierungsmittels 2-Methyloxiran mit dem Substituenten -$CH_2$-CH(OH)-$CH_3$ die Substituenten -$CH_2$CH[O-$CH_2$-CH(OH)-$CH_3$]-$CH_3$ und -$CH_2$-CH[O-CH($CH_3$)-$CH_2$-OH]-$CH_2$-OH entstehen, die wiederum mit 2-Methyloxiran oder anderen Funktionalisierungsmitteln oder Vernetzungsmitteln zu verschiedenen und strukturell komplexen Substituenten weiter reagieren können.

Für die Zwecke dieser Erfindung umfasst der Ausdruck "Hybridrest" Reste, die auf die ein- oder mehrfache Reaktion von Substituenten oder Linkern mit Vernetzungs- und/ oder Funktionalisierungsmitteln zurückzuführen ist. Die Linker und die in den Cyclodextrin-Monomeren enthaltenen Substituenten können ein- oder mehrfach durch einen oder mehrere Hybridreste substituiert ein.

In einer bevorzugten Ausführungsform ist der Hybridrest abgeleitet von

wenigstens einem Vernetzungsmittel ausgewählt aus der Gruppe aus 1,2-Dihalogenethan (z.B. 1,2-Dichlorethan), 1,3-Dihalogenpropan (z.B. 1,3-Dichlorpropan), Halogencarbonsäurehalogenid, [beispielsweise Halogenessigsäurehalogenid (z.B. Chloressigsäurechlorid) oder Halogenpropionsäurehalogenid (z.B. Chlorpropionsäurechlorid)], Epichlorhydrin, 4-Chlor-1,2-epoxybutan, 1,2,3,4-Diepoxybutan, Tetramethylendiisocyanat und Hexamethylendiisocyanat; wobei Halogen und Halogenid, unabhängig voneinander, vorzugsweise für -Cl, -Br oder -I, besonders bevorzugt für -Cl oder -Br und insbesondere für -Cl stehen; und/ oder

wenigstens einem Funktionalisierungsmittel ausgewählt aus der Gruppe aus $C_{1-6}$-Alkyl-Y, $C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-10}$-Cycloalkyl-C(=O)-OH, Y-$C_{1-6}$-Alkyl-C(=O)-O$C_{1-6}$-Alkyl, Y-$C_{3-10}$-Cycloalkyl-C(=O)-O-$C_{3-10}$-Cycloalkyl, [$C_{1-6}$-Alkyl-C(=O)]$_2$O, [$C_{3-7}$-Cycloalkyl-C(=O)]$_2$O, Dihydrofuran-2,5-dion, Furan-2,5-dion, Dihydro-2H-pyran-2,6(3H)-dion, Oxiran, ($C_{1-6}$-Alkyl)oxiran, ($C_{3-7}$-Cycloalkyl)oxiran, (Di-C1-6-Alkyl)oxiran, (Di-$C_{3-7}$-Cycloalkyl)oxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, ($C_{1-6}$-Alkyl)$_2$NH, ($C_{3-7}$-Cycloalkyl)$_2$NH, ($C_{1-6}$-Alkyl)$_3$N, ($C_{3-7}$-Cycloalkyl)$_3$N, [(Y-$C_{1-6}$-Alkyl)N($C_{1-6}$-Alkyl)$_3^+A^-$, 1,3-Propansulton und 1,4-Butansulton; wobei die $C_{1-6}$-Alkyl-Reste und die $C_{3-7}$-Cycloalkyl-Reste, unabhängig voneinander, unsubstituiert, ein- oder mehrfach mit gleichen oder verschiedenen Resten Z substituiert sein können; wobei die weiteren Substituenten Z zu keiner oder zu keiner wesentlichen Quervernetzung der Cyclodextrin-Monomere führen; Halogenid vorzugsweise für Chlorid, Bromid oder Iodid, besonders bevorzugt für Chlorid oder Bromid und insbesondere für Chlorid steht; und die Reste Y für Abgangsgruppen stehen, die, unabhängig voneinander, vorzugsweise ausgewählt sind aus der Gruppe bestehend aus -Cl, -Br, -I, -Mesyl, -Tosyl und Epoxy; besonders bevorzugt ausgewählt sind aus der Gruppe bestehend aus -Cl, -Br, -Mesyl und -Tosyl; und insbesondere für -Cl stehen; und die Substituenten Z, unabhängig voneinander, vorzugsweise ausgewählt sind aus der Gruppe bestehend aus -OH, =O, -C(=O)OH, -C(=O)O$CH_3$, -C(=O)O$CH_2CH_3$, -$NH_2$, -NH($CH_3$), -N($CH_3$)$_2$, -N($CH_3$)$_3^+A^-$, -NH($CH_2CH_3$), -N($CH_2CH_3$)$_2$ und -N($CH_2CH_3$)$_3^+$ $A^-$; besonders bevorzugt aus der Gruppe bestehend aus -OH, =O, -C(=O)OH, -C(=O)O$CH_3$ und -C(=O)O$CH_2CH_3$; und insbesondere ausgewählt sind aus der Gruppe bestehend aus -OH, =O und -C(=O)OH; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform enthält der Hybridrest wenigstens ein Strukturelement ausgewählt aus der Gruppe bestehend aus $\sim CH_2-CH_2-O\sim$, $\sim CH_2-CH(O\sim)-CH_3$, $\sim CH_2-CH(O\sim)-CH_2-CH_3$, $\sim CH(CH_3)-CH_2-O\sim$, $\sim CH(CH_2CH_3)-CH_2-O\sim$, $\sim CH_2-CH(O\sim)-CH_2-O\sim$, $\sim CH(CH_2-O\sim)-CH_2-O\sim$, $\sim CH_2-CH(O\sim)-C(=O)O\sim$, $\sim CH[C(=O)O\sim]-CH_2-O\sim$, $\sim CH[C(=O)O\sim]-CH_2-O\sim$, $\sim CH[C(=O)O\sim]-CH_2-O\sim$, $\sim CH_2-CH(O\sim)-CH_2-O\sim$, $\sim CH(CH_2O\sim)-CH_2O\sim$, $\sim CH_2-CH(O\sim)-CH_2-CH_2O\sim$, $\sim CH(CH_2O\sim)-CH_2-CH_2O\sim$, $\sim CH_2-CH(O\sim)-CH(O\sim)-CH_2O\sim$, $-CH(CH_2O\sim)-CH(CH_2O\sim)-O\sim$, $-CH_2-CH(O\sim)-CH(CH_2O\sim)-O\sim$, $-CH(O\sim)-CH_2-CH_2-CH_2O\sim$, $-CH(CH_2O\sim)-CH_2-CH_2O\sim$, wobei das Symbol "~" für eine kovalente Einfachbindung steht.

In einer weiteren bevorzugten Ausführungsform enthält der Hybridrest wenigstens ein Strukturelement ausgewählt aus der Gruppe bestehend aus $\sim CH_2-CH_2-O\#$, $\sim CH_2-CH(O\#)-CH_3$, $\sim CH_2-CH(O\#)-CH_2-CH_3$, $\sim CH(CH_3)-CH_2-O\#$, $\sim CH(CH_2CH_3)-CH_2-O\#$, $\sim CH_2-CH(O\#)-CH_2-O\#$, $\sim CH(CH_2-O\#)-CH_2-O\#$, $\sim CH_2-CH(O\#)-C(=O)O\#$, $\sim CH[C(=O)O\#]-CH_2-O\#$, $\sim CH[C(=O)O\#]-CH_2-O\#$, $\sim CH[C(=O)O\#]-CH_2-O\#$, $\sim CH_2-CH(O\#)-CH_2-O\#$, $\sim CH(CH_2O\#)-CH_2O\#$, $\sim CH_2-CH(O\#)-CH_2-CH_2O\#$, $\sim CH(CH_2O\#)-CH_2-CH_2O\#$, $\sim CH_2-CH(O\#)-CH(O\#)-CH_2O\#$, $-CH(CH_2O\#)-CH(CH_2O\#)-O\#$, $-CH_2-CH(O\#)-CH(CH_2O\#)-O\#$, $-CH(O\#)-CH_2-CH_2-CH_2O\#$, $-CH(CH_2O\#)-CH_2-CH_2O\#$; wobei das Symbol "~" für eine kovalente Einfachbindung zu einem N-, O- oder S-Atom steht und das Symbol "#" für eine kovalente Bindung zu einem H- oder C-Atom steht.

In einer weiteren bevorzugten Ausführungsform enthält der Hybridrest wenigstens ein Strukturelement ausgewählt aus der Gruppe bestehend aus $\S O-CH_2-CH_2-O\#$, $\S O-CH_2-CH(O\#)-CH_3$, $\S O-CH_2-CH(O\#)-CH_2-CH_3$, $\S O-CH(CH_3)-CH_2-O\#$, $\S O-CH(CH_2CH_3)-CH_2-O\#$, $\S O-CH_2-CH(O\#)-CH_2-O\#$, $\S O-CH(CH_2-O\#)-CH_2-O\#$, $\S O-CH_2-CH(O\#)-C(=O)O\#$, $\S O-CH[C(=O)O\#]-CH_2-O\#$, $\S O-CH[C(=O)O\#]-CH_2-O\#$, $\S O-CH[C(=O)O\#]-CH_2-O\#$, $\S O-CH_2-CH(O\#)-CH_2-O\#$, $\S O-CH(CH_2O\#)-CH_2O\#$, $\S O-CH_2-CH(O\#)-CH_2-CH_2O\#$, $\S O-CH(CH_2O\#)-CH_2-CH_2O\#$, $\S O-CH_2-CH(O\#)-CH(O\#)-CH_2O\#$, $\S O-CH(CH_2O\#)-CH(CH_2O\#)-O\#$, $\S O-CH_2-CH(O\#)-CH(CH_2O\#)-O\#$, $\S O-CH(O\#)-CH_2-CH_2-CH_2O\#$, $\S O-CH(CH_2O\#)-CH_2-CH_2O\#$; wobei das Symbol "#" für -H oder $-CH_2\S$ steht und das Symbol "§" für eine kovalente Einfachbindung zu einem C-Atom steht.

In einer weiteren bevorzugten Ausführungsform enthält der Hybridrest wenigstens ein Strukturelement ausgewählt aus der Gruppe bestehend aus $\S O-CH_2-CH_2-OH$, $\S O-CH_2-CH(OH)-CH_3$, $\S O-CH_2-CH(OH)-CH_2-CH_3$, $\S O-CH(CH_3)-CH_2-OH$, $\S O-CH(CH_2-CH_3)-CH_2-OH$, $\S O-CH_2-CH(OH)-CH_2-OH$, $\S O-CH(CH_2-OH)-CH_2-OH$, $\S O-CH_2-CH(OH)-C(=O)OH$, $\S O-CH_2-CH(OH)-C(=O)O-CH_3$, $\S O-CH_2-CH(OH)-C(=O)O-CH_2-CH_3$, $\S O-CH[C(=O)OH]-CH_2-OH$, $\S O-CH[C(=O)O-CH_3]-CH_2-OH$, $\S O-CH[C(=O)O-CH_2-CH_3]-CH_2-OH$, $\S O-CH_2-CH(OH)-CH_2-OH$, $\S O-CH(CH_2OH)-CH_2OH$, $\S O-CH_2-CH(OH)-CH_2-CH_2OH$, $\S O-CH(CH_2OH)-CH_2-CH_2OH$, $\S O-CH_2-CH(OH)-CH(OH)-CH_2OH$, $\S O-CH(CH_2OH)-CH(CH_2OH)-OH$, $\S O-CH_2-CH(OH)-CH(CH_2OH)-OH$, $\S O-CH(OH)-CH_2-CH_2-CH_2OH$ und $\S O-CH(CH_2-OH)-CH_2-CH_2OH$; wobei das Symbol "§" für eine kovalente Einfachbindung zu einem C-Atom steht.

In einer besonders bevorzugten Ausführungsform ist das erfindungsgemäße Copolymer ein Copolymer aus der Gruppe

(Glucosyl)-Cyclodextrin-Copolymer; (Galactosyl)-Cyclodextrin-Copolymer; (Maltosyl)-Cyclodextrin-Copolymer; (Maltriosyl)-Cyclodextrin-Copolymer; (Acetyl)-Cyclodextrin-Copolymer; (Propionyl)-Cyclodextrin-Copolymer; (Isobutyryl)-Cyclodextrin-Copolymer; (Butyryl)-Cyclodextrin-Copolymer; (Pivaloyl)-Cyclodextrin-Copolymer; (Pentanoyl)-Cyclodextrin-Copolymer; (Hexanoyl)-Cyclodextrin-Copolymer; (Cyclopropancarbonyl)-Cyclodextrin-Copolymer; (Benzoyl)-Cyclodextrin-Copolymer; (2-Phenylacetyl)-Cyclodextrin-Copolymer; (2-Carboxyacetyl)-Cyclodextrin-Copolymer; (3-Carboxypropanoyl)-Cyclodextrin-Copolymer; (4-Carboxybutanoyl)-Cyclodextrin-Copolymer; (2-Methoxy-2-oxoacetyl)-Cyclodextrin-Copolymer; (3-Methoxy-3-oxopropanoyl)-Cyclodextrin-Copolymer; (4-Methoxy-4-oxobutanoyl)-Cyclodextrin-Copolymer; (2-Hydroxyacetyl)-Cyclodextrin-Copolymer; (2-Hydroxypropanoyl)-Cyclodextrin-Copolymer; (3-Hydroxypropanoyl)-Cyclodextrin-Copolymer; (2-Hydroxybutanoyl)-Cyclodextrin-Copolymer; (3-Hydroxybutanoyl)-Cyclodextrin-Copolymer; (4-Hydroxybutanoyl)-Cyclodextrin-Copolymer; (2-Hydroxypentanoyl)-Cyclodextrin-Copolymer; (3-Hydroxypentanoyl)-Cyclodextrin-Copolymer; (4-Hydroxypentanoyl)-Cyclodextrin-Copolymer; (5-Hydroxypentanoyl)-Cyclodextrin-Copolymer; (2,3-Dihydroxypropanoyl)-Cyclodextrin-Copolymer; (2,3-Dihydroxybutanoyl)-Cyclodextrin-Copolymer; (2,4-Dihydroxybutanoyl)-Cyclodextrin-Copolymer; (3,4-Dihydroxybutanoyl)-Cyclodextrin-Copolymer; (2,3-Dihydroxypentanoyl)-Cyclodextrin-Copolymer; (2,4-Dihydroxypentanoyl)-Cyclodextrin-Copolymer; (2,5-Dihydroxypentanoyl)-Cyclodextrin-Copolymer; (3,4-Dihydroxypentanoyl)-Cyclodextrin-Copolymer; (3,5-Dihydroxypentanoyl)-Cyclodextrin-Copolymer; (4,5-Dihydroxypentanoyl)-Cyclodextrin-Copolymer; (2,3,4-Trihydroxybutanoyl)-Cyclodextrin-Copolymer; (2,3,4-Trihydroxypentanoyl)-Cyclodextrin-Copolymer; (2,3,5-Trihydroxypentanoyl)-Cyclodextrin-Copolymer; (2,4,5-Trihydroxypentanoyl)-Cyclodextrin-Copolymer; (3,4,5-Trihydroxypentanoyl)-Cyclodextrin-Copolymer; (2,3,4,5-Tetrahydroxypentanoyl)-Cyclodextrin-Copolymer; (2-Aminoacetyl) -Cyclodextrin-Copolymer; (2-Aminopropanoyl)-Cyclodextrin-Copolymer; (2-Aminobutanoyl)-Cyclodextrin-Copolymer; [2-(1*H*-Imidazol-1-yl)acetyl]-Cyclodextrin-Copolymer; [3-(1*H*-Imidazol-1-yl)propanoyl]-Cyclodextrin-Copolymer; [4-(1*H*-Imidazol-1-yl)butanoyl]-Cyclodextrin-Copolymer; [2-(1*H*-Imidazol-1-yl)acetyl]-Cyclodextrin-Copolymer; [3-(1*H*-Imidazol-1-yl)propanoyl]-Cyclodextrin-Copolymer;

[4-(1*H*-Imidazol-1-yl)butanoyl]-Cyclodextrin-Copolymer; [2-(Pyridin-3-yl)acetyl]-Cyclodextrin-Copolymer; [3-(Pyridin-3-yl)propanoyl]-Cyclodextrin-Copolymer; [4-(Pyridin-3-yl)butanoyl]-Cyclodextrin-Copolymer; (Methyl)-Cyclodextrin-Copolymer; (Ethyl)-Cyclodextrin-Copolymer; (*iso*-Propyl)-Cyclodextrin-Copolymer; (n-Propyl)-Cyclodextrin-Copolymer; (*iso*-Butyl)-Cyclodextrin-Copolymer; (n-Butyl)-Cyclodextrin-Copolymer; (sec-Butyl)-Cyclodextrin-Copolymer; (*tert*-Butyl)-Cyclodextrin-Copolymer, (Cyclopropyl)-Cyclodextrin-Copolymer; (Phenyl)-Cyclodextrin-Copolymer; (Benzyl)-Cyclodextrin-Copolymer; (Phenethyl)-Cyclodextrin-Copolymer; (Carboxymethyl)-Cyclodextrin-Copolymer; (1-Carboxyethyl)-Cyclodextrin-Copolymer; (2-Carboxyethyl)-Cyclodextrin-Copolymer; (1-Carboxypropyl)-Cyclodextrin-Copolymer; (2-Carboxypropyl)-Cyclodextrin-Copolymer; (3-Carboxypropyl)-Cyclodextrin-Copolymer; (1-Carboxypropan-2-yl)-Cyclodextrin-Copolymer; (4-Carboxybutyl)-Cyclodextrin-Copolymer; (5-Carboxypentyl)-Cyclodextrin-Copolymer; (5-Carboxypentyl)-Cyclodextrin-Copolymer; (2-Methoxy-2-oxoethyl)-Cyclodextrin-Copolymer; (3-Methoxy-2-oxopropyl)-Cyclodextrin-Copolymer; (4-Methoxy-4-oxobutyl)-Cyclodextrin-Copolymer; (5-Methoxy-5-oxopentyl)-Cyclodextrin-Copolymer; (6-Methoxy-6-oxohexyl)-Cyclodextrin-Copolymer; (2-Ethoxy-2-oxoethyl)-Cyclodextrin-Copolymer; (3-Ethoxy-2-oxopropyl)-Cyclodextrin-Copolymer; (4-Ethoxy-4-oxobutyl)-Cyclodextrin-Copolymer; (5-Ethoxy-5-oxopentyl)-Cyclodextrin-Copolymer; (6-Ethoxy-6-oxohexyl)-Cyclodextrin-Copolymer; (3-Carboxy-2-hydroxypropyl)-Cyclodextrin-Copolymer; (2-Hydroxy-4-methoxy-4-oxobutyl)-Cyclodextrin-Copolymer; (4-Ethoxy-2-hydroxy-4-oxobutyl)-Cyclodextrin-Copolymer; (1-Carboxy-2-hydroxyethyl)-Cyclodextrin-Copolymer; (3-Hydroxy-1-methoxy-1-oxopropan-2-yl)-Cyclodextrin-Copolymer; (1-Ethoxy-3-hydroxy-1-oxopropan-2-yl)-Cyclodextrin-Copolymer; (1-Carboxy-3-hydroxypropan-2-yl)-Cyclodextrin-Copolymer; (1-Hydroxy-4-methoxy-4-oxobutan-2-yl)-Cyclodextrin-Copolymer; (4-Ethoxy-1-hydroxy-4-oxobutan-2-yl)-Cyclodextrin-Copolymer; (Hydroxymethyl)-Cyclodextrin-Copolymer; (1-Hydroxyethyl)-Cyclodextrin-Copolymer; (2-Hydroxyethyl)-Cyclodextrin-Copolymer; (1-Hydroxypropyl)-Cyclodextrin-Copolymer; (2-Hydroxypropyl)-Cyclodextrin-Copolymer; (1-Hydroxypropan-2-yl)-Cyclodextrin-Copolymer; (2-Hydroxypropan-2-yl)-Cyclodextrin-Copolymer; (1-Hydroxybutyl)-Cyclodextrin-Copolymer; (2-Hydroxybutyl)-Cyclodextrin-Copolymer; (3-Hydroxybutyl)-Cyclodextrin-Copolymer; (4-Hydroxybutyl)-Cyclodextrin-Copolymer; (1-Hydroxypentyl)-Cyclodextrin-Copolymer; (2-Hydroxypentyl)-Cyclodextrin-Copolymer; (3-Hydroxypentyl)-Cyclodextrin-Copolymer; (4-Hydroxypentyl)-Cyclodextrin-Copolymer; (5-Hydroxypentyl)-Cyclodextrin-Copolymer; (1,2-Dihydroxyethyl)-Cyclodextrin-Copolymer; (1,2-Dihydroxypropyl)-Cyclodextrin-Copolymer; (1,3-Dihydroxypropyl)-Cyclodextrin-Copolymer; (2,3-Dihydroxypropyl)-Cyclodextrin-Copolymer; (1,2-Dihydroxybutyl)-Cyclodextrin-Copolymer; (1,3-Dihydroxybutyl)-Cyclodextrin-Copolymer; (1,4-Dihydroxybutyl)-Cyclodextrin-Copolymer; (2,3-Dihydroxybutyl)-Cyclodextrin-Copolymer; (2,4-Dihydroxybutyl)-Cyclodextrin-Copolymer; (3,4-Dihydroxybutyl)-Cyclodextrin-Copolymer; (1,2-Dihydroxypentyl)-Cyclodextrin-Copolymer; (1,3-Dihydroxypentyl)-Cyclodextrin-Copolymer; (1,4-Dihydroxypentyl)-Cyclodextrin-Copolymer; (1,5-Dihydroxypentyl)-Cyclodextrin-Copolymer; (2,3-Dihydroxypentyl)-Cyclodextrin-Copolymer; (2,4-Dihydroxypentyl)-Cyclodextrin-Copolymer; (2,5-Dihydroxypentyl)-Cyclodextrin-Copolymer; (3,4-Dihydroxypentyl)-Cyclodextrin-Copolymer; (3,5-Dihydroxypentyl)-Cyclodextrin-Copolymer; (4,5-Dihydroxypentyl)-Cyclodextrin-Copolymer; (1,2,3-Trihydroxypropyl)-Cyclodextrin-Copolymer; (1,2,3-Trihydroxybutyl)-Cyclodextrin-Copolymer; (1,2,4-Trihydroxybutyl)-Cyclodextrin-Copolymer; (1,3,4-Trihydroxybutyl)-Cyclodextrin-Copolymer; (2,3,4-Trihydroxybutyl)-Cyclodextrin-Copolymer; (1,2,3-Trihydroxypentyl)-Cyclodextrin-Copolymer; (1,2,4-Trihydroxypentyl)-Cyclodextrin-Copolymer; (1,2,5-Trihydroxypentyl)-Cyclodextrin-Copolymer; (1,3,4-Trihydroxypentyl)-Cyclodextrin-Copolymer; (1,3,5-Trihydroxypentyl)-Cyclodextrin-Copolymer; (1,4,5-Trihydroxypentyl)-Cyclodextrin-Copolymer; (2,3,4-Trihydroxypentyl)-Cyclodextrin-Copolymer; (2,3,5-Trihydroxypentyl)-Cyclodextrin-Copolymer, (2,4,5-Trihydroxypentyl)-Cyclodextrin-Copolymer; (3,4,5-Trihydroxypentyl)-Cyclodextrin-Copolymer; (1,2,3,4-Tetrahydroxybutyl)-Cyclodextrin-Copolymer; (1,2,3,4-Tetrahydroxypentyl)-Cyclodextrin-Copolymer; (1,2,4,5-Tetrahydroxypentyl)-Cyclodextrin-Copolymer; (2,3,4,5-Tetrahydroxypentyl)-Cyclodextrin-Copolymer; (1,2,3,4,5-Pentahydroxypentyl)-Cyclodextrin-Copolymer; [(Sulfooxy)methyl]-Cyclodextrin-Copolymer; [2-(Sulfooxy)ethyl]-Cyclodextrin-Copolymer; [3-(Sulfooxy)propyl]-Cyclodextrin-Copolymer; [4-(Sulfooxy)butyl]-Cyclodextrin-Copolymer; [(Phosphonooxy)methyl]-Cyclodextrin-Copolymer; [2-(Phosphonooxy)-ethyl]-Cyclodextrin-Copolymer; [3-(Phosphonooxy)propyl]-Cyclodextrin-Copolymer; [4-(Phosphonooxy)butyl]-Cyclodextrin-Copolymer; [4-(Phosphonooxy)butyl]-Cyclodextrin-Copolymer; (Sulfomethyl)-Cyclodextrin-Copolymer; (2-Sulfoethyl)-Cyclodextrin-Copolymer; (3-Sulfopropyl)-Cyclodextrin-Copolymer; (4-Sulfobutyl)-Cyclodextrin-Copolymer; (5-Sulfopentyl)-Cyclodextrin-Copolymer; (6-Sulfohexyl)-Cyclodextrin-Copolymer; (Phosphonomethyl)-Cyclodextrin-Copolymer; (2-Phosphonoethyl)-Cyclodextrin-Copolymer; (3-Phosphonopropyl)-Cyclodextrin-Copolymer; (4-Phosphonobutyl)-Cyclodextrin-Copolymer; (5-Phosphonopentyl)-Cyclodextrin-Copolymer; (6-Phosphonohexyl)-Cyclodextrin-Copolymer; (2-Phosphonovinyl)-Cyclodextrin-Copolymer; (3-Phosphonoallyl)-Cyclodextrin-Copolymer; (4-Phosphonobut-3-enyl)-Cyclodextrin-Copolymer; (5-Phosphonopent-4-enyl)-Cyclodextrin-Copolymer; (6-Phosphonohex-5-enyl)-Cyclodextrin-Copolymer; (Aminomethyl)-Cyclodextrin-Copolymer; (2-Aminoethyl)-Cyclodextrin-Copolymer; (3-Aminopropyl)-Cyclodextrin-Copolymer; (4-Aminobutyl)-Cyclodextrin-Copolymer; (5-Aminopentyl)-Cyclodextrin-Copolymer; (6-Aminohexyl)-Cyclodextrin-Copolymer; [(*N*,*N*-Dimethylamino)methyl]-Cyclodextrin-Copolymer; [2-(*N*,*N*-Dimethylamino)ethyl]-Cyclodextrin-Copolymer; [3-(*N*,*N*-Dimethylamino)propyl]-Cyclodextrin-Copolymer; [4-(*N*,*N*-Dimethylamino)butyl]-Cyclodextrin-Copolymer; [5-(*N*,*N*-Dimethylamino)pentyl]-Cyclodextrin-Copolymer; [6-(*N*,*N*-Dimethylamino) hexyl]-Cyclodextrin-Copolymer; [(*N*,*N*-Diethylamino)methyl]-Cyclodextrin-Copolymer; [2-(*N*,*N*-Diethylamino)ethyl]-Cyclodextrin-Copolymer; [3-(*N*,*N*-Diethylamino)propyl]-Cyclodextrin-Copolymer; [4-(*N,N*-Diethylamino)butyl]-Cyclodextrin-Copolymer; [5-(*N,N*-Diethylamino)pentyl]-Cyclodextrin-Copolymer; [6-(*N,N*-Diethylamino)hexyl]-Cyclodextrin-Copolymer; [(Trimethylammonio)methyl]-Cyclodextrin-Copolymer Chlorid; [2-(Trimethylammonio)ethyl]-Cyclodextrin-Copolymer Chlorid; [3-(Trimethylammonio)propyl]-Cyclodextrin-Copolymer Chlorid; [4-(Trimethylammonio)butyl]-Cyclodextrin-Copolymer Chlorid; [5-(Trimethylammonio)pentyl]-Cyclodextrin-Copolymer Chlorid; [6-(Trimethylammonio)hexyl]-Cyclodextrin-Copolymer Chlorid; [(Triethylammonio)methyl]-Cyclodextrin-Copolymer Chlorid; [2-(Triethylammonio)ethyl]-Cyclodextrin-Copolymer Chlorid; [3-(Triethylammonio)propyl]-Cyclodextrin-Copolymer Chlorid; [4-(Triethylammonio)-butyl]-Cyclodextrin-Copolymer Chlorid; [5-(Triethylammonio)pentyl]-Cyclodextrin-Copolymer Chlorid; [6-(Triethylammonio)hexyl]-Cyclodextrin-Copolymer Chlorid; [(1*H*-Imidazol-1-yl)methyl]-Cyclodextrin-Copolymer; [2-(1*H*-Imidazol-1-yl)ethyl]-Cyclodextrin-Copolymer; [3-(1*H*-Imidazol-1-yl)propyl]-Cyclodextrin-Copolymer; [4-(1*H*-Imidazol-1-yl)butyl]-Cyclodextrin-Copolymer; (Pyridin-3-ylmethyl)-Cyclodextrin-Copolymer; [2-(Pyridin-3-yl)ethyl]-Cyclodextrin-Copolymer; [3-(Pyridin-3-yl)propyl]-Cyclodextrin-Copolymer; [4-(Pyridin-3-yl)butyl]-Cyclodextrin-Copolymer; [(*β*-D-Glucopyranosyloxyuronsäure) methyl]-Cyclodextrin-Copolymer; [2-(*β*-D-Glucopyranosyloxyuronsäure)ethyl]-Cyclodextrin-Copolymer; [3-(*β*-D-Glucopyranosyloxyuronsäure)propyl]-Cyclodextrin-Copolymer; [4-(*β*-D-Glucopyranosyloxyuronsäure)butyl]-Cyclodextrin-Copolymer; [5-(*β*-D-Glucopyranosyloxyuronsäure)pentyl]-Cyclodextrin-Copolymer und [6-(*β*-D-Glucopyranosyloxyuronsäure)hexyl]-Cyclodextrin-Copolymer; wobei der Ausdruck "Cyclodextrin-Copolymer" für α-, β-, γ-, α/β-, α/γ-, β/γ- oder α/β/γ-Cyclodextrin-Copolymer, vorzugsweise für α-, β- oder γ-Cyclodextrin-Copolymer, bevorzugter für β-Cyclodextrin-Copolymer steht; und insbesondere für α-Cyclodextrin-Epichlorhydrin-Copolymer, β-Cyclodextrin-Epichlorhydrin-Copolymer, γ-Cyclodextrin-Epichlorhydrin-Copolymer, α-Cyclodextrin-4-Chlor-1,2-epoxybutan-Copolymer, β-Cyclodextrin-4-Chlor-1,2-epoxybutan-Copolymer, γ-Cyclodextrin-4-Chlor-1,2-epoxybutan-Copolymer, α-Cyclodextrin-1,2,3,4-Diepoxybutan-Copolymer, β-Cyclodextrin-1,2,3,4-Diepoxybutan-Copolymer, γ-Cyclodextrin-1,2,3,4-Diepoxybutan-Copolymer, α-Cyclodextrin-Tetramethylendiisocyanat-Copolymer, β-Cyclodextrin-Tetramethylendiisocyanat-Copolymer, γ-Cyclodextrin-Tetramethylendiisocyanat-Copolymer, α-Cyclodextrin-Hexamethylendiisocyanat-Copolymer, β-Cyclodextrin-Hexamethylendiisocyanat-Copolymer oder γ-Cyclodextrin-Hexamethylendiisocyanat-Copolymer steht.

In einer besonders bevorzugten Ausführungsform ist das erfindungsgemäße Copolymer ein Copolymer aus der Gruppe

(Acetyl)-α-Cyclodextrin-Epichlorhydrin-Copolymer; (2-Carboxyacetyl)-α-Cyclodextrin-Epichlorhydrin-Copolymer; (3-Carboxypropanoyl)-α-Cyclodextrin-Epichlorhydrin-Copolymer; (2-Hydroxyacetyl)-α-Cyclodextrin-Epichlorhydrin-Copolymer; (Methyl)-α-Cyclodextrin-Epichlorhydrin-Copolymer; (Ethyl)-α-Cyclodextrin-Epichlorhydrin-Copolymer; (Carboxymethyl)-α-Cyclodextrin-Epichlorhydrin-Copolymer; (1-Carboxyethyl)-α-Cyclodextrin-Epichlorhydrin-Copolymer; (2-Carboxyethyl)-α-Cyclodextrin-Epichlorhydrin-Copolymer; (Hydroxymethyl)-α-Cyclodextrin-Epichlorhydrin-Copolymer; (1-Hydroxyethyl)-α-Cyclodextrin-Epichlorhydrin-Copolymer; (2-Hydroxyethyl)-α-Cyclodextrin-Epichlorhydrin-Copolymer; (1-Hydroxypropyl)-α-Cyclodextrin-Epichlorhydrin-Copolymer; (2-Hydroxypropyl)-α-Cyclodextrin-Epichlorhydrin-Copolymer; (1-Hydroxypropan-2-yl)-α-Cyclodextrin-Epichlorhydrin-Copolymer; (2-Hydroxypropan-2-yl)-α-Cyclodextrin-Epichlorhydrin-Copolymer; [3-(Sulfooxy)propyl]-α-Cyclodextrin-Epichlorhydrin-Copolymer; [4-(Sulfooxy)butyl]-α-Cyclodextrin-Epichlorhydrin-Copolymer; (Acetyl)-β-Cyclodextrin-Epichlorhydrin-Copolymer; (2-Carboxyacetyl)-β-Cyclodextrin-Epichlorhydrin-Copolymer; (3-Carboxypropanoyl)-β-Cyclodextrin-Epichlorhydrin-Copolymer; (2-Hydroxyacetyl)-β-Cyclodextrin-Epichlorhydrin-Copolymer; (Methyl)-β-Cyclodextrin-Epichlorhydrin-Copolymer; (Ethyl)-β-Cyclodextrin-Epichlorhydrin-Copolymer; (Carboxymethyl)-β-Cyclodextrin-Epichlorhydrin-Copolymer; (1-Carboxyethyl)-β-Cyclodextrin-Epichlorhydrin-Copolymer; (2-Carboxyethyl)-β-Cyclodextrin-Epichlorhydrin-Copolymer; (Hydroxymethyl)-β-Cyclodextrin-Epichlorhydrin-Copolymer; (1-Hydroxyethyl)-β-Cyclodextrin-Epichlorhydrin-Copolymer; (2-Hydroxyethyl)-β-Cyclodextrin-Epichlorhydrin-Copolymer; (1-Hydroxypropyl)-β-Cyclodextrin-Epichlorhydrin-Copolymer; (2-Hydroxypropyl)-β-Cyclodextrin-Epichlorhydrin-Copolymer; (1-Hydroxypropan-2-yl)-β-Cyclodextrin-Epichlorhydrin-Copolymer; (2-Hydroxypropan-2-yl)-β-Cyclodextrin-Epichlorhydrin-Copolymer; [3-(Sulfooxy)propyl]-β-Cyclodextrin-Epichlorhydrin-Copolymer; [4-(Sulfooxy)butyl]-β-Cyclodextrin-Epichlorhydrin-Copolymer; (Acetyl)-γ-Cyclodextrin-Epichlorhydrin-Copolymer; (2-Carboxyacetyl)-γ-Cyclodextrin-Epichlorhydrin-Copolymer; (3-Carboxypropanoyl)-γ-Cyclodextrin-Epichlorhydrin-Copolymer; (2-Hydroxyacetyl)-γ-Cyclodextrin-Epichlorhydrin-Copolymer; (Methyl)-γ-Cyclodextrin-Epichlorhydrin-Copolymer; (Ethyl)-γ-Cyclodextrin-Epichlorhydrin-Copolymer; (Carboxymethyl)-γ-Cyclodextrin-Epichlorhydrin-Copolymer; (1-Carboxyethyl)-γ-Cyclodextrin-Epichlorhydrin-Copolymer; (2-Carboxyethyl)-γ-Cyclodextrin-Epichlorhydrin-Copolymer; (Hydroxymethyl)-γ-Cyclodextrin-Epichlorhydrin-Copolymer; (1-Hydroxyethyl)-γ-Cyclodextrin-Epichlorhydrin-Copolymer; (2-Hydroxyethyl)-γ-Cyclodextrin-Epichlorhydrin-Copolymer, (1-Hydroxypropyl)-γ-Cyclodextrin-Epichlorhydrin-Copolymer; (2-Hydroxypropyl)-γ-Cyclodextrin-Epichlorhydrin-Copolymer; (1-Hydroxypropan-2-yl)-γ-Cyclodextrin-Epichlorhydrin-Copolymer; (2-Hydroxypropan-2-yl)-γ-Cyclodextrin-Epichlorhydrin-Copolymer; [3-(Sulfooxy)propyl]-γ-Cyclodextrin-Epichlorhydrin-Copolymer und [4-(Sulfooxy)butyl]-γ-Cyclodextrin-Epichlorhydrin-Copolymer.

Ein weiterer bevorzugter Gegenstand dieser Erfindung betrifft Copolymere erhältlich durch ein Verfahren umfassend den Verfahrensschritt

Mischen wenigstens einer Cyclodextrin-Verbindung mit

(a) wenigstens einem Vernetzungsmittel oder
(b) wenigstens einem Vernetzungsmittel und wenigstens einem Funktionalisierungsmittel;

zur Verwendung in der Behandlung von Krankheiten des Urogenitalsystems.

In einer bevorzugten Ausführungsform sind die erfindungsgemäßen Copolymere erhältlich durch ein Verfahren umfassend den Verfahrensschritt

Mischen wenigstens einer α-, β- oder γ-Cyclodextrin-Verbindung mit

(a) wenigstens einem Vernetzungsmittel oder
(b) wenigstens einem Vernetzungsmittel und wenigstens einem Funktionalisierungsmittel.

In einer weiteren bevorzugten Ausführungsform sind die erfindungsgemäßen Copolymere erhältlich durch ein Verfahren umfassend den Verfahrensschritt

Mischen eines unsubstituierten oder ein- oder mehrfach substituierten α-, β- oder γ-Cyclodextrins mit

(a) einem Vernetzungsmittel oder
(b) einem Vernetzungsmittel und einem Funktionalisierungsmittel.

Vorzugsweise erfolgt der Verfahrensschritt "Mischen" in Gegenwart eines Lösungsmittels oder Lösungsmittelgemisches.

Vorzugsweise ist die Cyclodextrin-Verbindung eine Verbindung der allgemeinen Struktur I

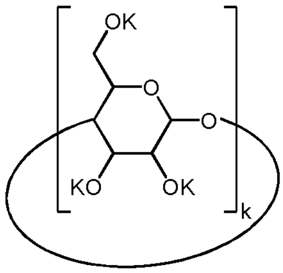

I,

worin

- k für 6, 7 oder 8 steht;
- jeder einzelne Rest K entweder

(a) für Wasserstoff steht; oder
(b) für Glycosyl, -C(=O)$R^6$ oder $R^7$ steht; wobei

$R^6$ und $R^7$, unabhängig voneinander, für $C_{1-6}$-Alkyl, $C_{3-7}$-Cycloalkyl, Phenyl oder 5- bis 7-gliedriges Heteroaryl stehen.

Im Sinne dieser Erfindung definiert die Variable "k" die Anzahl der Glucosyleinheiten im Ringsystem der Cyclodextrin-Verbindungen: für α-Cyclodextrin-Verbindungen ist k = 6; für β-Cyclodextrin-Verbindungen ist k = 7; und für γ-Cyclodextrin-Verbindungen ist k = 8.

Die Variable "k" steht vorzugsweise für 6 oder 7 und insbesondere für 7.

In einer bevorzugten Ausführungsform ist der Gesamtsubstitutionsgrad der Cyclodextrin-Verbindungen $0 < TDS_b < k \times 3$, wobei $TDS_b$ für den Gesamtsubstitutionsgrad der Verbindung der allgemeinen Struktur I in Bezug auf die Substituenten (b), d.h. Glycosyl, Glycosyl, -C(=O)$R^6$ und $R^7$ steht.

Im Falle, dass die Cyclodextrin-Verbindungen ein- oder mehrfach mit Resten substituiert sind, die nukleophile Gruppen tragen (z.B. -OH, -SH oder primäres, sekundäres oder tertiäres Amino), kann der Gesamtsubstitutionsgrad sowohl $0 < TDS_b < k \times 3$ als auch $TDS_b \geq k \times 3$ sein, da die nukleophilen Gruppen selbst substituiert sein können.

In Bezug auf die Verbindungen der allgemeinen Struktur I ist der Gesamtsubstitutionsgrad ($TDS_b$) in Bezug

auf die Substituenten (b) für a = 6 vorzugsweise $0 < TDS_b < 1$, $0 < TDS_b < 2$, $0 < TDS_b < 3$ oder $0 < TDS_b < 4$; bevorzugter $0 < TDS_b < 5$, $0 < TDS_b < 6$, $0 < TDS_b < 7$ oder $0 < TDS_b < 8$; noch bevorzugter $0 < TDS_b < 9$, $0 < TDS_b < 10$, $0 < TDS_b < 11$ oder $0 < TDS_b < 12$; am Bevorzugtesten $0 < TDS_b < 13$, $0 < TDS_b < 14$, $0 < TDS_b < 15$ oder $0 < TDS_b < 16$; und insbesondere $0 < TDS_b < 17$ oder $0 < TDS_b < 18$.

In Bezug auf die Verbindungen der allgemeinen Struktur I ist der Gesamtsubstitutionsgrad ($TDS_b$) in Bezug auf die Substituenten (b) für a = 7 vorzugsweise $0 < TDS_b < 1$, $0 < TDS_b < 2$, $0 < TDS_b < 3$, $0 < TDS_b < 4$ oder $0 < TDS_b < 5$; bevorzugter $0 < TDS_b < 6$, $0 < TDS_b < 7$, $0 < TDS_b < 8$, $0 < TDS_b < 9$ oder $0 < TDS_b < 10$; noch bevorzugter $0 < TDS_b < 11$, $0 < TDS_b < 12$, $0 < TDS_b < 13$, $0 < TDS_b < 14$ oder $0 < TDS_b < 15$; am Bevorzugtesten $0 < TDS_b < 16$, $0 < TDS_b < 17$, $0 < TDS_b < 18$ oder $0 < TDS_b < 19$; und insbesondere $0 < TDS_b < 20$ oder $0 < TDS_b < 21$.

In Bezug auf die Verbindungen der allgemeinen Struktur I ist der Gesamtsubstitutionsgrad ($TDS_b$) in Bezug auf die Substituenten (b) für a = 8 vorzugsweise $0 < TDS_b < 1$, $0 < TDS_b < 2$, $0 < TDS_b < 3$, $0 < TDS_b < 4$, $0 < TDS_b < 5$ oder $0 < TDS_b < 6$; bevorzugter $0 < TDS_b < 7$, $0 < TDS_b < 8$, $0 < TDS_b < 9$, $0 < TDS_b < 10$, $0 < TDS_b < 11$ oder $0 < TDS_b < 12$; noch bevorzugter $0 < TDS_b < 13$, $0 < TDS_b < 14$, $0 < TDS_b < 15$, $0 < TDS_b < 16$, $0 < TDS_b < 17$ oder $0 < TDS_b < 18$; am Bevorzugtesten $0 < TDS_b < 19$, $0 < TDS_b < 20$, $0 < TDS_b < 21$ oder $0 < TDS_b < 22$; und insbesondere $0 < TDS_b < 23$ oder $0 < TDS_b < 24$.

Unter dem Ausdruck "Gesamtsubstitutionsgrad" (total degree of substitution, TDS) versteht man im Sinne dieser Erfindung die durchschnittliche Anzahl an gleichen oder verschiedenen Substituenten, die in einer substituierten Cyclodextrin-Verbindung enthalten sind, wobei Wasserstoff nicht als Substituent gilt (vgl. J. Blanchard, S. Proniuk; Some Important Considerations in the Use of Cyclodextrins; Pharm. Res. 1999, 16(12), 1796-1798).

Beispielsweise kann der TDS in methyliertem β-Cyclodextrin Werte von > 0 bis ≤ 21 annehmen; wobei ein TDS von 21 bedeutet, dass alle Hydroxygruppen des β-Cylodextrins methyliert sind. Ein TDS von > 0 bis < 1 bedeutet, dass eine bestimmte Anzahl an β-Cyclodextrin-Molekülen keinen Methyl-Substituenten trägt und ein TDS von beispielsweise 8,3 bedeutet, dass jedes der β-Cyclodextrin-Moleküle im Durchschnitt 8,3 Methyl-Substituenten enthält. Im Fall von Cyclodextrinen mit nukleophilen Resten in den Substituenten (z.B. die nukleophile OH-Gruppe im Substituenten Hydroxypropyl der Verbindung Hydroxypropyl-β-Cyclodextrin) kann der TDS auch > 21 sein, da die nukleophilen Gruppen der Hydroxypropyl-Substituenten selbst substituiert sein können. Somit können sich in diesen Fällen TDS-Werte ergeben, die größer sind als die Anzahl der maximal möglichen Substitutionsstellen am Cyclodextrin-Ring.

In einer bevorzugten Ausführungsform ist das Glycosyl ein Monosaccharid, das ausgewählt ist aus der Gruppe bestehend aus Mannitol, Isomalt, Lactit, Sorbitol, Glucitol, Xylitol, Threit, Erythrit, Arabit, Glyceraldeyhd, Erythrose, Threose, Ribose, Arabinose, Xylose, Lyxose, Allose, Altrose, Glucose, Mannose, Gulose, Idose, Galactose, Talose, Dihydroxyaceton, Erythrulose, Ribulose, Xylulose, Psicose, Fructose, Sorbose, Tagatose, Glucosamin, Fucosamin, N-Acetylmannosamin, Hamamelose, Apiose, Gluconsäure, Galacturonsäure, Erythrarsäure, Ascorbinsäure, Glucuronsäure, Abequose, Apiose, Cladinose, Desoxyribose, Desoxyglucose, Digitalose, Digitoxose, Fucose, Fucosamin, Galactosamin, Glucosamin, Glucosaminitol, Glycerin, Glyceron, Mannosamin, Mannozuckersäure, Neuraminsäure, Rhamnose, Schleimsäure, Sedoheptulose, Streptose, Trehalosamin, Trehalose, Weinsäure und Glucarsäure. Besonders vorteilhaft ist Glucose.

Das Glycosyl kann auch ein Disaccharid, Trisaccharid, Oligosaccharid oder Polysaccharid sein, die vorzugsweise gleiche oder verschiedene Monomere der oben stehenden Monosaccharide enthalten.

In einer bevorzugten Ausführungsform ist Glycosyl ausgewählt aus der Gruppe bestehend aus Mannitol, Isomalt, Lactit, Sorbitol, Glucitol, Xylitol, Threit, Erythrit, Arabit, Glyceraldeyhd, Erythrose, Threose, Ribose, Arabinose, Xylose, Lyxose, Allose, Altrose, Glucose, Mannose, Gulose, Idose, Galactose, Talose, Dihydroxyaceton, Erythrulose, Ribulose, Xylulose, Psicose, Fructose, Sorbose, Tagatose, Glucosamin, Fucosamin, N-Acetylmannosamin, Hamamelose, Apiose, Gluconsäure, Galacturonsäure, Erythrarsäure, Ascorbinsäure, Glucuronsäure, Abequose, Apiose, Cladinose, Desoxyribose, Desoxyglucose, Digitalose, Digitoxose, Fucose, Fucosamin, Galactosamin, Glucosamin, Glucosaminitol, Glycerin, Glyceron, Mannosamin, Mannozuckersäure, Neuraminsäure, Rhamnose, Schleimsäure, Sedoheptulose, Streptose, Trehalosamin, Trehalose, Weinsäure, Glucarsäure, Maltose und Maltriose. Es ist besonders bevorzugt, dass Glycosyl ausgewählt ist aus der Gruppe bestehend aus Glucose, Galactose, Maltose und Maltriose.

In Bezug auf die Reste $R^6$ und $R^7$ ist $C_{1-6}$-Alkyl vorzugsweise ausgewählt aus der Gruppe bestehend aus Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl, Prop-1-enyl, iso-Butenyl, n-Butenyl, cis-2-Butenyl, trans-2-Butenyl, 1,2-Butadienyl, 1,3-Butadienyl, Pent-1-enyl, cis-Pent-2-enyl, trans-Pent-2-enyl, 2-Methyl-but-1-enyl, 2-Methyl-but-2-enyl, 3-Methyl-but-1-enyl, Hex-1-enyl, Hex-2-enyl und Hex-3-enyl. Besonders bevorzugt ist, dass $C_{1-6}$-Alkyl ausgewählt ist aus der Gruppe bestehend aus Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl und Prop-1-enyl. Die genannten $C_{1-6}$-Alkyl-Reste können unsubstituiert oder ein- oder mehrfach (z.B. zwei-, drei- vier- oder fünffach) mit Resten $R^8$ substituiert sein.

In Bezug auf die Reste $R^6$ und $R^7$ ist $C_{3-7}$-Cycloalkyl vorzugsweise ausgewählt aus der Gruppe bestehend aus Cyclopropyl, Cyclobutyl, Cyclopentyl, Cyclohexyl, Cycloheptyl, Cyclopropenyl, Cyclobutenyl, Cyclopentenyl, Cyclopentadienyl, Cyclohexenyl, Cyclohexadienyl, Cycloheptenyl, Cycloheptadienyl, Cycloheptatrienyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, Tetrahydropyranyl, Piperazinyl, Dioxanyl, Morpholinyl, Chinuclidinyl, Pyrazolinyl, Pyrazolinonyl, Pyranyl, Indolinyl, Chinolizinyl, Chromanyl, Isochromanyl, Chromenyl, Isochromenyl und Benzodioxolanyl. Es ist besonders bevorzugt, dass $C_{3-7}$-Cycloalkyl ausgewählt ist aus der Gruppe bestehend aus Cyclopropyl, Cyclobutyl, Cyclopentyl, Cyclohexyl, Cycloheptyl, Cyclopropenyl, Cyclobutenyl, Cyclopentenyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, Tetrahydropyranyl, Piperazinyl, Dioxanyl, Morpholinyl, Chinuclidinyl, Pyrazolinyl, Pyrazolinonyl und Pyranyl. Es ist weiterhin besonders bevorzugt, dass $C_{3-7}$-Cycloalkyl ausgewählt ist aus der Gruppe bestehend aus Cyclopropyl, Cyclobutyl, Cyclopentyl, Cyclohexyl, Tetrahydrofuranyl, Piperidinyl, Piperazinyl, Morpholinyl und Chinuclidinyl. Es ist insbesondere bevorzugt, dass $C_{3-7}$-Cycloalkyl ausgewählt ist aus der Gruppe bestehend aus Cyclopropyl, Cyclobutyl, Cyclopentyl, Cyclohexyl, Piperidinyl, Piperazinyl und Morpholinyl. Die genannten $C_{3-7}$-Cycloalkyl-Reste können unsubstituiert oder ein- oder mehrfach (z.B. zwei-, drei- vier- oder fünffach) mit Resten $R^8$ substituiert sein. Der $C_{3-7}$-Cycloalkyl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Verbindungen gebunden sein.

In Bezug auf die Reste $R^6$ und $R^7$ kann der Phenyl-Rest unsubstituiert oder ein- oder mehrfach (z.B. zwei-, drei- vier- oder fünffach) mit Resten $R^8$ substituiert sein. Der Phenyl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere gebunden sein.

In Bezug auf die Reste $R^6$ und $R^7$ ist 5- bis 7-gliedriges Heteroaryl vorzugsweise ausgewählt aus der Gruppe bestehend aus Pyrrolyl, Pyrazolyl, Imidazolyl, Thienyl, Furyl, Oxazolyl, Isoxazolyl, Thiazolyl, Isothiazolyl, Furazanyl, Oxadiazolyl, Pyridyl, Pyridazinyl, Pyrazinyl, Pyrimidinyl, Indolyl, Isoindolyl, Indolizinyl, Indazolyl, Benzotriazolyl, Chinolinyl, Isochinolinyl, Naphthyridinyl, Chinazolinyl, Phthalazinyl, Chinoxalinyl, Purinyl, Pteridinyl, Benzofuryl, Isobenzofuryl, Benzothienyl, Benzothiazolyl und Benzothiadiazolyl. In einer bevorzugten Ausführungsform ist 5- bis 7-gliedriges Heteroaryl ausgewählt aus der Gruppe bestehend aus Pyrrolyl, Pyrazolyl, Imidazolyl, Thienyl, Furyl, Oxazolyl, Isoxazolyl, Thiazolyl, Isothiazolyl, Pyridyl, Indolyl, Chinolinyl, Purinyl, Pteridinyl und Benzofuryl. In einer besonders bevorzugten Ausführungsform ist 5- bis 7-gliedriges Heteroaryl ausgewählt aus der Gruppe bestehend aus Pyrrolyl, Pyrazolyl, Imidazolyl, Thienyl, Furyl, Oxazolyl, Isoxazolyl, Thiazolyl, Isothiazolyl und Pyridyl. Die genannten 5-7-gliedrigen Heteroaryl-Reste können unsubstituiert oder ein- oder mehrfach (z.B. zwei-, drei- vier- oder fünffach) mit Resten $R^8$ substituiert sein. Der 5- bis 7-gliedrige Heteroaryl-Rest kann auch über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Verbindungen gebunden sein.

In einer bevorzugten Ausführungsform steht jeder einzelne Rest K entweder für Wasserstoff oder für einen Rest ausgewählt aus der Gruppe bestehend aus Glucose, Galactose, Maltose, Maltriose, $-C(=O)-C_{1-6}$-Alkyl, $-C(=O)-C_{3-7}$-Cycloalkyl, -C(=O)-Phenyl, -C(=O)-(5- bis 7-gliedriges Heteroaryl), $-C_{1-6}$-Alkyl, $-C_{3-7}$-Cycloalkyl, Phenyl und 5- bis 7-gliedriges Heteroaryl; wobei $C_{1-6}$-Alkyl, $C_{3-7}$-Cycloalkyl, Phenyl und 5- bis 7-gliedriges Heteroaryl unsubstituiert oder ein- oder mehrfach (z.B. zwei-, drei- vier- oder fünffach) mit gleichen oder verschiedenen Resten $R^8$ substituiert sein können; und $C_{3-7}$-Cycloalkyl, Phenyl und 5- bis 7-gliedriges Heteroaryl-Rest über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Verbindungen gebunden sein können.

In einer weiteren bevorzugten Ausführungsform steht jeder einzelne Rest K entweder für Wasserstoff oder für einen Rest ausgewählt aus der Gruppe bestehend aus Glucose, Galactose, Maltose, Maltriose, -C(=O)-Methyl, -C(=O)-Ethyl, -C(=O)-iso-Propyl, -C(=O)-n-Propyl, -C(=O)-iso-Butyl, -C(=O)-n-Butyl, -C(=O)-sec-Butyl, -C(=O)-tert-Butyl, -C(=O)-n-Pentyl, -C(=O)-iso-Pentyl, -C(=O)-neo-Pentyl, -C(=O)-iso-Hexyl, -C(=O)-n-Hexyl, neo-Hexyl, -C(=O)-3-Methylpentyl, -C(=O)-2,3-Dimethylbutyl, -C(=O)-Ethenyl, -C(=O)-Ethinyl, -C(=O)-Prop-1-enyl, $-C(=O)-C_{3-7}$-Cycloalkyl, -C(=O)-Cyclopropyl, -C(=O)-Cyclobutyl, -C(=O)-Cyclopentyl, -C(=O)-Cyclohexyl, -C(=O)-Piperidinyl, -C(=O)-Piperazinyl, -C(=O)-Morpholinyl, -C(=O)-Phenyl, -C(=O)-Imidazolyl, -C(=O)-Thienyl, -C(=O)-Furyl, -C(=O)-Oxazolyl, -C(=O)-Isoxazolyl, -C(=O)-Thiazolyl, -C(=O)-Isothiazolyl, -C(=O)-Pyridyl, Methyl, Ethyl, iso-Propyl, n-Propyl, iso-Butyl, n-Butyl, sec-Butyl, tert-Butyl, n-Pentyl, iso-Pentyl, neo-Pentyl, isoHexyl, n-Hexyl, neo-Hexyl, 3-Methylpentyl, 2,3-Dimethylbutyl, Ethenyl, Ethinyl, Prop-1-enyl, Cyclopropyl, Cyclobutyl, Cyclopentyl, Cyclohexyl, Piperidinyl, Piperazinyl, Morpholinyl, Phenyl, Pyrrolyl, Pyrazolyl, Imidazolyl, Thienyl, Furyl, Oxazolyl, Isoxazolyl, Thiazolyl, Isothiazolyl und Pyridyl; wobei die Reste unsubstituiert oder ein- oder mehrfach (z.B. zwei-, drei- vier- oder fünffach) mit Resten $R^8$ substituiert sein können; und die $C_{3-7}$-Cycloalkyl-, Phenyl- und 5- bis 7-gliedrigen Heteroaryl-Reste über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Verbindungen gebunden sein können.

$R^8$ ist vorzugsweise ausgewählt aus der Gruppe bestehend aus -C(=O)H, -C(=O)W, -C(=O)OH, -C(=O)OW, $-C(=O)NH_2$, -C(=O)NHW, $-C(=O)NW_2$, -C(=O)NHC(=O)W, -C(=O)NWC(=O)W, -OH, -OW, -OV, -OC(=O)H, -OC(=O)W, -OC(=O)OH, -OC(=O)OW, $-OC(=O)NH_2$, -OC(=O)NHW, $-OC(=O)NW_2$, -O-N=O, -OS(=O)H, -OS(=O)W, -OS(=O)OH, -OS(=O)OW, $-OS(=O)_2H$, $-OS(=O)_2W$, $-OS(=O)_2OH$, $-OS(=O)_2OW$, $-OS(=O)NH_2$, -OS(=O)NHW, $-OS(=O)NW_2$, $-OS(=O)_2NH_{23}$ $-OS(=O)_2NHW$, $-OS(=O)_2NW_2$, $-OP(=O)(W)_2$, -OP(=O)(OH)(W), -OP(=O)(OW)(W), $-OP(=O)(OH)_2$,

-OP(=O)(OW)(OH), -OP(=O)(OW)$_2$, -O-P(=NH)(W)$_2$, -OP(=NW)(W)$_2$, -OP(=O)(NH$_2$)$_2$, -OP(=O)(NW)(NH$_2$), -OP(=S)(W)$_2$, -OP(=S)(OH)(W), -OP(=S)(OW)(W), -OP(=S)(OH)$_2$, -OP(=S)(OW)(OH), -OP(=S)(OW)$_2$, -SH, -SW, -SV, -S(=O)H, -S(=O)W, -S(=O)OH, -S(=O)OW, -S(=O)$_2$H, -S(=O)$_2$W, -S(=O)$_2$OH, -S(=O)$_2$OW, -S(=O)NH$_2$, -S(=O)NHW, -S(=O)NW$_2$, -S(=O)$_2$NH$_2$, -S(=O)$_2$NHW, -S(=O)$_2$NW$_2$, -NH$_2$, -NHW, -NHV, -NW$_2$, -NV$_2$, -N(W)$_3$$^+$A$^-$, -NHC(=O)H, -NHC(=O)W, -N[C(=O)W]$_2$, -NWC(=O)W, -NHC(=O)NH$_2$, -NHC(=O)NHW, -NWC(=O)NHW, -NWC(=O)NW$_2$, -NWC(=O)NW$_2$, -NHC(=O)OH, -NHC(=O)OW, -NWC(=O)OW, -NHS(=O)H, -NHS(=O)W, -NWS(=O)H, -NWS(=O)W, -NHS(=O)OH, -NHS(=O)OW, -NWS(=O)OH, -NWS(=O)OW, -NHS(=O)$_2$H, -NHS(=O)$_2$W, -NWS(=O)$_2$H, -NWS(=O)$_2$W, -NHS(=O)$_2$OH, -NHS(=O)$_2$OW, -NWS(=O)$_2$OH, -NWS(=O)$_2$OW, -NHS(=O)NH$_2$, -NWS(=O)NHW, -NHS(=O)NW$_2$, -NWS(=O)NH$_2$, -NWS(=O)NW$_2$, -NHS(=O)$_2$NH$_2$, -NHS(=O)$_2$NHW, -NHS(=O)$_2$NW$_2$, -NWS(=O)$_2$NH$_2$, -NWS(=O)$_2$NHW, -NWS(=O)$_2$NW$_2$, =N-OH, =N-NH$_2$, -N=N-W, -PH$_2$, -PHW, -PW$_2$, -P(=O)W$_2$, -P(=O)(OH)(W), -P(=O)(OW)(W), -P(=O)(OH)$_2$, -P(=O)(OW)(OH), -P(=O)(OW)$_2$, -P(=NH)(W)$_2$, -P(=NW)(W)$_2$, -P(=O)(NH$_2$)$_2$, -P(=O)(NW$_2$)$_2$, -P(=S)(W)$_2$, -P(=S)(OH)(W), -P(=S)(OW)W, -P(=S)(OH)$_2$, -P(=S)(OW)(OH), -P(=S)(OW)$_2$, =NH, =NW, =O, =S, Pyrrolidinyl, Pyrazolinyl, Pyrazolidinyl, Imidazolinyl, Imidazolidinyl, Dioxolanyl, Piperidinyl, Piperazinyl, 4-W-Piperazinyl, Dioxanyl, Morpholinyl, Chinuclidinyl, Pyrazolinonyl, Indolinyl, Isoindolinyl, Chinolizinyl, Benzodioxolanyl, Pyrrolyl, Pyrazolyl, Imidazolyl, Thienyl, Oxazolyl, Isoxazolyl, Thiazolyl, Isothiazolyl, Furazanyl, Oxadiazolyl, Pyridyl, Pyridazinyl, Pyrazinyl, Pyrimidinyl, Indolyl, Isoindolyl, Indolizinyl, Indazolyl, Benzotriazolyl, Chinolinyl, Isochinolinyl, Naphthyridinyl, Chinazolinyl, Phthalazinyl, Chinoxalinyl, Purinyl, Pteridinyl, Benzothienyl, Benzothiazolyl und Benzothiadiazolyl; wobei der Rest W vorzugsweise für Methyl, Ethyl, n-Propyl, iso-Propyl, iso-Butyl, n-Butyl, sec-Butyl, tert-Butyl, Cyclopropyl, Cyclobutyl, Cyclopentyl, Cyclohexyl, Allyl, Vinyl oder Ethinyl steht; der Rest V für einen Hybridrest steht; und A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform ist R$^8$ ausgewählt aus der Gruppe bestehend aus -C(=O)H, -C(=O)W, -C(=O)OH, -C(=O)OW, -C(=O)NH$_2$, -C(=O)NHW, -C(=O)NW$_2$, -C(=O)NHC(=O)W, -C(=O)NWC(=O)W, -OH, -OW, -OV, -OC(=O)H, -OC(=O)W, -OC(=O)OH, -OC(=O)OW, -OC(=O)NH$_2$, -OC(=O)NHW, -OC(=O)NW$_2$, -OS(=O)H, -OS(=O)W, -OS(=O)OH, -OS(=O)OW, -OS(=O)$_2$H, -OS(=O)$_2$W, -OS(=O)$_2$OH, -OS(=O)$_2$OW, -OS(=O)NH$_2$, -OS(=O)NHW, -OS(=O)NW$_2$, -OS(=O)$_2$NH$_2$, -OS(=O)$_2$NHW, -OS(=O)$_2$NW$_2$, -OP(=O)(OH)(W), -OP(=O)(OH)$_2$, -OP(=O)(OW)(OH), -OP(=O)(OW)$_2$, -SH, -SV, -S(=O)H, -S(=O)W, -S(=O)OH, -S(=O)OW, -S(=O)$_2$H, -S(=O)$_2$W, -S(=O)$_2$OH, -S(=O)$_2$OW, -S(=O)NH$_2$, -S(=O)NHW, -S(=O)NW$_2$, -S(=O)$_2$NH$_2$, -S(=O)$_2$NHW, -S(=O)$_2$NW$_2$, -NH$_2$, -NHW, -NHV, -NW$_2$, -NV$_2$, -N(W)$_3$$^+$A$^-$, -NHC(=O)H, -NHC(=O)W, -N[C(=O)W]$_2$, -NWC(=O)W, -NHC(=O)NH$_2$, -NHC(=O)NHW, -NWC(=O)NW$_2$, -NWC(=O)NHW, -NWC(=O)NW$_2$, -NHC(=O)OH, -NHC(=O)OW, -NWC(=O)OW, -NHS(=O)H, -NWS(=O)H, -NHS(=O)OH, -NWS(=O)OH, -NHS(=O)$_2$OH, -NWS(=O)$_2$OH, =N-OH, =N-NH$_2$, -PH$_2$, -P(=O)W$_2$, -P(=O)(OH)(W), -P(=O)(OH)$_2$, -P(=O)(OW)(OH), -P(=O)(OW)$_2$, =NH, =NW, =O, =S, Pyrrolidinyl, Pyrazolinyl, Pyrazolidinyl, Imidazolinyl, Imidazolidinyl, Piperidinyl, Piperazinyl, 4-W-Piperazinyl, Morpholinyl, Chinuclidinyl, Pyrazolinonyl, Imidazolyl; wobei der Rest W vorzugsweise für Methyl, Ethyl, n-Propyl, iso-Propyl, Cyclopropyl, Allyl, Vinyl oder Ethinyl steht; der Rest V für einen Hybridrest steht; und A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform steht R$^8$ für einen ionischen, ionisierbaren, Hydroxy- oder Polyol-Rest. In diesem Zusammenhang ist R$^8$ vorzugsweise ausgewählt aus der Gruppe bestehend aus -C(=O)OH, -OH, -OV, -OC(=O)OH, -OC(=O)NH$_2$, -OS(=O)OH, -OS(=O)$_2$OH, -OP(=O)(OH)(W), -OP(=O)(OH)$_2$, -OP(=O)(OW)(OH), -OP(=O)(OW)$_2$, -SH, -SV, -S(=O)OH, -S(=O)$_2$OH, -NH$_2$, -NHW, -NHV, -NW$_2$, -NV$_2$, -N(W)$_3$$^+$A$^-$, -NHC(=O)OH, -NWC(=O)OH, -NHS(=O)OH, -NWS(=O)OH, -NHS(=O)$_2$OH, -NWS(=O)$_2$OH, =N-OH, =N-NH$_2$, -P(=O)(OH)(W), -P(=O)(OH)$_2$, -P(=O)(OW)(OH), -P(=O)(OW)$_2$, =NH, =NW, Piperidinyl, Morpholinyl und Imidazolyl; wobei der Rest W ausgewählt ist aus der Gruppe bestehend aus Methyl, Ethyl, n-Propyl, iso-Propyl, Cyclopropyl, Allyl, Vinyl und Ethinyl; der Rest V für einen Hybridrest steht; und A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform steht R$^8$ für einen ionischen, ionisierbaren, Hydroxy- oder Polyol-Rest. In diesem Zusammenhang ist R$^8$ vorzugsweise ausgewählt aus der Gruppe bestehend aus -C(=O)OH, -OH, -OV, -OS(=O)$_2$OH, -OP(=O)(OH)$_2$, -SH, -SV, -S(=O)$_2$OH, -NH$_2$, -NHW, -NHV, -NW$_2$, -NV$_2$, -N(W)$_3$$^+$A$^-$, -NHC(=O)OH, -NWC(=O)OH, -P(=O)(OH)(W), -P(=O)(OH)$_2$, -P(=O)(OW)(OH); wobei der Rest W ausgewählt ist aus der Gruppe bestehend aus Methyl, Ethyl, n-Propyl, iso-Propyl, Cyclopropyl, Allyl, Vinyl und Ethinyl; der Rest V für einen Hybridrest steht; und A$^-$ für ein anorganisches oder organisches Anion steht, das vorzugsweise ausgewählt ist aus der Gruppe bestehend aus Cl$^-$, Br$^-$, I$^-$, Dihydrogenphosphat, Hydrogenphosphat, Phosphat, Hydrogencarbonat, Carbonat, Acetat, Lactat und Citrat oder für andere physiologisch verträgliche Anionen steht.

Für die Zwecke dieser Erfindung versteht man unter einen "ionischen Rest" einen Substituenten, der eine permanente kationische oder anionische, vorzugsweise kationische Ladung trägt (z.B. quaternäres Ammonium; --

$N(CH_3)_3^+$, wobei das Symbol "--" für eine kovalente Einfachbindung zu einem C-Atom steht).

Im Sinne dieser Erfindung umfasst der Ausdruck "ionisierbarer Rest" Substituenten, die durch Abgabe eines Protons eine anionische Ladung (z.B. Carboxyl → Carboxylat) oder durch Aufnahme eines Protons eine kationische Ladung (z.B. primäres, sekundäres oder tertiäres Amin → primäres, sekundäres oder tertiäres Ammonium) enthalten.

Für die Zwecke dieser Erfindung umfasst der Ausdruck "Polyol" $C_{1-6}$-AlkylSubstituenten, die wenigstens zwei Hydroxy-Reste (z.B. zwei, drei, vier oder fünf) an dem gleichen oder an verschiedenen Kohlenstoffatomen des Alkyls enthalten (z.B. $-CH_2-CH(OH)-CH_2-CH_2-OH$).

In einer weiteren bevorzugten Ausführungsform sind die Reste $R^6$ und $R^7$, unabhängig voneinander ausgewählt aus der Gruppe bestehend aus Methyl, Ethyl, iso-Propyl, n-Propyl, iso-Butyl, n-Butyl, sec-Butyl, tert-Butyl, Ethenyl, Ethinyl, Prop-1-enyl, Cyclopropyl, Cyclobutyl, Cyclopentyl, Cyclohexyl, Phenyl, Benzyl, Phenethyl, $-CH_2-C(=O)-OH$, $-(CH_2)_2-C(=O)-OH$, $-(CH_2)_3-C(=O)-OH$, $-(CH_2)_4-C(=O)-OH$, $-(CH_2)_5-C(=O)-OH$, $-(CH_2)_6-C(=O)-OH$, $-CH_2-CH(OH)-C(=O)OH$, $-CH_2-CH(OH)-C(=O)O-CH_3$, $-CH_2-CH(OH)-C(=O)O-CH_2-CH_3$, $-CH(CH_2OH)-C(=O)OH$, $-CH(CH_2OH)-C(=O)O-CH_3$, $-CH(CH_2-OH)-C(=O)O-CH_2-CH_3$, $-CH_2-CH(OH)-CH_2-C(=O)-OH$, $-CH_2-CH(OH)-CH_2-C(=O)-O-CH_3$, $-CH_2-CH(OH)-CH_2-C(=O)-OCH_2-CH_3$, $-CH(CH_2-OH)-CH_2-C(=O)-O-CH_3$, $-CH(CH_2-OH)-CH_2-C(=O)-O-CH_2-CH_3$, $-CH_2-OH$, $-CH(OH)-CH_3$, $-(CH_2)_2-OH$, - $CH(OH)-CH_2-CH_3$, $-CH_2-CH(OH)-CH_3$, $-(CH_2)_3-OH$, $-CH(CH_3)-CH_2-OH$, $-CH(OH)-(CH_2)_2-CH_3$, $-CH_2-CH(OH)-CH_2-CH_3$, $-(CH_2)_2-CH(OH)-CH_3$, $-(CH_2)_4-OH$, $-(CH_2)_5-OH$, $-(CH_2)_6-OH$, $-CH(OH)-CH_2(OH)$, $-CH(OH)-CH(OH)-CH_3$, $-CH(OH)-CH_2-CH_2(OH)$, $-CH_2-CH(OH)-CH_2(OH)$, $-CH(OH)-CH(OH)-CH_2-CH_3$, $-CH(OH)-CH_2CH(OH)-CH_3$, $-CH(OH)-(CH_2)_2-CH_2(OH)$, $-CH_2-CH(OH)-CH(OH)-CH_3$, $-CH_2-CH(OH)-CH_2-CH_2(OH)$, $-(CH_2)_2-CH(OH)-CH_2(OH)$, $-CH(OH)-CH(OH)-CH_2(OH)$, $-CH(OH)-CH(OH)-CH(OH)-CH_3$, $-CH(OH)-CH(OH)-CH_2-CH_2(OH)$, $-CH(OH)-CH_2-CH(OH)-CH_2(OH)$, $-CH_2-CH(OH)-CH(OH)-CH_2(OH)$, $-CH(OH)-CH(OH)-CH(OH)-CH_2(OH)$, $-CH_2-O-S(=O)_2OH$, $-(CH_2)_2-O-S(=O)_2OH$, $-(CH_2)_3-O-S(=O)_2OH$, $-(CH_2)_4-O-S(=O)_2OH$, $-(CH_2)_5-O-S(=O)_2OH$, $-(CH_2)_6-O-S(=O)_2OH$, $-CH_2-O-P(=O)(OH)_2$, $-(CH_2)_2-O-P(=O)(OH)_2$, - $(CH_2)_3-O-P(=O)(OH)_2$, $-(CH_2)_4-O-P(=O)(OH)_2$, $-(CH_2)_5-O-P(=O)(OH)_2$, $-(CH_2)_6-O-P(=O)(OH)_2$, $-CH_2-SH$, $-CH(SH)-CH_3$, $-(CH_2)_2-SH$, $-CH(SH)-CH_2-CH_3$, $-CH_2-CH(SH)-CH_3$, $-(CH_2)_3-SH$, $-CH(SH)-(CH_2)_2-CH_3$, $-CH_2-CH(SH)-CH_2-CH_3$, $-(CH_2)_2-CH(SH)-CH_3$, $-(CH_2)_4-SH$, $-(CH_2)_5-SH$, $-(CH_2)_6-SH$, $-CH(SH)-CH_2(SH)$, $-CH(SH)-CH(SH)-CH_3$, $-CH(SH)-CH_2-CH_2(SH)$, $-CH_2-CH(SH)-CH_2(SH)$, $-CH(SH)-CH(SH)-CH_2-CH_3$, $-CH(SH)-CH_2CH(SH)-CH_3$, $-CH(SH)-(CH_2)_2-CH_2(SH)$, $-CH_2-CH(SH)-CH(SH)-CH_3$, $-CH_2-CH(SH)-CH_2-CH_2(SH)$, $-(CH_2)_2-CH(SH)-CH_2(SH)$, $-CH(SH)-CH(SH)-CH_2(SH)$, $-CH(SH)-CH(SH)-C(SH)-CH_3$, $-CH(SH)-CH(SH)-CH_2-CH_2(SH)$, $-CH(SH)-CH_2-CH(SH)-CH_2(SH)$, $-CH_2-CH(SH)-CH(SH)-CH_2(SH)$, $-CH(SH)-CH(SH)-CH(SH)-CH_2(SH)$, $-CH_2-S(=O)_2OH$, $-(CH_2)_2-S(=O)_2OH$, $-(CH_2)_3-S(=O)_2OH$, $-(CH_2)_4-S(=O)_2OH$, $-(CH_2)_5-S(=O)_2OH$, $-(CH_2)_6-S(=O)_2OH$, $-CH_2-NH_2$, $-(CH_2)_2-NH_2$, $-(CH_2)_3-NH_2$, $-(CH_2)_4-NH_2$, $-(CH_2)_5-NH_2$, $-(CH_2)_6-NH_2$, $-CH_2-NH(CH_3)$, $-(CH_2)_2-NH(CH_3)$, $-(CH_2)_3-NH(CH_3)$, $-(CH_2)_4-NH(CH_3)$, $-(CH_2)_5-NH(CH_3)$, $-(CH_2)_6-NH(CH_3)$, $-CH_2-NH(CH_2CH_3)$, $-(CH_2)_2-NH(CH_2CH_3)$, $-(CH_2)_3-NH(CH_2CH_3)$, $-(CH_2)_4-NH(CH_2CH_3)$, $-(CH_2)_5-NH(CH_2CH_3)$, $-(CH_2)_6-NH(CH_2CH_3)$, $-CH_2-N(CH_3)_2$, $-(CH_2)_2-N(CH_3)_2$, $-(CH_2)_3-N(CH_3)_2$, $-(CH_2)_4-N(CH_3)_2$, $-(CH_2)_5-N(CH_3)_2$, $-(CH_2)_6-N(CH_3)_2$, $-CH_2-N(CH_2CH_3)_2$, $-(CH_2)_2-N(CH_2CH_3)_2$, $-(CH_2)_3-N(CH_2CH_3)_2$, $-(CH_2)_4-N(CH_2CH_3)_2$, $-(CH_2)_5-N(CH_2CH_3)_2$, $-(CH_2)_6-N(CH_2CH_3)_2$, $-CH_2-N(CH_3)_3^+A^-$, $-(CH_2)_2-N(CH_3)_3^+A^-$, $-(CH_2)_3-N(CH_3)_3^+A^-$, $-(CH_2)_4-N(CH_3)_3^+A^-$, $-(CH_2)_5-N(CH_3)_3^+A^-$, $-(CH_2)_6-N(CH_3)_3^+A^-$, $-CH_2-N(CH_2CH_3)_3^+A^-$, $-(CH_2)_2-N(CH_2CH_3)_3^+A^-$, $-(CH_2)_3-N(CH_2CH_3)_3^+A^-$, $-(CH_2)_4-N(CH_2CH_3)_3^+A^-$, $-(CH_2)_5-N(CH_2CH_3)_3^+A^-$, $-(CH_2)_6-N(CH_2CH_3)_3^+A^-$, $-CH_2-P(=O)(OH)_2$, $-(CH_2)_2-P(=O)(OH)_2$, $-(CH_2)_3-P(=O)(OH)_2$, $-(CH_2)_4-P(=O)(OH)_2$, $-(CH_2)_5-P(=O)(OH)_2$, $-(CH_2)_6-P(=O)(OH)_2$, $-CH_2$-Pyrrolyl, $-(CH_2)_2$-Pyrrolyl, $-(CH_2)_3$-Pyrrolyl, $-(CH_2)_4$-Pyrrolyl, $-CH_2$-Pyrazolyl, $-(CH_2)_2$-Pyrazolyl, $-(CH_2)_3$-Pyrazolyl, $-(CH_2)_4$-Pyrazolyl, $-CH_2$-Imidazolyl, $-(CH_2)_2$-Imidazolyl, $-(CH_2)_3$-Imidazolyl, $-(CH_2)_4$-Imidazolyl, $-CH_2$-Oxazolyl, $-(CH_2)_2$-Oxazolyl, $-(CH_2)_3$-Oxazolyl, $-(CH_2)_4$-Oxazolyl, $-CH_2$-Isoxazolyl, $-(CH_2)_2$-Isoxazolyl, $-(CH_2)_3$-Isoxazolyl, $-(CH_2)_4$-Isoxazolyl, $-CH_2$-Isothiazolyl, $-(CH_2)_2$-Isothiazolyl, $-(CH_2)_3$-Isothiazolyl, $-(CH_2)_4$-Isothiazolyl, $-CH_2$-Pyridyl, $-(CH_2)_2$-Pyridyl, $-(CH_2)_3$-Pyridyl und $-(CH_2)_4$-Pyridyl; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht; wobei die Reste ggf. mit Hybridresten weiter substituiert sein können.

In einer weiteren bevorzugten Ausführungsform steht jeder einzelne Rest K entweder für Wasserstoff oder für einen Rest ausgewählt aus der Gruppe bestehend aus Glucose, Galactose, Maltose, Maltriose, $-C(=O)-C_{1-6}$-Alkyl, $-C(=O)-C_{3-7}$-Cycloalkyl, -C(=O)-Phenyl, -C(=O)-(5- bis 7-gliedriges Heteroaryl), $-C_{1-6}$-Alkyl, $-C_{3-7}$-Cycloalkyl, -Phenyl und -(5- bis 7-gliedriges Heteroaryl); wobei die $C_{3-7}$-Cycloalkyl-, Phenyl- und 5- bis 7-gliedrigen Heteroaryl-Reste über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die Cyclodextrin-Monomere gebunden sein können; und $C_{1-6}$-Alkyl, $C_{3-7}$-Cycloalkyl, Phenyl und 5- bis 7-gliedriges Heteroaryl unsubstituiert oder ein- oder mehrfach (z.B. zwei-, drei- vier- oder fünffach) mit gleichen oder verschiedenen Resten $R^8$ substituiert sein können; wobei $R^8$ ausgewählt ist aus der Gruppe bestehend aus -C(=O)OH, -OH, -OV, -OC(=O)OH, $-OC(=O)NH_2$, -OS(=O)OH, $-OS(=O)_2OH$, -OP(=O)(OH)(W), $-OP(=O)(OH)_2$, -OP(=O)(OW)(OH), $-OP(=O)(OW)_2$, -SH, -SV, -S(=O)OH, $-S(=O)_2OH$, $-NH_2$, -NHW, -NHV, $-NW_2$, $-NV_2$, $-N(W)_3^+A^-$, -NHC(=O)OH, -NWC(=O)OH, -NHS(=O)OH, -NWS(=O)OH, $-NHS(=O)_2OH$, $-NWS(=O)_2OH$, =N-OH, $=N-NH_2$, -P(=O)(OH)(W), $-P(=O)(OH)_2$, -P(=O)(OW)(OH),

$-P(=O)(OW)_2$, =NH, =NW, Pyrrolidinyl, Pyrazolinyl, Pyrazolidinyl, Imidazolinyl, Imidazolidinyl, Piperidinyl, Piperazinyl, 4-W-Piperazinyl, Morpholinyl, Chinuclidinyl, Pyrazolinonyl und Imidazolyl; wobei der Rest W ausgewählt ist aus der Gruppe bestehend aus Methyl, Ethyl, n-Propyl, iso-Propyl, Cyclopropyl, Allyl, Vinyl und Ethinyl; der Rest V für einen Hybridrest steht; und $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform steht jeder einzelne Rest K entweder für Wasserstoff oder für einen Rest ausgewählt aus der Gruppe bestehend aus Glucose, Galactose, Maltose, Maltriose, -C(=O)-Methyl, -C(=O)-Ethyl, -C(=O)-iso-Propyl, -C(=O)-n-Propyl, -C(=O)-iso-Butyl, -C(=O)-n-Butyl, -C(=O)-sec-Butyl, -C(=O)-tert-Butyl, -C(=O)-n-Pentyl, -C(=O)-iso-Pentyl, -C(=O)-neo-Pentyl, -C(=O)-iso-Hexyl, -C(=O)-n-Hexyl, -C(=O)-neo-Hexyl, -C(=O)-(3-Methylpentyl), -C(=O)-(2,3-Dimethylbutyl), -C(=O)-Ethenyl, -C(=O)-Ethinyl, -C(=O)-Prop-1-enyl, -C(=O)-Cyclopropyl, -C(=O)-Cyclobutyl, -C(=O)-Cyclopentyl, -C(=O)-Cyclohexyl, -C(=O)-Piperidinyl, -C(=O)-Piperazinyl, -C(=O)-Morpholinyl, -C(=O)-Phenyl, -C(=O)-Imidazolyl, -C(=O)-Thienyl, -C(=O)-Furyl, -C(=O)-Oxazolyl, -C(=O)-Isoxazolyl, -C(=O)-Thiazolyl, -C(=O)-Isothiazolyl, -C(=O)-Pyridyl, Methyl, Ethyl, iso-Propyl, n-Propyl, iso-Butyl, n-Butyl, sec-Butyl, tert-Butyl, n-Pentyl, iso-Pentyl, neo-Pentyl, isoHexyl, n-Hexyl, neo-Hexyl, 3-Methyl-pentyl, 2,3-Dimethylbutyl, Ethenyl, Ethinyl, Prop-1-enyl, Cyclopropyl, Cyclobutyl, Cyclopentyl, Cyclohexyl, Piperidinyl, Piperazinyl, Morpholinyl, Phenyl, Pyrrolyl, Pyrazolyl, Imidazolyl, Thienyl, Furyl, Oxazolyl, Isoxazolyl, Thiazolyl, Isothiazolyl und Pyridyl; wobei die $C_{3-7}$-Cycloalkyl-, Phenyl- und 5- bis 7-gliedrigen Heteroaryl-Reste über eine $C_{1-4}$-Alkylenbrücke, vorzugsweise $-CH_2-$, $-CH_2-CH_2-$, $-CH_2-CH_2-CH_2-$, oder $-CH_2-CH_2-CH_2-CH_2-$, an die entsprechenden Cyclodextrin-Monomere gebunden sein können; und die $C_{1-6}$-Alkyl-, $C_{3-7}$-Cycloalkyl-, Phenyl- und 5- bis 7-gliedrigen Heteroaryl-Reste unsubstituiert oder ein- oder mehrfach (z.B. zwei-, drei- vier- oder fünffach) mit Resten $R^8$ substituiert sein können; wobei $R^8$ vorzugsweise ausgewählt ist aus der Gruppe bestehend aus -C(=O)OH, -OH, -OV, $-OS(=O)_2OH$, $-OP(=O)(OH)_2$, -SH, -SV, $-S(=O)_2OH$, $-NH_2$, -NHW, -NHV, $-NW_2$, $-NV_2$, $-N(W)_3{}^+A^-$, -NHC(=O)OH, -NWC(=O)OH, -P(=O)(OH)(W), $-P(=O)(OH)_2$, -P(=O)(OW)(OH), Piperidinyl, Piperazinyl, Morpholinyl und Imidazolyl; wobei der Rest W ausgewählt ist aus der Gruppe bestehend aus Methyl, Ethyl, n-Propyl, iso-Propyl, Cyclopropyl, Allyl, Vinyl und Ethinyl; der Rest V für einen Hybridrest steht; und $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform steht jeder einzelne Rest K entweder für Wasserstoff oder für einen Rest ausgewählt aus der Gruppe bestehend aus Glucose, Galactose, Maltose, Maltriose, -C(=O)-Methyl, -C(=O)-Ethyl, -C(=O)-iso-Propyl, -C(=O)-n-Propyl, -C(=O)-iso-Butyl, -C(=O)-n-Butyl, -C(=O)-sec-Butyl, -C(=O)-tert-Butyl, -C(=O)-Ethenyl, -C(=O)-Ethinyl, -C(=O)-Prop-1-enyl, -C(=O)-Cyclopropyl, -C(=O)-Cyclobutyl, -C(=O)-Cyclopentyl, -C(=O)-Cyclohexyl, -C(=O)-Phenyl, -C(=O)-Benzyl, -C(=O)-Phenethyl, $-C(=O)-CH_2-C(=O)-OH$, $-C(=O)-(CH_2)_2-C(=O)-OH$, $-C(=O)-(CH_2)_3-C(=O)-OH$, $-C(=O)-(CH_2)_4-C(=O)-OH$, $-C(=O)-(CH_2)_5-C(=O)-OH$, $-C(=O)-(CH_2)_6-C(=O)-OH$, $-C(=O)-CH_2-OH$, $-C(=O)-CH(OH)-CH_3$, $-C(=O)-(CH_2)_2-OH$, $-C(=O)-CH(OH)-CH_2-CH_3$, $-C(=O)-CH_2-CH(OH)-CH_3$, $-C(=O)-(CH_2)_3-OH$, $-C(=O)-CH(OH)-(CH_2)_2-CH_3$, $-C(=O)-CH_2-CH(OH)-CH_2-CH_3$, $-C(=O)-(CH_2)_2-CH(OH)-CH_3$, $-C(=O)-(CH_2)_4-OH$, $-C(=O)-(CH_2)_5-OH$, $-C(=O)-(CH_2)_6-OH$, $-C(=O)-CH(OH)-CH_2(OH)$, $-C(=O)-CH(OH)-CH(OH)-CH_3$, $-C(=O)-CH(OH)-CH_2-CH_2(OH)$, $-C(=O)-CH_2-CH(OH)-CH_2(OH)$, $-C(=O)-CH(OH)-CH(OH)-CH_2-CH_3$, $-C(=O)-CH(OH)-CH_2-CH(OH)-CH_3$, $-C(=O)-CH(OH)-(CH_2)_2-CH_2(OH)$, $-C(=O)-CH_2-CH(OH)-CH(OH)-CH_3$, $-C(=O)-CH_2-CH(OH)-CH_2-CH_2(OH)$, $-C(=O)-(CH_2)_2-CH(OH)-CH_2(OH)$, $-C(=O)-CH(OH)-CH(OH)-CH_2(OH)$, $-C(=O)-CH(OH)-CH(OH)-CH(OH)-CH_3$, $-C(=O)-CH(OH)-CH(OH)-CH_2-CH_2(OH)$, $-C(=O)-CH(OH)-CH_2-CH(OH)-CH_2(OH)$, $-C(=O)-CH_2-CH(OH)-CH(OH)-CH_2(OH)$, $-C(=O)-CH(OH)-CH(OH)-CH(OH)-CH_2(OH)$, $-C(=O)-CH_2-NH_2$, $-C(=O)-(CH_2)_2-NH_2$, $-C(=O)-(CH_2)_3-NH_2$, $-C(=O)-(CH_2)_4-NH_2$, $-C(=O)-(CH_2)_5-NH_2$, $-C(=O)-(CH_2)_6-NH_2$, $-C(=O)-CH_2-NH(CH_3)$, $-C(=O)-(CH_2)_2-NH(CH_3)$, $-C(=O)-(CH_2)_3-NH(CH_3)$, $-C(=O)-(CH_2)_4-NH(CH_3)$, $-C(=O)-(CH_2)_5-NH(CH_3)$, $-C(=O)-(CH_2)_6-NH(CH_3)$, $-C(=O)-CH_2-NH(CH_2CH_3)$, $-C(=O)-(CH_2)_2-NH(CH_2CH_3)$, $-C(=O)-(CH_2)_3-NH(CH_2-CH_3)$, $-C(=O)-(CH_2)_4-NH(CH_2CH_3)$, $-C(=O)-(CH_2)_5-NH(CH_2CH_3)$, $-C(=O)-(CH_2)_6-NH(CH_2CH_3)$, $-C(=O)-CH_2-N(CH_3)_2$, $-C(=O)-(CH_2)_2-N(CH_3)_2$, $-C(=O)-(CH_2)_3-N(CH_3)_2$, $-C(=O)-(CH_2)_4-N(CH_3)_2$, $-C(=O)-(CH_2)_5-N(CH_3)_2$, $-C(=O)-(CH_2)_6-N(CH_3)_2$, $-C(=O)-CH_2-N(CH_2CH_3)_2$, $-C(=O)-(CH_2)_2-N(CH_2-CH_3)_2$, $-C(=O)-(CH_2)_3-N(CH_2CH_3)_2$, $-C(=O)-(CH_2)_4-N(CH_2CH_3)_2$, $-C(=O)-(CH_2)_5-N(CH_2CH_3)_2$, $-C(=O)-(CH_2)_6-N(CH_2CH_3)_2$, Methyl, Ethyl, iso-Propyl, n-Propyl, iso-Butyl, n-Butyl, sec-Butyl, tert-Butyl, Ethenyl, Ethinyl, Prop-1-enyl, Cyclopropyl, Cyclobutyl, Cyclopentyl, Cyclohexyl, Phenyl, Benzyl, Phenethyl, $-CH_2-C(=O)-OH$, $-(CH_2)_2-C(=O)-OH$, $-(CH_2)_3-C(=O)-OH$, $-(CH_2)_4-C(=O)-OH$, $-(CH_2)_5-C(=O)-OH$, $-(CH_2)_6-C(=O)-OH$, $-CH_2-CH(OH)-C(=O)OH$, $-CH_2-CH(OH)-C(=O)O-CH_3$, $-CH_2-CH(OH)-C(=O)O-CH_2-CH_3$, $-CH(CH_2OH)-C(=O)OH$, $-CH(CH_2OH)-C(=O)O-CH_3$, $-CH(CH_2-OH)-C(=O)O-CH_2-CH_3$, $-CH_2-CH(OH)-CH_2-C(=O)-OH$, $-CH_2-CH(OH)-CH_2-C(=O)-O-CH_3$, $-CH_2-CH(OH)-CH_2-C(=O)-OCH_2-CH_3$, $-CH(CH_2-OH)-CH_2-C(=O)-O-CH_3$, $-CH(CH_2-OH)-CH_2-C(=O)-O-CH_2-CH_3$, $-CH_2-OH$, $-CH(OH)-CH_3$, $-(CH_2)_2-OH$, $-CH(OH)-CH_2-CH_3$, $-CH_2-CH(OH)-CH_3$, $-(CH_2)_3-OH$, $-CH(CH_3)-CH_2-OH$, $-CH(OH)-(CH_2)_2-CH_3$, $-CH_2-CH(OH)-CH_2-CH_3$, $-(CH_2)_2-CH(OH)-CH_3$, $-(CH_2)_4-OH$, $-(CH_2)_5-OH$, $-(CH_2)_6-OH$, $-CH(OH)-CH_2(OH)$, $-CH(OH)-CH(OH)-CH_3$, $-CH(OH)-CH_2-CH_2(OH)$,

$-CH_2-CH(OH)-CH_2(OH)$, $-CH(OH)-CH(OH)-CH_2-CH_3$, $-CH(OH)-CH_2-CH(OH)-CH_3$, $-CH(OH)-(CH_2)_2-CH_2(OH)$, $-CH_2-CH(OH)-CH(OH)-CH_3$, $-CH_2-CH(OH)-CH_2-CH_2(OH)$, $-(CH_2)_2-CH(OH)-CH_2(OH)$, $-CH(OH)-CH(OH)-CH_2(OH)$, $-CH(OH)-CH(OH)-CH(OH)-CH_3$, $-CH(OH)-CH(OH)-CH_2-CH_2(OH)$, $-CH(OH)-CH_2-CH(OH)-CH_2(OH)$, $-CH_2-CH(OH)-CH(OH)-CH_2(OH)$, $-CH(OH)-CH(OH)-CH(OH)-CH_2(OH)$, $-CH_2-O-S(=O)_2OH$, $-(CH_2)_2-O-S(=O)_2OH$, $-(CH_2)_3-O-S(=O)_2OH$, $-(CH_2)_4-O-S(=O)_2OH$, $-(CH_2)_5-O-S(=O)_2OH$, $-(CH_2)_6-O-S(=O)_2OH$, $-CH_2-O-P(=O)(OH)_2$, $-(CH_2)_2-O-P(=O)(OH)_2$, $-(CH_2)_3-O-P(=O)(OH)_2$, $-(CH_2)_4-O-P(=O)(OH)_2$, $-(CH_2)_5-O-P(=O)(OH)_2$, $-(CH_2)_6-O-P(=O)(OH)_2$, $-CH_2-SH$, $-CH(SH)-CH_3$, $-(CH_2)_2-SH$, $-CH(SH)-CH_2-CH_3$, $-CH_2-CH(SH)-CH_3$, $-(CH_2)_3-SH$, $-CH(SH)-(CH_2)_2-CH_3$, $-CH_2-CH(SH)-CH_2-CH_3$, $-(CH_2)_2-CH(SH)-CH_3$, $-(CH_2)_4-SH$, $-(CH_2)_5-SH$, $-(CH_2)_6-SH$, $-CH_2-S(=O)_2OH$, $-(CH_2)_2-S(=O)_2OH$, $-(CH_2)_3-S(=O)_2OH$, $-(CH_2)_4-S(=O)_2OH$, $-(CH_2)_5-S(=O)_2OH$, $-(CH_2)_6-S(=O)_2OH$, $-CH_2-NH_2$, $-(CH_2)_2-NH_2$, - $(CH_2)_3-NH_2$, $-(CH_2)_4-NH_2$, $-(CH_2)_5-NH_2$, $-(CH_2)_6-NH_2$, $-CH_2-NH(CH_3)$, $-(CH_2)_2-NH(CH_3)$, $-(CH_2)_3-NH(CH_3)$, $-(CH_2)_4-NH(CH_3)$, $-(CH_2)_5-NH(CH_3)$, $-(CH_2)_6-NH(CH_3)$, $-CH_2-NH(CH_2CH_3)$, $-(CH_2)_2-NH(CH_2CH_3)$, $-(CH_2)_3-NH(CH_2CH_3)$, $-(CH_2)_4-NH(CH_2CH_3)$, $-(CH_2)_5-NH(CH_2CH_3)$, $-(CH_2)_6-NH(CH_2CH_3)$, $-CH_2-N(CH_3)_2$, $-(CH_2)_2-N(CH_3)_2$, $-(CH_2)_3-N(CH_3)_2$, $-(CH_2)_4-N(CH_3)_2$, $-(CH_2)_5-N(CH_3)_2$, $-(CH_2)_6-N(CH_3)_2$, $-CH_2-N(CH_2CH_3)_2$, $-(CH_2)_2-N(CH_2CH_3)_2$, $-(CH_2)_3-N(CH_2CH_3)_2$, $-(CH_2)_4-N(CH_2CH_3)_2$, $-(CH_2)_5-N(CH_2CH_3)_2$, $-(CH_2)_6-N(CH_2CH_3)_2$, $-CH_2-N(CH_3)_3^+A^-$, $-(CH_2)_2-N(CH_3)_3^+A^-$, - $(CH_2)_3-N(CH_3)_3^+A^-$, $-(CH_2)_4-N(CH_3)_3^+A^-$, $-(CH_2)_5-N(CH_3)_3^+A^-$, $-(CH_2)_6-N(CH_3)_3^+A^-$, $-CH_2-N(CH_2CH_3)_3^+A^-$, $-(CH_2)_2-N(CH_2CH_3)_3^+A^-$, $-(CH_2)_3-N(CH_2CH_3)_3^+A^-$, $-(CH_2)_4-N(CH_2CH_3)_3^+A^-$, $-(CH_2)_5-N(CH_2CH_3)_3^+A^-$, $-(CH_2)_6-N(CH_2CH_3)_3^+A^-$, $-CH_2-P(=O)(OH)_2$, $O(CH_2)_2-P(=O)(OH)_2$, $-(CH_2)_3-P(=O)(OH)_2$, $-(CH_2)_4-P(=O)(OH)_2$, $-(CH_2)_5-P(=O)(OH)_2$, $-(CH_2)_6-P(=O)(OH)_2$; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht; wobei die Reste ggf. mit Hybridresten weiter substituiert sein können.

Der Ausdruck "Vernetzungsmittel" steht im Sinne dieser Erfindung für Verbindungen die 2 oder mehr (z.B. 3, 4, 5 oder 6) gleiche oder verschiedene reaktive Gruppen wie beispielsweise -Cl, -Br, -I, -Tosyl, -Mesyl, -N=C=O, =O, -C(=O)H, -C(=O)Cl oder Epoxy enthalten und somit zur Vernetzung der Cyclodextrin-Verbindungen geeignet sind. Die reaktiven Gruppen der Vernetzungsmittel werden im Folgenden auch als Abgangsgruppen bezeichnet.

Die Vernetzungsmittel sind vorzugsweise bi- oder poylfunktionelle Vernetzungsmittel, bevorzugter homobifunktionelle. homopolyfunktionelle, heterobifunktionelle oder heteropolyfunktionelle Vernetzungsmittel und insbesondere homo- oder heterobifunktionelle Vernetzungsmittel.

Für die Zwecke dieser Erfindung steht der Ausdruck "bifunktionelle Vernetzungsmittel" für Verbindungen, die zwei gleiche oder verschiedene Abgangsgruppen enthalten (z.B. -Cl, -Br, -I, -Tosyl, -Mesyl, -N=C=O, =O, -C(=O)H, -C(=O)Cl, Epoxy). Besonders vorteilhaft sind die Abgangsgruppen -Cl, -Br, -I, -Tosyl, -Mesyl, -N=C=O und Epoxy. Insbesondere vorteilhaft sind -Cl, -Br, -I, -N=C=O und Epoxy.

Für die Zwecke dieser Erfindung umfasst der Begriff "Abgangsgruppe" nicht nur reaktive Gruppen wie beispielsweise -Cl, -Br, -I, -Tosyl oder -Mesyl, die das Vernetzungsmittel während der Vernetzungsreaktion nukleofug verlassen, sondern auch solche Abgangsgruppen wie beispielsweise -N=C=O oder Epoxy, deren Atome das Vernetzungsmittel während der Vernetzungsreaktion nicht verlassen, jedoch in andere funktionelle Gruppen wie beispielsweise -NHC(=O)- oder Hydroxyalkyl umgewandelt werden (zur Definition der Begriffe wird verwiesen auf: P. Muller. Glossary of terms used in physical organic chemistry (IUPAC Recommendations 1994). Pure Appl. Chem., 1994, 66(5), 1077-1184).

Im Fall, dass das bifunktionelle Vernetzungsmittel zwei gleiche Abgangsgruppen enthält, wird dieses im Sinne dieser Erfindung als "homobifunktionelles Vernetzungsmittel" bezeichnet (z.B. 1,2-Dichlorethan). Im Fall, dass die beiden Abgangsgruppen des bifunktionellen Vernetzungsmittels voneinander verschieden sind, wird dieses für die Zwecke dieser Erfindung als "heterobifunktionelles Vernetzungsmittel" bezeichnet (z.B. Epichlorhydrin). Die zwei gleichen oder voneinander verschiedenen Abgangsgruppen der entsprechenden homo- oder heterobifunktionellen Vernetzungsmittel sind vorzugsweise ausgewählt aus der Gruppe bestehend aus -Cl, -Br, -I, -Tosyl, -Mesyl, -N=C=O, =O, -C(=O)H, -C(=O)Cl und Epoxy. Es ist besonders bevorzugt, dass die Abgangsgruppen ausgewählt sind aus der Gruppe bestehend aus -Cl, -Br, -I, -Tosyl, -Mesyl, -N=C=O und Epoxy. Insbesondere vorteilhaft sind -Cl, -Br, -I, -N=C=O und Epoxy.

Der Ausdruck "polyfunktionelle Vernetzungsmittel" steht im Sinne dieser Erfindung für Verbindungen, die drei oder mehr (z.B. 4, 5 oder 6) gleiche oder verschiedene Abgangsgruppen enthalten, die vorzugsweise ausgewählt sind aus der Gruppe bestehend aus -Cl, -Br, -I, -Tosyl, -Mesyl, -N=C=O, =O, -C(=O)H, -C(=O)Cl und Epoxy. Es ist besonders bevorzugt, dass die Abgangsgruppen ausgewählt sind aus der Gruppe bestehend aus -Cl, -Br, -I, -Tosyl, -Mesyl, -N=C=O und Epoxy. Insbesondere vorteilhaft sind -Cl, -Br, -I, -N=C=O und Epoxy.

Im Fall, dass das polyfunktionelle Vernetzungsmittel drei oder mehr (z.B. 4, 5 oder 6) gleiche Abgangsgruppen enthält, wird dieses im Sinne dieser Erfindung als "homopolyfunktionelles Vernetzungsmittel" bezeichnet. Im Fall, dass das polyfunktionelle Vernetzungsmittel wenigstens zwei voneinander verschiedene Abgangsgruppen enthält, wird dieses für die Zwecke dieser Beschreibung als "heteropolyfunktionelles Vernetzungsmittel" bezeichnet. Die gleichen oder verschiedenen Abgangsgruppen der entsprechenden homo- und heteropolyfunktionellen Vernetzungsmittel sind vorzugsweise ausgewählt aus der Gruppe bestehend aus -Cl, -Br, -I, -Tosyl, -Mesyl, -N=C=O, =O, -C(=O)H, -C(=O)Cl und Epoxy. Es ist besonders bevorzugt, dass die Abgangsgruppen ausgewählt sind aus der Gruppe bestehend aus -Cl, -Br,

-I, -Tosyl, -Mesyl, -N=C=O und Epoxy. Insbesondere vorteilhaft sind -Cl, -Br, -I, -N=C=O und Epoxy.

In einer bevorzugten Ausführungsform ist das Vernetzungsmittel ein $C_{1-6}$-Kohlenwasserstoff, 3- bis 7-gliedriger cyclischer Kohlenwasserstoff, Benzen (Benzol) oder 5- bis 7-gliedriger Heteroaromat, die jeweils mit zwei oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

In einer weiteren bevorzugten Ausführungsform ist das Vernetzungsmittel ein $C_{1-6}$-Kohlenwasserstoff oder 3- bis 7-gliedriger cyclischer Kohlenwasserstoff, die jeweils mit zwei oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

Im Sinne dieser Erfindung umfasst der Ausdruck "$C_{1-6}$-Kohlenwasserstoff" acyclische, gesättigte oder ungesättigte Kohlenwasserstoffe, die verzweigt- oder geradkettig und 1, 2, 3, 4, 5 oder 6 Kohlenstoffatome enthalten. Der Ausdruck "$C_{1-6}$-Kohlenwasserstoff" umfasst $C_{1-6}$-Alkane, $C_{2-6}$-Alkene und $C_{2-6}$-Alkine. $C_{2-6}$-Alkene enthalten mindestens eine C-C-Doppelbindung und $C_{2-6}$-Alkine mindestens eine C-C-Dreifachbindung. Vorzugsweise ist $C_{1-6}$-Kohlenwasserstoff ausgewählt aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Ethen, Ethin, Prop-1-en, iso-Buten, n-Buten, cis-2-Buten, trans-2-Buten, 1,2-Butadien, 1,3-Butadien, Pent-1-en, cis-Pent-2-en, trans-Pent-2-en, 2-Methyl-but-1-en, 2-Methyl-but-2-en, 3-Methyl-but-1-en, Hex-1-en, Hex-2-en und Hex-3-en. Die $C_{1-6}$-Kohlenwasserstoffe sind zwei- oder mehrfach mit gleichen oder verschiedenen Abgangsgruppen substituiert, wobei die Abgangsgruppen in jeder beliebigen und möglichen Position des $C_{1-6}$-Kohlenwasserstoffs sein können.

In Bezug auf das Vernetzungsmittel umfasst der Ausdruck "3- bis 7-gliedriger cyclischer Kohlenwasserstoff" für die Zwecke dieser Erfindung cyclische, gesättigte oder ungesättigte (aber nicht aromatische) Kohlenwasserstoffe, die 3, 4, 5, 6 oder 7 Kohlenstoffatome enthalten. Ein oder zwei Kohlenstoffatome können jedoch durch gleiche oder verschiedene Heteroatome, die ausgewählt sind aus der Gruppe bestehend aus N, O und S, ersetzt sein. Vorzugsweise ist 3- bis 7-gliedriger cyclischer Kohlenwasserstoff ausgewählt aus der Gruppe bestehend aus Cyclopropan, Cyclobutan, Cyclopentan, Cyclohexan, Cycloheptan, Cyclopropen, Cyclobuten, Cyclopenten, Cyclopentadien, Cyclohexen, Cyclohexadien, Cyclohepten, Cycloheptadien, Cycloheptatrien, Pyrrolin, Pyrrolidin, Tetrahydrofuran, Pyrazolin, Pyrazolidin, Imidazolin, Imidazolidin, Dioxolan, Piperidin, N-Methylpiperidin, Tetrahydropyran, Piperazin, N,N-Dimethylpiperazin, Dioxan, Morpholin, N-Methylmorpholin, Pyrazolin, Pyrazolinon und Pyranyl. Die 3- bis 7-gliedrigen cyclischen Kohlenwasserstoffe sind wenigstens zweifach mit gleichen oder verschiedenen Abgangsgruppen substituiert, wobei die Abgangsgruppen in jeder beliebigen und möglichen Position des 3- bis 7-gliedrigen cyclischen Kohlenwasserstoffs sein können.

In Bezug auf das Vernetzungsmittel ist der aromatische Kohlenwasserstoff Benzen im Sinne dieser Erfindung wenigstens zweifach (z.B. zwei-, drei oder vierfach) mit gleichen oder verschiedenen Abgangsgruppen substituiert, wobei die Abgangsgruppen in jeder beliebigen und möglichen Position des Benzens sein können. Das Benzen kann ggf. auch Teil eine bi- oder polycyclischen Systems sein (z.B. Naphthalin).

Der Ausdruck "5- bis 7-gliedriger Heteroaromat" umfasst für die Zwecke dieser Erfindung 5-, 6- oder 7-gliedrige cyclische Aromaten, die mindestens 1, 2, 3, 4 oder 5 Heteroatome enthalten, wobei die Heteroatome gleich oder verschieden sind und ausgewählt sind aus der Gruppe bestehend aus N, O und S. Der Heteroaromat kann ggf. auch Teil eine bi- oder polycyclischen Systems sein. Es ist bevorzugt, dass der 5- bis 7-gliedriger Heteroaromat ausgewählt ist aus der Gruppe bestehend aus Pyrrol, Pyrazol, Imidazol, Thiophen, Furan, Oxazol, Isoxazol, Thiazol, Isothiazol, Furazan, Oxadiazol, Pyridin, Pyridazin, Pyrazin und Pyrimidin. Die 5- bis 7-gliedrigen Heteroaromaten sind zwei- oder mehrfach mit gleichen oder verschiedenen Abgangsgruppen substituiert, wobei die Abgangsgruppen in jeder beliebigen und möglichen Position des 5- bis 7-gliedrigen Heteroaromaten sein können.

In Bezug auf $C_{1-6}$-Kohlenwasserstoff versteht man unter dem Begriff "wenigstens zweifach substituiert" im Sinne dieser Erfindung die Substitution zweier oder mehrerer Wasserstoffreste durch wenigstens zwei gleiche oder verschiedene Abgangsgruppen, die entweder an gleichen oder verschiedenen Kohlenstoffatomen des $C_{1-6}$-Kohlenwasserstoffs mehrfach (z.B. zweifach) substituiert sind, beispielsweise zweifach am gleichen Kohlenstoffatom wie im Falle von Di(oxiran-2-yl)methan, oder an verschiedenen Stellen wie im Falle von 1,2-Dichlorethan. Die Mehrfachsubstitution kann mit dem gleichen (z.B. 1,2-Dichlorethan) oder mit verschiedenen Abgangsgruppen (z.B. Epichlorhydrin) erfolgen.

In Bezug auf 3- bis 7-gliedriger cyclischer Kohlenwasserstoff, Benzen und 5- bis 7-gliedriger Heteroaromat versteht man im Sinne dieser Erfindung unter "wenigstens zweifach substituiert" die Substitution zweier oder mehrerer Wasserstoffreste der Ringsysteme durch wenigstens zwei gleiche oder verschiedene Abgangsgruppen. Die Mehrfachsubstitution erfolgt dabei mit gleichen oder verschiedenen Abgangsgruppen und an verschiedenen oder falls möglich an gleichen Ringgliedern der Ringsysteme.

In einer weiteren bevorzugten Ausführungsform ist das Vernetzungsmittel ausgewählt aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Ethen, Ethin, Prop-1-en, iso-Buten, n-Buten, cis-2-Buten, trans-2-Buten, 1,2-Butadien, 1,3-Butadien, Pent-1-en, cis-Pent-2-en, trans-Pent-2-en, 2-Methyl-but-1-en, 2-Methyl-but-2-en, 3-Methyl-but-1-en, Hex-1-en, Hex-2-en Hex-3-en, Cyclopropan, Cyclobutan, Cyclopentan, Cyclohexan, Cycloheptan, Cyclopropen, Cyclobuten, Cyclopenten, Cyclopentadien, Cyclohexen, Cyclohexadien, Cyclohepten, Cycloheptadien, Cycloheptatrien, Pyrrolin, Pyrrolidin, Tetrahydrofuran, Pyrazolin, Pyrazolidin, Imidazolin, Imidazolidin, Dioxolan, Piperidin, N-Methylpiperidin, Tetrahydropyran, Piperazin, N,N-Dimethylpiperazin, Dioxan, Morpholin, N-Methylmorpholin, Pyrazolin, Pyrazolinon, Pyran, Benzen, Pyrrol, Pyrazol, Imidazol, Thiophen, Furan, Oxazol, Isoxazol, Thiazol, Isothiazol, Furazan, Oxadiazol, Pyridin, Pyridazin, Pyrazin und Pyrimidin, die jeweils mit 2 oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

In einer weiteren bevorzugten Ausführungsform ist das Vernetzungsmittel ausgewählt aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Cyclopropan, Cyclobutan, Cyclopentan, Cyclohexan, Cycloheptan, Tetrahydrofuran, Dioxolan, Tetrahydropyran, Dioxan, Benzen, Pyrrol, Pyrazol, Imidazol, Thiophen, Furan, Oxazol, Isoxazol, Thiazol, Isothiazol, Furazan, Oxadiazol, Pyridin, Pyridazin, Pyrazin und Pyrimidin, die jeweils mit 2 oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

In einer weiteren bevorzugten Ausführungsform ist das Vernetzungsmittel ausgewählt aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Cyclopropan, Cyclobutan, Cyclopentan, Cyclohexan und Cycloheptan, die jeweils mit 2 oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

In einer weiteren bevorzugten Ausführungsform ist das Vernetzungsmittel ausgewählt aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Cyclopropan, Cyclobutan, Cyclopentan und Cyclohexan, die jeweils mit 2 gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

In einer weiteren bevorzugten Ausführungsform ist das Vernetzungsmittel ausgewählt aus der Gruppe bestehend aus Methan, Ethan, n-Propan, n-Butan, n-Pentan, n-Hexan, Cyclopropan, Cyclobutan, Cyclopentan und Cyclohexan, die jeweils mit 2 gleichen oder verschiedenen Abgangsgruppen substituiert sind, wobei die Abgangsgruppen ausgewählt sind aus der Gruppe bestehend aus -Cl, -Br, -I, -N=C=O, =O, -C(=O)H, -C(=O)Cl und Epoxy.

In einer weiteren bevorzugten Ausführungsform ist das Vernetzungsmittel ausgewählt aus der Gruppe bestehend aus Glyoxal, 1,2-Dihalogenethan (z.B. 1,2-Dichlorethan), 1,3-Dihalogenpropan (z.B. 1,3-Dichlorpropan), Halogencarbonsäurehalogenid [beispielsweise Halogenessigsäurehalogenid (z.B. Chloressigsäurechlorid) oder Halogenpropionsäurehalogenid (z.B. Chlorpropionsäurechlorid)], Epichlorhydrin, 4-Chlor-1,2-epoxybutan, 1,2,3,4-Diepoxybutan, Tetramethylendiisocyanat und Hexamethylendiisocyanat, wobei Halogen und Halogenid, unabhängig voneinander, vorzugsweise für -Cl, -Br oder -I, besonders bevorzugt für -Cl oder -Br und insbesondere für -Cl stehen.

In einer weiteren bevorzugten Ausführungsform ist das Vernetzungsmittel ausgewählt ist aus der Gruppe bestehend aus Glyoxal, Epichlorhydrin, 4-Chlor-1,2-epoxybutan und 1,2,3,4-Diepoxybutan. Besonders vorteilhaft sind die Vernetzungsmittel Epichlorhydrin, 4-Chlor-1,2-epoxybutan und 1,2, 3,4-Diepoxybutan.

Das Vernetzungsmittel Epichlorhydrin ist insbesondere bevorzugt.

Für die Zwecke dieser Erfindung steht der Ausdruck "Funktionalisierungsmittel" für Verbindungen, die einer oder mehr, vorzugsweise nur eine reaktive Abgangsgruppe (z.B. -Cl, -Br, -I, -Tosyl, -Mesyl, -C(=O)Cl, Epoxy) enthalten und die weitere funktionelle Gruppen enthalten können, die zu keiner oder zu keiner wesentlichen Vernetzung der Cyclodextrin-Verbindungen führen (z.B. -OH, Alkyloxy, -C(=O)OH). Typische Funktionalisierungsmittel sind beispielsweise Alkylhalogenide, cyclische Alkansultone, Carbonsäurehalogenide, Carbonsäureanhydride, Halogencarbonsäuren, Halogencarbonsäureester, (Halogenalkyl)trialkylammonium-Salze und Oxirane.

Der Ausdruck "zu keiner wesentlichen Vernetzung der Cyclodextrin-Verbindungen führen" bedeutet im Sinne dieser Erfindung, dass vorzugsweise $< 10$ mol%, bevorzugter $< 5,0$ mol%, noch bevorzugter $< 2,0$ mol%, am Bevorzugtesten $< 1,0$ mol% und insbesondere eine analytisch nicht-nachweisbare Menge der Cyclodextrin-Monomere in den erfindungsgemäßen Copolymeren über Linker quervernetzt sind, die von den eingesetzten Funktionalisierungsmitteln abgeleitet sind; bezogen auf die Gesamtstoffmenge an Linkern des erfindungsgemäßen Copolymers.

Das Funktionalisierungsmittel kann jedoch auch Ammoniak, ein primäres, sekundäres oder tertiäres Amin sein, das durch Reaktion mit den Vernetzungsmitteln zu der Entstehung von Amino- und/ oder Ammonium-Substituenten in den erfindungsgemäßen Verbindungen führt. Beispielsweise kann das an eine Cyclodextrin-Verbindung oder ein Cyclodextrin-Monomer einseitig gebundene von Epichlorhydrin abgeleitete Strukturelement $-CH_2-CH(OH)-CH_2-Cl$ mit $NH(CH_3)_2$ zu - $CH_2-CH(OH)-CH_2-N(CH_3)_2$ reagieren.

Das Funktionalisierungsmittel ist vorzugsweise ausgewählt aus der Gruppe bestehend aus $C_{1-6}$-Alkyl-Y,

$C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-7}$-Cycloalkyl-C(=O)-OH, Y-$C_{1-6}$-Alkyl-C(=O)-O-$C_{1-6}$-Alkyl, Y-$C_{3-7}$-Cycloalkyl-C(=O)-O-$C_{3-7}$-Cycloalkyl, [$C_{1-6}$-Alkyl-C(=O)]$_2$O, [$C_{3-7}$-Cycloalkyl-C(=O)]$_2$O, Dihydrofuran-2,5-dion, Furan-2,5-dion, Dihydro-2H-pyran-2,6(3H)-dion, Oxiran, $C_{1-6}$-Alkyloxiran, $C_{3-7}$-Cycloalkyloxiran, Di-$C_{1-6}$-Alkyloxiran, Di-$C_{3-7}$-Cycloalkyloxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, ($C_{1-6}$-Alkyl)$_2$NH, ($C_{3-7}$-Cycloalkyl)$_2$NH, ($C_{1-6}$-Alkyl)$_3$N, ($C_{3-7}$-Cycloalkyl)$_3$N, [(Y-$C_{1-6}$-Alkyl)N($C_{1-6}$-Alkyl)$_3$]$^+$ Halogenid, 1,3-Propansulton und 1,4-Butansulton; wobei $C_{1-6}$-Alkyl und $C_{3-7}$-Cycloalkyl, unabhängig voneinander, unsubstituiert oder ein- oder mehrfach mit gleichen oder verschiedenen Resten Z substituiert sein können, wobei die weiteren Substituenten Z zu keiner oder zu keiner wesentlichen Vernetzung der Cyclodextrin-Monomere bzw. -Verbindungen führen; Halogenid vorzugsweise für Chlorid, Bromid oder Iodid, besonders bevorzugt für Chlorid oder Bromid und insbesondere für Chlorid steht; und die Reste Y für Abgangsgruppen stehen.

Die Abgangsgruppen Y sind, unabhängig voneinander, vorzugsweise ausgewählt aus der Gruppe bestehend aus -Cl, -Br, -I, -Mesyl, -Tosyl und Epoxy. Besonders vorteilhaft sind -Cl, -Br, -Mesyl und -Tosyl; insbesondere -Cl.

Die Substituenten Z sind, unabhängig voneinander, vorzugsweise ausgewählt aus der Gruppe bestehend aus -OH, =O, -C(=O)OH, -C(=O)$OCH_3$, -C(=O)$OCH_2CH_3$, -$NH_2$, -NH($CH_3$), -N($CH_3$)$_2$, -N($CH_3$)$_3^+A^-$, -NH($CH_2CH_3$), -N($CH_2CH_3$)$_2$ und -N($CH_2CH_3$)$_3^+A^-$, wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht. Besonders vorteilhaft sind -OH, =O, -C(=O)OH, -C(=O)$OCH_3$ und -C(=O)$OCH_2CH_3$; insbesondere -OH, =O und -C(=O)OH.

In Bezug auf das Funktionalisierungsmittel umfasst der Ausdruck "$C_{1-6}$-Alkyl" im Sinne dieser Erfindung acyclische, gesättigte oder ungesättigte Kohlenwasserstoffreste, die 1, 2, 3, 4, 5 oder 6 Kohlenstoffatome enthalten, verzweigt- oder geradkettig sind, eine Abgangsgruppe Y enthalten und weitere Substituenten Z enthalten können, wobei die weiteren Substituenten Z jedoch zu keiner oder zu keiner wesentlichen Vernetzung der Cyclodextrin-Monomere bzw. - Verbindungen führen. Die Abgangsgruppe und ggf. die Substituenten Z können in jeder beliebigen und möglichen Position des $C_{1-6}$-Alkyls sein. $C_{1-6}$-Alkyl umfasst $C_{1-6}$-Alkanyl, $C_{2-6}$-Alkenyl und $C_{2-6}$-Alkinyl. Dabei weisen $C_{2-6}$-Alkenyle mindestens eine C-C-Doppelbindung und $C_{2-6}$-Alkinyle mindestens eine C-C-Dreifachbindung auf, wohingegen $C_{1-6}$-Alkanyle vollständig gesättigt sind.

In Bezug auf das Funktionalisierungsmittel ist $C_{1-6}$-Alkyl vorzugsweise ausgewählt aus der Gruppe bestehend aus Methyl, Ethyl, iso-Propyl, n-Propyl, iso-Butyl, n-Butyl, sec-Butyl, tert-Butyl, n-Pentyl, iso-Pentyl, neo-Pentyl, iso-Hexyl, n-Hexyl, neo-Hexyl, 3-Methylpentyl, 2,3-Dimethylbutyl, Ethenyl, Ethinyl, Prop-1-enyl, iso-Butenyl, n-Butenyl, cis-2-Butenyl, trans-2-Butenyl, 1,2-Butadienyl, 1,3-Butadienyl, Pent-1-enyl, cis-Pent-2-enyl, trans-Pent-2-enyl, 2-Methyl-but-1-enyl, 2-Methyl-but-2-enyl, 3-Methyl-but-1-enyl, Hex-1-enyl, Hex-2-enyl und Hex-3-enyl. Es ist besonders bevorzugt, dass $C_{1-6}$-Alkyl ausgewählt ist aus der Gruppe bestehend aus Methyl, Ethyl, iso-Propyl, n-Propyl, iso-Butyl, n-Butyl, sec-Butyl, tert-Butyl, Ethenyl, Ethinyl und Prop-1-enyl. Es ist insbesondere bevorzugt, dass $C_{1-6}$-Alkyl ausgewählt ist aus der Gruppe bestehend aus Methyl, Ethyl, iso-Propyl, n-Propyl, iso-Butyl, n-Butyl, sec-Butyl und tert-Butyl. In Bezug auf das Funktionalisierungsmittel enthält das -$C_{1-6}$-Alkyl eine Abgangsgruppe Y und kann mit einem oder mehreren gleichen oder verschiedenen Substituenten Z substituiert sein.

In Bezug auf das Funktionalisierungsmittel umfasst der Ausdruck "$C_{3-7}$-Cycloalkyl" für die Zwecke dieser Erfindung cyclische Kohlenwasserstoffreste mit 3, 4, 5, 6 oder 7 Kohlenstoffatomen, die gesättigt oder ungesättigt (aber nicht aromatisch) sind, eine Abgangsgruppe Y enthalten und weitere Substituenten Z enthalten können, wobei die weiteren Substituenten Z zu keiner oder zu keiner wesentlichen Vernetzung der Cyclodextrin-Monomere führen. Die Abgangsgruppe und ggf. die Substituenten Z können in jeder beliebigen und möglichen Position des $C_{3-7}$-Cycloalkyls sein. Der Ausdruck "$C_{3-7}$-Cycloalkyl" umfasst auch gesättigte oder ungesättigte (aber nicht aromatische) Cycloalkyle, in denen ein oder zwei Kohlenstoffatome durch ein Heteroatom N, O oder S ersetzt sind. Das gesättigte, ungesättigte, aber nicht aromatische $C_{3-7}$-Cycloalkyl kann mit einem oder mehreren gesättigten, ungesättigten oder aromatischen Resten, wie beispielsweise Cyclohexyl, Cyclohexenyl, Cyclohexadienyl oder Benzen (z.B. Indolin), kondensiert sein.

In Bezug auf das Funktionalisierungsmittel sind die $C_{3-7}$-Cycloalkyl-Reste, unabhängig voneinander, vorzugsweise ausgewählt aus der Gruppe bestehend aus Cyclopropyl, Cyclobutyl, Cyclopentyl, Cyclohexyl, Cycloheptyl, Cyclopropenyl, Cyclobutenyl, Cyclopentenyl, Cyclopentadienyl, Cyclohexenyl, Cyclohexadienyl, Cycloheptenyl, Cycloheptadienyl, Cycloheptatrienyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, N-Methylpiperidinyl, Tetrahydropyranyl, Piperazinyl, N,N-Dimethylpiperazinyl, Dioxanyl, Morpholinyl, N-Methylmorpholinyl, Chinuclidinyl, Pyrazolinyl, Pyrazolinonyl, Pyranyl, Indolinyl, Chinolizinyl, Chromanyl, Isochromanyl, Chromenyl, Isochromenyl und Benzodioxolanyl. Es ist besonders bevorzugt, dass $C_{3-7}$-Cycloalkyl ausgewählt ist aus der Gruppe bestehend aus Cyclopropyl, Cyclobutyl, Cyclopentyl, Cyclohexyl, Pyrrolinyl, Pyrrolidinyl, Tetrahydrofuranyl, Pyrazolinyl, Pyrazolidinyl, Imidazolinyl, Imidazolidinyl, Dioxolanyl, Piperidinyl, N-Methylpiperidinyl Tetrahydropyranyl, Piperazinyl, N,N-Dimethylpiperazinyl, Dioxanyl, Morpholinyl, N-Methylmorpholinyl, Chinuclidinyl, Pyrazolinyl, Pyrazolinonyl und Pyranyl. Es ist insbesondere bevorzugt, dass $C_{3-7}$-Cycloalkyl ausgewählt ist aus der Gruppe bestehend aus Cyclopropyl, Cyclobutyl, Cyclopentyl und Cyclohexyl. In Bezug auf das Funktionalisierungsmittel

enthält $C_{3-7}$-Cycloalkyl eine Abgangsgruppe Y und kann mit einem oder mehreren gleichen oder verschiedenen Substituenten Z substituiert sein.

In einer bevorzugten Ausführungsform sind die Funktionalisierungsmittel ausgewählt aus der Gruppe bestehend aus $CH_3$-$Y^1$, $CH_3$-$CH_2$-$Y^1$, $CH_3$-$CH_2$-$CH_2$-$Y^1$, $(CH_3)_2CH$-$Y^1$, $CH_3$-$CH_2$-$CH_2$-$CH_2$-$Y^1$, $(CH_3)_2CH$-$CH_2$-$Y^1$, $CH_3$-$CH_2$-$CH(CH_3)$-$Y^1$, $(CH_3)_3C$-$Y^1$, $CH_2$=CH-$Y^1$, $CH_2$=CH-$CH_2$-$Y^1$, Cyclopropyl-$Y^1$, $Y^1$-$CH_2$-C(=O)-OH, $Y^1$-$CH_2$-$CH_2$-C(=O)-OH, $Y^1$-$CH(CH_3)$-C(=O)-OH, $Y^1$-$CH_2$-C(=O)-O-$CH_3$, Y'-$CH_2$-$CH_2$-C(=O)-O-$CH_3$, $Y^1$-$CH(CH_3)$-C(=O)-O-$CH_3$, $Y^1$-$CH_2$-C(=O)-O-$CH_2CH_3$, $Y^1$-$CH_2$-$CH_2$-C(=O)-O-$CH_2$-$CH_3$, $Y^1$-$CH(CH_3)$-C(=O)-O-$CH_2$-$CH_3$, $CH_3$-C(=O)$Y^2$, $CH_3$-$CH_2$-C(=O)$Y^2$, $(CH_3)_2CH$-C(=O)$Y^2$, $CH_3$-$CH_2$-$CH_2$-C(=O)$Y^2$, $(CH_3)_3C$-C(=O)$Y^2$, $CH_3$-$CH_2$-$CH(CH_3)$-C(=O)$Y^2$, $(CH_3)_2CH$-$CH_2$-C(=O)$Y^2$, $CH_2$=CH-C(=O)$Y^2$, $CH_2$=CH-$CH_2$-C(=O)$Y^2$, Cyclopropyl-C(=O)$Y^2$, Dihydrofuran-2,5-dion, Furan-2,5-dion, Dihydro-2H-pyran-2,6(3H)-dion, $[CH_3$-C(=O)$]_2O$, $[CH_3$-$CH_2$-C(=O)$]_2O$, $[CH_3$-$CH_2$-$CH_2$-C(=O)$]_2O$, $[(CH_3)_2CH$-C(=O)$]_2O$, $[CH_3$-$CH_2$-$CH_2$-$CH_2$-C(=O)$]_2O$, $[CH_3$-$CH_2$-$CH(CH_3)$-C(=O)$]_2O$, $[(CH_3)_2CH$-$CH_2$-C(=O)$]_2O$, $[(CH_3)_3C$-C(=O)$]_2O$, $[CH_2$=CH-C(=O)$]_2O$, $[CH_2$=CH-$CH_2$-C(=O)$]_2O$, [Cyclopropyl-C(=O)$]_2O$, $CH_2$=CH-C(=O)-OH, $CH_3$-CH=CH-C(=O)-OH, $(CH_3)_2C$=CH-C(=O)-OH, $CH_3$-$CH_2$-CH=CH-C(=O)-OH, $CH_3$-CH=$C(CH_3)$-C(=O)-OH, $CH_2$=CH-C(=O)-O-$CH_3$, $CH_3$-CH=CH-C(=O)-O-$CH_3$, $(CH_3)_2C$=CH-C(=O)-O-$CH_3$, $CH_3$-$CH_2$-CH=CH-C(=O)-O-$CH_3$, $CH_3$-CH=$C(CH_3)$-C(=O)-O-$CH_3$, $CH_2$=CH-C(=O)-O-$CH_2$-$CH_3$, $CH_3$-CH=CH-C(=O)-O-$CH_2$-$CH_3$, $(CH_3)_2C$=CH-C(=O)-O-$CH_2$-$CH_3$, $CH_3$-$CH_2$-CH=CH-C(=O)-O-$CH_2$-$CH_3$, $CH_3$-CH=$C(CH_3)$-C(=O)O-$CH_2$-$CH_3$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, $(CH_3$-$O)_2S(=O)_2$, $(CH_3$-$CH_2$-$O)_2S(=O)_2$, $(CH_3$-$O)_2P(=O)Cl$, $(CH_3$-$CH_2$-$O)_2P(=O)Cl$, $[(CH_3)_2CH$-$O]_2P(=O)Cl$, $NH_3$, $CH_3$-$NH_2$, $(CH_3)_2NH$, $(CH_3)_3N$, $CH_3$-$CH_2$-$NH_2$, $(CH_3$-$CH_2)_2NH$, $(CH_3$-$CH_2)_3N$, $[Cl$-$CH_2$-$CH_2$-$N(CH_3)_3]^+Cl^-$, $[Cl$-$CH_2$-$CH_2$-$N(CH_2$-$CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

Die erfindungsgemäßen Copolymere sind vorzugsweise erhältlich durch das Mischen (Zusammenbringen) von wenigstens einer α-, β- oder γ-Cyclodextrin-Verbindung mit einem oder mehreren Vernetzungsmitteln, vorzugsweise nur einem Vernetzungsmittel; vorzugsweise in Gegenwart wenigstens eines Lösungsmittels.

Die erfindungsgemäßen Copolymere sind vorzugsweise auch erhältlich durch das Mischen (Zusammenbringen) eines Gemisches, das verschiedene α-, verschiedene β- oder verschiedene γ-Cyclodextrin-Verbindungen enthält oder durch das Mischen (Zusammenbringen) eines Gemisches, das α- und β-, α- und γ-, β- und γ- oder α-, β- und γ-Cyclodextrin-Verbindungen enthält, mit einem oder mehreren Vernetzungsmitteln, vorzugsweise nur einem Vernetzungsmittel; vorzugsweise in Gegenwart wenigstens eines Lösungsmittels.

In einer bevorzugten Ausführungsform wird eine α-, β- oder γ-Cyclodextrin-Verbindung oder eine Mischung, die wenigstens zwei verschiedene der genannten Cyclodextrin-Verbindungen enthält, ggf. in Gegenwart eines Lösungsmittels, gemischt mit wenigstens einem, vorzugsweise nur einem Vernetzungsmittel ausgewählt aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Ethen, Ethin, Prop-1-en, iso-Buten, n-Buten, cis-2-Buten, trans-2-Buten, 1,2-Butadien, 1,3-Butadien, Pent-1-en, cis-Pent-2-en, trans-Pent-2-en, 2-Methyl-but-1-en, 2-Methyl-but-2-en, 3-Methyl-but-1-en, Hex-1-en, Hex-2-en Hex-3-en, Cyclopropan, Cyclobutan, Cyclopentan, Cyclohexan, Cycloheptan, Cyclopropen, Cyclobuten, Cyclopenten, Cyclopentadien, Cyclohexen, Cyclohexadien, Cyclohepten, Cycloheptadien, Cycloheptatrien, Pyrrolin, Pyrrolidin, Tetrahydrofuran, Pyrazolin, Pyrazolidin, Imidazolin, Imidazolidin, Dioxolan, Piperidin, N-Methylpiperidin, Tetrahydropyran, Piperazin, N,N-Dimethylpiperazin, Dioxan, Morpholin, N-Methylmorpholin, Pyrazolin, Pyrazolinon, Pyran, Benzen, Pyrrol, Pyrazol, Imidazol, Thiophen, Furan, Oxazol, Isoxazol, Thiazol, Isothiazol, Furazan, Oxadiazol, Pyridin, Pyridazin, Pyrazin und Pyrimidin, die jeweils mit 2 oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy.

In einer bevorzugten Ausführungsform wird eine α-, β- oder γ-Cyclodextrin-Verbindung oder eine Mischung, die wenigstens zwei verschiedene der genannten Cyclodextrin-Verbindungen enthält, ggf. in Gegenwart eines Lösungsmittels, gemischt mit wenigstens einem, vorzugsweise nur einem Vernetzungsmittel ausgewählt aus der Gruppe bestehend aus Glyoxal, 1,2-Dihalogenethan (z.B. 1,2-Dichlorethan), 1,3-Dihalogenpropan (z.B. 1,3-Dichlorpropan), Halogencarbonsäurehalogenid, [beispielsweise Halogenessigsäurehalogenid (z.B. Chloressigsäurechlorid) oder Halogenpropionsäurehalogenid (z.B. Chlorpropionsäurechlorid)], Epichlorhydrin, 4-Chlor-1,2-epoxybutan, 1,2,3,4-Diepoxybutan, Tetramethylendiisocyanat und Hexamethylendiisocyanat; wobei Halogen und Halogenid, unabhängig voneinander, vorzugsweise für -Cl, -Br oder -I, besonders bevorzugt für -Cl oder -Br und insbesondere für -Cl stehen.

In einer weiteren bevorzugten Ausführungsform wird eine α-, β- oder γ-Cyclodextrin-Verbindung oder eine Mischung, die wenigstens zwei verschiedene der genannten Cyclodextrin-Verbindungen enthält, ggf. in Gegenwart eines Lösungsmittels, gemischt mit dem Vernetzungsmittel Epichlorhydrin und ggf. einem weiteren Vernetzungsmittel ausgewählt aus der Gruppe bestehend aus Glyoxal, 1,2-Dihalogenethan (z.B. 1,2-Dichlorethan), 1,3-Dihalogenpropan

(z.B. 1,3-Dichlorpropan), Halogencarbonsäurehalogenid [beispielsweise Halogenessigsäurehalogenid (z.B. Chloressigsäurechlorid) oder Halogenpropionsäurehalogenid (z.B. Chlorpropionsäurechlorid)], 4-Chlor-1,2-epoxybutan, 1,2,3,4-Diepoxybutan, Tetramethylendiisocyanat und Hexamethylendiisocyanat; wobei Halogen und Halogenid, unabhängig voneinander, vorzugsweise für -Cl, -Br oder -I, besonders bevorzugt für -Cl oder -Br und insbesondere für -Cl stehen.

In einer weiteren bevorzugten Ausführungsform wird eine α-, β- oder γ-Cyclodextrin-Verbindung oder eine Mischung, die wenigstens zwei verschiedene der genannten Cyclodextrin-Verbindungen enthält, ggf. in Gegenwart eines Lösungsmittels, gemischt mit dem Vernetzungsmittel Epichlorhydrin und keinem weiteren Vernetzungsmittel oder mit dem Vernetzungsmittel 1,2,3,4-Diepoxybutan und keinem weiteren Vernetzungsmittel.

In einer besonders bevorzugten Ausführungsform sind die erfindungsgemäßen Copolymere erhältlich durch Mischen von

einer oder wenigstens zwei Cyclodextrin-Verbindungen, vorzugsweise nur einer Cyclodextrin-Verbindung ausgewählt aus der Gruppe bestehend aus (Glucosyl)-Cyclodextrin; (Galactosyl)-Cyclodextrin; (Maltosyl)-Cyclodextrin; (Maltriosyl)-Cyclodextrin; (Acetyl)-Cyclodextrin; (Propionyl)-Cyclodextrin; (Isobutyryl)-Cyclodextrin; (Butyryl)-Cyclodextrin; (Pivaloyl)-Cyclodextrin; (Pentanoyl)-Cyclodextrin; (Hexanoyl)-Cyclodextrin; (Cyclopropancarbonyl)-Cyclodextrin; (Benzoyl)-Cyclodextrin; (2-Phenylacetyl)-Cyclodextrin; (2-Carboxyacetyl)-Cyclodextrin; (3-Carboxypropanoyl)-Cyclodextrin; (4-Carboxybutanoyl)-Cyclodextrin; (2-Methoxy-2-oxoacetyl)-Cyclodextrin; (3-Methoxy-3-oxopropanoyl)-Cyclodextrin; (4-Methoxy-4-oxobutanoyl)-Cyclodextrin; (2-Hydroxyacetyl)-Cyclodextrin; (2-Hydroxypropanoyl)-Cyclodextrin; (3-Hydroxypropanoyl)-Cyclodextrin; (2-Hydroxybutanoyl)-Cyclodextrin; (3-Hydroxybutanoyl)-Cyclodextrin; (4-Hydroxybutanoyl)-Cyclodextrin; (2-Hydroxypentanoyl)-Cyclodextrin; (3-Hydroxypentanoyl)-Cyclodextrin; (4-Hydroxypentanoyl)-Cyclodextrin; (5-Hydroxypentanoyl)-Cyclodextrin; (2,3-Dihydroxy-propanoyl)-Cyclodextrin; (2,3-Dihydroxybutanoyl)-Cyclodextrin; (2,4-Dihydroxybutanoyl)-Cyclodextrin; (3,4-Dihydroxybutanoyl)-Cyclodextrin; (2,3-Dihydroxypentanoyl)-Cyclodextrin; (2,4-Dihydroxypentanoyl)-Cyclodextrin; (2,5-Dihydroxypentanoyl)-Cyclodextrin; (3,4-Dihydroxypentanoyl)-Cyclodextrin; (3,5-Dihydroxypentanoyl)-Cyclodextrin; (4,5-Dihydroxypentanoyl)-Cyclodextrin; (2,3,4-Trihydroxybutanoyl)-Cyclodextrin; (2,3,4-Trihydroxypentanoyl)-Cyclodextrin; (2,3,5-Trihydroxypentanoyl)-Cyclodextrin; (2,4,5-Trihydroxypentanoyl)-Cyclodextrin; (3,4,5-Trihydroxypentanoyl)-Cyclodextrin; (2,3,4,5-Tetrahydroxypentanoyl)-Cyclodextrin; (2-Aminoacetyl)-Cyclodextrin; (2-Aminopropanoyl)-Cyclodextrin; (2-Aminobutanoyl)-Cyclodextrin; [2-(1*H*-Imidazol-1-yl)acetyl]-Cyclodextrin; [3-(1*H*-Imidazol-1-yl)propanoyl]-Cyclodextrin; [4-(1*H*-Imidazol-1-yl)butanoyl]-Cyclodextrin; [2-(1*H*-Imidazol-1-yl)acetyl]-Cyclodextrin; [3-(1*H*-Imidazol-1-yl)propanoyl]-Cyclodextrin; [4-(1*H*-Imidazol-1-yl)butanoyl]-Cyclodextrin; [2-(Pyridin-3-yl)acetyl]-Cyclodextrin; [3-(Pyridin-3-yl)propanoyl]-Cyclodextrin; [4-(Pyridin-3-yl)butanoyl]-Cyclodextrin; (Methyl)-Cyclodextrin; (Ethyl)-Cyclodextrin; (*iso*-Propyl)-Cyclodextrin, (n-Propyl)-Cyclodextrin; (*iso*-Butyl)-Cyclodextrin; (*n*-Butyl)-Cyclodextrin; (sec-Butyl)-Cyclodextrin; (*tert*-Butyl)-Cyclodextrin; (Cyclopropyl)-Cyclodextrin; (Phenyl)-Cyclodextrin; (Benzyl)-Cyclodextrin; (Phenethyl)-Cyclodextrin; (Carboxymethyl)-Cyclodextrin; (1-Carboxyethyl)-Cyclodextrin; (2-Carboxyethyl)-Cyclodextrin; (1-Carboxypropyl)-Cyclodextrin; (2-Carboxypropyl)-Cyclodextrin; (3-Carboxypropyl)-Cyclodextrin; (1-Carboxypropan-2-yl)-Cyclodextrin; (4-Carboxybutyl)-Cyclodextrin; (5-Carboxypentyl)-Cyclodextrin; (5-Carboxypentyl)-Cyclodextrin; (2-Methoxy-2-oxoethyl)-Cyclodextrin; (3-Methoxy-2-oxopropyl)-Cyclodextrin; (4-Methoxy-4-oxobutyl)-Cyclodextrin; (5-Methoxy-5-oxopentyl)-Cyclodextrin; (6-Methoxy-6-oxohexyl)-Cyclodextrin; (2-Ethoxy-2-oxoethyl)-Cyclodextrin; (3-Ethoxy-2-oxopropyl)-Cyclodextrin; (4-Ethoxy-4-oxobutyl)-Cyclodextrin; (5-Ethoxy-5-oxopentyl)-Cyclodextrin; (6-Ethoxy-6-oxohexyl)-Cyclodextrin; (3-Carboxy-2-hydroxypropyl)-Cyclodextrin; (2-Hydroxy-4-methoxy-4-oxobutyl)-Cyclodextrin; (4-Ethoxy-2-hydroxy-4-oxobutyl)-Cyclodextrin; (1-Carboxy-2-hydroxyethyl)-Cyclodextrin; (3-Hydroxy-1-methoxy-1-oxopropan-2-yl)-Cyclodextrin; (1-Ethoxy-3-hydroxy-1-oxopropan-2-yl)-Cyclodextrin; (1-Carboxy-3-hydroxypropan-2-yl)-Cyclodextrin; (1-Hydroxy-4-methoxy-4-oxobutan-2-yl)-Cyclodextrin; (4-Ethoxy-1-hydroxy-4-oxobutan-2-yl)-Cyclodextrin; (Hydroxymethyl)-Cyclodextrin; (1-Hydroxyethyl)-Cyclodextrin; (2-Hydroxyethyl)-Cyclodextrin; (1-Hydroxypropyl)-Cyclodextrin; (2-Hydroxypropyl)-Cyclodextrin; (3-Hydroxypropyl)-Cyclodextrin; (1-Hydroxypropan-2-yl)-Cyclodextrin; (2-Hydroxypropan-2-yl)-Cyclodextrin; (1-Hydroxybutyl)-Cyclodextrin; (2-Hydroxybutyl)-Cyclodextrin; (3-Hydroxybutyl)-Cyclodextrin; (4-Hydroxybutyl)-Cyclodextrin; (1-Hydroxypentyl)-Cyclodextrin; (2-Hydroxypentyl)-Cyclodextrin; (3-Hydroxypentyl)-Cyclodextrin; (4-Hydroxypentyl)-Cyclodextrin; (5-Hydroxypentyl)-Cyclodextrin; (1,2-Dihydroxyethyl)-Cyclodextrin; (1,2-Dihydroxypropyl)-Cyclodextrin; (1,3-Dihydroxypropyl)-Cyclodextrin; (2,3-Dihydroxypropyl)-Cyclodextrin; (1,2-Dihydroxybutyl)-Cyclodextrin; (1,3-Dihydroxybutyl)-Cyclodextrin; (1,4-Dihydroxybutyl)-Cyclodextrin; (2,3-Dihydroxybutyl)-Cyclodextrin; (2,4-Dihydroxybutyl)-Cyclodextrin; (3,4-Dihydroxybutyl)-Cyclodextrin; (1,2-Dihydroxypentyl)-Cyclodextrin; (1,3-Dihydroxypentyl)-Cyclodextrin; (1,4-Dihydroxy-pentyl)-Cyclodextrin; (1,5-Dihydroxypentyl)-Cyclodextrin; (2,3-Dihydroxypentyl)-Cyclodextrin; (2,4-Dihydroxypentyl)-Cyclodextrin; (2,5-Dihydroxypentyl)-Cyclodextrin; (3,4-Dihydroxypentyl)-Cyclodextrin; (3,5-Dihydroxypentyl)-Cyclodextrin; (4,5-Dihydroxypentyl)-Cyclodextrin; (1,2,3-Trihydroxypropyl)-Cyclodextrin; (1,2,3-Trihydroxybutyl)-Cyclodextrin; (1,2,4-Trihydroxybutyl)-Cyclodextrin; (1,3,4-Trihydroxybutyl)-Cyclodextrin; (2,3,4-Trihydroxybutyl)-Cyclodextrin; (1,2,3-Trihydroxy-pentyl)-Cyclodextrin; (1,2,4-Trihydroxypentyl)-Cyclodextrin; (1,2,5-Trihydroxypentyl)-Cyclodextrin; (1,3,4-Trihydroxypentyl)-Cyclodextrin; (1,3,5-Trihydroxypentyl)-Cyclodextrin; (1,4,5-Trihydroxypentyl)-Cyclodextrin; (2,3,4-Trihydroxypentyl)-Cyclodextrin; (2,3,5-Trihydroxypentyl)-Cyclodextrin; (2,4,5-Trihydroxypentyl)-Cyclodextrin; (3,4,5-Trihydroxypentyl)-Cyclodextrin; (1,2,3,4-Tetrahydroxybutyl)-Cyclodextrin; (1,2,3,4-Tetrahydroxypentyl)-Cyclodextrin;

(1,2,4,5-Tetrahydroxypentyl)-Cyclodextrin; (2,3,4,5-Tetrahydroxypentyl)-Cyclodextrin; (1,2,3,4,5-Pentahydroxypentyl)-Cyclodextrin; [(Sulfooxy)methyl]-Cyclodextrin; [2-(Sulfooxy)ethyl]-Cyclodextrin; [3-(Sulfooxy)propyl]-Cyclodextrin; [4-(Sulfooxy)butyl]-Cyclodextrin; [(Phosphonooxy)methyl]-Cyclodextrin; [2-(Phosphonooxy)ethyl]-Cyclodextrin; [3-(Phosphonooxy)propyl]-Cyclodextrin; [4-(Phosphonooxy)butyl]-Cyclodextrin; [4-(Phosphonooxy)butyl]-Cyclodextrin; (Sulfomethyl)-Cyclodextrin; (2-Sulfoethyl)-Cyclodextrin; (3-Sulfopropyl)-Cyclodextrin; (4-Sulfobutyl)-Cyclodextrin; (5-Sulfopentyl)-Cyclodextrin; (6-Sulfohexyl)-Cyclodextrin; (Phosphonomethyl)-Cyclodextrin; (2-Phosphonoethyl)-Cyclodextrin; (3-Phosphonopropyl)-Cyclodextrin; (4-Phosphonobutyl)-Cyclodextrin; (5-Phosphonopentyl)-Cyclodextrin; (6-Phosphonohexyl)-Cyclodextrin; (2-Phosphonovinyl)-Cyclodextrin; (3-Phosphonoallyl)-Cyclodextrin; (4-Phosphonobut-3-enyl)-Cyclodextrin; (5-Phosphonopent-4-enyl)-Cyclodextrin; (6-Phosphonohex-5-enyl)-Cyclodextrin; (Aminomethyl)-Cyclodextrin; (2-Aminoethyl)-Cyclodextrin; (3-Aminopropyl)-Cyclodextrin; (4-Aminobutyl)-Cyclodextrin; (5-Aminopentyl)-Cyclodextrin; (6-Aminohexyl)-Cyclodextrin; [(*N,N*-Dimethylamino)methyl]-Cyclodextrin; [2-(*N,N*-Di-methylamino)ethyl]-Cyclodextrin; [3-(*N,N*-Dimethylamino)propyl]-Cyclodextrin; [4-(*N,N*-Dimethylamino)butyl]-Cyclodextrin; [5-(*N,N*-Dimethylamino)pentyl]-Cyclodextrin; [6-(*N,N*-Dimethylamino) hexyl]-Cyclodextrin; [(*N,N*-Diethylamino)methyl]-Cyclodextrin; [2-(*N,N*-Diethylamino)ethyl]-Cyclodextrin; [3-(*N,N*-Diethylamino)propyl]-Cyclodextrin; [4-(*N,N*-Diethylamino)butyl]-Cyclodextrin; [5-(*N,N*-Diethylamino)pentyl]-Cyclodextrin; [6-(*N,N*-Diethylamino)hexyl]-Cyclodextrin; [(Trimethylammonio)methyl]-Cyclodextrin Chlorid; [2-(Trimethylammonio)ethyl]-Cyclodextrin Chlorid; [3-(Trimethylammonio)propyl]-Cyclodextrin Chlorid; [4-(Trimethylammonio)butyl]-Cyclodextrin Chlorid; [5-(Trimethylammonio)pentyl]-Cyclodextrin Chlorid; [6-(Trimethylammonio)hexyl]-Cyclodextrin Chlorid; [(Triethylammonio)methyl]-Cyclodextrin Chlorid; [2-(Triethylammonio)ethyl]-Cyclodextrin Chlorid; [3-(Triethylammonio)propyl]-Cyclodextrin Chlorid; [4-(Triethylammonio)butyl]-Cyclodextrin Chlorid; [5-(Triethylammonio)pentyl]-Cyclodextrin Chlorid; [6-(Triethyl-ammonio)hexyl]-Cyclodextrin Chlorid; [(1*H*-Imidazol-1-yl)methyl]-Cyclodextrin; [2-(1*H*-Imidazol-1-yl)ethyl]-Cyclodextrin; [3-(1*H*-Imidazol-1-yl)propyl]-Cyclodextrin; [4-(1*H*-Imidazol-1-yl)butyl]-Cyclodextrin; (Pyridin-3-ylmethyl)-Cyclodextrin; [2-(Pyridin-3-yl)ethyl]-Cyclodextrin; [3-(Pyridin-3-yl)propyl]-Cyclodextrin; [4-(Pyridin-3-yl)butyl]-Cyclodextrin; [(*β*-D-Glucopyranosyloxyuronsäure) methyl]-Cyclodextrin; [2-(*β*-D-Glucopyranosyloxyuronsäure)ethyl]-Cyclodextrin; [3-(*β*-D-Glucopyranosyl-oxyuronsäure)propyl]-Cyclodextrin; [4-(*β*-D-Glucopyranosyloxyuronsäure)butyl]-Cyclodextrin; [5-(*β*-D-Glucopyranosyloxyuronsäure)pentyl]-Cyclodextrin; und [6-(*β*-D-Glucopyranosyloxyuronsäure)hexyl]-Cyclodextrin; wobei Cyclodextrin für α-, β- oder γ-Cyclodextrin, vorzugsweise α- oder β-Cyclodextrin und insbesondere für β-Cyclodextrin steht;
mit einem oder wenigstens zwei verschiedenen Vernetzungsmitteln, vorzugsweise nur einem Vernetzungsmittel ausgewählt aus der Gruppe bestehend aus Epichlorhydrin, 4-Chlor-1,2-epoxybutan, 1,2,3,4-Diepoxybutan, Tetramethylendiisocyanat und Hexamethylendiisocyanat; wobei das Vernetzungsmittel Epichlorhydrin insbesondere bevorzugt ist;
jeweils ggf. in Gegenwart wenigstens eines Lösungsmittels; wobei
der Ausdruck "Cyclodextrin" für α-, β- oder γ-Cyclodextrin, vorzugsweise für α- oder β-Cyclodextrin und insbesondere für β-Cyclodextrin steht.

In einer weiteren besonders bevorzugten Ausführungsform wird (Carboxymethyl)-Cyclodextrin, (3-Sulfopropyl)-Cyclodextrin oder (4-Sulfobutyl)-Cyclodextrin mit Epichlorhydrin gemischt; vorzugsweise in Gegenwart eines Lösungsmittels.

In Bezug auf die α-Cyclodextrin-Verbindungen ist der Gesamtsubstitutionsgrad vorzugsweise 0 < TDS < 1, 0 < TDS < 2, 0 < TDS < 3 oder 0 < TDS < 4; bevorzugter 0 < TDS < 5, 0 < TDS < 6, 0 < TDS < 7 oder 0 < TDS < 8; noch bevorzugter 0 < TDS < 9, 0 < TDS < 10, 0 < TDS < 11 oder 0 < TDS < 12; am Bevorzugtesten 0 < TDS < 13, 0 < TDS < 14, 0 < TDS < 15 oder 0 < TDS < 16; und insbesondere 0 < TDS < 17 oder 0 < TDS < 18.

In Bezug auf die β-Cyclodextrin-Verbindungen ist der Gesamtsubstitutionsgrad vorzugsweise 0 < TDS < 1, 0 < TDS < 2, 0 < TDS < 3, 0 < TDS < 4 oder 0 < TDS < 5; bevorzugter 0 < TDS < 6, 0 < TDS < 7, 0 < TDS < 8, 0 < TDS < 9 oder 0 < TDS < 10; noch bevorzugter 0 < TDS < 11, 0 < TDS < 12, 0 < TDS < 13, 0 < TDS < 14 oder 0 < TDS < 15; am Bevorzugtesten 0 < TDS < 16, 0 < TDS < 17, 0 < TDS < 18 oder 0 < TDS < 19; und insbesondere 0 < TDS < 20 oder 0 < TDS < 21.

In Bezug auf die γ-Cyclodextrin-Verbindungen ist der Gesamtsubstitutionsgrad vorzugsweise 0 < TDS < 1, 0 < TDS < 2, 0 < TDS < 3, 0 < TDS < 4, 0 < TDS < 5 oder 0 < TDS < 6; bevorzugter 0 < TDS < 7, 0 < TDS < 8, 0 < TDS < 9, 0 < TDS < 10, 0 < TDS < 11 oder 0 < TDS < 12; noch bevorzugter 0 < TDS < 13, 0 < TDS < 14, 0 < TDS < 15, 0 < TDS < 16, 0 < TDS < 17 oder 0 < TDS < 18; am Bevorzugtesten 0 < TDS < 19, 0 < TDS < 20, 0 < TDS < 21 oder 0 < TDS < 22; und insbesondere 0 < TDS < 23 oder 0 < TDS < 24.

Das Mischen der Cyclodextrin-Verbindungen mit Vernetzungsmitteln kann in wässrigen oder organischen Lösungsmitteln erfolgen. Vorzugsweise erfolgt das Mischen in Wasser, Dimethylsulfoxid (DMSO), Dimethylformamid (DMF), N-Methyl-2-pyrrolidon (NMP) oder Mischungen davon. Die Umsetzung kann auch in wasserfreiem DMSO, wasserfreiem DMF, wasserfreiem NMP oder Mischungen davon unter vorzugsweise wasserfreien Bedingungen durchgeführt werden. Als Lösungsmittel besonders vorteilhaft sind Wasser, DMSO und DMF sowie Mischungen davon; insbesondere Wasser oder DMF sowie Mischungen davon.

Das Mischen erfolgt vorzugsweise im alkalischen Milieu, in der Regel bei einem pH-Wert > 7.

Das Mischen erfolgt vorzugsweise bei 7,5 ≤ pH ≤ 14, bevorzugter bei 7,5 ≤ pH ≤ 13, noch bevorzugter bei 8 ≤

pH ≤ 13, am Bevorzugtesten bei 8 ≤ pH ≤ 12,5 und insbesondere bei 9 ≤ pH ≤ 12.

Die Einstellung des pH-Wertes der Reaktionslösung oder -dispersion erfolgt vorzugsweise mit Basen, wie beispielsweise basischen Alkalimetallsalzen, basischen Erdalkalimetallsalzen, Hydriden, Alkoxiden oder organischen Basen wie beispielsweise Pyridin oder Diisopropylamin. Besonders vorteilhaft sind Natriumhydroxid, Natriumhydrogencarbonat, Natriumcarbonat, Kaliumhydroxid, Kaliumhydrogencarbonat, Kaliumcarbonat, Natriumhydrid, Kaliumhydrid, Natriummethanolat, Kalium-tert-butoxid, Pyridin und Diisopropylamin.

Da die Copolymerisierung bzw. Vernetzung der Cyclodextrin-Verbindungen unter basischen Reaktionsbedingungen erfolgt, können Substituenten, die im alkalischen Milieu nicht stabil sind, strukturell verändert werden. Beispielsweise kann die Ester-Gruppe der Cyclodextrin-Verbindung (2-Methoxy-2-oxoethyl)-Cyclodextrin zu einer Carboxyl- bzw. Carboxylat-Gruppe gespalten werden, wobei die Ausgangsverbindung (2-Methoxy-2-oxoethyl)-Cyclodextrin zu (Carboxymethyl)-Cyclodextrin umgewandelt wird. Diese Umwandlung kann, bezogen auf die Gesamtanzahl der Ester-Gruppen, vollständig oder unvollständig erfolgen. Folglich können im entsprechenden erfindungsgemäßen Copolymer alle oder nur ein Teil der Ester- zu Carboxyl-Gruppen umgewandelt sein.

In einer bevorzugten Ausführungsform werden die Cyclodextrin-Verbindungen in einem Lösungsmittel oder Lösungsmittelgemisch gelöst oder dispergiert und anschließend werden vorzugsweise unter Rühren der Dispersion oder Lösung die Vernetzungsmittel darin gelöst oder dispergiert. Die Zugabe der Vernetzungsmittel kann auf einmal oder sukzessive in Portionen erfolgen. Die Vernetzungsmittel können vor der Zugabe in einem Lösungsmittel (z.B. Wasser, DMF, DMSO, NMP) gelöst oder dispergiert werden und als Lösung oder Dispersion dem Reaktionsgemisch auf einmal oder sukzessive in Portionen zugegeben oder mittels Tropftrichter, Pipette oder Spritze zugetropft werden. Die Zugabe bzw. Zutropfung kann mit Hilfe einer Spritzenpumpe erfolgen.

In einer weiteren bevorzugten Ausführungsform werden die Vernetzungsmittel in einem Lösungsmittel oder Lösungsmittelgemisch gelöst oder dispergiert und anschließend wird vorzugsweise unter Rühren der Lösung oder Dispersion die Cyclodextrin-Verbindungen in dieser Lösung oder Dispersion gelöst oder dispergiert. Die Zugabe der Cyclodextrin-Verbindungen kann auf einmal oder sukzessive in Portionen erfolgen. Die Cyclodextrin-Verbindungen können vor der Zugabe in einem Lösungsmittel gelöst oder dispergiert werden und als Lösung oder Dispersion dem Reaktionsgemisch auf einmal oder sukzessive in Portionen zugegeben oder mittels Tropftrichter, Pipette oder Spritze zugetropft werden. Die Zugabe bzw. Zutropfung kann mit Hilfe einer Spritzenpumpe erfolgen.

In einer weiteren bevorzugten Ausführungsform werden die Vernetzungsmittel und Cyclodextrin-Verbindungen gleichzeitig in einem Lösungsmittel oder Lösungsmittelgemisch gelöst oder dispergiert.

Beim Mischen kann es zu einem plötzlichen und unerwünschten Anstieg der Reaktionstemperatur kommen. Deshalb ist es vorteilhaft, das Reaktionsgemisch ggf. zu kühlen, um eine im Wesentlichen konstante Reaktionstemperatur zu gewährleisten. Die Kühlung des Reaktionsgemisches kann über dem Eisbad oder Wasserbad erfolgen.

Die Alkalisierung des Reaktionsgemisches kann vor, während oder nach Zugabe des Vernetzungsmittels, vor, während oder nach Zugabe der Cyclodextrin-Verbindungen oder vor, während oder nach Zugabe einer Mischung davon erfolgen.

Die Messung des alkalischen pH-Wertes erfolgt vorzugsweise mit Hilfe von pH-Papier oder einem pH-Meter. In einer bevorzugten Ausführungsform wird der pH-Wert während der Umsetzung fortlaufend oder in bestimmten Zeitintervallen gemessen. Falls der pH-Wert unter einen bestimmten Wert fällt (z.B. pH < 8 oder < 7,5), kann im Reaktionsgemisch ein alkalischer pH-Wert durch Basenzugabe wieder hergestellt werden.

Die Umsetzung erfolgt vorzugsweise bei einer Reaktionstemperatur (Temperatur des Reaktionsgemisches) von 5,0 bis 90 °C, bevorzugter 10 bis 75 °C, noch bevorzugter 15 bis 50 °C, am Bevorzugtesten 15 bis 30 °C und insbesondere 20 bis 25 °C. Nach dem Mischen der Cyclodextrin-Verbindungen mit den Vernetzungsmitteln, ggf. in Gegenwart Lösungsmittels oder Lösungsmittelgemisches, wird die Temperatur des Reaktionsgemisches vorzugsweise auf 5 bis 90 °C, bevorzugter auf 10 bis 75 °C, noch bevorzugter auf 15 bis 50 °C, am Bevorzugtesten auf 15 bis 30 °C und insbesondere auf 20 bis 25 °C eingestellt.

In einer bevorzugten Ausführungsform wird eine Gesamtstoffmenge von ≥ 0,5 mol, ≥ 1,0 mol, ≥ 2,0 mol oder ≥ 3,0 mol, bevorzugter ≥ 4,0 mol, ≥ 5,0 mol oder ≥ 6,0 mol, noch bevorzugter ≥ 7,0 mol, ≥ 8,0 mol oder ≥ 9,0 mol, am Bevorzugtesten ≥ 10 mol, ≥ 15 mol oder ≥ 20 mol, und insbesondere ≥ 25 mol an Vernetzungsmittel bezogen auf eine Gesamtstoffmenge von 1,0 mol an Cyclodextrin-Verbindungen in das Reaktionsgemisch eingesetzt (Äquivalente).

In einer weiteren bevorzugten Ausführungsform ist das Verhältnis der Gesamtstoffmenge an Vernetzungsmittel zu der Gesamtstoffmenge an Cyclodextrin-Verbindungen ≥ 1 : 1, bevorzugter ≥ 2 : 1, noch bevorzugter ≥ 3 : 1, am Bevorzugtesten ≥ 5 : 1 und insbesondere ≥ 10 : 1.

Die Umsetzung (Vernetzungsreaktion) kann mittels geeigneter analytischer Methoden verfolgt werden. Beispielsweise können zu bestimmten Zeitpunkten Stichproben des Reaktionsgemisches genommen werden und mittels LC/MS oder NMR analysiert werden. Auf diese Weise kann das im Reaktionsgemisch zu einem bestimmten Zeitpunkt vorliegende Reaktionsprodukt bestimmt werden. Falls die Stichprobenanalyse ergibt, dass das gewünschte Reaktionsprodukt vorliegt, so kann die Umsetzung beendet und das gewünschte Reaktionsprodukt isoliert bzw. aufgereinigt werden.

Zur Beendigung der Umsetzung wird das Reaktionsgemisch vorzugsweise neutralisiert, d.h. der pH-Wert der Reaktionslösung oder -dispersion wird vorzugsweise auf 6,5 ≤ pH ≤ 7,5, besonders bevorzugt auf 6,8 ≤ pH ≤ 7,2 eingestellt. Die Neutraliserung erfolgt vorzugsweise mit organischen (z.B. Trifluoressigsäure, Essigsäure) oder anorganischen Säuren (z.B. Salzsäure, Schwefelsäure, Salpetersäure).

In einer weiteren bevorzugten Ausführungsform wird das Reaktionsgemisch nach der Umsetzung bzw. Neutralisierung dialysiert, vorzugsweise salzfrei dialysiert, wobei die in diesem Dialyseverfahren eingesetzte Dialysemembran einen MWCO ("Molecular Weight Cut-Off') von vorzugsweise 0,50 kDa oder 1,0 kDa, bevorzugter von 2,0 kDa oder 3,0 kDa, noch bevorzugter von 4,0 kDa oder 5,0 kDa, am Bevorzugtesten von 10 kDa und insbesondere von ≥ 15 kDa aufweist.

Vorzugsweise hat die salzfrei dialysierte Lösung einen Restgehalt von ≤ 10 Gew.-%, bevorzugter ≤ 5,0 Gew.-%, noch bevorzugter ≤ 2,5 Gew.-% und insbesondere ≤ 1,0 Gew.-% an Verbindungen mit einem Molekulargewicht kleiner als 1,5 kDa, bezogen auf die Gesamtmasse an erfindungsgemäßen Copolymer.

Das Reaktionsgemisch bzw. die im Wesentlichen salzfreie Dialyselösung kann vor der weiteren Aufreinigung bzw. Isolierung vorzugsweise mit Hilfe eines Rotationsverdampfers eingeengt werden. Eine Einengung des Reaktionsgemisches kann auch bereits vor der Dialyse, vorzugsweise salzfreien Dialyse erfolgen.

Die Isolierung oder Aufreinigung der erfindungsgemäßen Copolymere erfolgt vorzugsweise mittels Ultraflitration, Säulenchromatographie (z.B. an Kieselgel oder Aktivkohle), präparativer HPLC oder Rekristallisation. Das erhaltene erfindungsgemäße Copolymer kann lyophilisiert werden.

In einer bevorzugten Ausführungsform beträgt der Stoffmengenanteil der im erfindungsgemäßen Copolymer enthaltenen Linker 20 bis 95 mol%, bevorzugter 25 bis 80 mol%, noch bevorzugter 30 bis 70 mol%, am Bevorzugtesten 30 bis 60 mol% und insbesondere 30 bis 50 mol%, bezogen auf die Gesamtstoffmenge der Monomere, die im erfindungsgemäßen Copolymer enthalten sind.

Der Stoffmengenanteil der im erfindungsgemäßen Copolymer enthaltenen Cyclodextrin-Monomere beträgt vorzugsweise 5,0 bis 80 mol%, bevorzugter 10 bis 75 mol%, noch bevorzugter 20 bis 70 mol%, am Bevorzugtesten 30 bis 70 mol% und insbesondere 40 bis 70 mol%, bezogen auf die Gesamtstoffmenge der Monomere, die im erfindungsgemäßen Copolymer enthalten sind.

In einer weiteren bevorzugten Ausführungsform ist die Summe aus dem Stoffmengenanteil der Linker und dem Stoffmengenanteil der Cyclodextrin-Monomere ≥ 25 mol%, ≥ 30 mol% oder ≥ 40 mol%, bevorzugter ≥ 50 mol% oder ≥ 60 mol%, noch bevorzugter ≥ 70 mol% oder ≥ 80 mol%, am Bevorzugtesten ≥ 90 mol% oder ≥ 95 mol%, und insbesondere 100 mol%, bezogen auf die Gesamtstoffmenge der Monomere, die im erfindungsgemäßen Copolymer enthalten sind.

Die Wasserlöslichkeit der erfindungsgemäßen Copolymere ist bei 23 °C vorzugsweise ≥ 5,0 g $l^{-1}$, ≥ 10 g $l^{-1}$, ≥ 25 g $l^{-1}$ oder ≥ 50 g $l^{-1}$, bevorzugter ≥ 75 g $l^{-1}$, ≥ 100 g $l^{-1}$, ≥ 125 g $l^{-1}$ oder ≥ 150 g $l^{-1}$, noch bevorzugter ≥ 175 g $l^{-1}$, ≥ 200 g $l^{-1}$, ≥ 225 g $l^{-1}$ oder ≥ 250 g $l^{-1}$, am Bevorzugtesten ≥ 300 g $l^{-l}$ oder ≥ 400 g $l^{-1}$ und insbesondere ≥ 500 g $l^{-1}$.

Die Osmolalität einer 50 mM wässrigen Lösung an erfindungsgemäßen Copolymer ist vorzugsweise ≥ 50 mosm $kg^{-1}$, ≥ 75 mosm $kg^{-1}$ oder ≥ 100 mosm $kg^{-1}$, bevorzugter ≥ 125 mosm $kg^{-1}$, ≥ 150 mosm $kg^{-1}$ oder ≥ 175 mosm $kg^{-1}$, noch bevorzugter ≥ 200 mosm $kg^{-1}$ oder ≥ 225 mosm $kg^{-1}$, am Bevorzugtesten ≥ 250 mosm $kg^{-1}$ oder ≥ 275 mosm $kg^{-1}$ und insbesondere ≥ 300 mosm $kg^{-1}$.

Der Polymerisationsgrad der erfindungsgemäßen Copolymere ist vorzugsweise ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

Das mittlere Molekulargewicht der erfindungsgemäßen Copolymere ist vorzugsweise ≥ 2,0 kDa, bevorzugter ≥ 2,5 kDa, noch bevorzugter ≥ 3,0 kDa, am Bevorzugtesten ≥ 4,0 kDa und insbesondere ≥ 5,0 kDa. Das mittlere Molekulargewicht wird vorzugsweise mit Hilfe eines Membranosmometers gemessen (destilliertes Wasser, 23 °C).

In einer weiteren bevorzugten Ausführungsform ist das mittleres Molekulargewicht (M) der erfindungsgemäßen Polymere vorzugsweise 2,0 ≤ M ≤ 100 kDa, bevorzugter 2,0 ≤ M ≤ 50 kDa, noch bevorzugter 2,0 ≤ M ≤ 25 kDa, am Bevorzugtesten 2,0 ≤ M ≤ 15 kDa und insbesondere 2,0 ≤ M ≤ 10 kDa.

In einer weiteren besonders bevorzugten Ausführungsform hat das erfindungsgemäße Copolymer, das durch das Mischen wenigstens einer Cyclodextrin-Verbindung mit wenigstens einem Vernetzungsmittel erhältlich ist, vorzugsweise einen Polymerisationsgrad von ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

In einer weiteren bevorzugten Ausführungsform ist der mittlere Partikeldurchmesser der erfindungsgemäßen Copolymere, die durch das Mischen wenigstens einer Cyclodextrin-Verbindung mit wenigstens einem Vernetzungsmittel erhältlich sind, ≤ 1000 μm, ≤ 900 μm oder ≤ 800 μm, bevorzugter ≤ 700 μm, ≤ 600 μm oder ≤ 500 μm, noch bevorzugter ≤ 400 μm, ≤ 300 μm oder ≤ 200 μm, am Bevorzugtesten ≤ 100 μm, ≤ 75 μm oder ≤ 50 μm und insbesondere ≤ 25 μm.

Die erfindungsgemäßen Copolymere sind vorzugsweise erhältlich durch das Mischen (Zusammenbringen) von wenigstens einer α-, β- oder γ-Cyclodextrin-Verbindung mit einem oder mehreren Vernetzungsmitteln, vorzugsweise mit einem Vernetzungsmittel und einem oder mehreren Funktionalisierungsmitteln, vorzugsweise nur einem Funktionalisierungsmittel; vorzugsweise in Gegenwart wenigstens eines Lösungsmittels.

Die erfindungsgemäßen Copolymere sind vorzugsweise auch erhältlich durch das Mischen (Zusammenbringen) eines Gemisches, das verschiedene α-, verschiedene β- oder verschiedene γ-Cyclodextrin-Verbindungen enthält oder durch das Mischen (Zusammenbringen) eines Gemisches, das α- und β-, α- und γ-, β- und γ- oder α-, β- und γ-Cyclodextrin-Verbindungen enthält, mit einem oder mehreren Vernetzungsmitteln, vorzugsweise nur einem Vernetzungsmittel und einem oder mehreren Funktionalisierungsmittel, vorzugsweise nur einem Funktionalisierungsmittel; vorzugsweise in Gegenwart wenigstens eines Lösungsmittels.

In einer bevorzugten Ausführungsform wird wenigstens eine, vorzugsweise nur eine Cyclodextrin-Verbindung gemischt mit wenigstens einem, vorzugsweise nur einem Vernetzungsmittel und wenigstens einem, vorzugsweise nur einem Funktionalisierungsmittel, ggf. in Gegenwart wenigstens eines Lösungsmittels; wobei

das Vernetzungsmittel ausgewählt ist aus der Gruppe bestehend aus Methan, Ethan, iso-Propan, n-Propan, iso-Butan, n-Butan, sec-Butan, tert-Butan, n-Pentan, iso-Pentan, neo-Pentan, iso-Hexan, n-Hexan, neo-Hexan, 3-Methylpentan, 2,3-Dimethylbutan, Ethen, Ethin, Prop-1-en, iso-Buten, n-Buten, cis-2-Buten, trans-2-Buten, 1,2-Butadien, 1,3-Butadien, Pent-1-en, cis-Pent-2-en, trans-Pent-2-en, 2-Methyl-but-1-en, 2-Methyl-but-2-en, 3-Methyl-but-1-en, Hex-1-en, Hex-2-en Hex-3-en, Cyclopropan, Cyclobutan, Cyclopentan, Cyclohexan, Cycloheptan, Cyclopropen, Cyclobuten, Cyclopenten, Cyclopentadien, Cyclohexen, Cyclohexadien, Cyclohepten, Cycloheptadien, Cycloheptatrien, Pyrrolin, Pyrrolidin, Tetrahydrofuran, Pyrazolin, Pyrazolidin, Imidazolin, Imidazolidin, Dioxolan, Piperidin, N-Methylpiperidin, Tetrahydropyran, Piperazin, N,N-Dimethylpiperazin, Dioxan, Morpholin, N-Methylmorpholin, Pyrazolin, Pyrazolinon, Pyran, Benzen, Pyrrol, Pyrazol, Imidazol, Thiophen, Furan, Oxazol, Isoxazol, Thiazol, Isothiazol, Furazan, Oxadiazol, Pyridin, Pyridazin, Pyrazin und Pyrimidin, die jeweils mit 2 oder mehr (z.B. 3, 4, 5 oder 6) gleichen oder verschiedenen Abgangsgruppen substituiert sind, wobei die Abgangsgruppen ausgewählt sind aus der Gruppe bestehend aus -Cl, -Br, -I, -Tosyl, -Mesyl, -N=C=O, =O, -C(=O)H, -C(=O)Cl und Epoxy; und

das Funktionalisierungsmittel ausgewählt ist aus der Gruppe bestehend aus $C_{1-6}$-Alkyl-Y, $C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-7}$-Cycloalkyl-C(=O)-OH, Y-$C_{1-6}$-Alkyl-C(=O)-O-$C_{1-6}$-Alkyl, Y-$C_{3-7}$-Cycloalkyl-C(=O)-O-$C_{3-7}$-Cycloalkyl, $[C_{1-6}\text{-Alkyl-C(=O)}]_2O$, $[C_{3-7}\text{-Cycloalkyl-C(=O)}]_2O$, Dihydrofuran-2,5-dion, Furan-2,5-dion, Dihydro-2H-pyran-2,6(3H)-dion, Oxiran, $C_{1-6}$-Alkyloxiran, $C_{3-7}$-Cycloalkyloxiran, Di-$C_{1-6}$-Alkyloxiran, Di-$C_{3-7}$-Cycloalkyloxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, $(C_{1-6}\text{-Alkyl})_2NH$, $(C_{3-7}\text{-Cycloalkyl})_2NH$, $(C_{1-6}\text{-Alkyl})_3N$, $(C_{3-7}\text{-Cycloalkyl})_3N$, $[(Y\text{-}C_{1-6}\text{-Alkyl})N(C_{1-6}\text{-Alkyl})_3]^+$ Halogenid, 1,3-Propansulton und 1,4-Butansulton; wobei $C_{1-6}$-Alkyl und $C_{3-7}$-Cycloalkyl, unabhängig voneinander, unsubstituiert oder ein- oder mehrfach mit gleichen oder verschiedenen Resten Z substituiert sein können; wobei die weiteren Substituenten Z zu keiner oder zu keiner wesentlichen Quervernetzung der Cyclodextrin-Monomere führen; Halogenid vorzugsweise für Chlorid, Bromid oder Iodid, besonders bevorzugt für Chlorid oder Bromid und insbesondere für Chlorid steht; und die Reste Y für Abgangsgruppen stehen; wobei die Abgangsgruppen Y, unabhängig voneinander, vorzugsweise ausgewählt sind aus der Gruppe bestehend aus -Cl, -Br, -I, -Mesyl, -Tosyl und Epoxy; besonders bevorzugt ausgewählt sind aus der Gruppe bestehend aus -Cl, -Br, -Mesyl und -Tosyl; und insbesondere für -Cl stehen; und die Substituenten Z, unabhängig voneinander, vorzugsweise ausgewählt sind aus der Gruppe bestehend aus -OH, =O, -C(=O)OH, $-C(=O)OCH_3$, $-C(=O)OCH_2CH_3$, $-NH_2$, $-NH(CH_3)$, $-N(CH_3)_2$, $-N(CH_3)_3^+A^-$, $-NH(CH_2CH_3)$, $-N(CH_2CH_3)_2$ und $-N(CH_2CH_3)_3^+A^-$; besonders bevorzugt aus der Gruppe bestehend aus -OH, =O, -C(=O)OH, $-C(=O)OCH_3$ und $-C(=O)OCH_2CH_3$; und insbesondere ausgewählt sind aus der Gruppe bestehend aus -OH, =O und -C(=O)OH; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer bevorzugten Ausführungsform wird wenigstens eine, vorzugsweise nur eine Cyclodextrin-Verbindung gemischt mit wenigstens einem, vorzugsweise nur einem Vernetzungsmittel und wenigstens einem, vorzugsweise nur einem Funktionalisierungsmittel, ggf. in Gegenwart wenigstens eines Lösungsmittels; wobei

das Vernetzungsmittel ausgewählt ist aus der Gruppe bestehend aus Glyoxal, 1,2-Dihalogenethan (z.B. 1,2-Dichlorethan), 1,3-Dihalogenpropan (z.B. 1,3-Dichlorpropan), Halogencarbonsäurehalogenid [beispielsweise Halogenessigsäurehalogenid (z.B. Chloressigsäurechlorid) oder Halogenpropionsäurehalogenid (z.B. Chlorpropionsäurechlorid)], Epichlorhydrin, 4-Chlor-1,2-epoxybutan, 1,2,3,4-Diepoxybutan, Tetramethylendiisocyanat und Hexamethylendiisocyanat; wobei Halogen und Halogenid, unabhängig voneinander, vorzugsweise für -Cl, -Br oder -I, besonders bevorzugt für -Cl oder -Br und insbesondere für -Cl stehen; und

das Funktionalisierungsmittel ausgewählt ist aus der Gruppe bestehend aus $C_{1-6}$-Alkyl-Y, $C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-7}$-Cycloalkyl-C(=O)-OH, Y-$C_{1-6}$-Alkyl-C(=O)-O-$C_{1-6}$-Alkyl, Y-$C_{3-7}$-Cycloalkyl-C(=O)-O-$C_{3-7}$-Cycloalkyl, $[C_{1-6}\text{-Alkyl-C(=O)}]_2O$, $[C_{3-7}\text{Cycloalkyl-C(=O)}]_2O$, Dihydrofuran-2,5-dion, Furan-2,5-dion, Dihydro-2H-pyran-2,6(3H)-dion, Oxiran, $C_{1-6}$-Alkyloxiran, $C_{3-7}$-Cycloalkyloxiran, Di-$C_{1-6}$-Alkyloxiran, Di-$C_{3-7}$-Cycloalkyloxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, $(C_{1-6}\text{-Alkyl})_2NH$, $(C_{3-7}\text{-Cycloalkyl})_2NH$, $(C_{1-6}\text{-Alkyl})_3N$, $(C_{3-7}\text{-Cycloalkyl})_3N$, $[(Y\text{-}C_{1-6}\text{-Alkyl})N(C_{1-6}\text{-Alkyl})_3]^+$ Halogenid, 1,3-Propansulton und 1,4-Butansulton; wobei $C_{1-6}$-Alkyl und $C_{3-7}$-Cycloalkyl, unabhängig voneinander, unsubstituiert oder ein- oder mehrfach mit

gleichen oder verschiedenen Resten Z substituiert sein können; wobei die weiteren Substituenten Z zu keiner oder zu keiner wesentlichen Quervernetzung der Cyclodextrin-Monomere führen; Halogenid vorzugsweise für Chlorid, Bromid oder Iodid, besonders bevorzugt für Chlorid oder Bromid und insbesondere für Chlorid steht; und die Reste Y für Abgangsgruppen stehen; wobei die Abgangsgruppen Y, unabhängig voneinander, vorzugsweise ausgewählt sind aus der Gruppe bestehend aus -Cl, -Br, -I, -Mesyl, -Tosyl und Epoxy; besonders bevorzugt ausgewählt sind aus der Gruppe bestehend aus -Cl, -Br, -Mesyl und -Tosyl; und insbesondere für -Cl stehen; und die Substituenten Z, unabhängig voneinander, vorzugsweise ausgewählt sind aus der Gruppe bestehend aus -OH, =O, -C(=O)OH, $-C(=O)OCH_3$, $-C(=O)OCH_2CH_3$, $-NH_2$, $-NH(CH_3)$, $-N(CH_3)_2$, $-N(CH_3)_3^+A^-$, $-NH(CH_2CH_3)$, $-N(CH_2CH_3)_2$ und $-N(CH_2CH_3)_3^+A^-$; besonders bevorzugt aus der Gruppe bestehend aus -OH, =O, -C(=O)OH, $-C(=O)OCH_3$ und $-C(=O)OCH_2CH_3$; und insbesondere ausgewählt sind aus der Gruppe bestehend aus -OH, =O und -C(=O)OH; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht.

In einer weiteren bevorzugten Ausführungsform wird wenigstens eine Cyclodextrin-Verbindung, vorzugsweise nur eine Cyclodextrin-Verbindung gemischt mit wenigstens einem, vorzugsweise nur einem Vernetzungsmittel und wenigstens einem, vorzugsweise nur einem Funktionalisierungsmittel, ggf. in Gegenwart wenigstens eines Lösungsmittels; wobei

das Vernetzungsmittel vorzugsweise ausgewählt ist aus der Gruppe bestehend aus Glyoxal, 1,2-Dihalogenethan (z.B. 1,2-Dichlorethan), 1,3-Dihalogenpropan (z.B. 1,3-Dichlorpropan), Halogencarbonsäurehalogenid [beispielsweise Halogenessigsäurehalogenid (z.B. Chloressigsäurechlorid), Halogenpropionsäurehalogenid (z.B. Chlorpropionsäurechlorid)], Epichlorhydrin, 4-Chlor-1,2-epoxybutan, 1,2,3,4-Diepoxybutan, Tetramethylendiisocyanat und Hexamethylendiisocyanat; wobei Halogen und Halogenid, unabhängig voneinander, vorzugsweise für -Cl, -Br oder -I, besonders bevorzugt für -Cl oder -Br und insbesondere für -Cl stehen; und

das Funktionalisierungsmittel ausgewählt ist aus der Gruppe bestehend aus $CH_3-Y^1$, $CH_3-CH_2-Y^1$, $CH_3-CH_2-CH_2-Y^1$, $(CH_3)_2CH-Y^1$, $CH_3-CH_2-CH_2-CH_2-Y^1$, $(CH_3)_2CH-CH_2-Y^1$, $CH_3-CH_2-CH(CH_3)-Y^1$, $(CH_3)_3C-Y^1$, $CH_2=CH-Y^1$, $CH_2=CH-CH_2-Y^1$, Cyclopropyl-$Y^1$, $Y^1-CH_2-C(=O)-OH$, $Y^1-CH_2-CH_2-C(=O)-OH$, $Y^1-CH(CH_3)-C(=O)-OH$, $Y^1-CH_2-C(=O)-O-CH_3$, $Y^1-CH_2-CH_2-C(=O)-O-CH_3$, $Y^1-CH(CH_3)-C(=O)-O-CH_3$, $Y^1-CH_2-C(=O)-O-CH_2CH_3$, $Y^1-CH_2-CH_2-C(=O)-O-CH_2-CH_3$, $Y^1-CH(CH_3)-C(=O)-O-CH_2-CH_3$, $CH_3-C(=O)Y^2$, $CH_3-CH_2-C(=O)Y^2$, $(CH_3)_2CH-C(=O)Y^2$, $CH_3-CH_2-CH_2-C(=O)Y^2$, $(CH_3)_3C-C(=O)Y^2$, $CH_3-CH_2-CH(CH_3)-C(=O)Y^2$, $(CH_3)_2CH-CH_2-C(=O)Y^2$, $CH_2=CH-C(=O)Y^2$, $CH_2=CH-CH_2-C(=O)Y^2$, Cycloproyl-$C(=O)Y^2$, Dihydrofuran-2,5-dion, Furan-2,5-dion, Dihydro-2H-pyran-2,6(3H)-dion, $[CH_3-C(=O)]_2O$, $[CH_3-CH_2-C(=O)]_2O$, $[CH_3-CH_2-CH_2-C(=O)]_2O$, $[(CH_3)_2CH-C(=O)]_2O$, $[CH_3-CH_2-CH_2-CH_2-C(=O)]_2O$, $[CH_3-CH_2-CH(CH_3)-C(=O)]_2O$, $[(CH_3)_2CH-CH_2-C(=O)]_2O$, $[(CH_3)_3C-C(=O)]_2O$, $[CH_2=CH-C(=O)]_2O$, $[CH_2=CH-CH_2-C(=O)]_2O$, [Cyclopropyl-$C(=O)]_2O$, $CH_2=CH-C(=O)-OH$, $CH_3-CH=CH-C(=O)-OH$, $(CH_3)_2C=CH-C(=O)-OH$, $CH_3-CH_2-CH=CH-C(=O)-OH$, $CH_3-CH=C(CH_3)-C(=O)-OH$, $CH_2=CH-C(=O)-O-CH_3$, $CH_3-CH=CH-C(=O)-O-CH_3$, $(CH_3)_2C=CH-C(=O)-O-CH_3$, $CH_3-CH_2-CH=CH-C(=O)-O-CH_3$, $CH_3-CH=C(CH_3)-C(=O)-O-CH_3$, $CH_2=CH-C(=O)-O-CH_2-CH_3$, $CH_3-CH=CH-C(=O)-O-CH_2-CH_3$, $(CH_3)_2C=CH-C(=O)-O-CH_2-CH_3$, $CH_3-CH_2-CH=CH-C(=O)-O-CH_2-CH_3$, $CH_3-CH=C(CH_3)-C(=O)O-CH_2-CH_3$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, $Cl-CH_2-CH_2-OH$, $(CH_3-O)_2S(=O)_2$, $(CH_3-CH_2-O)_2S(=O)_2$, $(CH_3-O)_2P(=O)Cl$, $(CH_3-CH_2-O)_2P(=O)Cl$, $[(CH_3)_2CH-O]_2P(=O)Cl$, $NH_3$, $CH_3-NH_2$, $(CH_3)_2NH$, $(CH_3)_3N$, $CH_3-CH_2-NH_2$, $(CH_3-CH_2)_2NH$, $(CH_3-CH_2)_3N$, $[Cl-CH_2-CH_2-N(CH_3)_3]^+Cl^-$, $[Cl-CH_2-CH_2-N(CH_2-CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

In einer weiteren bevorzugten Ausführungsform wird wenigstens eine Cyclodextrin-Verbindung, vorzugsweise nur eine Cyclodextrin-Verbindung gemischt mit nur einem Vernetzungsmittel ausgewählt aus der Gruppe bestehend aus Epichlorhydrin, 1,2,3,4-Diepoxybutan, 4-Chlor-1,2-epoxybutan, Tetramethylendiisocyanat und Hexamethylendiisocyanat und nur einem Funktionalisierungsmittel, ggf. in Gegenwart wenigstens eines Lösungsmittels; wobei das Funktionalisierungsmittel ausgewählt ist aus der Gruppe bestehend aus $CH_3-Y^1$, $CH_3-CH_2-Y^1$, Cyclopropyl-$Y^1$, $Y^1-CH_2-C(=O)-OH$, $Y^1-CH_2-C(=O)-O-CH_3$, $Y^1-CH_2-C(=O)-O-CH_2-CH_3$, $CH_3-C(=O)Y^2$, Dihydrofuran-2,5-dion, Furan-2,5-dion, Dihydro-2H-pyran-2,6(3H)-dion, $[CH_3-C(=O)]_2O$, [Cyclopropyl-$C(=O)]_2O$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, $Cl-CH_2-CH_2-OH$, $(CH_3-O)_2S(=O)_2$, $(CH_3-CH_2-O)_2S(=O)_2$, $CH_3-NH_2$, $(CH_3)_2NH$, $(CH_3)_3N$, $CH_3-CH_2-NH_2$, $(CH_3-CH_2)_2NH$, $(CH_3-CH_2)_3N$, $[Cl-CH_2-CH_2-N(CH_3)_3]^+Cl^-$, $[Cl-CH_2-CH_2-N(CH_2-CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und $Y^2$ vorzugsweise für -Cl, -Br oder -I, besonders bevorzugt für -Cl oder -Br und insbesondere für -Cl steht.

In einer weiteren bevorzugten Ausführungsform wird eine Cyclodextrin-Verbindung gemischt mit dem Vernetzungsmittel Epichlorhydrin und keinem weiteren Vernetzungsmittel oder mit dem Vernetzungsmittel 1,2,3,4-Diepoxybutan und keinem weiteren Vernetzungsmittel und nur einem Funktionalisierungsmittel, ggf. in Gegenwart wenigstens eines Lösungsmittels; wobei das Funktionalisierungsmittel ausgewählt ist aus der Gruppe bestehend aus $CH_3$-$Y^1$, $CH_3$-$CH_2$-$Y^1$, Cyclopropyl-$Y^1$, $Y^1$-$CH_2$-C(=O)-OH, $Y^1$-$CH_2$-C(=O)-O-$CH_3$, $Y^1$-$CH_2$-C(=O)-O-$CH_2$-$CH_3$, $CH_3$-C(=O)$Y^2$, Dihydrofuran-2,5-dion, Furan-2,5-dion, Dihydro-2H-pyran-2,6(3H)-dion, [$CH_3$-C(=O)]$_2$O, [Cyclopropyl-C(=O)]$_2$O, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, ($CH_3$-O)$_2$S(=O)$_2$, ($CH_3$-$CH_2$-O)$_2$S(=O)$_2$, $CH_3$-$NH_2$, $(CH_3)_2NH$, $(CH_3)_3N$, $CH_3$-$CH_2$-$NH_2$, ($CH_3$-$CH_2$)$_2$NH, ($CH_3$-$CH_2$)$_3$N, [Cl-$CH_2$-$CH_2$-N($CH_3$)$_3$]$^+$$Cl^-$, [Cl-$CH_2$-$CH_2$-N($CH_2$-$CH_3$)$_3$]$^+$$Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

In einer besonders bevorzugten Ausführungsform sind die erfindungsgemäßen Copolymere erhältlich durch Mischen von

wenigstens einer, vorzugsweise nur einer Cyclodextrin-Verbindungen ausgewählt aus der Gruppe bestehend aus (Glucosyl)-Cyclodextrin; (Galactosyl)-Cyclodextrin; (Maltosyl)-Cyclodextrin; (Maltriosyl)-Cyclodextrin; (Acetyl)-Cyclodextrin; (Propionyl)-Cyclodextrin; (Isobutyryl)-Cyclodextrin; (Butyryl)-Cyclodextrin; (Pivaloyl)-Cyclodextrin; (Pentanoyl)-Cyclodextrin; (Hexanoyl)-Cyclodextrin; (Cyclopropancarbonyl)-Cyclodextrin; (Benzoyl)-Cyclodextrin; (2-Phenylacetyl)-Cyclodextrin; (2-Carboxyacetyl)-Cyclodextrin; (3-Carboxypropanoyl)-Cyclodextrin; (4-Carboxybutanoyl)-Cyclodextrin; (2-Methoxy-2-oxoacetyl)-Cyclodextrin; (3-Methoxy-3-oxopropanoyl)-Cyclodextrin; (4-Methoxy-4-oxobutanoyl)-Cyclodextrin; (2-Hydroxyacetyl)-Cyclodextrin; (2-Hydroxypropanoyl)-Cyclodextrin; (3-Hydroxypropanoyl)-Cyclodextrin; (2-Hydroxybutanoyl)-Cyclodextrin; (3-Hydroxybutanoyl)-Cyclodextrin; (4-Hydroxybutanoyl)-Cyclodextrin; (2-Hydroxypentanoyl)-Cyclodextrin; (3-Hydroxypentanoyl)-Cyclodextrin; (4-Hydroxypentanoyl)-Cyclodextrin; (5-Hydroxypentanoyl)-Cyclodextrin; (2,3-Dihydroxypropanoyl)-Cyclodextrin; (2,3-Dihydroxybutanoyl)-Cyclodextrin; (2,4-Dihydroxybutanoyl)-Cyclodextrin; (3,4-Dihydroxybutanoyl)-Cyclodextrin; (2,3-Dihydroxypentanoyl)-Cyclodextrin; (2,4-Dihydroxypentanoyl)-Cyclodextrin; (2,5-Dihydroxypentanoyl)-Cyclodextrin; (3,4-Dihydroxypentanoyl)-Cyclodextrin; (3,5-Dihydroxypentanoyl)-Cyclodextrin; (4,5-Dihydroxypentanoyl)-Cyclodextrin; (2,3,4-Trihydroxybutanoyl)-Cyclodextrin; (2,3,4-Trihydroxypentanoyl)-Cyclodextrin; (2,3,5-Trihydroxypentanoyl)-Cyclodextrin; (2,4,5-Trihydroxypentanoyl)-Cyclodextrin; (3,4,5-Trihydroxypentanoyl)-Cyclodextrin; (2,3,4,5-Tetrahydroxypentanoyl)-Cyclodextrin; (2-Aminoacetyl) -Cyclodextrin; (2-Aminopropanoyl)-Cyclodextrin; (2-Aminobutanoyl)-Cyclodextrin; [2-(1*H*-Imidazol-1-yl)acetyl]-Cyclodextrin; [3-(1*H*-Imidazol-1-yl)propanoyl]-Cyclodextrin, [4-(1*H*-Imidazol-1-yl)butanoyl]-Cyclodextrin; [2-(1*H*-Imidazol-1-yl)acetyl]-Cyclodextrin; [3-(1*H*-Imidazol-1-yl)propanoyl]-Cyclodextrin; [4-(1*H*-Imidazol-1-yl)butanoyl]-Cyclodextrin; [2-(Pyridin-3-yl)acetyl]-Cyclodextrin; [3-(Pyridin-3-yl)propanoyl]-Cyclodextrin; [4-(Pyridin-3-yl)butanoyl]-Cyclodextrin; (Methyl)-Cyclodextrin; (Ethyl)-Cyclodextrin; (iso-Propyl)-Cyclodextrin; (n-Propyl)-Cyclodextrin; (*iso*-Butyl)-Cyclodextrin; (n-Butyl)-Cyclodextrin; (sec-Butyl)-Cyclodextrin; (*tert*-Butyl)-Cyclodextrin, (Cyclopropyl)-Cyclodextrin; (Phenyl)-Cyclodextrin; (Benzyl)-Cyclodextrin; (Phenethyl)-Cyclodextrin; (Carboxymethyl)-Cyclodextrin; (1-Carboxyethyl)-Cyclodextrin; (2-Carboxyethyl)-Cyclodextrin; (1-Carboxypropyl)-Cyclodextrin; (2-Carboxypropyl)-Cyclodextrin; (3-Carboxypropyl)-Cyclodextrin; (1-Carboxypropan-2-yl)-Cyclodextrin; (4-Carboxybutyl)-Cyclodextrin; (5-Carboxypentyl)-Cyclodextrin; (5-Carboxypentyl)-Cyclodextrin; (2-Methoxy-2-oxoethyl)-Cyclodextrin; (3-Methoxy-2-oxopropyl)-Cyclodextrin; (4-Methoxy-4-oxobutyl)-Cyclodextrin; (5-Methoxy-5-oxopentyl)-Cyclodextrin; (6-Methoxy-6-oxohexyl)-Cyclodextrin; (2-Ethoxy-2-oxoethyl)-Cyclodextrin; (3-Ethoxy-2-oxopropyl)-Cyclodextrin; (4-Ethoxy-4-oxobutyl)-Cyclodextrin; (5-Ethoxy-5-oxopentyl)-Cyclodextrin; (6-Ethoxy-6-oxohexyl)-Cyclodextrin; (3-Carboxy-2-hydroxypropyl)-Cyclodextrin; (2-Hydroxy-4-methoxy-4-oxobutyl)-Cyclodextrin; (4-Ethoxy-2-hydroxy-4-oxobutyl)-Cyclodextrin; (1-Carboxy-2-hydroxyethyl)-Cyclodextrin; (3-Hydroxy-1-methoxy-1-oxopropan-2-yl)-Cyclodextrin; (1-Ethoxy-3-hydroxy-1-oxopropan-2-yl)-Cyclodextrin; (1-Carboxy-3-hydroxypropan-2-yl)-Cyclodextrin; (1-Hydroxy-4-methoxy-4-oxobutan-2-yl)-Cyclodextrin; (4-Ethoxy-1-hydroxy-4-oxobutan-2-yl)-Cyclodextrin; (Hydroxymethyl)-Cyclodextrin; (1-Hydroxyethyl)-Cyclodextrin; (2-Hydroxyethyl)-Cyclodextrin; (1-Hydroxypropyl)-Cyclodextrin; (2-Hydroxypropyl)-Cyclodextrin; (1-Hydroxypropan-2-yl)-Cyclodextrin; (2-Hydroxypropan-2-yl)-Cyclodextrin; (1-Hydroxybutyl)-Cyclodextrin; (2-Hydroxybutyl)-Cyclodextrin; (3-Hydroxybutyl)-Cyclodextrin; (4-Hydroxybutyl)-Cyclodextrin; (1-Hydroxypentyl)-Cyclodextrin; (2-Hydroxypentyl)-Cyclodextrin; (3-Hydroxypentyl)-Cyclodextrin; (4-Hydroxypentyl)-Cyclodextrin; (5-Hydroxypentyl)-Cyclodextrin; (1,2-Dihydroxyethyl)-Cyclodextrin; (1,2-Dihydroxypropyl)-Cyclodextrin; (1,3-Dihydroxypropyl)-Cyclodextrin; (2,3-Dihydroxypropyl)-Cyclodextrin; (1,2-Dihydroxybutyl)-Cyclodextrin; (1,3-Dihydroxybutyl)-Cyclodextrin; (1,4-Dihydroxybutyl)-Cyclodextrin; (2,3-Dihydroxybutyl)-Cyclodextrin; (2,4-Dihydroxybutyl)-Cyclodextrin; (3,4-Dihydroxybutyl)-Cyclodextrin; (1,2-Dihydroxypentyl)-Cyclodextrin; (1,3-Dihydroxypentyl)-Cyclodextrin; (1,4-Dihydroxypentyl)-Cyclodextrin; (1,5-Dihydroxypentyl)-Cyclodextrin; (2,3-Dihydroxypentyl)-Cyclodextrin; (2,4-Dihydroxypentyl)-Cyclodextrin; (2,5-Dihydroxypentyl)-Cyclodextrin; (3,4-Dihydroxypentyl)-Cyclodextrin; (3,5-Dihydroxypentyl)-Cyclodextrin; (4,5-Dihydroxypentyl)-Cyclodextrin; (1,2,3-Trihydroxypropyl)-Cyclodextrin; (1,2,3-Trihydroxybutyl)-Cyclodextrin; (1,2,4-Trihydroxybutyl)-Cyclodextrin; (1,3,4-Trihydroxybutyl)-Cyclodextrin; (2,3,4-Trihydroxybutyl)-Cyclodextrin; (1,2,3-Trihydroxypentyl)-Cyclodextrin; (1,2,4-Trihydroxypentyl)-Cyclodextrin; (1,2,5-Trihydroxypentyl)-Cyclodextrin; (1,3,4-Trihydroxypentyl)-Cyclodextrin; (1,3,5-Trihydroxypentyl)-Cyclodextrin; (1,4,5-Trihydroxypentyl)-Cyclodextrin; (2,3,4-Trihydroxypentyl)-Cyclodextrin; (2,3,5-Trihydroxypentyl)-Cyclodextrin; (2,4,5-Trihydroxypentyl)-Cyclodextrin; (3,4,5-Trihydroxypentyl)-Cyclodextrin; (1,2,3,4-Tetrahydroxybutyl)-Cyclodextrin; (1,2,3,4-Tetrahydroxypentyl)-Cyclodextrin; (1,2,4,5-Tetrahydroxypentyl)-Cyclodextrin; (2,3,4,5-Tetrahydroxypentyl)-Cyclodextrin; (1,2,3,4,5-Pentahydroxypentyl)-Cyclodextrin; [(Sulfooxy)methyl]-Cyclodextrin; [2-(Sulfooxy)ethyl]-Cyclodextrin; [3-(Sulfooxy)propyl]-Cyclodextrin; [4-(Sulfooxy)butyl]-Cyclodextrin; [(Phosphonooxy)methyl]-Cyclodextrin; [2-(Phosphonooxy)ethyl]-Cyclodextrin; [3-(Phosphonooxy)propyl]-Cyclodextrin; [4-(Phosphonooxy)butyl]-Cyclodextrin; [4-(Phosphonooxy)butyl]-Cyclodextrin; (Sulfomethyl)-Cyclodextrin; (2-Sulfoethyl)-Cyclodextrin; (3-Sulfopropyl)-Cyclodextrin; (4-Sulfobutyl)-Cyclodextrin; (5-Sulfopentyl)-Cyclodextrin; (6-Sulfohexyl)-Cyclodextrin; (Phosphonomethyl)-Cyclodextrin; (2-Phosphonoethyl)-Cyclodextrin; (3-Phosphonopropyl)-Cyclodextrin; (4-Phosphonobutyl)-Cyclodextrin; (5-Phosphonopentyl)-Cyclodextrin; (6-Phosphonohexyl)-Cyclodextrin; (2-Phosphonovinyl)-Cyclodextrin; (3-Phosphonoallyl)-Cyclodextrin; (4-Phosphonobut-3-enyl)-Cyclodextrin; (5-Phosphonopent-4-enyl)-Cyclodextrin; (6-Phosphonohex-5-enyl)-Cyclodextrin; (Aminomethyl)-Cyclodextrin; (2-Aminoethyl)-Cyclodextrin; (3-Aminopropyl)-Cyclodextrin; (4-Aminobutyl)-Cyclodextrin; (5-Aminopentyl)-Cyclodextrin; (6-Aminohexyl)-Cyclodextrin; [(*N,N*-Dimethylamino)methyl]-Cyclodextrin; [2-(*N,N*-Dimethylamino)ethyl]-Cyclodextrin; [3-(*N,N*-Dimethylamino)propyl]-Cyclodextrin; [4-(*N,N*-Dimethylamino)butyl]-Cyclodextrin; [5-(*N,N*-Dimethylamino)pentyl]-Cyclodextrin; [6-(*N,N*-Dimethylamino) hexyl]-Cyclodextrin; [(*N,N*-Diethylamino)methyl]-Cyclodextrin; [2-(*N,N*-Diethylamino)ethyl]-Cyclodextrin; [3-(*N,N*-Diethylamino)propyl]-Cyclodextrin; [4-(*N,N*-Diethylamino)butyl]-Cyclodextrin; [5-(*N,N*-Diethylamino)pentyl]-Cyclodextrin; [6-(*N,N*-Diethylamino)hexyl]-Cyclodextrin; [(Trimethylammonio)methyl]-Cyclodextrin Chlorid; [2-(Trimethylammonio)ethyl]-Cyclodextrin Chlorid; [3-(Trimethylammonio)propyl]-Cyclodextrin Chlorid; [4-(Trimethylammonio)butyl]-Cyclodextrin Chlorid; [5-(Trimethylammonio)pentyl]-Cyclodextrin Chlorid; [6-(Trimethylammonio)hexyl]-Cyclodextrin Chlorid; [(Triethylammonio)methyl]-Cyclodextrin Chlorid; [2-(Triethylammonio)ethyl]-Cyclodextrin Chlorid; [3-(Triethylammonio)propyl]-Cyclodextrin Chlorid; [4-(Triethylammonio)butyl]-Cyclodextrin Chlorid; [5-(Triethylammonio)pentyl]-Cyclodextrin Chlorid; [6-(Triethylammonio)hexyl]-Cyclodextrin Chlorid; [(1*H*-Imidazol-1-yl)methyl]-Cyclodextrin; [2-(1*H*-Imidazol-1-yl)ethyl]-Cyclodextrin; [3-(1*H*-Imidazol-1-yl)propyl]-Cyclodextrin; [4-(1*H*-Imidazol-1-yl)butyl]-Cyclodextrin; (Pyridin-3-ylmethyl)-Cyclodextrin; [2-(Pyridin-3-yl)ethyl]-Cyclodextrin; [3-(Pyridin-3-yl)propyl]-Cyclodextrin; [4-(Pyridin-3-yl)butyl]-Cyclodextrin; [($\beta$-D-Glucopyranosyloxyuronsäure) methyl]-Cyclodextrin; [2-($\beta$-D-Glucopyranosyloxyuronsäure)ethyl]-Cyclodextrin; [3-($\beta$-D-Glucopyranosyloxyuronsäure)propyl]-Cyclodextrin; [4-($\beta$-D-Glucopyranosyloxyuronsäure)butyl]-Cyclodextrin; [5-($\beta$-D-Glucopyranosyloxyuronsäure)pentyl]-Cyclodextrin; und [6-($\beta$-D-Glucopyranosyloxyuronsäure)hexyl]-Cyclodextrin; wobei der Ausdruck "Cyclodextrin" für $\alpha$-, $\beta$- oder $\gamma$-Cyclodextrin, vorzugsweise für $\alpha$- oder $\beta$-Cyclodextrin und insbesondere für $\beta$-Cyclodextrin steht;
mit wenigstens einem, vorzugsweise nur einem Vernetzungsmittel ausgewählt aus der Gruppe bestehend aus Epichlorhydrin, 4-Chlor-1,2-epoxybutan, 1,2,3,4-Diepoxybutan, Tetramethylendiisocyanat und Hexamethylendiisocyanat; wobei das Vernetzungsmittel Epichlorhydrin insbesondere bevorzugt ist; und
mit wenigstens einem, vorzugsweise nur einem Funktionalisierungsmittel ausgewählt aus der Gruppe bestehend aus $CH_3$-$Y^1$, $CH_3$-$CH_2$-$Y^1$, Cyclopropyl-$Y^1$, $Y^1$-$CH_2$-C(=O)-OH, $Y^1$-$CH_2$-C(=O)-O-$CH_3$, $Y^1$-$CH_2$-C(=O)-O-$CH_2$-$CH_3$, $CH_3$-C(=O)$Y^2$, Dihydrofuran-2,5-dion, Furan-2,5-dion, Dihydro-2H-pyran-2,6(3H)-dion, $[CH_3\text{-}C(=O)]_2O$, $[\text{Cyclopropyl-}C(=O)]_2O$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, $(CH_3\text{-}O)_2S(=O)_2$, $(CH_3\text{-}CH_2\text{-}O)_2S(=O)_2$, $CH_3$-$NH_2$, $(CH_3)_2NH$, $(CH_3)_3N$, $CH_3$-$CH_2$-$NH_2$, $(CH_3\text{-}CH_2)_2NH$, $(CH_3\text{-}CH_2)_3N$, $[Cl\text{-}CH_2\text{-}CH_2\text{-}N(CH_3)_3]^+Cl^-$, $[Cl\text{-}CH_2\text{-}CH_2\text{-}N(CH_2\text{-}CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und $Y^2$ vorzugsweise für -Cl, -Br oder -I, besonders bevorzugt für -Cl oder -Br und insbesondere für -Cl steht;
ggf. in Gegenwart wenigstens eines Lösungsmittels.

In einer weiteren besonders bevorzugten Ausführungsform wird unsubstituiertes $\alpha$-. $\beta$- oder $\gamma$-Cydodextrin mit dem Vernetzungsmittel Epichlorhydrin und dem Funktionalisierungsmittel 1,3-Butansulton, 1,4-Butansulton, 2-Chloressigsäure, 2-Chloressigsäuremethylester oder 2-Chloressigsäureethylester umgesetzt; ggf. in Gegenwart eines Lösungsmittels.

Das Mischen der Cyclodextrin-Verbindungen mit Vernetzungsmitteln und Funktionalisierungsmitteln kann in wässrigen oder organischen Lösungsmitteln erfolgen. Vorzugsweise erfolgt das Mischen in Wasser, Dimethylsulfoxid (DMSO), Dimethylformamid (DMF), N-Methyl-2-pyrrolidon (NMP) oder Mischungen davon. Die Umsetzung kann auch in wasserfreiem DMSO, wasserfreiem DMF, wasserfreiem NMP oder Mischungen davon unter vorzugsweise wasserfreien Bedingungen durchgeführt werden. Als Lösungsmittel besonders vorteilhaft sind Wasser, DMSO und DMF sowie Mischungen davon; insbesondere Wasser oder DMF sowie Mischungen davon.

Das Mischen erfolgt vorzugsweise im alkalischen Milieu, in der Regel bei einem pH-Wert > 7.

Das Mischen erfolgt vorzugsweise bei $7{,}5 \leq pH \leq 14$, bevorzugter bei $7{,}5 \leq pH \leq 13$, noch bevorzugter bei $8 \leq$

pH ≤ 13, am Bevorzugtesten bei 8 ≤ pH ≤ 12,5 und insbesondere bei 9 ≤ pH ≤ 12.

Die Einstellung des pH-Wertes der Reaktionslösung oder -dispersion erfolgt vorzugsweise mit Basen, wie beispielsweise basischen Alkalimetallsalzen, basischen Erdalkalimetallsalzen, Hydriden, Alkoxiden oder organischen Basen wie beispielsweise Pyridin oder Diisopropylamin. Besonders vorteilhaft sind Natriumhydroxid, Natriumhydrogencarbonat, Natriumcarbonat, Kaliumhydroxid, Kaliumhydrogencarbonat, Kaliumcarbonat, Natriumhydrid, Kaliumhydrid, Natriummethanolat, Kalium-tert-butoxid, Pyridin und Diisopropylamin.

Da die Copolymerisierung bzw. Vernetzung der Cyclodextrin-Verbindungen unter basischen Reaktionsbedingungen erfolgt, können Substituenten, die im alkalischen Milieu nicht stabil sind, strukturell verändert werden. Diese Veränderung kann basenlabile Gruppen in Cyclodextrin-Verbindungen, Vernetzungsmitteln und Funktionalisierungsmitteln betreffen. Beispielsweise kann die Ester-Gruppe der Cyclodextrin-Verbindung (2-Methoxy-2-oxoethyl)-Cyclodextrin zu einer Carboxyl- bzw. Carboxylat-Gruppe gespalten werden, wobei die Ausgangsverbindung (2-Methoxy-2-oxoethyl)-Cyclodextrin zu (Carboxymethyl)-Cyclodextrin umgewandelt wird. Diese Umwandlung kann, bezogen auf die Gesamtanzahl der Ester-Gruppen, vollständig oder unvollständig erfolgen. Folglich können im entsprechenden erfindungsgemäßen Copolymer alle oder nur ein Teil der Ester- zu Carboxyl-Gruppen umgewandelt sein.

In einer bevorzugten Ausführungsform werden die Cyclodextrin-Verbindungen in einem Lösungsmittel oder Lösungsmittelgemisch gelöst oder dispergiert und anschließend werden vorzugsweise unter Rühren der Dispersion oder Lösung die Vernetzungsmittel und die Funktionalisierungsmittel darin gelöst oder dispergiert. Die Zugabe der Vernetzungsmittel und der Funktionalisierungsmittel kann in beliebiger Reihenfolge oder gleichzeitig, auf einmal oder sukzessive in Portionen erfolgen. Die Vernetzungsmittel und/ oder Funktionalisierungsmittel können vor der Zugabe in einem Lösungsmittel (z.B. Wasser, DMF, DMSO, NMP) gelöst oder dispergiert werden und als Lösung oder Dispersion dem Reaktionsgemisch auf einmal oder sukzessive in Portionen zugegeben oder mittels Tropftrichter, Pipette oder Spritze zugetropft werden. Die Zugabe bzw. Zutropfung kann mit Hilfe einer Spritzenpumpe erfolgen.

In einer weiteren bevorzugten Ausführungsform werden die Vernetzungsmittel und Funktionalisierungsmittel in einer beliebigen Reihenfolge oder gleichzeitig in einem Lösungsmittel oder Lösungsmittelgemisch gelöst oder dispergiert und anschließend werden, vorzugsweise unter Rühren der Lösung oder Dispersion, die Cyclodextrin-Verbindungen in dieser Lösung oder Dispersion gelöst oder dispergiert. Die Zugabe der Cyclodextrin-Verbindungen kann auf einmal oder sukzessive in Portionen erfolgen. Die Cyclodextrin-Verbindungen können vor der Zugabe in einem Lösungsmittel gelöst oder dispergiert werden und als Lösung oder Dispersion dem Reaktionsgemisch auf einmal oder sukzessive in Portionen zugegeben oder mittels Tropftrichter, Pipette oder Spritze zugetropft werden. Die Zugabe bzw. Zutropfung kann mit Hilfe einer Spritzenpumpe erfolgen.

In einer weiteren bevorzugten Ausführungsform werden die Vernetzungsmittel, Funktionalisierungsmittel und Cyclodextrin-Verbindungen gleichzeitig in einem Lösungsmittel oder Lösungsmittelgemisch gelöst oder dispergiert.

In einer besonders bevorzugten Ausführungsform werden zunächst unsubstituierte oder substituierte Cyclodextrin-Verbindungen mit Vernetzungsmitteln in Gegenwart eines Lösungsmittels gemischt und zu vernetzten Copolymeren umgesetzt. Die dabei entstehenden vernetzten Copolymere können nach der Vernetzung aufgereinigt und isoliert werden. Anschließend kann das Funktionalisierungsmittel mit dem aufgereinigten oder isolierten Copolymer in Gegenwart eines Lösungsmittels gemischt und umgesetzt werden; oder das Funktionalisierungsmittel wird mit dem Reaktionsgemisch, das die nicht-isolierten bzw. nicht-aufgereinigten Copolymere enthält, gemischt und umgesetzt.

In einer weiteren besonders bevorzugten Ausführungsform werden zunächst unsubstituierte oder substituierte Cyclodextrin-Verbindungen mit Funktionalisierungsmitteln in Gegenwart eines Lösungsmittels gemischt und zu substituierten Cyclodextrin-Verbindungen umgesetzt. Die dabei entstehenden substituierten Cyclodextrin-Verbindungen können nach der Vernetzung aufgereinigt und isoliert werden. Anschließend kann das Vernetzungsmittelmittel mit dem aufgereinigten oder isolierten substituierten Cyclodextrin-Verbindungen in Gegenwart eines Lösungsmittels gemischt und umgesetzt werden; oder das Vernetzungsmittel wird mit dem Reaktionsgemisch, das die nicht-isolierten bzw. nicht-aufgereinigten substituierten Cyclodextrin-Verbindungen enthält, gemischt und umgesetzt.

Beim Mischen kann es zu einem plötzlichen und unerwünschten Anstieg der Reaktionstemperatur kommen. Deshalb ist es vorteilhaft, das Reaktionsgemisch ggf. zu kühlen, um eine im Wesentlichen konstante Reaktionstemperatur zu gewährleisten. Die Kühlung des Reaktionsgemisches kann über dem Eisbad oder Wasserbad erfolgen.

Die Alkalisierung des Reaktionsgemisches kann vor, während oder nach Zugabe der Vernetzungsmittel, vor, während oder nach Zugabe der Cyclodextrin-Verbindungen, vor, während oder nach der Zugabe der Funktionalisierungsmittel oder vor, während oder nach Zugabe einer Mischung davon erfolgen.

Die Messung des alkalischen pH-Wertes erfolgt vorzugsweise mit Hilfe von pH-Papier oder einem pH-Meter. In einer bevorzugten Ausführungsform wird der pH-Wert während der Umsetzung fortlaufend oder in bestimmten Zeitintervallen gemessen. Falls der pH-Wert unter einen bestimmten Wert fällt (z.B. pH < 8 oder < 7,5), kann im Reaktionsgemisch ein alkalischer pH-Wert durch Basenzugabe wieder hergestellt werden.

Die Umsetzung erfolgt vorzugsweise bei einer Reaktionstemperatur (Temperatur des Reaktionsgemisches) von 5,0 bis 90 °C, bevorzugter 10 bis 75 °C, noch bevorzugter 15 bis 50 °C, am Bevorzugtesten 15 bis 30 °C und insbesondere 20 bis 25 °C. Nach dem Mischen der Cyclodextrin-Verbindungen mit den Vernetzungs- und Funktionalisierungsmitteln, ggf. in Gegenwart Lösungsmittels oder Lösungsmittelgemisches, wird die Temperatur des Reaktionsgemisches vorzugsweise auf 5 bis 90 °C, bevorzugter auf 10 bis 75 °C, noch bevorzugter auf 15 bis 50 °C, am Bevorzugtesten auf 15 bis 30 °C und insbesondere auf 20 bis 25 °C eingestellt.

In einer bevorzugten Ausführungsform wird eine Gesamtstoffmenge von ≥ 0,5 mol, ≥ 1,0 mol, ≥ 2,0 mol oder ≥ 3,0 mol, bevorzugter ≥ 4,0 mol, ≥ 5,0 mol oder ≥ 6,0 mol, noch bevorzugter ≥ 7,0 mol, ≥ 8,0 mol oder ≥ 9,0 mol, am Bevorzugtesten ≥ 10 mol, ≥ 15 mol oder ≥ 20 mol, und insbesondere ≥ 25 mol an Vernetzungsmittel bezogen auf eine Gesamtstoffmenge von 1,0 mol an Cyclodextrin-Verbindungen in das Reaktionsgemisch eingesetzt (Äquivalente).

In einer weiteren bevorzugten Ausführungsform wird eine Gesamtstoffmenge von ≥ 0,5 mol, ≥ 1,0 mol, ≥ 2,0 mol oder ≥ 3,0 mol, bevorzugter ≥ 4,0 mol, ≥ 5,0 mol oder ≥ 6,0 mol, noch bevorzugter ≥ 7,0 mol, ≥ 8,0 mol oder ≥ 9,0 mol, am Bevorzugtesten ≥ 10 mol, ≥ 15 mol oder ≥ 20 mol, und insbesondere ≥ 25 mol an Funktionalisierungsmittel bezogen auf eine Gesamtstoffmenge von 1,0 mol an Cyclodextrin-Verbindungen in das Reaktionsgemisch eingesetzt (Äquivalente).

In einer weiteren bevorzugten Ausführungsform ist das Verhältnis der Gesamtstoffmenge an Vernetzungsmitteln zu der Gesamtstoffmenge an Cyclodextrin-Verbindungen ≥ 1 : 1, bevorzugter ≥ 2 : 1, noch bevorzugter ≥ 3 : 1, am Bevorzugtesten ≥ 5 : 1 und insbesondere ≥ 10 : 1.

In einer weiteren bevorzugten Ausführungsform ist das Verhältnis der Gesamtstoffmenge an Funktionalisierungsmitteln zu der Gesamtstoffmenge an Cyclodextrin-Verbindungen ≥ 1 : 1, bevorzugter ≥ 2 : 1, noch bevorzugter ≥ 3 : 1, am Bevorzugtesten ≥ 5 : 1 und insbesondere ≥ 10 : 1.

In einer weiteren bevorzugten Ausführungsform ist das Verhältnis der Gesamtstoffmenge an Funktionalisierungsmitteln zu der Gesamtstoffmenge an Vernetzungsmitteln ≥ 1 : 1, bevorzugter ≥ 2 : 1, noch bevorzugter ≥ 3 : 1, am Bevorzugtesten ≥ 5 : 1 und insbesondere ≥ 10 : 1.

Die Umsetzung (Vernetzungsreaktion) kann mittels geeigneter analytischer Methoden verfolgt werden. Beispielsweise können zu bestimmten Zeitpunkten Stichproben des Reaktionsgemisches genommen werden und mittels LC/MS oder NMR analysiert werden. Auf diese Weise kann das im Reaktionsgemisch zu einem bestimmten Zeitpunkt vorliegende Reaktionsprodukt bestimmt werden. Falls die Stichprobenanalyse ergibt, dass das gewünschte Reaktionsprodukt vorliegt, so kann die Umsetzung beendet und das gewünschte Reaktionsprodukt isoliert bzw. aufgereinigt werden.

Zur Beendigung der Umsetzung wird das Reaktionsgemisch vorzugsweise neutralisiert, d.h. der pH-Wert der Reaktionslösung oder -dispersion wird vorzugsweise auf 6,5 ≤ pH ≤ 7,5, besonders bevorzugt auf 6,8 ≤ pH ≤ 7,2 eingestellt. Die Neutraliserung erfolgt vorzugsweise mit organischen (z.B. Trifluoressigsäure, Essigsäure) oder anorganischen Säuren (z.B. Salzsäure, Schwefelsäure, Salpetersäure).

In einer weiteren bevorzugten Ausführungsform wird das Reaktionsgemisch nach der Umsetzung bzw. Neutralisierung dialysiert, vorzugsweise salzfrei dialysiert, wobei die in diesem Dialyseverfahren eingesetzte Dialysemembran einen MWCO ("Molecular Weight Cut-Off') von vorzugsweise 0,50 kDa oder 1,0 kDa, bevorzugter von 2,0 kDa oder 3,0 kDa, noch bevorzugter von 4,0 kDa oder 5,0 kDa, am Bevorzugtesten von 10 kDa und insbesondere von ≥ 15 kDa aufweist.

Vorzugsweise hat das salzfrei dialysierte erfindungsgemäße Copolymer einen Restgehalt von ≤ 10 Gew.-%, bevorzugter ≤ 5,0 Gew.-%, noch bevorzugter ≤ 2,5 Gew.-% und insbesondere ≤ 1,0 Gew.-% an Verbindungen mit einem Molekulargewicht kleiner als 1,5 kDa, bezogen auf die Gesamtmasse an erfindungsgemäßen Copolymer.

Das Reaktionsgemisch bzw. die im Wesentlichen salzfreie Dialyselösung kann vor der weiteren Aufreinigung bzw. Isolierung vorzugsweise mit Hilfe eines Rotationsverdampfers eingeengt werden. Eine Einengung des Reaktionsgemisches kann auch bereits vor der Dialyse, vorzugsweise salzfreien Dialyse erfolgen.

Die Isolierung oder Aufreinigung der erfindungsgemäßen Copolymere erfolgt vorzugsweise mittels Ultraflitration, Säulenchromatographie (z.B. an Kieselgel oder Aktivkohle), präparativer HPLC oder Rekristallisation. Das erhaltene erfindungsgemäße Copolymer kann lyophilisiert werden.

In einer bevorzugten Ausführungsform beträgt der Stoffmengenanteil der im erfindungsgemäßen Copolymer enthaltenen Linker 20 bis 95 mol%, bevorzugter 25 bis 80 mol%, noch bevorzugter 30 bis 70 mol%, am Bevorzugtesten 30 bis 60 mol% und insbesondere 30 bis 50 mol%, bezogen auf die Gesamtstoffmenge der Monomere, die im erfindungsgemäßen Copolymer enthalten sind.

Der Stoffmengenanteil der im erfindungsgemäßen Copolymer enthaltenen Cyclodextrin-Monomere beträgt vorzugsweise 5,0 bis 80 mol%, bevorzugter 10 bis 75 mol%, noch bevorzugter 20 bis 70 mol%, am Bevorzugtesten 30 bis 70 mol% und insbesondere 40 bis 70 mol%, bezogen auf die Gesamtstoffmenge der Monomere, die im erfindungsgemäßen Copolymer enthalten sind.

In einer weiteren bevorzugten Ausführungsform ist die Summe aus dem Stoffmengenanteil der Linker und dem Stoffmengenanteil der Cyclodextrin-Monomere ≥ 25 mol%, ≥ 30 mol% oder ≥ 40 mol%, bevorzugter ≥ 50 mol% oder ≥ 60 mol%, noch bevorzugter ≥ 70 mol% oder ≥ 80 mol%, am Bevorzugtesten ≥ 90 mol% oder ≥ 95 mol%, und insbesondere 100 mol%, bezogen auf die Gesamtstoffmenge der Monomere, die im erfindungsgemäßen Copolymer enthalten sind.

Die Wasserlöslichkeit der erfindungsgemäßen Copolymere ist bei 23 °C vorzugsweise ≥ 5,0 g $l^{-1}$, ≥ 10 g $l^{-1}$, ≥

25 g $l^{-1}$ oder ≥ 50 g $l^{-1}$, bevorzugter ≥ 75 g $l^{-1}$, ≥ 100 g $l^{-1}$, ≥ 125 g $l^{-1}$ oder ≥ 150 g $l^{-1}$, noch bevorzugter ≥ 175 g $l^{-1}$, ≥ 200 g $l^{-1}$, ≥ 225 g $l^{-1}$ oder ≥ 250 g $l^{-1}$, am Bevorzugtesten ≥ 300 g $l^{-1}$ oder ≥ 400 g $l^{-1}$ und insbesondere ≥ 500 g $l^{-1}$.

Die Osmolalität einer 50 mM wässrigen Lösung an erfindungsgemäßen Copolymer ist vorzugsweise ≥ 50 mosm $kg^{-1}$, ≥ 75 mosm $kg^{-1}$ oder ≥ 100 mosm $kg^{-1}$, bevorzugter ≥ 125 mosm $kg^{-1}$, ≥ 150 mosm $kg^{-1}$ oder ≥ 175 mosm $kg^{-1}$, noch bevorzugter ≥ 200 mosm $kg^{-1}$ oder ≥ 225 mosm $kg^{-1}$, am Bevorzugtesten ≥ 250 mosm $kg^{-1}$ oder ≥ 275 mosm $kg^{-1}$ und insbesondere ≥ 300 mosm $kg^{-1}$.

Der Polymerisationsgrad der erfindungsgemäßen Copolymere ist vorzugsweise ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

Das mittlere Molekulargewicht der erfindungsgemäßen Copolymere ist vorzugsweise ≥ 2,0 kDa, bevorzugter ≥ 2,5 kDa, noch bevorzugter ≥ 3,0 kDa, am Bevorzugtesten ≥ 4,0 kDa und insbesondere ≥ 5,0 kDa. Das mittlere Molekulargewicht wird vorzugsweise mit Hilfe eines Membranosmometers gemessen (destilliertes Wasser, 23 °C).

In einer weiteren bevorzugten Ausführungsform ist das mittleres Molekulargewicht (M) der erfindungsgemäßen Polymere vorzugsweise 2,0 ≤ M ≤ 100 kDa, bevorzugter 2,0 ≤ M ≤ 50 kDa, noch bevorzugter 2,0 ≤ M ≤ 25 kDa, am Bevorzugtesten 2,0 ≤ M ≤ 15 kDa und insbesondere 2,0 ≤ M ≤ 10 kDa.

In einer weiteren besonders bevorzugten Ausführungsform hat das erfindungsgemäße Copolymer, das durch das Mischen wenigstens einer Cyclodextrin-Verbindung mit wenigstens einem Vernetzungsmittel und wenigstens einem Funktionalisierungsmittel erhältlich ist, vorzugsweise einen Polymerisationsgrad von ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

In einer weiteren bevorzugten Ausführungsform ist der mittlere Partikeldurchmesser der erfindungsgemäßen Copolymere, die durch das Mischen wenigstens einer Cyclodextrin-Verbindung mit wenigstens einem Vernetzungsmittel und wenigstens einem Funktionalisierungsmittel erhältlich sind, ≤ 1000 μm, ≤ 900 μm oder ≤ 800 μm, bevorzugter ≤ 700 μm, ≤ 600 μm oder ≤ 500 μm, noch bevorzugter ≤ 400 μm, ≤ 300 μm oder ≤ 200 μm, am Bevorzugtesten ≤ 100 μm, ≤ 75 μm oder ≤ 50 μm und insbesondere ≤ 25 μm.

Ein weiterer bevorzugter Gegenstand dieser Erfindung sind Copolymere erhältlich durch ein Verfahren umfassend den Verfahrensschritt

Mischen wenigstens eines Cyclodextrin-Polymers mit wenigstens einem Funktionalisierungsmittel;

zur Verwendung in der Behandlung von Krankheiten des Urogenitalsystems.

Für die Zwecke dieser Erfindung umfasst der Ausdruck "Cyclodextrin-Polymer" unsubstituierte oder ein- oder mehrfach substituierte Copolymere, die wie oben stehend definiert sind, d.h. die wenigstens zwei Cyclodextrin-Monomere und wenigstens einen Linker enthalten; wobei die substituierten Cyclodextrin-Polymere wenigstens ein unsubstituiertes -OH, wenigstens ein unsubstituiertes -SH oder wenigstens eine primäre, sekundäre oder tertiäre Aminogruppe enthalten.

Im Sinne dieser Erfindung kann "das wenigstens eine unsubstituierte -OH" Bestandteil der Glucosyleinheiten der Cyclodextrin-Monomere, der Linker, der Substituenten der Cyclodextrin-Monomere und/ oder der Substituenten Linker sein. "Das wenigstens eine -SH" oder "die wenigstens eine primäre, sekundäre oder tertiäre Aminogruppe" sind für die Zwecke dieser Erfindung vorzugsweise Bestandteile der Substituenten der Cyclodextrin-Monomere oder der Substituenten der Linker. Die in den Cyclodextrin-Polymeren enthaltenen Cyclodextrin-Monomere und Linker sowie deren bevorzugte Ausführungsformen sind wie oben stehend definiert.

Ferner umfasst der Ausdruck "Cyclodextrin-Polymer" für die Zecke dieser Erfindung Copolymere, die erhältlich sind nach dem vorstehend definierten Verfahren, d.h. durch das Verfahren umfassend den Verfahrensschritt

Mischen wenigstens einer Cyclodextrin-Verbindung mit

(a) wenigstens einem Vernetzungsmittel oder
(b) wenigstens einem Vernetzungsmittel und wenigstens einem Funktionalisierungsmittel;

einschließlich der vorstehend definierten bevorzugten Ausführungsformen dieser Copolymere; wobei die substituierten Cyclodextrin-Polymere wenigstens ein unsubstituiertes -OH, wenigstens ein unsubstituiertes -SH oder wenigstens eine primäre, sekundäre oder tertiäre Aminogruppe enthalten.

In einer bevorzugten Ausführungsform enthält das Cyclodextrin-Polymer zwei oder mehr unsubstituierte Hydroxygruppen, zwei oder mehr unsubstituierte Thiolgruppen oder zwei oder mehr primäre, sekundäre oder tertiäre Aminogruppen.

In einer weiteren bevorzugten Ausführungsform enthalten substituierte α-Cyclodextrin-Polymere durchschnittlich 1, 2, 3, 4, 5, 6, 7, 8, 9, 10, 11, 12, 13, 14, 15, 16 oder 17 unsubstituierte Hydroxygruppen bezogen auf ein α-Cydodextrin-Monomer des α-Cyclodextrin-Polymers.

In einer weiteren bevorzugten Ausführungsform enthalten substituierte β-Cyclodextrin-Polymere durchschnittlich 1, 2, 3, 4, 5, 6, 7, 8, 9, 10, 11, 12, 13, 14, 15, 16, 17, 18, 19 oder 20 unsubstituierte Hydroxygruppen bezogen auf

ein β-Cyclodextrin-Monomer des β-Cyclodextrin-Polymers.

In einer weiteren bevorzugten Ausführungsform enthalten substituierte γ-Cyclodextrin-Polymere durchschnittlich 1, 2, 3, 4, 5, 6, 7, 8, 9, 10, 11, 12, 13, 14, 15, 16, 17, 18, 19, 20, 21, 22 oder 23 unsubstituierte Hydroxygruppen bezogen auf ein γ-Cyclodextrin-Monomer des γ-Cydodextrin-Polymers.

In einer weiteren bevorzugten Ausführungsform enthalten substituierte α/β-Cyclodextrin-Polymere durchschnittlich 1, 2, 3, 4, 5, 6, 7, 8, 9, 10, 11, 12, 13, 14, 15, 16, 17, 18, 19 oder 20 unsubstituierte Hydroxygruppen bezogen auf ein Cyclodextrin-Monomer des α/β-Cyclodextrin-Polymers.

In einer weiteren bevorzugten Ausführungsform enthalten substituierte α/γ-Cyclodextrin-Polymere durchschnittlich 1, 2, 3, 4, 5, 6, 7, 8, 9, 10, 11, 12, 13, 14, 15, 16, 17, 18, 19, 20, 21, 22 oder 23 unsubstituierte Hydroxygruppen bezogen auf ein Cyclodextrin-Monomer des α/γ-Cydodextrin-Polymers.

In einer weiteren bevorzugten Ausführungsform enthalten substituierte β/γ-Cyclodextrin-Polymere durchschnittlich 1, 2, 3, 4, 5, 6, 7, 8, 9, 10, 11, 12, 13, 14, 15, 16, 17, 18, 19, 20, 21, 22 oder 23 unsubstituierte Hydroxygruppen bezogen auf ein Cyclodextrin-Monomer des β/γ-Cyclodextrin-Polymers.

In einer weiteren bevorzugten Ausführungsform enthalten substituierte α/β/γ-Cyclodextrin-Polymere durchschnittlich 1, 2, 3, 4, 5, 6, 7, 8, 9, 10, 11, 12, 13, 14, 15, 16, 17, 18, 19, 20, 21, 22 oder 23 unsubstituierte Hydroxygruppen bezogen auf ein Cyclodextrin-Monomer des α/β/γ-Cyclodextrin-Polymers.

Die im Verfahren verwendeten Funktionalisierungsmittel einschließlich den bevorzugten Ausführungsformen sind wie oben stehend definiert.

Die erfindungsgemäßen Copolymere sind demnach erhältlich durch ein Verfahren umfassend den Verfahrensschritt

Mischen wenigstens eines, bereits copolymerisierten bzw. vernetzten, unsubstituierten oder ein- oder mehrfach substituierten Cyclodextrin-Polymers mit wenigstens einem Funktionalisierungsmittel.

In einer bevorzugten Ausführungsform sind die erfindungsgemäßen Copolymere erhältlich durch ein Verfahren umfassend den Verfahrensschritt

Mischen wenigstens eines, bereits copolymerisierten bzw. vernetzten, unsubstituierten Cyclodextrin-Polymers mit wenigstens einem Funktionalisierungsmittel.

Der Ausdruck Cyclodextrin-Polymer umfasst vorzugsweise α-Cyclodextrin-Polymere, β-Cyclodextrin-Polymere, γ-Cyclodextrin-Polymere, α/β-Cyclodextrin-Polymere, α/γ-Cyclodextrin-Polymere und α/β/γ-Cyclodextrin-Polymere sowie Mischungen, die wenigstens zwei verschiedene der genannten Cyclodextrin-Polymere enthalten; wobei die Cyclodextrin-Polymere unsubstituiert oder ein- oder mehrfach substituiert, vorzugsweise unsubstituiert sind und wenigstens ein unsubstituiertes -OH, wenigstens ein unsubstituiertes -SH oder wenigstens eine primäre, sekundäre oder tertiäre Aminogruppe enthalten.

Im Sinne dieser Erfindung steht der Ausdruck "α-Cyclodextrin-Polymer" für Copolymere, die zwei oder mehr gleiche oder verschiedene α-Cyclodextrin-Monomere und einen oder mehrere gleiche oder verschiedene Linker enthalten; der Ausdruck "β-Cyclodextrin-Polymer" für Copolymere, die zwei oder mehr gleiche oder verschiedene β-Cyclodextrin-Monomere und einen oder mehrere gleiche oder verschiedene Linker enthalten; der Ausdruck "γ-Cyclodextrin-Polymer" für Copolymere, die zwei oder mehr gleiche oder verschiedene y-Cyclodextrin-Monomere und einen oder mehrere gleiche oder verschiedene Linker enthalten; der Ausdruck " α/β-Cyclodextrin-Polymer" für Copolymere, die einen oder mehrere gleiche oder verschiedene α-Cyclodextrin-Monomere, einen oder mehrere gleiche oder verschiedene β-Cyclodextrin-Monomere und einen oder mehrere gleiche oder verschiedene Linker enthalten; der Ausdruck "α/γ-Cydodextrin-Polymer" für Copolymere, die einen oder mehrere gleiche oder verschiedene α-Cyclodextrin-Monomere, einen oder mehrere gleiche oder verschiedene y-Cyclodextrin-Monomere und einen oder mehrere gleiche oder verschiedene Linker enthalten; der Ausdruck "β/γ-Cyclodextrin-Polymer" für Copolymere, die einen oder mehrere gleiche oder verschiedene β-Cyclodextrin-Monomere, einen oder mehrere gleiche oder verschiedene y-Cyclodextrin-Monomere und einen oder mehrere gleiche oder verschiedene Linker enthalten; der Ausdruck "α/β/γ-Cyclodextrin-Polymer" für Copolymere, die einen oder mehrere gleiche oder verschiedene α-Cydodextrin-Monomere, einen oder mehrere gleiche oder verschiedene β-Cyclodextrin-Monomere, einen oder mehrere gleiche oder verschiedene y-Cyclodextrin-Monomere und zwei oder mehr gleiche oder verschiedene Linker enthalten; wobei die Cyclodextrin-Polymere unsubstituiert oder ein- oder mehrfach substituiert, vorzugsweise unsubstituiert sind und wenigstens ein unsubstituiertes -OH, wenigstens ein unsubstituiertes -SH oder wenigstens eine primäre, sekundäre oder tertiäre Aminogruppe enthalten.

In einer weiteren bevorzugten Ausführungsform steht der Ausdruck "Cyclodextrin-Polymer" für

- α-, β- und γ-Cyclodextrin-Epichlorhydrin-Copolymer,
- α-, β- und γ-Cyclodextrin-4-Chlor-1,2-epoxybutan-Copolymer,

- α-, β- und γ-Cyclodextrin-1,2,3,4-Diepoxybutan-Copolymer,
- α-, β- und γ-Cyclodextrin-Tetramethylendiisocyanat-Copolymer oder
- α-, β- und γ-Cyclodextrin-Hexamethylendiisocyanat-Copolymer;

d.h. die unsubstituierten oder ein- oder mehrfach substituierten Cyclodextrin-Polymere sind vorzugsweise copolymerisiert bzw. vernetzt mit Epichlorhydrin, 4-Chlor-1,2-epoxybutan, 1,2,3,4-Diepoxybutan, Tetramethylendiisocyanat oder Hexamethylendiisocyanat.

Besonders vorteilhaft sind unsubstituierte oder ein- oder mehrfach substituierte α-, β- und γ-Cyclodextrin-Epichlorhydrin-Copolymere.

In einer bevorzugten Ausführungsform beträgt der Stoffmengenanteil der im Cyclodextrin-Polymer enthaltenen Linker 20 bis 95 mol%, bevorzugter 25 bis 80 mol%, noch bevorzugter 30 bis 70 mol%, am Bevorzugtesten 30 bis 60 mol% und insbesondere 30 bis 50 mol%, bezogen auf die Gesamtstoffmenge der Monomere, die im Cyclodextrin-Polymer enthalten sind.

Der Stoffmengenanteil der im Cyclodextrin-Polymer enthaltenen Cyclodextrin-Monomere beträgt vorzugsweise 5,0 bis 80 mol%, bevorzugter 10 bis 75 mol%, noch bevorzugter 20 bis 70 mol%, am Bevorzugtesten 30 bis 70 mol% und insbesondere 40 bis 70 mol%, bezogen auf die Gesamtstoffmenge der Monomere, die im Cyclodextrin-Polymer enthalten sind.

In einer weiteren bevorzugten Ausführungsform ist die Summe aus dem Stoffmengenanteil der Linker und dem Stoffmengenanteil der Cyclodextrin-Monomere ≥ 25 mol%, ≥ 30 mol% oder ≥ 40 mol%, bevorzugter ≥ 50 mol% oder ≥ 60 mol%, noch bevorzugter ≥ 70 mol% oder ≥ 80 mol%, am Bevorzugtesten ≥ 90 mol% oder ≥ 95 mol%, und insbesondere 100 mol%, bezogen auf die Gesamtstoffmenge der Monomere, die im Cyclodextrin-Polymer enthalten sind.

Die Cyclodextrin-Polymere können eine schlechte Wasserlöslichkeit aufweisen. Durch das Mischen mit Funktionalisierungsmittel kann die Wasserlöslichkeit der Cyclodextrin-Polymere erhöht werden.

Die Wasserlöslichkeit der Cyclodextrin-Polymere ist bei 23 °C vorzugsweise ≤ 1,0 g $l^{-1}$, ≤ 2,5 g $l^{-1}$, ≤ 5,0 g $l^{-1}$ oder ≤ 7,5 g $l^{-1}$, bevorzugter ≤ 10 g $l^{-1}$, ≤ 20 g $l^{-1}$, ≤ 40 g $l^{-1}$ oder ≤ 60 g $l^{-1}$, noch bevorzugter ≤ 80 g $l^{-1}$, ≤ 100 g $l^{-1}$, ≤ 125 g $l^{-1}$ oder ≤ 150 g $l^{-1}$, am Bevorzugtesten ≤ 175 g $l^{-1}$ oder ≤ 200 g $l^{-1}$ und insbesondere ≤ 225 g $l^{-1}$.

Die Cyclodextrin-Polymere können eine geringe Osmolalität aufweisen. Durch das Mischen mit Funktionalisierungsmittel kann die Osmolalität der Cyclodextrin-Polymere erhöht werden.

Die Osmolalität einer 50 mM wässrigen Lösung an Cyclodextrin-Polymer ist vorzugsweise ≤ 10 mosm $kg^{-1}$, ≤ 25 mosm $kg^{-1}$ oder ≤ 50 mosm $kg^{-1}$, bevorzugter ≤ 75 mosm $kg^{-1}$, ≤ 100 mosm $kg^{-1}$ oder ≤ 125 mosm $kg^{-1}$, noch bevorzugter ≤ 150 mosm $kg^{-1}$ oder ≤ 175 mosm $kg^{-1}$, am Bevorzugtesten ≤ 200 mosm $kg^{-1}$ oder ≤ 225 mosm $kg^{-1}$ und insbesondere ≥ 300 mosm $kg^{-1}$.

Der Polymerisationsgrad der Cyclodextrin-Polymere ist vorzugsweise ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

Der mittlere Partikeldurchmesser der erfindungsgemäßen Copolymere ist vorzugsweise ≤ 1000 μm, ≤ 900 μm oder ≤ 800 μm, bevorzugter ≤ 700 μm, ≤ 600 μm oder ≤ 500 μm, noch bevorzugter ≤ 400 μm, ≤ 300 μm oder ≤ 200 μm, am Bevorzugtesten ≤ 100 μm, ≤ 75 μm oder ≤ 50 μm und insbesondere ≤ 25 μm.

Das mittlere Molekulargewicht der Cyclodextrin-Polymere ist vorzugsweise ≥ 2,0 kDa, bevorzugter ≥ 2,5 kDa, noch bevorzugter ≥ 3,0 kDa, am Bevorzugtesten ≥ 4,0 kDa und insbesondere ≥ 5,0 kDa. Das mittlere Molekulargewicht wird vorzugsweise mit Hilfe eines Membranosmometers gemessen (destilliertes Wasser, 23 °C).

In einer weiteren bevorzugten Ausführungsform ist das mittleres Molekulargewicht (M) der Cyclodextrin-Polymere vorzugsweise 2,0 ≤ M ≤ 100 kDa, bevorzugter 2,0 ≤ M ≤ 50 kDa, noch bevorzugter 2,0 ≤ M ≤ 25 kDa, am Bevorzugtesten 2,0 ≤ M ≤ 15 kDa und insbesondere 2,0 ≤ M ≤ 10 kDa.

In einer weiteren besonders bevorzugten Ausführungsform hat das Cyclodextrin-Polymer vorzugsweise einen Polymerisationsgrad von ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

Die erfindungsgemäßen Copolymere sind vorzugsweise erhältlich durch ein Verfahren umfassend den Verfahrensschritt Mischen von α-Cyclodextrin-Polymer mit einem oder mehreren (z.B. 2, 3 oder 4) Funktionalisierungsmitteln, vorzugsweise mit nur einem Funktionalisierungsmittel; Mischen von β-Cyclodextrin-Polymer mit einem oder mehreren (z.B. 2, 3 oder 4) Funktionalisierungsmitteln, vorzugsweise mit nur einem Funktionalisierungsmittel; Mischen von γ-Cyclodextrin-Polymer mit einem oder mehreren (z.B. 2, 3 oder 4) Funktionalisierungsmitteln, vorzugsweise mit nur einem Funktionalisierungsmittel; Mischen von α/β-Cyclodextrin-Polymer mit einem oder mehreren (z.B. 2, 3 oder 4) Funktionalisierungsmitteln, vorzugsweise mit nur einem Funktionalisierungsmittel; Mischen von α/γ-Cyclodextrin-Polymer mit einem oder mehreren (z.B. 2, 3 oder 4) Funktionalisierungsmitteln, vorzugsweise mit nur einem Funktionalisierungsmittel; Mischen von β/γ-Cyclodextrin-Polymer mit einem oder mehreren (z.B. 2, 3 oder 4) Funktionalisierungsmitteln, vorzugsweise mit nur einem Funktionalisierungsmittel; oder Mischen von α/β/γ-Cyclodextrin-Polymer mit einem oder mehreren (z.B. 2, 3 oder 4) Funktionalisierungsmitteln, vorzugsweise mit nur einem Funktionalisierungsmittel; ggf. jeweils in Gegenwart wenigstens eines Lösungsmittels.

Die erfindungsgemäßen Copolymere sind vorzugsweise auch erhältlich durch ein Verfahren umfassen den Verfahrensschritt Mischen (Zusammenbringen) beliebiger Gemische der oben genannten Cyclodextrin-Polymere mit wenigstens einem Funktionalisierungsmittel. Vorteilhaft ist, dass die erfindungsgemäßen Copolymere erhältlich sind durch das Mischen (Zusammenbringen) von Gemischen, die α- und β-Cyclodextrin-Polymer, α- und γ-Cyclodextrin-Polymer, β- und γ-Cyclodextrin-Polymer oder α-, β- und γ-Cyclodextrin-Polymer enthalten, mit einem oder mehreren Funktionalisierungsmitteln, vorzugsweise mit nur einem Funktionalisierungsmittel, ggf. in der Gegenwart wenigstens eines Lösungsmittels.

In einer bevorzugten Ausführungsform wird wenigstens ein, vorzugsweise nur ein Cyclodextrin-Polymer gemischt mit wenigstens einem, vorzugsweise nur einem Funktionalisierungsmittel, ggf. in Gegenwart wenigstens eines Lösungsmittels; wobei das Funktionalisierungsmittel ausgewählt ist aus der Gruppe bestehend aus $C_{1-6}$-Alkyl-Y, $C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-7}$-Cycloalkyl-C(=O)-OH, Y-$C_{1-6}$-Alkyl-C(=O)-O-$C_{1-6}$-Alkyl, Y-$C_{3-7}$-Cycloalkyl-C(=O)-O-$C_{3-7}$-Cycloalkyl, $[C_{1-6}\text{-Alkyl-C(=O)}]_2O$, $[C_{3-7}\text{-Cycloalkyl-C(=O)}]_2O$, Dihydrofuran-2,5-dion, Furan-2,5-dion, Dihydro-2H-pyran-2,6(3H)-dion, Oxiran, $C_{1-6}$-Alkyloxiran, $C_{3-7}$-Cycloalkyloxiran, Di-$C_{1-6}$-Alkyloxiran, Di-$C_{3-7}$-Cycloalkyloxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, $(C_{1-6}\text{-Alkyl})_2NH$, $(C_{3-7}\text{-Cycloalkyl})_2NH$, $(C_{1-6}\text{-Alkyl})_3N$, $(C_{3-7}\text{-Cycloalkyl})_3N$, $[(Y\text{-}C_{1-6}\text{-Alkyl})N(C_{1-6}\text{-Alkyl})_3]^+$ Halogenid, 1,3-Propansulton und 1,4-Butansulton; wobei $C_{1-6}$-Alkyl und $C_{3-7}$-Cycloalkyl, unabhängig voneinander, unsubstituiert oder ein- oder mehrfach mit gleichen oder verschiedenen Resten Z substituiert sein können; wobei die weiteren Substituenten Z zu keiner oder zu keiner wesentlichen Quervernetzung der Cyclodextrin-Monomere führen; Halogenid vorzugsweise für Chlorid, Bromid oder Iodid, besonders bevorzugt für Chlorid oder Bromid und insbesondere für Chlorid steht; und die Reste Y für Abgangsgruppen stehen; wobei die Abgangsgruppen Y, unabhängig voneinander, vorzugsweise ausgewählt sind aus der Gruppe bestehend aus -Cl, -Br, -I, -Mesyl, -Tosyl und Epoxy; besonders bevorzugt ausgewählt sind aus der Gruppe bestehend aus -Cl, -Br, -Mesyl und -Tosyl; und insbesondere für -Cl stehen; und die Substituenten Z, unabhängig voneinander, vorzugsweise ausgewählt sind aus der Gruppe bestehend aus -OH, =O, -C(=O)OH, -C(=O)$OCH_3$, -C(=O)$OCH_2CH_3$, -$NH_2$, -NH($CH_3$), -N$(CH_3)_2$, -N$(CH_3)_3{}^+A^-$, -NH($CH_2CH_3$), -N$(CH_2CH_3)_2$ und -N$(CH_2CH_3)_3{}^+A^-$; besonders bevorzugt aus der Gruppe bestehend aus -OH, =O, -C(=O)OH, -C(=O)$OCH_3$ und -C(=O)$OCH_2CH_3$; und insbesondere ausgewählt sind aus der Gruppe bestehend aus -OH, =O und -C(=O)OH; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht In einer weiteren bevorzugten Ausführungsform wird wenigstens ein, vorzugsweise nur ein Cyclodextrin-Polymer gemischt mit wenigstens einem, vorzugsweise nur einem Funktionalisierungsmittel, ggf. in Gegenwart wenigstens eines Lösungsmittels; wobei das Funktionalisierungsmittel ausgewählt ist aus der Gruppe bestehend aus $CH_3$-$Y^1$, $CH_3$-$CH_2$-$Y^1$, $CH_3$-$CH_2$-$CH_2$-$Y^1$, $(CH_3)_2$CH-$Y^1$, $CH_3$-$CH_2$-$CH_2$-$CH_2$-$Y^1$, $(CH_3)_2$CH-$CH_2$-$Y^1$, $CH_3$-$CH_2$-CH($CH_3$)-$Y^1$, $(CH_3)_3$C-$Y^1$, $CH_2$=CH-$Y^1$, $CH_2$=CH-$CH_2$-$Y^1$, Cyclopropyl-$Y^1$, $Y^1$-$CH_2$-C(=O)-OH, $Y^1$-$CH_2$-$CH_2$-C(=O)-OH, $Y^1$-CH($CH_3$)-C(=O)-OH, $Y^1$-$CH_2$-C(=O)-O-$CH_3$, $Y^1$-$CH_2$-$CH_2$-C(=O)-O-$CH_3$, $Y^1$-CH($CH_3$)-C(=O)-O-$CH_3$, $Y^1$-$CH_2$-C(=O)-O-$CH_2CH_3$, $Y^1$-$CH_2$-$CH_2$-C(=O)-O-$CH_2$-$CH_3$, $Y^1$-CH($CH_3$)-C(=O)-O-$CH_2$-$CH_3$, $CH_3$-C(=O)$Y^2$, $CH_3$-$CH_2$-C(=O)$Y^2$, $(CH_3)_2$CH-C(=O)$Y^2$, $CH_3$-$CH_2$-$CH_2$-C(=O)$Y^2$, $(CH_3)_3$C-C(=O)$Y^2$, $CH_3$-$CH_2$-CH($CH_3$)-C(=O)$Y^2$, $(CH_3)_2$CH-$CH_2$-C(=O)$Y^2$, $CH_2$=CH-C(=O)$Y^2$, $CH_2$=CH-$CH_2$-C(=O)$Y^2$, Cycloproyl-C(=O)$Y^2$, Dihydrofuran-2,5-dion, Furan-2,5-dion, Dihydro-2H-pyran-2,6(3H)-dion, $[CH_3\text{-C(=O)}]_2O$, $[CH_3\text{-}CH_2\text{-C(=O)}]_2O$, $[CH_3\text{-}CH_2\text{-}CH_2\text{-C(=O)}]_2O$, $[(CH_3)_2CH\text{-C(=O)}]_2O$, $[CH_3\text{-}CH_2\text{-}CH_2\text{-}CH_2\text{-C(=O)}]_2O$, $[CH_3\text{-}CH_2\text{-CH(}CH_3\text{)-C(=O)}]_2O$, $[(CH_3)_2CH\text{-}CH_2\text{-C(=O)}]_2O$, $[(CH_3)_3C\text{-C(=O)}]_2O$, $[CH_2\text{=CH-C(=O)}]_2O$, $[CH_2\text{=CH-}CH_2\text{-C(=O)}]_2O$, $[\text{Cyclopropyl-C(=O)}]_2O$, $CH_2$=CH-C(=O)-OH, $CH_3$-CH=CH-C(=O)-OH, $(CH_3)_2$C=CH-C(=O)-OH, $CH_3$-$CH_2$-CH=CH-C(=O)-OH, $CH_3$-CH=C($CH_3$)-C(=O)-OH, $CH_2$=CH-C(=O)-O-$CH_3$, $CH_3$-CH=CH-C(=O)-O-$CH_3$, $(CH_3)_2$C=CH-C(=O)-O-$CH_3$, $CH_3$-$CH_2$-CH=CH-C(=O)-O-$CH_3$, $CH_3$-CH=C($CH_3$)-C(=O)-O-$CH_3$, $CH_2$=CH-C(=O)-O-$CH_2$-$CH_3$, $CH_3$-CH=CH-C(=O)-O-$CH_2$-$CH_3$, $(CH_3)_2$C=CH-C(=O)-O-$CH_2$-$CH_3$, $CH_3$-$CH_2$-CH=CH-C(=O)-O-$CH_2$-$CH_3$, $CH_3$-CH=C($CH_3$)-C(=O)O-$CH_2$-$CH_3$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, $(CH_3\text{-O})_2S(=O)_2$, $(CH_3\text{-}CH_2\text{-O})_2S(=O)_2$, $(CH_3\text{-O})_2P(=O)Cl$, $(CH_3\text{-}CH_2\text{-O})_2P(=O)Cl$, $[(CH_3)_2CH\text{-O}]_2P(=O)Cl$, $NH_3$, $CH_3$-$NH_2$, $(CH_3)_2NH$, $(CH_3)_3N$, $CH_3$-$CH_2$-$NH_2$, $(CH_3\text{-}CH_2)_2NH$, $(CH_3\text{-}CH_2)_3N$, $[Cl\text{-}CH_2\text{-}CH_2\text{-N(}CH_3)_3]^+Cl^-$, $[Cl\text{-}CH_2\text{-}CH_2\text{-N(}CH_2\text{-}CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

In einer weiteren bevorzugten Ausführungsform wird nur ein Cyclodextrin-Polymer gemischt mit nur einem Funktionalisierungsmittel, ggf. in Gegenwart wenigstens eines Lösungsmittels; wobei das Funktionalisierungsmittel ausgewählt ist aus der Gruppe bestehend aus $CH_3$-$Y^1$, $CH_3$-$CH_2$-$Y^1$, Cyclopropyl-$Y^1$, $Y^1$-$CH_2$-C(=O)-OH, $Y^1$-$CH_2$-C(=O)-O-$CH_3$, $Y^1$-$CH_2$-C(=O)-O-$CH_2$-$CH_3$, $CH_3$-C(=O)$Y^2$, Dihydrofuran-2,5-dion, Furan-2,5-dion, Dihydro-2H-pyran-2,6(3H)-dion, $[CH_3\text{-C(=O)}]_2O$, $[\text{Cyclopropyl-C(=O)}]_2O$, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, $Cl-CH_2-CH_2-OH$, $(CH_3-O)_2S(=O)_2$, $(CH_3-CH_2-O)_2S(=O)_2$, $CH_3-NH_2$, $(CH_3)_2NH$, $(CH_3)_3N$, $CH_3-CH_2-NH_2$, $(CH_3-CH_2)_2NH$, $(CH_3-CH_2)_3N$, $[Cl-CH_2-CH_2-N(CH_3)_3]^+Cl^-$, $[Cl-CH_2-CH_2-N(CH_2-CH_3)_3]^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und die Reste $Y^2$, unabhängig voneinander, vorzugsweise für -Cl, -Br oder -I, besonders bevorzugt für -Cl oder -Br und insbesondere für -Cl stehen.

In einer weiteren bevorzugten Ausführungsform sind die erfindungsgemäßen Copolymere erhältlich durch in Verfahren umfassend den Verfahrensschritt Mischen von wenigstens einem, vorzugsweise nur einem Cyclodextrin-Polymer ausgewählt aus der Gruppe bestehend aus (Glucosyl)-Cyclodextrin-Copolymer; (Galactosyl)-Cyclodextrin-Copolymer; (Maltosyl)-Cyclodextrin-Copolymer; (Maltriosyl)-Cyclodextrin-Copolymer; (Acetyl)-Cyclodextrin-Copolymer; (Propionyl)-Cyclodextrin-Copolymer; (Isobutyryl)-Cyclodextrin-Copolymer; (Butyryl)-Cyclodextrin-Copolymer; (Pivaloyl)-Cyclodextrin-Copolymer; (Pentanoyl)-Cyclodextrin-Copolymer; (Hexanoyl)-Cyclodextrin-Copolymer; (Cyclopropancarbonyl)-Cyclodextrin-Copolymer; (Benzoyl)-Cyclodextrin-Copolymer; (2-Phenylacetyl)-Cyclodextrin-Copolymer; (2-Carboxyacetyl)-Cyclodextrin-Copolymer; (3-Carboxypropanoyl)-Cyclodextrin-Copolymer; (4-Carboxybutanoyl)-Cyclodextrin-Copolymer; (2-Methoxy-2-oxoacetyl)-Cyclodextrin-Copolymer; (3-Methoxy-3-oxopropanoyl)-Cyclodextrin-Copolymer; (4-Methoxy-4-oxobutanoyl)-Cyclodextrin-Copolymer; (2-Hydroxyacetyl)-Cyclodextrin-Copolymer; (2-Hydroxypropanoyl)-Cyclodextrin-Copolymer; (3-Hydroxypropanoyl)-Cyclodextrin-Copolymer; (2-Hydroxybutanoyl)-Cyclodextrin-Copolymer; (3-Hydroxybutanoyl)-Cyclodextrin-Copolymer; (4-Hydroxybutanoyl)-Cyclodextrin-Copolymer; (2-Hydroxypentanoyl)-Cyclodextrin-Copolymer; (3-Hydroxypentanoyl)-Cyclodextrin-Copolymer; (4-Hydroxypentanoyl)-Cyclodextrin-Copolymer; (5-Hydroxypentanoyl)-Cyclodextrin-Copolymer; (2,3-Dihydroxypropanoyl)-Cyclodextrin-Copolymer; (2,3-Dihydroxybutanoyl)-Cyclodextrin-Copolymer; (2,4-Dihydroxybutanoyl)-Cyclodextrin-Copolymer; (3,4-Dihydroxybutanoyl)-Cyclodextrin-Copolymer; (2,3-Dihydroxypentanoyl)-Cyclodextrin-Copolymer; (2,4-Dihydroxy-pentanoyl)-Cyclodextrin-Copolymer; (2,5-Dihydroxypentanoyl)-Cyclodextrin-Copolymer; (3,4-Dihydroxypentanoyl)-Cyclodextrin-Copolymer; (3,5-Dihydroxypentanoyl)-Cyclodextrin-Copolymer; (4,5-Dihydroxypentanoyl)-Cyclodextrin-Copolymer; (2,3,4-Trihydroxybutanoyl)-Cyclodextrin-Copolymer; (2,3,4-Trihydroxypentanoyl)-Cyclodextrin-Copolymer; (2,3,5-Trihydroxypentanoyl)-Cyclodextrin-Copolymer; (2,4,5-Trihydroxypentanoyl)-Cyclodextrin-Copolymer; (3,4,5-Trihydroxypentanoyl)-Cyclodextrin-Copolymer; (2,3,4,5-Tetrahydroxypentanoyl)-Cyclodextrin-Copolymer; (2-Aminoacetyl)-Cyclodextrin-Copolymer; (2-Aminopropanoyl)-Cyclodextrin-Copolymer; (2-Aminobutanoyl)-Cyclodextrin-Copolymer; [2-(1*H*-Imidazol-1-yl)acetyl]-Cyclodextrin-Copolymer; [3-(1*H*-Imidazol-1-yl)propanoyl]-Cyclodextrin-Copolymer; [4-(1*H*-Imidazol-1-yl)butanoyl]-Cyclodextrin-Copolymer; [2-(1*H*-Imidazol-1-yl)acetyl]-Cyclodextrin-Copolymer; [3-(1*H*-Imidazol-1-yl)propanoyl]-Cyclodextrin-Copolymer; [4-(1*H*-Imidazol-1-yl)butanoyl]-Cyclodextrin-Copolymer; [2-(Pyridin-3-yl)acetyl]-Cyclodextrin-Copolymer; [3-(Pyridin-3-yl)propanoyl]-Cyclodextrin-Copolymer; [4-(Pyridin-3-yl)butanoyl]-Cyclodextrin-Copolymer; (Methyl)-Cyclodextrin-Copolymer; (Ethyl)-Cyclodextrin-Copolymer; (*iso*-Propyl)-Cyclodextrin-Copolymer; (n-Propyl)-Cyclodextrin-Copolymer; (*iso*-Butyl)-Cyclodextrin-Copolymer; (*n*-Butyl)-Cyclodextrin-Copolymer; (*sec*-Butyl)-Cyclodextrin-Copolymer; (*tert*-Butyl)-Cyclodextrin-Copolymer; (Cyclopropyl)-Cyclodextrin-Copolymer; (Phenyl)-Cyclodextrin-Copolymer; (Benzyl)-Cyclodextrin-Copolymer; (Phenethyl)-Cyclodextrin-Copolymer; (Carboxymethyl)-Cyclodextrin-Copolymer; (1-Carboxyethyl)-Cyclodextrin-Copolymer; (2-Carboxyethyl)-Cyclodextrin-Copolymer; (1-Carboxypropyl)-Cyclodextrin-Copolymer; (2-Carboxypropyl)-Cyclodextrin-Copolymer; (3-Carboxypropyl)-Cyclodextrin-Copolymer; (1-Carboxypropan-2-yl)-Cyclodextrin-Copolymer; (4-Carboxybutyl)-Cyclodextrin-Copolymer; (5-Carboxypentyl)-Cyclodextrin-Copolymer; (5-Carboxypentyl)-Cyclodextrin-Copolymer; (2-Methoxy-2-oxoethyl)-Cyclodextrin-Copolymer; (3-Methoxy-2-oxopropyl)-Cyclodextrin-Copolymer; (4-Methoxy-4-oxo-butyl)-Cyclodextrin-Copolymer; (5-Methoxy-5-oxopentyl)-Cyclodextrin-Copolymer; (6-Methoxy-6-oxohexyl)-Cyclodextrin-Copolymer; (2-Ethoxy-2-oxoethyl)-Cyclodextrin-Copolymer; (3-Ethoxy-2-oxopropyl)-Cyclodextrin-Copolymer; (4-Ethoxy-4-oxobutyl)-Cyclodextrin-Copolymer; (5-Ethoxy-5-oxopentyl)-Cyclodextrin-Copolymer; (6-Ethoxy-6-oxohexyl)-Cyclodextrin-Copolymer; (3-Carboxy-2-hydroxypropyl)-Cyclodextrin-Copolymer; (2-Hydroxy-4-methoxy-4-oxobutyl)-Cyclodextrin-Copolymer; (4-Ethoxy-2-hydroxy-4-oxobutyl)-Cyclodextrin-Copolymer; (1-Carboxy-2-hydroxyethyl)-Cyclodextrin-Copolymer; (3-Hydroxy-1-methoxy-1-oxopropan-2-yl)-Cyclodextrin-Copolymer; (1-Ethoxy-3-hydroxy-1-oxopropan-2-yl)-Cyclodextrin-Copolymer; (1-Carboxy-3-hydroxypropan-2-yl)-Cyclodextrin-Copolymer; (1-Hydroxy-4-methoxy-4-oxobutan-2-yl)-Cyclodextrin-Copolymer; (4-Ethoxy-1-hydroxy-4-oxobutan-2-yl)-Cyclodextrin-Copolymer; (Hydroxymethyl)-Cyclodextrin-Copolymer; (1-Hydroxyethyl)-Cyclodextrin-Copolymer; (2-Hydroxyethyl)-Cyclodextrin-Copolymer; (1-Hydroxypropyl)-Cyclodextrin-Copolymer; (2-Hydroxypropyl)-Cyclodextrin-Copolymer; (1-Hydroxypropan-2-yl)-Cyclodextrin-Copolymer; (2-Hydroxypropan-2-yl)-Cyclodextrin-Copolymer; (1-Hydroxybutyl)-Cyclodextrin-Copolymer; (2-Hydroxybutyl)-Cyclodextrin-Copolymer; (3-Hydroxybutyl)-Cyclodextrin-Copolymer; (4-Hydroxybutyl)-Cyclodextrin-Copolymer; (1-Hydroxypentyl)-Cyclodextrin-Copolymer; (2-Hydroxypentyl)-Cyclodextrin-Copolymer; (3-Hydroxypentyl)-Cyclodextrin-Copolymer; (4-Hydroxy-pentyl)-Cyclodextrin-Copolymer; (5-Hydroxypentyl)-Cyclodextrin-Copolymer; (1,2-Di-hydroxyethyl)-Cyclodextrin-Copolymer; (1,2-Dihydroxypropyl)-Cyclodextrin-Copolymer; (1,3-Dihydroxypropyl)-Cyclodextrin-Copolymer; (2,3-Dihydroxypropyl)-Cyclodextrin-Copolymer; (1,2-Dihydroxybutyl)-Cyclodextrin-Copolymer; (1,3-Dihydroxybutyl)-Cyclodextrin-Copolymer; (1,4-Dihydroxybutyl)-Cyclodextrin-Copolymer; (2,3-Dihydroxybutyl)-Cyclodextrin-Copolymer; (2,4-Dihydroxybutyl)-Cyclodextrin-Copolymer; (3,4-Dihydroxybutyl)-Cyclodextrin-Copolymer; (1,2-Dihydroxypentyl)-Cyclodextrin-Copolymer; (1,3-Dihydroxypentyl)-Cyclodextrin-Copolymer; (1,4-Dihydroxypentyl)-Cyclodextrin-Copolymer; (1,5-Dihydroxypentyl)-Cyclodextrin-Copolymer; (2,3-Dihydroxypentyl)-Cyclodextrin-Copolymer; (2,4-Dihydroxypentyl)-Cyclodextrin-Copolymer; (2,5-Dihydroxypentyl)-Cyclodextrin-Copolymer; (3,4-Dihydroxypentyl)-Cyclodextrin-Copolymer; (3,5-Dihydroxypentyl)-Cyclodextrin-Copolymer; (4,5-Dihydroxypentyl)-Cyclodextrin-Copolymer; (1,2,3-Tri-hydroxypropyl)-Cyclodextrin-Copolymer; (1,2,3-Trihydroxybutyl)-Cyclodextrin-Copolymer; (1,2,4-Trihydroxybutyl)-Cyclodextrin-Copolymer; (1,3,4-Trihydroxybutyl)-Cyclodextrin-Copolymer; (2,3,4-Trihydroxybutyl)-Cyclodextrin-Copolymer; (1,2,3-Tri-hydroxypentyl)-Cyclodextrin-Copolymer; (1,2,4-Trihydroxypentyl)-Cyclodextrin-Copolymer; (1,2,5-Trihydroxypentyl)-Cyclodextrin-Copolymer; (1,3,4-Tri-hydroxypentyl)-Cyclodextrin-Copolymer; (1,3,5-Trihydroxypentyl)-Cyclodextrin-Copolymer; (1,4,5-Trihydroxypentyl)-Cyclodextrin-Copolymer; (2,3,4-Trihydroxypentyl)-Cyclodextrin-Copolymer; (2,3,5-Trihydroxypentyl)-Cyclodextrin-Copolymer; (2,4,5-Trihydroxypentyl)-Cyclodextrin-Copolymer; (3,4,5-Trihydroxypentyl)-Cyclodextrin-Copolymer; (1,2,3,4-Tetrahydroxybutyl)-Cyclodextrin-Copolymer; (1,2,3,4-Tetrahydroxypentyl)-Cyclodextrin-Copolymer; (1,2,4,5-Tetrahy-droxypentyl)-Cyclodextrin-Copolymer; (2,3,4,5-Tetrahydroxypentyl)-Cyclodextrin-Copolymer; (1,2,3,4,5-Pentahydroxypentyl)-Cyclodextrin-Copolymer; [(Sulfooxy)methyl]-Cyclodextrin-Copolymer; [2-(Sulfooxy)ethyl]-Cyclodextrin-Copolymer; [3-(Sulfooxy)propyl]-Cyclodextrin-Copolymer; [4-(Sulfooxy)butyl]-Cyclodextrin-Copolymer; [(Phosphonooxy)methyl]-Cyclodextrin-Copolymer; [2-(Phosphonooxy)ethyl]-Cyclodextrin-Copolymer; [3-(Phosphonooxy)propyl]-Cyclodextrin-Copolymer; [4-(Phosphonooxy)butyl]-Cyclodextrin-Copolymer; [4-(Phosphonooxy)butyl]-Cyclodextrin-Copolymer; (Sulfomethyl)-Cyclodextrin-Copolymer; (2-Sulfoethyl)-Cyclodextrin-Copolymer; (3-Sulfopropyl)-Cyclodextrin-Copolymer; (4-Sulfobutyl)-Cyclodextrin-Copolymer; (5-Sulfopentyl)-Cyclodextrin-Copolymer; (6-Sulfohexyl)-Cyclodextrin-Copolymer; (Phosphonomethyl)-Cyclodextrin-Copolymer; (2-Phosphonoethyl)-Cyclodextrin-Copolymer; (3-Phosphonopropyl)-Cyclodextrin-Copolymer; (4-Phosphonobutyl)-Cyclodextrin-Copolymer; (5-Phosphonopentyl)-Cyclodextrin-Copolymer; (6-Phosphonohexyl)-Cyclodextrin-Copolymer; (2-Phosphonovinyl)-Cyclodextrin-Copolymer; (3-Phosphonoallyl)-Cyclodextrin-Copolymer; (4-Phosphonobut-3-enyl)-Cyclodextrin-Copolymer; (5-Phosphonopent-4-enyl)-Cyclodextrin-Copolymer; (6-Phosphonohex-5-enyl)-Cyclodextrin-Copolymer; (Aminomethyl)-Cyclodextrin-Copolymer; (2-Aminoethyl)-Cyclodextrin-Copolymer; (3-Aminopropyl)-Cyclodextrin-Copolymer; (4-Aminobutyl)-Cyclodextrin-Copolymer; (5-Aminopentyl)-Cyclodextrin-Copolymer; (6-Aminohexyl)-Cyclodextrin-Copolymer; [(*N*,*N*-Dimethylamino)methyl]-Cyclodextrin-Copolymer; [2-(*N*,*N*-Dimethylamino)ethyl]-Cyclodextrin-Copolymer; [3-(*N,N*-Dimethylamino)propyl]-Cyclodextrin-Copolymer; [4-(*N*,*N*-Dimethylamino)butyl]-Cyclodextrin-Copolymer; [5-(*N*,*N*-Dimethylamino)pentyl]-Cyclodextrin-Copolymer, [6-(*N*,*N*-Dimethylamino) hexyl]-Cyclodextrin-Copolymer; [(*N*,*N*-Diethylamino)methyl]-Cyclodextrin-Copolymer; [2-(*N*,*N*-Diethylamino)ethyl]-Cyclodextrin-Copolymer; [3-(*N*,*N*-Diethylamino)propyl]-Cyclodextrin-Copolymer; [4-(*N*,*N*-Diethylamino)butyl]-Cyclodextrin-Copolymer; [5-(*N*,*N*-Diethylamino)pentyl]-Cyclodextrin-Copolymer; [6-(*N*,*N*-Diethylamino)hexyl]-Cyclodextrin-Copolymer; [(Trimethylammonio)methyl]-Cyclodextrin-Copolymer Chlorid; [2-(Trimethylammonio)ethyl]-Cyclodextrin-Copolymer Chlorid; [3-(Trimethylammonio)propyl]-Cyclodextrin-Copolymer Chlorid; [4-(Trimethylammonio)butyl]-Cyclodextrin-Copolymer Chlorid; [5-(Trimethylam-monio)pentyl]-Cyclodextrin-Copolymer Chlorid; [6-(Trimethylammonio)hexyl]-Cyclodextrin-Copolymer Chlorid; [(Triethylammonio)methyl]-Cyclodextrin-Copolymer Chlorid; [2-(Triethylammonio)ethyl]-Cyclodextrin-Copolymer Chlorid; [3-(Triethylammonio)propyl]-Cyclodextrin-Copolymer Chlorid; [4-(Triethylammonio)-butyl]-Cyclodextrin-Copolymer Chlorid; [5-(Triethylammonio)pentyl]-Cyclodextrin-Copolymer Chlorid; [6-(Triethylammonio)hexyl]-Cyclodextrin-Copolymer Chlorid; [(1*H*-Imidazol-1-yl)methyl]-Cyclodextrin-Copolymer; [2-(1*H*-Imidazol-1-yl)ethyl]-Cyclodextrin-Copolymer; [3-(1*H*-Imidazol-1-yl)propyl]-Cyclodextrin-Copolymer; [4-(1*H*-Imidazol-1-yl)butyl]-Cyclodextrin-Copolymer; (Pyridin-3-ylmethyl)-Cyclodextrin-Copolymer; [2-(Pyridin-3-yl)ethyl]-Cyclodextrin-Copolymer; [3-(Pyridin-3-yl)propyl]-Cyclodextrin-Copolymer; [4-(Pyridin-3-yl)butyl]-Cyclodextrin-Copolymer; [(*β*-D-Glucopyranosyloxyuronsäure) methyl]-Cyclodextrin-Copolymer; [2-(*β*-D-Gluco-pyranosyloxyuronsäure)ethyl]-Cyclodextrin-Copolymer; [3-(*β*-D-Glucopyranosyloxy-uronsäure)propyl]-Cyclodextrin-Copolymer; [4-(*β*-D-Glucopyranosyloxyuronsäure)butyl]-Cyclodextrin-Copolymer; [5-(*β*-D-Glucopyranosyloxyuronsäure)pentyl]-Cyclodextrin-Copolymer; und [6-(*β*-D-Glucopy-ranosyloxyuronsäure)hexyl]-Cyclodextrin-Copolymer; wobei der Ausdruck "Cyclodextrin-Copolymer" für α-, β-, γ, α/β-, α/γ-, β/γ oder α/β/γ-Cyclodextrin-Copolymer, vorzugsweise für α-, β- oder γ-Cyclodextrin-Copolymer, bevorzugter für β-Cyclodextrin-Copolymer steht; und insbesondere für α-Cyclodextrin-Epichlorhydrin-Copolymer, β-Cyclodextrin-Epichlorhydrin-Copolymer, γ-Cyclodextrin-Epichlorhydrin-Copolymer, α-Cyclodextrin-4-Chlor-1,2-epoxybutan-Copolymer, β-Cyclodextrin-4-Chlor-1,2-epoxybutan-Copolymer, γ-Cyclodextrin-4-Chlor-1,2-epoxybutan-Copolymer, α-Cyclodextrin-1,2,3,4-Diepoxybutan-Copolymer, β-Cyclodextrin-1,2,3,4-Diepoxybutan-Copolymer, γ-Cyclodextrin-1,2,3,4-Diepoxybutan-Copolymer, α-Cyclodextrin-Tetramethylendiisocyanat-Copolymer, β-Cyclodextrin-Tetramethylendiisocyanat-Copolymer, γ-Cyclodextrin-Tetramethylendiisocyanat-Copolymer, α-Cyclodextrin-Hexamethylendiisocyanat-Copolymer, β-Cyclodextrin-Hexamethylendiisocyanat-Copolymer oder γ-Cyclodextrin-Hexamethylendiisocyanat-Copolymer steht;

mit wenigstens einem, vorzugsweise nur einem Funktionalisierungsmittel ausgewählt aus der Gruppe bestehend aus $CH_3$-$Y^1$, $CH_3$-$CH_2$-$Y^1$, Cyclopropyl-$Y^1$, $Y^1$-$CH_2$-C(=O)-OH, $Y^1$-$CH_2$-C(=O)-O-$CH_3$, $Y^1$-$CH_2$-C(=O)-O-$CH_2$-$CH_3$,

$CH_3$-C(=O)$Y^2$, Dihydrofuran-2,5-dion, Furan-2,5-dion, Dihydro-2H-pyran-2,6(3H)-dion, [$CH_3$-C(=O)]$_2$O, [Cyclopropyl-C(=O)]$_2$O, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäure-ethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, ($CH_3$-O)$_2$S(=O)$_2$, ($CH_3$-$CH_2$-O)$_2$S(=O)$_2$, $CH_3$-$NH_2$, $(CH_3)_2NH$, $(CH_3)_3N$, $CH_3$-$CH_2$-$NH_2$, ($CH_3$-$CH_2$)$_2$NH, ($CH_3$-$CH_2$)$_3$N, [Cl-$CH_2$-$CH_2$-N$(CH_3)_3$]$^-Cl^-$, [Cl-$CH_2$-$CH_2$-N($CH_2$-$CH_3$)$_3$]$^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und $Y^2$ vorzugsweise für -Cl, -Br oder -I, besonders bevorzugt für -Cl oder -Br und insbesondere für -Cl steht;
ggf. in Gegenwart wenigstens eines Lösungsmittels.

Die erfindungsgemäßen Copolymere sind vorzugsweise erhältlich durch ein Verfahren umfassend den Verfahrensschritt Mischen von

- unsubstituiertem α-Cyclodextrin-Epichlorhydrin-Copolymer;
- unsubstituiertem α-Cyclodextrin-4-Chlor-1,2-epoxybutan-Copolymer;
- unsubstituiertem α-Cyclodextrin-1,2,3,4-Diepoxybutan-Copolymer;
- unsubstituiertem β-Cyclodextrin-Epichlorhydrin-Copolymer,
- unsubstituiertem β-Cyclodextrin-1,2,3,4-Diepoxybutan-Copolymer;
- unsubstituiertem γ-Cyclodextrin-Epichlorhydrin-Copolymer;
- unsubstituiertem γ-Cyclodextrin-4-Chlor-1,2-epoxybutan-Copolymer;
- unsubstituiertem γ-Cyclodextrin-1,2,3,4-Diepoxybutan-Copolymer; oder
- einer Mischung, die wenigstens zwei verschiedene der vorstehend genannten Cyclodextrin-Polymere enthält;

mit einem Funktionalisierungsmittel, wobei das Funktionalisierungsmittel vorzugsweise ausgewählt ist aus der Gruppe bestehend aus $CH_3$-$Y^1$, $CH_3$-$CH_2$-$Y^1$, Cyclopropyl-$Y^1$, $Y^1$-$CH_2$-C(=O)-OH, $Y^1$-$CH_2$-C(=O)-O-$CH_3$, $Y^1$-$CH_2$-C(=O)-O-$CH_2$-$CH_3$, $CH_3$-C(=O)$Y^2$, Dihydrofuran-2,5-dion, Furan-2,5-dion, Dihydro-2H-pyran-2,6(3H)-dion, [$CH_3$-C(=O)]$_2$O, [Cyclopropyl-C(=O)]$_2$O, Oxiran, 2-Methyloxiran, 2-Ethyloxiran, Oxiran-2-yl-methanol, Oxiran-2-carbonsäure, Oxiran-2-carbonsäuremethylester, Oxiran-2-carbonsäureethylester, 2-(Oxiran-2-yl)essigsäure, 2-(Oxiran-2-yl)essigsäuremethylester, 2-(Oxiran-2-yl)essigsäureethylester, Cl-$CH_2$-$CH_2$-OH, ($CH_3$-O)$_2$S(=O)$_2$, ($CH_3$-$CH_2$-O)$_2$S(=O)$_2$, $CH_3$-$NH_2$, $(CH_3)_2NH$, $(CH_3)_3N$, $CH_3$-$CH_2$-$NH_2$, ($CH_3$-$CH_2$)$_2$NH, ($CH_3$-$CH_2$)$_3$N, [Cl-$CH_2$-$CH_2$-N$(CH_3)_3$]$^+Cl^-$, [Cl-$CH_2$-$CH_2$-N($CH_2$-$CH_3$)$_3$]$^+Cl^-$, 1,3-Propansulton und 1,4-Butansulton; wobei die Reste $Y^1$, unabhängig voneinander, vorzugsweise für -Cl, -Br, -I, -Tosyl, -Mesyl oder Epoxy, besonders bevorzugt für -Cl, -Br oder Epoxy und insbesondere für -Cl stehen; und $Y^2$ vorzugsweise für -Cl, -Br oder -I, besonders bevorzugt für -Cl oder -Br und insbesondere für -Cl steht;
ggf. in Gegenwart wenigstens eines Lösungsmittels.

In einer weiteren bevorzugten Ausführungsform sind die erfindungsgemäßen Copolymere erhältlich durch ein Verfahren umfassend den Verfahrensschritt Mischen von:

- unsubstituiertem α-Cyclodextrin-Epichlorhydrin-Copolymer mit 1,3-Propansulton, 1,4-Butansulton, Chloressigsäure, Chloressigsäuremethylester oder Chloressigsäureethylester;
- unsubstituiertem α-Cyclodextrin-4-Chlor-1,2-epoxybutan-Copolymer mit 1,3-Propansulton, 1,4-Butansulton, Chloressigsäure, Chloressigsäuremethylester oder Chloressigsäureethylester;
- unsubstituiertem α-Cyclodextrin-1,2,3,4-Diepoxybutan-Copolymer mit 1,3-Propansulton, 1,4-Butansulton, Chloressigsäure, Chloressigsäuremethylester oder Chloressigsäureethylester;
- unsubstituiertem β-Cyclodextrin-Epichlorhydrin-Copolymer mit 1,3-Propansulton, 1,4-Butansulton, Chloressigsäure, Chloressigsäuremethylester oder Chloressigsäureethylester;
- unsubstituiertem β-Cyclodextrin-4-Chlor-1,2-epoxybutan-Copolymer mit 1,3-Propansulton, 1,4-Butansulton, Chloressigsäure, Chloressigsäuremethylester oder Chloressigsäureethylester;
- unsubstituiertem β-Cyclodextrin-1,2,3,4-Diepoxybutan-Copolymer mit 1,3-Propansulton, 1,4-Butansulton, Chloressigsäure, Chloressigsäuremethylester oder Chloressigsäureethylester;
- unsubstituiertem γ-Cyclodextrin-Epichlorhydrin-Copolymer mit 1,3-Propansulton, 1,4-Butansulton, Chloressigsäure, Chloressigsäuremethylester oder Chloressigsäureethylester;
- unsubstituiertem γ-Cyclodextrin-4-Chlor-1,2-epoxybutan-Copolymer mit 1,3-Propansulton, 1,4-Butansulton, Chloressigsäure, Chloressigsäuremethylester oder Chloressigsäureethylester; oder
- unsubstituiertem γ-Cyclodextrin-1,2,3,4-Diepoxybutan-Copolymer mit 1,3-Propansulton, 1,4-Butansulton, Chloressigsäure, Chloressigsäuremethylester oder Chloressigsäureethylester;

ggf. in Gegenwart wenigstens eines Lösungsmittels.

In einer weiteren bevorzugten Ausführungsform wird ein unsubstituiertes α-. β- oder γ-Cyclodextrin-Epichlorhydrin-Copolymer mit nur einem Funktionalisierungsmittel aus der Gruppe 1,3-Butansulton, 1,4-Butansulton, 2-Chloressigsäure, 2-Chloressigsäuremethylester und 2-Chloressigsäureethylester gemischt; ggf. in Gegenwart eines Lösungsmittels.

Das Mischen der Cyclodextrin-Polymere mit Funktionalisierungsmitteln kann in wässrigen oder organischen Lösungsmitteln erfolgen. Vorzugsweise erfolgt das Mischen in Wasser, Dimethylsulfoxid (DMSO), Dimethylformamid (DMF), N-Methyl-2-pyrrolidon (NMP) oder Mischungen davon. Die Umsetzung kann auch in wasserfreiem DMSO, wasserfreiem DMF, wasserfreiem NMP oder Mischungen davon unter vorzugsweise wasserfreien Bedingungen durchgeführt werden. Als Lösungsmittel besonders vorteilhaft sind Wasser, DMSO und DMF sowie Mischungen davon; insbesondere Wasser oder DMF sowie Mischungen davon.

Das Mischen erfolgt vorzugsweise im alkalischen Milieu, in der Regel bei einem pH-Wert > 7.

Das Mischen erfolgt vorzugsweise bei 7,5 ≤ pH ≤ 14, bevorzugter bei 7,5 ≤ pH ≤ 13, noch bevorzugter bei 8 ≤ pH ≤ 13, am Bevorzugtesten bei 8 ≤ pH ≤ 12,5 und insbesondere bei 9 ≤ pH ≤ 12.

Die Einstellung des pH-Wertes der Reaktionslösung oder -dispersion erfolgt vorzugsweise mit Basen, wie beispielsweise basischen Alkalimetallsalzen, basischen Erdalkalimetallsalzen, Hydriden, Alkoxiden oder organischen Basen wie beispielsweise Pyridin oder Diisopropylamin. Besonders vorteilhaft sind Natriumhydroxid, Natriumhydrogencarbonat, Natriumcarbonat, Kaliumhydroxid, Kaliumhydrogencarbonat, Kaliumcarbonat, Natriumhydrid, Kaliumhydrid, Natriummethanolat, Kalium-tert-butoxid, Pyridin und Diisopropylamin.

Da die Funktionalisierung der Cyclodextrin-Polymere unter basischen Reaktionsbedingungen erfolgt, können Substituenten, die im alkalischen Milieu nicht stabil sind, strukturell verändert werden. Diese Veränderung kann basenlabile Gruppen der Cyclodextrin-Polymere, Vernetzungsmittel und Funktionalisierungsmittel betreffen. Beispielsweise kann die Ester-Gruppe des Cyclodextrin-Polymers (2-Methoxy-2-oxoethyl)-Cyclodextrin-Epichlorhydrin-Copolymer zu einer Carboxyl- bzw. Carboxylat-Gruppe gespalten werden, wobei das (2-Methoxy-2-oxoethyl)-Cyclodextrin-Epichlorhydrin-Copolymer zu (Carboxymethyl)-Cyclodextrin-Epichlorhydrin-Copolymer umgewandelt wird. Diese Umwandlung kann, bezogen auf die Gesamtanzahl der Ester-Gruppen, vollständig oder unvollständig erfolgen. Folglich können im entsprechenden erfindungsgemäßen Copolymer alle oder nur ein Teil der Ester- zu Carboxyl-Gruppen umgewandelt sein.

In einer bevorzugten Ausführungsform werden die Cyclodextrin-Polymere in einem Lösungsmittel oder Lösungsmittelgemisch gelöst oder dispergiert und anschließend werden vorzugsweise unter Rühren der Dispersion oder Lösung die Funktionalisierungsmittel darin gelöst oder dispergiert. Die Zugabe der Funktionalisierungsmittel kann auf einmal oder sukzessive in Portionen erfolgen. Die Funktionalisierungsmittel können vor der Zugabe in einem Lösungsmittel (z.B. Wasser, DMF, DMSO, NMP) gelöst oder dispergiert werden und als Lösung oder Dispersion dem Reaktionsgemisch auf einmal oder sukzessive in Portionen zugegeben oder mittels Tropftrichter, Pipette oder Spritze zugetropft werden. Die Zugabe bzw. Zutropfung kann mit Hilfe einer Spritzenpumpe erfolgen.

In einer weiteren bevorzugten Ausführungsform werden die Funktionalisierungsmittel in einem Lösungsmittel oder Lösungsmittelgemisch gelöst oder dispergiert und anschließend werden, vorzugsweise unter Rühren der Lösung oder Dispersion, die Cyclodextrin-Polymere in dieser Lösung oder Dispersion gelöst oder dispergiert. Die Zugabe der Cyclodextrin-Polymere kann auf einmal oder sukzessive in Portionen erfolgen. Die Cyclodextrin-Polymere können vor der Zugabe in einem Lösungsmittel gelöst oder dispergiert werden und als Lösung oder Dispersion dem Reaktionsgemisch auf einmal oder sukzessive in Portionen zugegeben oder mittels Tropftrichter, Pipette oder Spritze zugetropft werden. Die Zugabe bzw. Zutropfung kann mit Hilfe einer Spritzenpumpe erfolgen.

In einer weiteren bevorzugten Ausführungsform werden die Funktionalisierungsmittel und Cyclodextrin-Polymere gleichzeitig in einem Lösungsmittel oder Lösungsmittelgemisch gelöst oder dispergiert.

Beim Mischen kann es zu einem plötzlichen und unerwünschten Anstieg der Reaktionstemperatur kommen. Deshalb ist es vorteilhaft, das Reaktionsgemisch ggf. zu kühlen, um eine im Wesentlichen konstante Reaktionstemperatur zu gewährleisten. Die Kühlung des Reaktionsgemisches kann über dem Eisbad oder Wasserbad erfolgen.

Die Alkalisierung des Reaktionsgemisches kann vor, während oder nach Zugabe der Cyclodextrin-Polymere, vor, während oder nach der Zugabe der Funktionalisierungsmittel oder vor, während oder nach Zugabe einer Mischung davon erfolgen.

Die Messung des alkalischen pH-Wertes erfolgt vorzugsweise mit Hilfe von pH-Papier oder einem pH-Meter. In einer bevorzugten Ausführungsform wird der pH-Wert während der Umsetzung fortlaufend oder in bestimmten Zeitintervallen gemessen. Falls der pH-Wert unter einen bestimmten Wert fällt (z.B. pH < 8 oder < 7,5), kann im Reaktionsgemisch ein alkalischer pH-Wert durch Basenzugabe wieder hergestellt werden.

Die Umsetzung erfolgt vorzugsweise bei einer Reaktionstemperatur (Temperatur des Reaktionsgemisches) von 5,0 bis 90 °C, bevorzugter 10 bis 75 °C, noch bevorzugter 15 bis 50 °C, am Bevorzugtesten 15 bis 30 °C und insbesondere 20 bis 25 °C. Vor, während oder nach dem Mischen der Cyclodextrin-Polymere mit den Funktionalisierungsmitteln, ggf. in Gegenwart Lösungsmittels oder Lösungsmittelgemisches, wird die Temperatur des Reaktionsgemisches vorzugsweise auf 5 bis 90 °C, bevorzugter auf 10 bis 75 °C, noch bevorzugter auf 15 bis 50 °C, am Bevorzugtesten auf 15 bis 30 °C und insbesondere auf 20 bis 25 °C eingestellt.

In einer bevorzugten Ausführungsform wird eine Gesamtstoffmenge von ≥ 5,0 mol, ≥ 10 mol, ≥ 20 mol oder ≥

30 mol, bevorzugter ≥ 40 mol, ≥ 50 mol oder ≥ 60 mol, noch bevorzugter ≥ 70 mol, ≥ 80 mol oder ≥ 90 mol, am Bevorzugtesten ≥ 100 mol, ≥ 150 mol oder ≥ 200 mol, und insbesondere ≥ 250 mol an Funktionalisierungsmittel bezogen auf eine Gesamtstoffmenge von 1,0 mol an Cyclodextrin-Polymeren in das Reaktionsgemisch eingesetzt (Äquivalente).

In einer weiteren bevorzugten Ausführungsform ist das Verhältnis der Gesamtstoffmenge an Funktionalisierungsmitteln zu der Gesamtstoffmenge an Cyclodextrin-Polymeren ≥ 1 : 1, bevorzugter ≥ 10 : 1, noch bevorzugter ≥ 25 : 1, am Bevorzugtesten ≥ 50 : 1 und insbesondere ≥ 100 : 1.

Die Umsetzung kann mittels geeigneter analytischer Methoden verfolgt werden. Beispielsweise können zu bestimmten Zeitpunkten Stichproben des Reaktionsgemisches genommen werden und mittels LC/MS oder NMR analysiert werden. Auf diese Weise kann das im Reaktionsgemisch zu einem bestimmten Zeitpunkt vorliegende Reaktionsprodukt bestimmt werden. Falls die Stichprobenanalyse ergibt, dass das gewünschte Reaktionsprodukt vorliegt, so kann die Umsetzung beendet und das gewünschte Reaktionsprodukt isoliert bzw. aufgereinigt werden.

Zur Beendigung der Umsetzung wird das Reaktionsgemisch vorzugsweise neutralisiert, d.h. der pH-Wert der Reaktionslösung oder -dispersion wird vorzugsweise auf 6,5 ≤ pH ≤ 7,5, besonders bevorzugt auf 6,8 ≤ pH ≤ 7,2 eingestellt. Die Neutraliserung erfolgt vorzugsweise mit organischen (z.B. Trifluoressigsäure, Essigsäure) oder anorganischen Säuren (z.B. Salzsäure, Schwefelsäure, Salpetersäure).

In einer weiteren bevorzugten Ausführungsform wird das Reaktionsgemisch nach der Umsetzung bzw. Neutralisierung dialysiert, vorzugsweise salzfrei dialysiert, wobei die in diesem Dialyseverfahren eingesetzte Dialysemembran einen MWCO ("Molecular Weight Cut-Off') von vorzugsweise 0,50 kDa oder 1,0 kDa, bevorzugter von 2,0 kDa oder 3,0 kDa, noch bevorzugter von 4,0 kDa oder 5,0 kDa, am Bevorzugtesten von 10 kDa und insbesondere von ≥ 15 kDa aufweist.

Vorzugsweise hat das salzfrei dialysierte erfindungsgemäße Copolymer einen Restgehalt von ≤ 10 Gew.-%, bevorzugter ≤ 5,0 Gew.-%, noch bevorzugter ≤ 2,5 Gew.-% und insbesondere ≤ 1,0 Gew.-% an Verbindungen mit einem Molekulargewicht kleiner als 1,5 kDa, bezogen auf die Gesamtmasse an erfindungsgemäßen Copolymer.

Das Reaktionsgemisch bzw. die im Wesentlichen salzfreie Dialyselösung kann vor der weiteren Aufreinigung bzw. Isolierung vorzugsweise mit Hilfe eines Rotationsverdampfers eingeengt werden. Eine Einengung des Reaktionsgemisches kann auch bereits vor der Dialyse, vorzugsweise salzfreien Dialyse erfolgen.

Die Isolierung oder Aufreinigung der erfindungsgemäßen Copolymere erfolgt vorzugsweise mittels Ultraflitration, Säulenchromatographie (z.B. an Kieselgel oder Aktivkohle), präparativer HPLC oder Rekristallisation. Das erhaltene erfindungsgemäße Copolymer kann lyophilisiert werden.

In einer bevorzugten Ausführungsform beträgt der Stoffmengenanteil der im erfindungsgemäßen Copolymer enthaltenen Linker 20 bis 95 mol%, bevorzugter 25 bis 80 mol%, noch bevorzugter 30 bis 70 mol%, am Bevorzugtesten 30 bis 60 mol% und insbesondere 30 bis 50 mol%, bezogen auf die Gesamtstoffmenge der Monomere, die im erfindungsgemäßen Copolymer enthalten sind.

Der Stoffmengenanteil der im erfindungsgemäßen Copolymer enthaltenen Cyclodextrin-Monomere beträgt vorzugsweise 5,0 bis 80 mol%, bevorzugter 10 bis 75 mol%, noch bevorzugter 20 bis 70 mol%, am Bevorzugtesten 30 bis 70 mol% und insbesondere 40 bis 70 mol%, bezogen auf die Gesamtstoffmenge der Monomere, die im erfindungsgemäßen Copolymer enthalten sind.

In einer weiteren bevorzugten Ausführungsform ist die Summe aus dem Stoffmengenanteil der Linker und dem Stoffmengenanteil der Cyclodextrin-Monomere ≥ 25 mol%, ≥ 30 mol% oder ≥ 40 mol%, bevorzugter ≥ 50 mol% oder ≥ 60 mol%, noch bevorzugter ≥ 70 mol% oder ≥ 80 mol%, am Bevorzugtesten ≥ 90 mol% oder ≥ 95 mol%, und insbesondere 100 mol%, bezogen auf die Gesamtstoffmenge der Monomere, die im erfindungsgemäßen Copolymer enthalten sind.

Die Wasserlöslichkeit der erfindungsgemäßen Copolymere ist bei 23 °C vorzugsweise ≥ 5,0 g $l^{-1}$, ≥ 10 g $l^{-1}$, ≥ 25 g $l^{-1}$ oder ≥ 50 g $l^{-1}$, bevorzugter ≥ 75 g $l^{-1}$, ≥ 100 g $l^{-1}$, ≥ 125 g $l^{-1}$ oder ≥ 150 g $l^{-1}$, noch bevorzugter ≥ 175 g $l^{-1}$, ≥ 200 g $l^{-1}$, ≥ 225 g $l^{-1}$ oder ≥ 250 g $l^{-1}$, am Bevorzugtesten ≥ 300 g $l^{-1}$ oder ≥ 400 g $l^{-1}$ und insbesondere ≥ 500 g $l^{-1}$.

Die Osmolalität einer 50 mM wässrigen Lösung an erfindungsgemäßen Copolymer ist vorzugsweise ≥ 50 mosm $kg^{-1}$, ≥ 75 mosm $kg^{-1}$ oder ≥ 100 mosm $kg^{-1}$, bevorzugter ≥ 125 mosm $kg^{-1}$, ≥ 150 mosm $kg^{-1}$ oder ≥ 175 mosm $kg^{-1}$, noch bevorzugter ≥ 200 mosm $kg^{-1}$ oder ≥ 225 mosm $kg^{-1}$, am Bevorzugtesten ≥ 250 mosm $kg^{-1}$ oder ≥ 275 mosm $kg^{-1}$ und insbesondere ≥ 300 mosm $kg^{-1}$.

Der Polymerisationsgrad der erfindungsgemäßen Copolymere ist vorzugsweise ≥ 2,0 oder ≥ 3,0, bevorzugter ≥ 4,0 oder ≥ 5,0, noch bevorzugter ≥ 6,0 oder ≥ 7,0, am Bevorzugtesten ≥ 8,0 oder ≥ 9,0 und insbesondere ≥ 10.

Das mittlere Molekulargewicht der erfindungsgemäßen Copolymere ist vorzugsweise ≥ 2,0 kDa, bevorzugter ≥ 2,5 kDa, noch bevorzugter ≥ 3,0 kDa, am Bevorzugtesten ≥ 4,0 kDa und insbesondere ≥ 5,0 kDa. Das mittlere Molekulargewicht wird vorzugsweise mit Hilfe eines Membranosmometers gemessen (destilliertes Wasser, 23 °C).

In einer weiteren bevorzugten Ausführungsform ist das mittleres Molekulargewicht (M) der erfindungsgemäßen Polymere vorzugsweise 2,0 ≤ M ≤ 100 kDa, bevorzugter 2,0 ≤ M ≤ 50 kDa, noch bevorzugter 2,0 ≤ M ≤ 25 kDa, am Bevorzugtesten 2,0 ≤ M ≤ 15 kDa und insbesondere 2,0 ≤ M ≤ 10 kDa.

In einer weiteren besonders bevorzugten Ausführungsform hat das erfindungsgemäße Copolymer vorzugsweise einen Polymerisationsgrad von ≥ 2,0 oder ≥ 3,0, bevorzugter ≥ 4,0 oder ≥ 5,0, noch bevorzugter ≥ 6,0 oder ≥ 7,0,

am Bevorzugtesten ≥ 8,0 oder ≥ 9,0 und insbesondere ≥ 10.

In einer weiteren bevorzugten Ausführungsform ist der mittlere Partikeldurchmesser der erfindungsgemäßen Copolymere ≤ 1000 μm, ≤ 900 μm oder ≤ 800 μm, bevorzugter ≤ 700 μm, ≤ 600 μm oder ≤ 500 μm, noch bevorzugter ≤ 400 μm, ≤ 300 μm oder ≤ 200 μm, am Bevorzugtesten ≤ 100 μm, ≤ 75 μm oder ≤ 50 μm und insbesondere ≤ 25 μm.

In einer bevorzugten Ausführungsform sind die erfindungsgemäßen Copolymere zur Verwendung in der Behandlung der Niereninsuffizienz geeignet.

In einer weiteren bevorzugten Ausführungsform sind die erfindungsgemäßen Copolymere zur Verwendung in der Dialysebehandlung geeignet.

In einer weiteren bevorzugten Ausführungsform sind die erfindungsgemäßen Copolymere, u.a. aufgrund ihrer osmotischen Wirksamkeit, zur Verwendung in der Hämodialyse- und / oder Peritonealdialysebehandlung geeignet.

Ein weiterer Gegenstand dieser Erfindung betrifft Dialyselösungen enthaltend wenigstens ein erfindungsgemäßes Copolymer.

In einer bevorzugten Ausführungsform ist die erfindungsgemäße Dialyselösung eine Hämodialyselösung oder eine Peritonealdialyselösung.

Darreichungsformen, die in der Dialysebehandlung eingesetzt werden, sind vorzugsweise Konzentrate in Mehrkomponenten-Systemen oder gebrauchsfertige Dialyselösungen.

Für die Zwecke dieser Erfindung umfasst der Ausdruck "Dialyselösung" eine gebrauchsfertige Darreichungsform zur Dialysebehandlung, d.h. eine flüssige Zubereitung, die als solche zur Applikation geeignet ist. Insbesondere muss die Dialyselösung vor der Applikation nicht verdünnt und/ oder mit anderen Zubereitungen gemischt werden.

Im Gegensatz zu den vorstehend beschriebenen Dialyselösungen werden Konzentrate, die entweder in flüssiger, halbfester oder fester Form vorliegen können, vor der Applikation mit Wasser oder wässrigen Lösungen verdünnt oder in Wasser oder wässrigen Lösungen gelöst. In analoger Weise müssen die Komponenten eines Mehrkomponenten-Systems vor der Applikation miteinander gemischt werden um eine gebrauchsfertige Dialyselösung zu erhalten. Konzentrate und Mehrkomponenten-Systeme können also als Vorstufe der erfindungsgemäßen Dialyselösung angesehen werden.

Die erfindungsgemäße Dialyselösung ist vorzugsweise eine Hämodialyse- oder eine Peritonealdialyselösung. Hämodialyse- und Peritonealdialyselösungen enthalten üblicherweise Elektrolyte in einer Konzentration, die im Wesentlichen der Plasma-Elektrolyt-Konzentration entspricht. Elektrolyte umfassen üblicherweise Natrium-, Kalium-, Calcium-, Magnesium- und Chlorid-Ionen.

Dialyselösungen haben üblicherweise einen physiologisch verträglichen pH-Wert. Dies wird vorzugsweise erreicht durch Puffer (Puffer-Systeme), die selbst zum Gesamtgehalt an Elektrolyten beitragen können. Die Puffer sind vorzugsweise Hydrogencarbonat, Lactat oder Pyruvat.

Ferner besitzen Dialyselösungen üblicherweise eine physiologisch verträgliche Osmolarität. Dies wird in der Regel erreicht durch die in der Dialyselösung enthaltenen Elektrolyte und erfindungsgemäßen Copolymere, die als osmotisch aktive Substanzen (Osmotika) in der gewünschten Konzentration physiologisch verträglich sind.

Die erfindungsgemäße Dialyselösung besitzt eine Osmolarität im Bereich von vorzugsweise 200 bis 550 mosm $l^{-1}$.

Im Fall, dass die erfindungsgemäße Dialyselösung eine Hämodialyselösung ist, beträgt die Osmolarität vorzugsweise 200 bis 350 mosm $l^{-1}$ oder 210 bis 340 mosm $l^{-1}$, bevorzugter 220 bis 330 mosm $l^{-1}$, noch bevorzugter 230 bis 320 mosm $l^{-1}$, am Bevorzugtesten 240 bis 310 mosm $l^{-1}$ und insbesondere 250 bis 300 mosm $l^{-1}$. Verfahren zur Messung der Osmolarität und des osmotischen Drucks sind dem Fachmann bekannt. Beispielsweise können diese mit Hilfe eines Membranosmometers bestimmt werden.

Im Fall, dass die erfindungsgemäße Dialyselösung eine Peritonealdialyselösung ist, beträgt die Osmolarität vorzugsweise 200 bis 570 mosm $l^{-1}$ oder 210 bis 560 mosm $l^{-1}$, bevorzugter 220 bis 550 mosm $l^{-1}$, noch bevorzugter 230 bis 540 mosm $l^{-1}$, am Bevorzugtesten 240 bis 530 mosm $l^{-1}$ und insbesondere 250 bis 520 mosm $l^{-1}$. In einer bevorzugten Ausführungsform beträgt die Osmolarität 250 ± 50 mosm $l^{-1}$ oder 250 ± 45 mosm $l^{-1}$, bevorzugter 250 ± 35 mosm $l^{-1}$, noch bevorzugter 250 ± 25 mosm $l^{-1}$, am Bevorzugtesten 250 ± 15 mosm $l^{-1}$ und insbesondere 250 ± 10 mosm $l^{-1}$. In einer weiteren bevorzugten Ausführungsform beträgt die Osmolarität 300 ± 50 mosm $l^{-1}$ oder 300 ± 45 mosm $l^{-1}$, bevorzugter 300 ± 35 mosm $l^{-1}$, noch bevorzugter 300 ± 25 mosm $l^{-1}$, am Bevorzugtesten 300 ± 15 mosm $l^{-1}$ und insbesondere 300 ± 10 mosm $l^{-1}$. In einer weiteren bevorzugten Ausführungsform beträgt die Osmolarität 350 ± 50 mosm $l^{-1}$ oder 350 ± 45 mosm $l^{-1}$, bevorzugter 350 ± 35 mosm $l^{-1}$, noch bevorzugter 350 ± 25 mosm $l^{-1}$, am Bevorzugtesten 350 ± 15 mosm $l^{-1}$ und insbesondere 300 ± 10 mosm $l^{-1}$. In einer weiteren bevorzugten Ausführungsform beträgt die Osmolarität 400 ± 50 mosm $l^{-1}$ oder 400 ± 45 mosm $l^{-1}$, bevorzugter 400 ± 35 mosm $l^{-1}$, noch bevorzugter 400 ± 25 mosm $l^{-1}$, am Bevorzugtesten 400 ± 15 mosm $l^{-1}$ und insbesondere 300 ± 10 mosm $l^{-1}$. In einer weiteren bevorzugten Ausführungsform beträgt die Osmolarität 450 ± 50 mosm $l^{-1}$ oder 450 ± 45 mosm $l^{-1}$, bevorzugter 450 ± 35 mosm $l^{-1}$, noch bevorzugter 450 ± 25 mosm $l^{-1}$, am Bevorzugtesten 450 ± 15 mosm $l^{-1}$ und insbesondere 450 ± 10 mosm $l^{-1}$. In einer weiteren bevorzugten Ausführungsform beträgt die Osmolarität 500 ± 50 mosm $l^{-1}$ oder 500 ± 45 mosm $l^{-1}$, bevorzugter 500 ± 35 mosm $l^{-1}$, noch bevorzugter 500 ± 25 mosm $l^{-1}$, am Bevorzugtesten 500 ± 15 mosm

$l^{-1}$ und insbesondere 500 ± 10 mosm $l^{-1}$.

Die erfindungsgemäße Dialyselösung hat einen pH-Wert vorzugsweise von 4,9 bis 8,0, bevorzugter von 5,2 bis 7,8; gemessen bei Raumtemperatur (20 bis 23 °C). In einer bevorzugten Ausführungsform ist der pH-Wert 5,5 ± 1,0 oder 5,5 ± 0.8, bevorzugter 5,5 ± 0,7 oder 5,5 ± 0,6, noch bevorzugter 5,5 ± 0,5 oder 5,5 ± 0,4, am Bevorzugtesten 5,5 ± 0,3 oder 5,5 ± 0,2 und insbesondere 5,5 ± 0,1. In einer weiteren bevorzugten Ausführungsform ist der pH-Wert 6,0 ± 1,0 oder 6,0 ± 0.8, bevorzugter 6,0 ± 0,7 oder 6,0 ± 0,6, noch bevorzugter 6,0 ± 0,5 oder 6,0 ± 0,4, am Bevorzugtesten 6,0 ± 0,3 oder 6,0 ± 0,2 und insbesondere 6,0 ± 0,1. In einer weiteren bevorzugten Ausführungsform ist der pH-Wert 6,5 ± 1,0 oder 6,5 ± 0.8, bevorzugter 6,5 ± 0,7 oder 6,5 ± 0,6, noch bevorzugter 6,5 ± 0,5 oder 6,5 ± 0,4, am Bevorzugtesten 6,5 ± 0,3 oder 6,5 ± 0,2 und insbesondere 6,5 ± 0,1. In einer weiteren bevorzugten Ausführungsform ist der pH-Wert 7,0 ± 1,0 oder 7,0 ± 0.8, bevorzugter 7,0 ± 0,7 oder 7,0 ± 0,6, noch bevorzugter 7,0 ± 0,5 oder 7,0 ± 0,4, am Bevorzugtesten 7,0 ± 0,3 oder 7,0 ± 0,2 und insbesondere 7,0 ± 0,1. In einer weiteren bevorzugten Ausführungsform ist der pH-Wert 7,4 ± 1,0 oder 7,4 ± 0.8, bevorzugter 7,4 ± 0,7 oder 7,4 ± 0,6, noch bevorzugter 7,4 ± 0,5 oder 7,4 ± 0,4, am Bevorzugtesten 7,4 ± 0,3 oder 7,4 ± 0,2 und insbesondere 7,4 ± 0,1.

Die erfindungsgemäße Dialyselösung enthält ein oder mehrere (z.B. zwei, drei, vier oder fünf) erfindungsgemäße Copolymere; wobei die erfindungsgemäßen Copolymere wie oben stehend definiert sind.

Die erfindungsgemäße Dialyselösung enthält erfindungsgemäßes Copolymer in einer Gesamtkonzentration von vorzugsweise 0,010 mM bis 1,0 M oder 0,01 bis 750 mM, bevorzugter 0,10 bis 500 mM, noch bevorzugter 1,0 bis 250 mM, am Bevorzugtesten 10 bis 100 mM und insbesondere 15 bis 90 mM. In einer bevorzugten Ausführungsform ist die Gesamtkonzentration an erfindungsgemäßen Copolymer 25 ± 24 mM, bevorzugter 25 ± 20 mM, noch bevorzugter 25 ± 15 mM, am Bevorzugtesten 25 ± 10 mM und insbesondere 25 ± 5 mM. In einer weiteren bevorzugten Ausführungsform ist die Gesamtkonzentration an erfindungsgemäßen Copolymer 50 ± 25 mM, bevorzugter 50 ± 20 mM, noch bevorzugter 50 ± 15 mM, am Bevorzugtesten 50 ± 10 mM und insbesondere 50 ± 5 mM. In einer weiteren bevorzugten Ausführungsform ist die Gesamtkonzentration an erfindungsgemäßen Copolymer 75 ± 25 mM, bevorzugter 75 ± 20 mM, noch bevorzugter 75 ± 15 mM, am Bevorzugtesten 75 ± 10 mM und insbesondere 75 ± 5 mM. In einer weiteren bevorzugten Ausführungsform ist die Gesamtkonzentration an erfindungsgemäßen Copolymer 100 ± 25 mM, bevorzugter 100 ± 20 mM, noch bevorzugter 100 ± 15 mM, am Bevorzugtesten 100 ± 10 mM und insbesondere 100 ± 5 mM. Die Gesamtkonzentration ist vorzugsweise berechnet mittels des mittleren Molekulargewichts der erfindungsgemäßen Copolymere.

Die erfindungsgemäße Dialyselösung enthält erfindungsgemäßes Copolymer in einer Gesamtmassenkonzentration von vorzugsweise 0,01 g $l^{-1}$ bis 1,0 kg $l^{-1}$, bevorzugter von 0,1 bis 750 g $l^{-1}$, noch bevorzugter von 1,0 bis 500 g $l^{-1}$, am Bevorzugtesten 10 bis 250 g $l^{-1}$ und insbesondere von 100 bis 200 g $l^{-1}$. In einer bevorzugten Ausführungsform ist die Gesamtmassenkonzentration 25 ± 24 g $l^{-1}$, bevorzugter 25 ± 20 g $l^{-1}$, noch bevorzugter 25 ± 15 g $l^{-1}$, am Bevorzugtesten 25 ± 10 g $l^{-1}$ und insbesondere 25 ± 5 g $l^{-1}$. In einer weiteren bevorzugten Ausführungsform ist die Gesamtmassenkonzentration 50 ± 25 g $l^{-1}$, bevorzugter 50 ± 20 g $l^{-1}$, noch bevorzugter 50 ± 15 g $l^{-1}$, am Bevorzugtesten 50 ± 10 g $l^{-1}$ und insbesondere 50 ± 5 g $l^{-1}$. In einer weiteren bevorzugten Ausführungsform ist die Gesamtmassenkonzentration 75 ± 25 g $l^{-1}$, bevorzugter 75 ± 20 g $l^{-1}$, noch bevorzugter 75 ± 15 g $l^{-1}$, am Bevorzugtesten 75 ± 10 g $l^{-1}$ und insbesondere 75 ± 5 g $l^{-1}$. In einer weiteren bevorzugten Ausführungsform ist die Gesamtmassenkonzentration 100 ± 25 g $l^{-1}$, bevorzugter 100 ± 20 g $l^{-1}$, noch bevorzugter 100 ± 15 g $l^{-1}$, am Bevorzugtesten 100 ± 10 g $l^{-1}$ und insbesondere 100 ± 5 g $l^{-1}$. In einer weiteren bevorzugten Ausführungsform ist die Gesamtmassenkonzentration 200 ± 25 g $l^{-1}$, bevorzugter 200 ± 20 g $l^{-1}$, noch bevorzugter 200 ± 15 g $l^{-1}$, am Bevorzugtesten 200 ± 10 g $l^{-1}$ und insbesondere 200 ± 5 g $l^{-1}$.

Die erfindungsgemäße Dialyselösung kann auch weitere osmotisch aktive Substanzen wie beispielsweise Glucose, Polyglucose, Mannitol oder Glycerol enthalten.

Die erfindungsgemäße Dialyselösung enthält vorzugsweise einen oder mehrere Elektrolyte.

Im Sinne dieser Erfindung steht der Ausdruck "Elektrolyt" für eine Substanz, die freie Ionen enthält und elektrische Konduktivität aufweist. Vorzugsweise dissoziiert das Elektrolyt vollständig in Kationen und Anionen ohne den pH-Wert einer wässrigen Zusammensetzung im Wesentlichen zu ändern. Diese Eigenschaft grenzt Elektrolyte von Puffersubstanzen ab. Vorzugsweise liegen die Elektrolyte in einer Konzentration vor, die in einer im Wesentlichen vollständigen Dissoziation in Wasser resultiert.

Bevorzugte Elektrolyte sind ausgewählt aus der Gruppe der Alkalimetalle wie beispielsweise $Na^+$ und $K^+$ und der Erdalkalimetalle wie beispielsweise $Ca^{2+}$ und $Mg^{2+}$. Ein bevorzugtes Anion ist $Cl^-$.

Die erfindungsgemäße Dialyselösung kann weitere Anionen wie beispielsweise Hydrogencarbonat, Dihydrogenphosphat, Hydrogenphosphat, Phosphat, Acetat, Lactat und Pyruvat enthalten; diese Anionen (in geeigneten Kombinationen mit Kationen) werden jedoch aufgrund Ihrer Pufferkapazität im Sinne dieser Erfindung nicht als Elektrolyte sondern als Puffer bezeichnet.

In einer bevorzugten Ausführungsform enthält die erfindungsgemäße Dialyselösung $Na^+$-Ionen. Die Konzentration an $Na^+$-Ionen ist vorzugsweise 10 bis 200 mM oder 50 bis 190 mM, bevorzugter 100 bis 180 mM oder 110 bis 170 mM, noch bevorzugter 115 bis 165 mM oder 120 bis 160 mM, am Bevorzugtesten 125 bis 155 mM und insbesondere

130 bis 150 mM. In einer weiteren bevorzugten Ausführungsform enthält die erfindungsgemäße Dialyselösung keine $Na^+$-Ionen.

In einer bevorzugten Ausführungsform enthält die erfindungsgemäße Dialyselösung $K^+$-Ionen. Die Konzentration an $K^+$-Ionen ist vorzugsweise 0,10 bis 20 mM, bevorzugter 0,25 bis 15 mM, noch bevorzugter 0,50 bis 10 mM, am Bevorzugtesten 0,75 bis 7,5 mM und insbesondere 1,0 bis 5,0 mM. In einer weiteren bevorzugten Ausführungsform ist die Konzentration an $K^+$-Ionen 1,0 $\pm$ 0,75, 2,0 $\pm$ 0,75, 3,0 $\pm$ 0,75, 4,0 $\pm$ 0,75 oder 5,0 $\pm$ 0,75 mM und insbesondere 1,0 $\pm$ 0,50, 2,0 $\pm$ 0,50, 3,0 $\pm$ 0,50, 4,0 $\pm$ 0,50 oder 5,0 $\pm$ 0,50. In einer weiteren bevorzugten Ausführungsform enthält die erfindungsgemäße Dialyselösung keine $K^+$-Ionen.

In einer bevorzugten Ausführungsform enthält die erfindungsgemäße Dialyselösung $Ca^{2+}$-Ionen. Die Konzentration an $Ca^{2+}$-Ionen ist vorzugsweise 0,10 bis 10 mM, bevorzugter 0,20 bis 7,5 mM, noch bevorzugter 0,30 bis 5,0 mM, am Bevorzugtesten 0,40 bis 2,5 mM und insbesondere 0,50 bis 2,0 mM. In einer weiteren bevorzugten Ausführungsform ist die Konzentration an $Ca^{2+}$-Ionen 1,0 $\pm$ 0,75, 2,0 $\pm$ 0,75, 3,0 $\pm$ 0,75, 4,0 $\pm$ 0,75 oder 5,0 $\pm$ 0,75 mM und insbesondere 1,0 $\pm$ 0,50, 2,0 $\pm$ 0,50, 3,0 $\pm$ 0,50, 4,0 $\pm$ 0,50 oder 5,0 $\pm$ 0,50. In einer weiteren bevorzugten Ausführungsform enthält die erfindungsgemäße Dialyselösung keine $Ca^{2+}$-Ionen.

In einer bevorzugten Ausführungsform enthält die erfindungsgemäße Dialyselösung $Mg^{2+}$-Ionen. Die Konzentration an $Mg^{2+}$-Ionen ist vorzugsweise 0,010 bis 10 mM, bevorzugter 0,050 bis 7,5 mM, noch bevorzugter 0,10 bis 5,0 mM, am Bevorzugtesten 0,20 bis 2,5 mM und insbesondere 0,25 bis 1,5 mM. In einer weiteren bevorzugten Ausführungsform ist die Konzentration an $Ca^{2+}$-Ionen 0,25 $\pm$ 0,20, 0,30 $\pm$ 0,20, 0,40 $\pm$ 0,20, 0,50 $\pm$ 0,20 oder 1,0 $\pm$ 0,20 mM und insbesondere 0,25 $\pm$ 0,10, 0,30 $\pm$ 0,10, 0,40 $\pm$ 0,10, 0,50 $\pm$ 0,10 oder 1,0 $\pm$ 0,10 mM. In einer weiteren bevorzugten Ausführungsform enthält die erfindungsgemäße Dialyselösung keine $Mg^{2+}$-Ionen.

In einer bevorzugten Ausführungsform enthält die erfindungsgemäße Dialyselösung $Cl^-$-Ionen. Die Konzentration an $Cl^-$-Ionen ist vorzugsweise 10 bis 300 mM, bevorzugter 25 bis 250 mM, noch bevorzugter 50 bis 200 mM, am Bevorzugtesten 75 bis 150 mM und insbesondere 80 bis 125 mM. In einer weiteren bevorzugten Ausführungsform enthält die erfindungsgemäße Dialyselösung keine $Cl^-$-Ionen.

Die erfindungsgemäße Dialyselösung enthält vorzugsweise einen oder mehrere Puffer.

Geeignete Puffer sind dem Fachmann bekannt. Üblicherweise umfassen Puffer Lactat-, Hydrogencarbonat-, Carbonat-, Dihydrogenphosphat-, Hydrogenphosphat-, Phosphat-, Pyruvat-, Citrat-, Isocitrat-, Succinat-, Fumarat-, Acetat- und Lactat-Salze. Der Fachmann weiß, dass das entsprechende Kation der vorstehend genannten Anionen Bestandteil des Puffers ist, der zur Einstellung des pH-Wertes benutzt wird (z.B. $Na^+$ als Bestandteil des Puffers $NaHCO_3$). Wenn das Puffersalz jedoch in Wasser dissoziiert hat es auch die Wirkung eines Elektrolyts. Für die Zwecke dieser Beschreibung berechnen sich die Konzentrationen an Kationen oder Anionen und die Gesamtkonzentration an Ionen, unabhängig davon, ob sie als Bestandteil von Elektrolyten, Puffern oder anderen Verbindungen (z.B. als Salz der erfindungsgemäßen Copolymere) eingesetzt werden.

In einer bevorzugten Ausführungsform enthält der Puffer Hydrogencarbonat. Hydrogencarbonat ist ein gut verträgliches Puffersystem, das im alkalischen Milieu mit Carbonat und im sauren Milieu mit $H_2CO_3$ bzw. $CO_2$ im Gleichgewicht steht. Neben Hydrogencarbonat sind auch andere Puffer-Systeme einsetzbar, die im Bereich von pH 7 eine Pufferwirkung ausüben; z.B. auch Verbindungen, die im Körper zu Hydrogencarbonat metabolisiert werden wie Lactat oder Pyruvat.

In einer weiteren bevorzugten Ausführungsform enthält der Puffer das Salz einer schwachen Säure, vorzugsweise Lactat. Die Säurestärke ($pK_s$) der schwachen Säure ist vorzugsweise $\leq 5$. Der Puffer kann auch ein Gemisch von Substanzen mit Pufferwirkung sein, z.B. ein Gemisch enthaltend Hydrogencarbonat und ein Salz einer schwachen Säure (z.B. Lactat). Eine geringe Hydrogencarbonat-Konzentration hat den Vorteil, dass der $CO_2$-Druck im Behältnis gering ist. Eine herkömmliche Polyolefin-Folie kann als $CO_2$-Barriere benutzt werden.

In einer bevorzugten Ausführungsform ist die erfindungsgemäße Dialyselösung gepuffert durch Hydrogencarbonat. Die Hydrogencarbonat-Konzentration ist vorzugsweise 1,0 bis 200 mM, bevorzugter 5,0 bis 150 mM, noch bevorzugter 10 bis 100 mM, am Bevorzugtesten 20 bis 75 mM oder 25 bis 50 mM und insbesondere 30 bis 40 mM. In einer weiteren bevorzugten Ausführungsform enthält die erfindungsgemäße Dialyselösung kein Hydrogencarbonat.

In einer bevorzugten Ausführungsform ist die erfindungsgemäße Dialyselösung gepuffert durch Lactat. Die Lactat-Konzentration ist vorzugsweise 1,0 bis 200 mM, bevorzugter 5,0 bis 150 mM, noch bevorzugter 10 bis 100 mM, am Bevorzugtesten 20 bis 75 mM oder 25 bis 50 mM und insbesondere 30 bis 40 mM. In einer weiteren bevorzugten Ausführungsform enthält die erfindungsgemäße Dialyselösung kein Lactat.

In einer bevorzugten Ausführungsform ist die erfindungsgemäße Dialyselösung gepuffert durch Acetat. Die Acetat-Konzentration ist vorzugsweise 1,0 bis 100 mM, bevorzugter 1,0 bis 50 mM, noch bevorzugter 1,0 bis 25 mM, am Bevorzugtesten 1,0 bis 10 mM oder 2,0 bis 7,5 mM und insbesondere 2,5 bis 7,0 mM. In einer weiteren bevorzugten Ausführungsform enthält die erfindungsgemäße Dialyselösung kein Acetat.

Das Gesamtvolumen an Dialyselösung ist nicht beschränkt. Üblicherweise beträgt das Volumen mehrere Liter (geeignetes Verabreichungsvolumen für einen Patienten) bis einige hundert Liter (geeignetes Vorratsvolumen für mehr als einen Patienten).

Wie bereits oben stehend ausgeführt ist unter dem Ausdruck "Dialyselösung" im Sinne dieser Erfindung eine gebrauchsfertige Dialyselösung zu verstehen, d.h. die Dialyselösung kann direkt für die Dialysebehandlung (Hämodialyse oder Peritonealdialyse) verwendet werden.

In einer bevorzugten Ausführungsform ist die erfindungsgemäße Dialyselösung eine Peritonealdialyselösung wie nachstehend beschrieben.

Die Peritonealdialyselösung ist biochemisch so abgestimmt, dass sie die mit Nierenversagen einhergehende metabolische Azidose im Wesentlichen korrigiert. Die Peritonealdialyselösung enthält Hydrogencarbonat vorzugsweise in annähernd physiologischen Konzentrationen. In einer bevorzugten Ausführungsform enthält die Peritonealdialyselösung Hydrogencarbonat in einer Konzentration von ca. 20 bis 30 mM. In einer weiteren bevorzugten Ausführungsform enthält die Peritonealdialyselösung eine Hydrogencarbonat-Konzentration von 25 mM.

Ferner enthält die Peritonealdialyselösung vorzugsweise Kohlenstoffdioxid mit einem Partialdruck ($pCO_2$) von weniger als 60 mmHg. In einer bevorzugten Ausführungsform ist $pCO_2$ der Peritonealdialyselösung im Wesentlichen gleich zum $pCO_2$, der in Blutgefäßen gemessen wird.

Ferner hat die Peritonealdialyselösung vorzugsweise einen pH-Wert von ca. 7,4. Daher ist die Peritonealdialyselösung eine physiologisch verträgliche Lösung.

Die Peritonealdialyselösung enthält vorzugsweise eine schwache Säure mit einem $pK_s \leq 5$.Die schwachen Säuren sind vorzugsweise Verbindungen, die als physiologische Stoffwechselprodukte im Glucose-Metabolismus auftreten. Die schwache Säure ist vorzugsweise ausgewählt aus Gruppe bestehend aus Lactat, Pyruvat, Citrat, Isocitrat, Ketoglutarat, Succinat, Fumarat, Malat und Oxaloacetat. Diese Säuren können entweder allein oder als Gemisch in der Peritonealdialyselösung enthalten sein. Die schwachen Säuren sind vorzugsweise in einer Konzentration von 10 bis 20 mEq $l^{-1}$ und im Wesentlichen als Natrium-Salze in der Peritonealdialyselösung enthalten. In der Peritonealdialyselösung ist die schwache Säure vorzugsweise in einer Menge enthalten, die der täglichen metabolischen Wasserstoffproduktion von ca. 1 mEq/kg/Tag entspricht.

Die Peritonealdialyselösung enthält wenigstens ein erfindungsgemäßes Copolymer wie oben stehend definiert.

Die erfindungsgemäße Peritonealdialyselösung enthält vorzugsweise eine Konzentration an Hydrogencarbonat und weist einen $pCO_2$ auf, wie sie bei gesunden, nicht-niereninsuffizienten Patienten gemessen werden. Die schwache Säure diffundiert entlang des Konzentrationsgradienten von der Dialyselösung ins Blut des Dialysepatienten und korrigiert somit die metabolische Azidose der Dialysepatienten.

Ein weiterer Gegenstand dieser Erfindung betrifft Mehrkomponenten-Systeme zur Herstellung der oben beschriebenen gebrauchsfertigen Dialyselösungen. Die Herstellung erfolgt vorzugsweise in einer detailliert beschriebenen Art und Weise, d.h. durch Befolgung einer entsprechenden Anleitung (Protokoll). Besagte Herstellung kann manuell, z.B. durch Mischen einzelner Komponenten oder Verdünnen einer Komponente mit Wasser, erfolgen. Die Herstellung kann jedoch auch automatisiert erfolgen, z.B. mittels einer Vorrichtung die für diesen Prozess geeignet ist und kommerziell erhältlich sein kann. Die Herrichtung muss nicht zwangsläufig zu einer Dialyselösung mit statischer (gleich bleibender) Zusammensetzung führen sondern kann auch zu einer Dialyselösung führen, die kontinuierlich ihre Zusammensetzung ändert, wobei diese Änderung durch eine geeignete Vorrichtung überwacht werden kann. Beispielsweise kann das erfindungsgemäße Copolymer in einer Dialyselösung enthalten sein, die kontinuierlich während der Dialysebehandlung verdünnt wird, so dass der Patient einer abnehmenden Copolymer-Konzentration ausgesetzt ist.

In einer bevorzugten Ausführungsform sind die erfindungsgemäßen Dialyselösungen zur Verwendung in der Behandlung der Niereninsuffizienz geeignet.

In einer weiteren bevorzugten Ausführungsform sind die erfindungsgemäßen Dialyselösungen zur Verwendung in der Dialysebehandlung geeignet.

In einer weiteren bevorzugten Ausführungsform sind die erfindungsgemäßen Dialyselösungen zur Verwendung in der Hämodialyse- und / oder Peritonealdialysebehandlung geeignet.

Ein weiterer Gegenstand dieser Erfindung betrifft ein Kit, das für die Herstellung der oben beschriebenen erfindungsgemäßen Dialyselösungen konfiguriert ist, wobei das Kit

- eine erste Komponente,
- eine zweite Komponente und
- ggf. eine weitere oder mehrere weitere Komponenten

umfasst, und
die Herstellung der erfindungsgemäßen Dialyselösung durch Mischen der ersten Komponente mit der zweiten Komponente und ggf. der/ den weiteren Komponente(n) erfolgt.

Das Kit umfasst wenigstens eine erste Komponente und eine zweite Komponente. Das Kit kann auch weitere Komponenten umfassen, z.B. eine dritte und eine vierte Komponente. Vorzugsweise besteht das Kit aus zwei Komponenten, die vorzugsweise voneinander verschieden sind.

Im Sinne dieser Erfindung umfasst der Ausdruck "Komponente" flüssige, halbfeste oder feste Zusammensetzungen, die zueinander gleich oder voneinander verschieden sein können, wobei durch Mischen aller Komponenten des Kits die erfindungsgemäße gebrauchsfertige Dialyselösung erhalten wird.

Die erste und die zweite Komponente können, unabhängig voneinander, fest, halbfest oder flüssig sein. Im Falle, dass die Komponenten flüssig sind, können sie Lösungen oder Dispersionen (z.B. Dispersionen oder Suspensionen) sein.

In einer bevorzugten Ausführungsform ist die erste Komponente flüssig, vorzugsweise reines Wasser oder eine wässrige Lösung, und die zweite Komponente ist ebenfalls flüssig. In einer weiteren bevorzugten Ausführungsform ist die erste Komponente flüssig, vorzugsweise reines Wasser oder eine wässrige Lösung, und die zweite Komponente ist fest, vorzugsweise ein pulverförmiges Gemisch.

Die erste Komponente ist vorzugsweise eine Lösung, die osmotisch wirksame Substanzen (z.B. erfindungsgemäßes Copolymer), Calcium-Ionen, Magnesium-Ionen, Hydronium-Ionen und Chlorid-Ionen enthält.

Das erfindungsgemäße Kit kann verschiedenartig ausgestaltet sein. Beispielsweise können die einzelnen Komponenten in voneinander separierten Behältnissen (z.B. einzelne Beutel) vorliegen. Das erfindungsgemäße Kit ist jedoch vorzugsweise ein Mehrkammer-Behältnis-System (z.B. flexibles oder starres Mehrkammer-Behältnis-System), vorzugsweise ein flexibles Mehrkammer-Beutel-System.

Das erfindungsgemäße Kit ist vorzugsweise ein Mehrkammer-Behältnis-System, das die erste Komponente, die zweite Komponente und ggf. eine oder mehrere weitere Komponenten in Kammern enthält, die voneinander durch lösbare bzw. brechbare Trennsysteme (z.B. Trennbrechteile) getrennt sind, wobei die erste Komponente, die zweite Komponente und ggf. die eine oder mehreren weiteren Komponenten nach dem Lösen bzw. Brechen des Trennsystems miteinander gemischt werden können, um die erfindungsgemäße Dialyselösung zu erhalten.

Das Mehrkammer-Behältnis kann als Kunststoffbehältnis vorliegen (z.B. Mehrkammer-Kunststoffbeutel), der jeweils eine abgetrennte Kammer für jede einzelne Komponente enthält. Vorzugsweise enthält das Kunststoffbehältnis die einzelnen Komponentenlösungen in Kammern, die jeweils durch Trennelemente voneinander abgetrennt sind.

Das Mehrkammer-Behältnis ist vorzugsweise ein Zweikammer-Beutel mit einer ersten Kammer und einer zweiten Kammer, wobei die Kammern durch ein lösbares bzw. brechbares Trennsystem voneinander getrennt sind, und die erste Kammer die erste Komponente enthält und die zweite Kammer die zweite Komponente enthält. Das Lösen bzw. Brechen des Trennsystems führt zum Mischen der beiden Komponenten und resultiert in der gebrauchsfertigen Dialyselösung. Die erste Kammer und die zweite Kammer sind im Mehrkammer-Behältnis vorzugsweise angrenzend angeordnet und durch das Trennsystem voneinander abgetrennt. Das Trennsystem ist vorzugsweise eine Trennnaht (z.B. lösbare oder brechbare Schweißnaht). Die Trennnaht öffnet sich vorzugsweise durch das Anlegen eines Druckes auf eine der Kammern, woraufhin die Trennnaht bricht bzw. sich löst und sich der Inhalt der beiden Kammern mischt und das Gemisch als gebrauchsfertige Dialyselösung in der Dialysebehandlung eingesetzt werden kann.

Die erste Komponente des erfindungsgemäßen Kits ist vorzugsweise eine sterile Lösung, die eine Säure enthält und einen pH-Wert $\leq$ 6,0 aufweist; die zweite Komponente ist vorzugsweise ebenfalls eine sterile Lösung, die vorzugsweise einen Puffer enthält und einen pH-Wert $\geq$ 7,0 aufweist.

Das erfindungsgemäße Copolymer kann in der ersten Komponente oder in der zweiten Komponente als auch in beiden Komponenten in gleichen oder verschiedenen Konzentrationen enthalten sein. In einer bevorzugten Ausführungsform ist das erfindungsgemäße Copolymer nur in der ersten (sauren) Komponente enthalten. In einer weiteren bevorzugten Ausführungsform ist das erfindungsgemäße Copolymer nur in der zweiten (basischen) Komponente enthalten. Die erste Komponente und/ oder die zweite Komponente und/ oder die ggf. weitere(n) Komponente(n) können einen oder mehrere Elektrolyte enthalten.

Der Fachmann erkennt, dass das Mischen der einzelnen Komponenten üblicherweise einen Verdünnungseffekt für den Fall nach sich zieht, dass die Komponenten die Inhaltsstoffe in unterschiedlichen Konzentrationen enthalten. Falls beispielsweise das erfindungsgemäße Copolymer ausschließlich in einer der Komponenten enthalten ist, führt das Mischen dieser Komponente mit wenigstens einer anderen Komponente zu einem Anstieg des Volumens in Bezug auf die Copolymer-Komponente und somit zu einer Verdünnung, d.h. Abnahme der Copolymer-Konzentration. Folglich enthält die Komponente das erfindungsgemäße Copolymer in einer höheren Konzentration als die gebrauchsfertige Dialyselösung.

Vorzugsweise ist die Konzentration an erfindungsgemäßen Copolymer in der Komponente nahe an der Sättigungskonzentration bei einer Temperatur von 5 °C um eine ausreichende Lagerstabilität bei höheren Temperaturen sicher zu stellen.

In einer bevorzugten Ausführungsform ist die Gesamtmassenkonzentration an erfindungsgemäßen Copolymer in der Komponente 0,01 g $l^{-1}$ bis 1,0 kg $l^{-1}$, bevorzugter 0,1 bis 750 g $l^{-1}$, noch bevorzugter 1,0 bis 500 g $l^{-1}$, am Bevorzugtesten 10 bis 250 g $l^{-1}$ und insbesondere 100 bis 200 g $l^{-1}$. In einer weiteren bevorzugten Ausführungsform ist die Gesamtmassenkonzentration an erfindungsgemäßen Copolymer in der Komponente 25 $\pm$ 24 g $l^{-1}$, bevorzugter 25 $\pm$ 20 g $l^{-1}$, noch bevorzugter 25 $\pm$ 15 g $l^{-1}$, am Bevorzugtesten 25 $\pm$ 10 g $l^{-1}$ und insbesondere 25 $\pm$ 5 g $l^{-1}$. In einer weiteren bevorzugten Ausführungsform ist die Gesamtmassenkonzentration an erfindungsgemäßen Copolymer in der Komponente 50 $\pm$ 25 g $l^{-1}$, bevorzugter 50 $\pm$ 20 g $l^{-1}$, noch bevorzugter 50 $\pm$ 15 g $l^{-1}$, am Bevorzugtesten 50 $\pm$ 10 g $l^{-1}$

und insbesondere 50 ± 5 g $l^{-1}$. In einer weiteren bevorzugten Ausführungsform ist die Gesamtmassenkonzentration an erfindungsgemäßen Copolymer in der Komponente 75 ± 25 g $l^{-1}$, bevorzugter 75 ± 20 g $l^{-1}$, noch bevorzugter 75 ± 15 g $l^{-1}$, am Bevorzugtesten 75 ± 10 g $l^{-1}$ und insbesondere 75 ± 5 g $l^{-1}$. In einer weiteren bevorzugten Ausführungsform ist die Gesamtmassenkonzentration an erfindungsgemäßen Copolymer in der Komponente 100 ± 25 g $l^{-1}$, bevorzugter 100 ± 20 g $l^{-1}$, noch bevorzugter 100 ± 15 g $l^{-1}$, am Bevorzugtesten 100 ± 10 g $l^{-1}$ und insbesondere 100 ± 5 g $l^{-1}$. In einer weiteren bevorzugten Ausführungsform ist die Gesamtmassenkonzentration an erfindungsgemäßen Copolymer in der Komponente 200 ± 25 g $l^{-1}$, bevorzugter 200 ± 20 g $l^{-1}$, noch bevorzugter 200 ± 15 g $l^{-1}$, am Bevorzugtesten 200 ± 10 g $l^{-1}$ und insbesondere 200 ± 5 g $l^{-1}$.

In einer bevorzugten Ausführungsform enthält die zweite Komponente die Gesamtmenge an erfindungsgemäßen Copolymer und einen geeigneten Puffer, der den pH-Wert der zweiten Komponente auf über 7,0, bevorzugter auf über 7,5, noch bevorzugter auf über 8,0, am Bevorzugtesten auf über 8,5 und insbesondere auf über 9,0 einstellt. Dies kann vorzugsweise durch Hydrogencarbonat-Ionen erreicht werden, die beispielsweise in Form von dissoziiertem Natrium-Hydrogencarbonat und oder Kalium-Hydrogencarbonat vorliegen können. In einer weiteren bevorzugten Ausführungsform ist die zweite Komponente fest und umfasst ein pulverförmiges Gemisch enthaltend wenigstens ein erfindungsgemäßes Copolymer und wenigstens einen Puffer, z.B. Natrium- und/ oder Kalium-Hydrogencarbonat.

Der Mehrkammer-Beutel ist vorzugsweise geeignet für die Herstellung einer Dialyselösung, die zur Verwendung in der Peritonealdialysebehandlung verwendet werden kann, und die folgenden Inhaltsstoffe vorzugsweise in folgenden Konzentrationen enthält:

| | |
|---|---|
| $Ca^{2+}$ | 0,5 bis 5 mval $l^{-1}$; |
| $Mg^{2+}$ | 0 bis 3,0 mval $l^{-1}$; |
| $Cl^{-}$ | 90,5 bis 121 mval $l^{-1}$; |
| $K^{+}$ | 0 bis 4,0 mval $l^{-1}$; |
| $HCO_3^{-}$ | 25 bis 40 mval $L^{-1}$; wobei |

eine Kammer des Mehrkammer-Beutel-Systems ein erstes saures Konzentrat und eine andere Kammer ein zweites basisches Konzentrat enthält; wobei das saure Konzentrat $Ca^{2+}$-Ionen enthält und das basische Konzentrat $HCO_3^{-}$-Ionen aber keine $Ca^{2+}$-Ionen enthält; und die zwei Konzentrate nach Lösen bzw. Brechen des Trennsystems (z.B. Trennnaht) miteinander gemischt werden können; wobei das Mischen der zwei Konzentrate zur Herstellung der gebrauchsfertigen Dialyselösung führt und der pH der gebrauchsfertigen Dialyselösung 7,2 bis 7,4 ist.

Vorzugsweise enthält das basische Konzentrat wenigstens ein erfindungsgemäßes Copolymer, wohingegen das saure Konzentrat kein erfindungsgemäßes Copolymer und kein weiteres osmotisch aktives Polymer (z.B. Polyglucose) enthält.

Vorzugsweise enthält das basische Konzentrat eine Menge an Hydrogencarbonat, die zu einer Hydrogencarbonat-Konzentration der gebrauchsfertigen Dialyselösung von wenigstens 20 mM führt. Vorzugsweise ist die Hydrogencarbonat-Konzentration der basischen Komponente so hoch, dass die gebrauchsfertige Dialyselösung eine Hydrogencarbonat-Konzentration von 25 bis 40 mM aufweist.

Der pH-Wert des basischen, gepufferten zweiten Konzentrats wird vorzugsweise mit Salzsäure eingestellt.

Vorzugsweise werden die beiden Konzentrate in einem Volumenverhältnis von 10:1 bis 1:10 oder 8:1 bis 1:8, bevorzugter 5:1 bis 1:5 oder 3:1 bis 1:3, noch bevorzugter 2:1 bis 1:2 und insbesondere 1:1 miteinander gemischt.

Ein weiterer Gegenstand betrifft ein Verfahren zur Herstellung einer Dialyselösung, worin das gewünschte Mischverhältnis automatisch durch eine Dialysemaschine oder einen Peritonealdialyse-Cycler erfolgt.

In einer bevorzugten Ausführungsform betrifft die Erfindung eine feste Zusammensetzung, die zur Herstellung der erfindungsgemäßen Dialyselösung durch das Lösen in einem definierten Volumen eines Lösungsmittels (z.B. Wasser) geeignet ist. Vorzugsweise ist die feste Zusammensetzung eine vorstehend beschriebene Komponente und somit ein Bestandteil des erfindungsgemäßen Kits.

Die erfindungsgemäße feste Zusammensetzung enthält vorzugsweise ein Hydrogencarbonat-Salz wie beispielsweise Natrium- oder Kalium-Hydrogencarbonat. Das Stoffmengenverhältnis von Hydrogencarbonat zu erfindungsgemäßen Copolymer in der festen Zusammensetzung ist vorzugsweise 1:100 bis 100:1, bevorzugter 1:50 bis 50:1, noch bevorzugter 1:25 bis 25:1, am Bevorzugtesten 1:10 bis 10:1 und insbesondere 1:5 bis 5:1.

Das definierte Volumen an Lösungsmittel, das zur Herstellung der erfindungsgemäßen Dialyselösung durch Lösen der festen Zusammensetzung benötigt wird ist vorzugsweise 1,0 bis 2000 l. Vorzugsweise ist das Lösungsmittel gereinigtes Wasser, sterilisiertes Wasser oder Wasser für Injektionszwecke, das ggf. einen oder mehrere der oben beschriebenen Elektrolyte, eine oder mehrere osmotisch wirksame Substanzen (z.B. wenigstens ein erfindungsgemäßes Copolymer) und/ oder einen oder mehrere der oben beschriebenen Puffer enthalten kann.

Ein weiterer Gegenstand dieser Erfindung betrifft die Verwendung wenigstens eines erfindungsgemäßen Copolymers zur Herstellung der erfindungsgemäßen Dialyselösung (Hämodialyselösung oder Peritonealdialyselösung);

wobei die Dialyselösung vorzugsweise zur Verwendung in der Behandlung von Krankheiten des Urogenitalsystems, bevorzugter zur Verwendung in der Dialysebehandlung und insbesondere zur Verwendung in der Hämodialysebehandlung oder Peritonealdialysebehandlung geeignet ist.

Ein weiterer Gegenstand dieser Erfindung betrifft die Verwendung eines erfindungsgemäßen Kits zur Herstellung der erfindungsgemäßen Dialyselösung (Hämodialyselösung oder Peritonealdialyselösung); wobei die Dialyselösung vorzugsweise zur Verwendung in der Behandlung von Krankheiten des Urogenitalsystems, bevorzugter zur Verwendung in der Dialysebehandlung und insbesondere zur Verwendung in der Hämodialysebehandlung oder Peritonealdialysebehandlung geeignet ist.

Ein weiterer Gegenstand dieser Erfindung betrifft die Verwendung einer erfindungsgemäßen festen Zusammensetzung zur Herstellung der erfindungsgemäßen Dialyselösung (Hämodialyselösung oder Peritonealdialyselösung); wobei die Dialyselösung vorzugsweise zur Verwendung in der Behandlung von Krankheiten des Urogenitalsystems, bevorzugter zur Verwendung in der Dialysebehandlung und insbesondere zur Verwendung in der Hämodialysebehandlung oder Peritonealdialysebehandlung geeignet ist.

## Beispiele

### Beispiel 1

β-Cyclodextrin (2 g) wird in wasserfreiem Dimethylformamid (10 mL) gelöst. Zu dieser Lösung wird Epichlorhydrin (1 mmol) langsam zugegeben. Die Lösung wird für 4 Stunden bei 60 °C gerührt und anschließend über Wasser gekühlt. Zu dieser gekühlten Lösung wird Ethanol zugegeben und der entstehende Niederschlag wird abfiltriert, mit kaltem Ethanol gewaschen und anschließend in Vakuum getrocknet.

### Beispiel 2

Das Natriumsalz von (2-Carboxyethyl)-β-cyclodextrin mit einem Substitutitonsgrad von -2,5 (3 g) wird in wasserfreiem Dimethylformamid (10 mL) gelöst. Zu dieser Lösung wird Epichlorhydrin (1,2 mmol) langsam zugegeben. Die Lösung wird über Nacht bei 60 °C gerührt und anschließend auf Raumtemperatur abgekühlt. Die Lösung wird im Rotationsverdampfer eingeengt. Der entstandene Niederschlag wird abfiltriert, mit kaltem Wasser gewaschen und anschließend im Vakkum getrocknet.

### Beispiel 3

6-O-Maltosyl-β-Cyclodextrin mit einem Substitutionsgrad von 1,5 (3 g) wird in 10 mL wasserfreiem Dimethylformamid gelöst. Zu dieser Lösung wird Tetramethylendiisocyanat (1,6 mmol) langsam zugegeben. Die Lösung wird über Nacht bei 60 °C gerührt und anschließend auf Raumtemperatur abgekühlt. Die Lösung wird im Rotationsverdampfer eingeengt. Der entstandene Niederschlag wird abfiltriert, mit kaltem Ethanol gewaschen und anschließend im Vakkum getrocknet.

### Osmotische Wirksamkeit

Von Beispielverbindung 2 wird eine halbgesättigte Lösung in destilliertem Wasser hergestellt. Diese Lösung (50 mL) wird in eine Kammer einer Pfeffer'schen Zelle eingebracht (Kammer A). In die andere Kammer (Kammer B) der Pfeffer'schen Zelle wird destilliertes Wasser eingebracht (50 mL). Beide Kammern sind über eine semipermeable Membran verbunden (Cellulosemembran, Cut Off 1500 Da). Innerhalb von vier Stunden gehen ca. 4,7 mL der Lösung der Kammer B in die Lösung der Kammer A über.

## Patentansprüche

1. Copolymer enthaltend

   wenigstens zwei Cyclodextrin-Monomere und
   wenigstens einen Linker;

   zur Verwendung in der Behandlung von Krankheiten des Urogenitalsystems, **dadurch gekennzeichnet, dass** die Wasserlöslichkeit des Copolymers bei 23 °C $\geq 5{,}0\ g\ l^{-1}$ ist.

**2.** Copolymer nach Anspruch 1, **dadurch gekennzeichnet, dass** der Stoffmengenanteil der Linker 20 bis 95 mol% beträgt, bezogen auf die Gesamtstoffmenge der Monomere.

**3.** Copolymer nach Anspruch 1 oder 2, **dadurch gekennzeichnet, dass** der Stoffmengenanteil der Cyclodextrin-Monomere 5,0 bis 80 mol% beträgt, bezogen auf die Gesamtstoffmenge der Monomere.

**4.** Copolymer nach einem oder mehreren der Ansprüche 1 bis 3, **dadurch gekennzeichnet, dass** die Summe aus dem Stoffmengenanteil der Linker und dem Stoffmengenanteil der Cyclodextrin-Monomere ≥ 25 mol% ist.

**5.** Copolymer nach einem oder mehreren der Ansprüche 1 bis 4, **dadurch gekennzeichnet, dass** die Osmolalität einer 50 mM wässrigen Lösung an Copolymer ≥ 50 mosm $kg^{-1}$ ist.

**6.** Copolymer nach einem oder mehreren der Ansprüche 1 bis 5, **dadurch gekennzeichnet, dass** die Cyclodextrin-Monomere, gleich oder verschieden, abgeleitet sind von α-, β- oder γ-Cyclodextrin-Verbindungen, die jeweils unsubstituiert oder ein- oder mehrfach mit gleichen oder verschiedenen Resten substituiert sind.

**7.** Copolymer nach Anspruch 6, **dadurch gekennzeichnet, dass** die Reste abgeleitet sind von wenigstens einem Funktionalisierungsmittelausgewählt aus der Gruppe bestehend aus $C_{1-6}$-Alkyl-Y, $C_{3-7}$-Cycloalkyl-Y, $C_{1-6}$-Alkyl-C(=O)-Y, $C_{3-7}$-Cycloalkyl-C(=O)-Y, Y-$C_{1-6}$-Alkyl-C(=O)-OH, Y-$C_{3-7}$-Cycloalkyl-C(=O)-OH, Y-$C_{1-6}$-Alkyl-C(=O)-O-$C_{1-6}$-Alkyl, Y-$C_{3-7}$-Cycloalkyl-C(=O)-O-$C_{3-7}$-Cycloalkyl, $[C_{1-6}$-Alkyl-C(=O)$]_2$O, $[C_{3-7}$-Cycloalkyl-C(=O)$]_2$O, Dihydrofuran-2,5-dion, Furan-2,5-dion, Dihydro-2H-pyran-2,6(3H)-dion, Oxiran, $C_{1-6}$-Alkyloxiran, $C_{3-7}$-Cycloalkyloxiran, Di-$C_{1-6}$-Alkyloxiran, Di-$C_{3-7}$-Cycloalkyloxiran, 2-Chlorethanol, Di-$C_{1-6}$-Alkylsulfat, Di-$C_{3-7}$-Cycloalkylsulfat, Di-$C_{1-6}$-Alkylphosphorochloridat, Di-$C_{3-7}$-Cycloalkylphosphorochloridat, $NH_3$, ($C_{1-6}$-Alkyl)$NH_2$, ($C_{3-7}$-Cycloalkyl)$NH_2$, ($C_{1-6}$-Alkyl)$_2$NH, ($C_{3-7}$-Cycloalkyl)$_2$NH, ($C_{1-6}$-Alkyl)$_3$N, ($C_{3-7}$-Cycloalkyl)$_3$N, $[(Y-C_{1-6}$-Alkyl)N($C_{1-6}$-Alkyl)$_3]^+$ Halogenid, 1,3-Propansulton und 1,4-Butansulton; wobei $C_{1-6}$-Alkyl und $C_{3-7}$-Cycloalkyl, unabhängig voneinander, unsubstituiert oder ein- oder mehrfach mit gleichen oder verschiedenen Resten Z substituiert sein können, wobei die weiteren Substituenten Z zu keiner oder zu keiner wesentlichen Vernetzung der Cyclodextrin-Monomere bzw. -Verbindungen führen; wobei die Substituenten Z, unabhängig voneinander, vorzugsweise ausgewählt sind aus der Gruppe bestehend aus -OH, =O, -C(=O)OH, -C(=O)$OCH_3$, -C(=O)$OCH_2CH_3$, -$NH_2$, -NH($CH_3$), -N$(CH_3)_2$, -N$(CH_3)_3^+A^-$, -NH($CH_2CH_3$), -N$(CH_2CH_3)_2$ und -N$(CH_2CH_3)_3^+A^-$; wobei $A^-$ für ein anorganisches oder organisches Anion steht, das vorzugsweise ausgewählt ist aus der Gruppe bestehend aus $Cl^-$, $Br^-$, $I^-$, Dihydrogenphosphat, Hydrogenphosphat, Phosphat, Hydrogencarbonat, Carbonat, Acetat, Lactat und Citrat oder für andere physiologisch verträgliche Anionen steht;und die Reste Y für Abgangsgruppen stehen.

**8.** Copolymer nach einem oder mehreren der Ansprüche 1 bis 7, **dadurch gekennzeichnet, dass** die Linker abgeleitet sind von bi- und/ oder polyfunktionellen Vernetzungsmitteln.

**9.** Copolymer nach einem oder mehreren der Ansprüche 1 bis 8 zur Verwendung in der Behandlung von Niereninsuffizienz.

**10.** Copolymer nach einem oder mehreren der Ansprüche 1 bis 9 zur Verwendung in der Dialysebehandlung.

**11.** Hämodialyse- oder Peritonealdialyselösung enthaltend wenigstens ein Copolymer nach einem oder mehreren der Ansprüche 1 bis 10.

**12.** Kit, das für die Herstellung der Hämodialyse- oder Peritonealdialyselösungg nach Anspruch 11 konfiguriert ist, umfassend

- eine erste Komponente,
- eine zweite Komponente und
- ggf. eine oder mehrere weitere Komponenten; wobei

durch Mischen der ersten Komponente mit der zweiten Komponente, und ggf. mit der weiteren oder den weiteren Komponenten, die Hämodialyse- oder Peritonealdialyselösung nach Anspruch 11 erhalten wird.

**13.** Feste Zusammensetzung geeignet zur Herstellung der Hämodialyse- oder Peritonealdialyselösung nach Anspruch 11, **dadurch gekennzeichnet, dass** die Hämodialyse- oder Peritonealdialyselösung nach Anspruch 11 durch Lösen der feste Zusammensetzung in einem Lösungsmittel erhalten wird.

14. Verwendung wenigstens eines Copolymers nach einem oder mehreren der Ansprüche 1 bis 10 oder eines Kits nach Anspruch 12 oder einer festen Zusammensetzung nach Anspruch 13 zur Herstellung der Hämodialyse- oder Peritonealdialyselösung nach Anspruch 11.

## Claims

1. A copolymer, containing
at least two cyclodextrin monomers and
at least one linker;
for use in the treatment of diseases of the urogenital system, **characterized in that** the water solubility of the copolymer is ≥5.0 $gL^{-1}$ at 23°C.

2. The copolymer according to claim 1, **characterized in that** the substance quantity of the linkers is 20 to 95 mol%, based on the total substance quantity of monomers.

3. The copolymer according to claim 1 or 2, **characterized in that** the substance quantity of the cyclodextrin monomers is 5.0 to 80 mol% based on the total substance quantity of monomers.

4. The copolymer according to any one or more of claims 1 to 3, **characterized in that** the sum of the substance quantity of the linkers and the substance quantity of the cyclodextrin monomers is ≥25 mol%.

5. The copolymer according to any one or more of claims 1 to 4, **characterized in that** the osmolality of a 50-mM aqueous solution of copolymer is ≥50 mosm $kg^{-1}$.

6. The copolymer according to any one or more of claims 1 to 5, **characterized in that** the cyclodextrin monomers are the same or different and are derived from α-, β- or γ-cyclodextrin compounds, each being unsubstituted or substituted one or more times with the same or different radicals.

7. The copolymer according to claim 6, **characterized in that** the radicals are derived from at least one functionalization agent selected from the group consisting of $C_{1-6}$-alkyl-Y, $C_{3-7}$-cycloalkyl-Y, $C_{1-6}$-alkyl-C(=O)-Y, $C_{3-7}$-cycloalkyl-C(=O)-Y, Y-$C_{1-6}$-alkyl-C(=O)-OH, Y-$C_{3-7}$-cycloalkyl-C(=O)-OH, Y-$C_{1-6}$-alkyl-C(=O)-O-$C_{1-6}$-alkyl, Y-$C_{3-7}$-cycloalkyl-C(=O)-O-$C_{3-7}$-cycloalkyl, $[C_{1-6}$-alkyl-C(=O)$]_2$O, $[C_{3-7}$-cycloalkyl-C(=O)$]_2$O, dihydrofuran-2,5-dione, furan-2,5-dione, dihydro-2H-pyran-2,6(3H)-dione, oxirane, $C_{1-6}$-alkyloxirane, $C_{3-7}$-cycloalkyloxirane, di-$C_{1-6}$-alkyloxirane, di-$C_{3-7}$-cycloalkyloxirane, 2-chlorethanol, di-$C_{1-6}$-alkyl sulfate, di-$C_{3-7}$-cycloalkyl sulfate, di-$C_{1-6}$-alkyl phosphorochloridate, di-$C_{3-7}$-cycloalkyl phosphorochloridate, $NH_3$, ($C_{1-6}$-alkyl)$NH_2$, ($C_{3-7}$-cycloalkyl)$NH_2$, ($C_{1-6}$-alkyl)$_2$NH, ($C_{3-7}$-cycloalkyl)$_2$NH, ($C_{1-6}$-alkyl)$_3$N, [(Y-$C_{1-6}$-alkyl)$_3$]$^+$ halide, 1,3-propanesultone and 1,4-butanesultone; wherein $C_{1-6}$-alkyl and $C_{3-7}$-cycloalkyl, independently of one another, may be unsubstituted or substituted one or more times with the same or different Z radicals, wherein the additional substituents Z do not result in any or any significant crosslinking of the cyclodextrin monomers and/or compounds; wherein the substituents Z, independently of one another, are preferably selected from the group consisting of OH, =O, -C(=O)OH, (=O)$OCH_3$, -C(=O)$OCH_2CH_3$, -$NH_2$, -NH($CH_3$), -N$(CH_3)_2$, -N $(CH_3)_3{}^+A^-$, -NH($CH_2CH_3$), -N$(CH_2CH_3)_2$ and -N$(CH_2CH_3)_3{}^+A^-$; wherein $A^-$ stands for an organic or inorganic anion,which is preferably selected from the group consisting of $Cl^-$, $Br^-$, $I^-$, dihydrogen phosphate, hydrogen phosphate, phosphate, bicarbonate, carbonate, acetate, lactate and citrate or for other physiologically compatible anions; and the Y radicals stand for departing groups.

8. The copolymer according to any one or more of claims 1 to 7, **characterized in that** the linkers are derived from bifunctional and/or polyfunctional crosslinking agents.

9. The copolymer according to any one or more of claims 1 to 8 for use in the treatment of renal insufficiency.

10. The copolymer according to any one or more of claims 1 to 9 for use in dialysis treatment.

11. A hemodialysis or peritoneal dialysis solution, containing at least one copolymer according to any one or more of claims 1 to 10.

12. A kit configured for production of hemodialysis solution or peritoneal dialysis solution according to claim 11, comprising

- a first component,
- a second component and
- optionally one or more additional components; wherein

the hemodialysis solution or peritoneal dialysis solution according to claim 11 is obtained by mixing the first component with the second component and optionally with the additional or other components with which the hemodialysis solution or peritoneal dialysis solution according to claim 11 is obtained.

**13.** A solid composition suitable for producing the hemodialysis solution or peritoneal dialysis solution according to claim 11, **characterized in that** the hemodialysis solution or peritoneal dialysis solution according to claim 11 is obtained by dissolving the solid components in a solvent.

**14.** Use of at least one copolymer according to any one or more of claims 1 to 10 or a kit according to claim 12 or a solid composition according to claim 13, for preparing the hemodialysis solution or peritoneal dialysis solution according to claim 11.

**Revendications**

**1.** Copolymère contenant
au moins deux monomères de cyclodextrine et
au moins un liant ;
destiné à être utilisé dans le traitement des maladies du système urogénital, **caractérisé en ce que** la solubilité dans l'eau du copolymère à 23 °C est ≥ 5,0 g $l^{-1}$.

**2.** Copolymère selon la revendication 1, **caractérisé en ce que** la fraction molaire des liants est de 20 à 95 % en moles par rapport à la fraction molaire totale des monomères.

**3.** Copolymère selon la revendication 1 ou 2, **caractérisé en ce que** la fraction molaire des liants est de 5,0 à 80 % en moles par rapport à la fraction molaire totale des monomères.

**4.** Copolymère selon une ou plusieurs des revendications 1 à 3, **caractérisé en ce que** la somme de la fraction molaire des liants et de la fraction molaire des monomères de cyclodextrine est ≥ 25 % en moles.

**5.** Copolymère selon une ou plusieurs des revendications 1 à 4, **caractérisé en ce que** l'osmolarité d'une solution aqueuse de 50 mM de copolymère est ≥ 50 mosm $kg^{-1}$.

**6.** Copolymère selon une ou plusieurs des revendications 1 à 5, **caractérisé en ce que** les monomères de cyclodextrine, identiques ou différents, sont dérivés de composés de α-, β- ou y-cyclodextrine qui sont respectivement insubstitués ou substitués une ou plusieurs fois avec des radicaux identiques ou différents.

**7.** Copolymère selon la revendication 6, **caractérisé en ce que** les radicaux sont sélectionnés parmi au moins un agent de fonctionnalisation du groupe composé de $C_{1-6}$-alkyle-Y, $C_{3-7}$-cycloalkyle-Y, $C_{1-6}$-alkyle-C(=0)-Y, $C_{3-7}$-cycloalkyle-C(=0)-Y, Y- $C_{1-6}$-alkyle-C(=0)-OH, Y- $C_{3-7}$-cycloalkyle-C(=0)-OH, Y- $C_{1-6}$-alkyle-C(=0)-0- $C_{1-6}$-alkyle,Y-C3-7-cycloalkyle-C-O- $C_{3-7}$-cycloalkyle, [$C_{1-6}$-alkyle-C(=0)]$_2$0, [$C_{3-7}$-cycloalkyle-C(=0)]$_2$0, di-hydrofurane-2,5-dione, furane-2,5-dione, dihydro-2H-pyrane-2,6(3H)-dione, oxirane, $C_{1-6}$-alkyloxirane, $C_{3-7}$-cycloalkyloxirane, Di-$C_{1-6}$-alkyloxirane, Di- $C_{3-7}$-cycloalkyloxirane, 2-chloréthanol, Di- $C_{3-7}$-alkylsulfate, Di-$C_{3-7}$-cycloalkylsulfate, Di-$C_{1-6}$-alkylphosphorochloridate, Di-$C_{3-7}$-cycloalkyle-phosphorochloridate, NH3, ($C_{1-6}$-alkyle)NH2, ($C_{3-7}$-cycloalkyle)NH2, $(C_{1-6}\text{-alkyle})_2NH$, $(C_{3-7}\text{-cycloalkyle})_2NH$, ($C_{1-6}$-alkyle)3N, $(C_{3-7}\text{-cycloalkyle})_3N$, [(Y- $C_{1-6}$-alkyle)NC1-6-alkyle)$_3$]+ halogénure, 1,3-propane sultone et 1,4-butane sultone ; sachant que $C_{1-6}$-alkyle et $C_{3-7}$-cycloalkyle, peuvent être insubstitués l'un par rapport à l'autre ou substitués une ou plusieurs fois avec des radicaux Z identiques ou différents, les autres substituants Z n'entraînant aucune ou aucune réticulation substantielle des monomères ou composés de cyclodextrine, les substituants Z étant de préférence sélectionnés dans le groupe composé de -OH, =O, -C(=O)OH, -C(=O)$OCH_3$, -C(=O)$OCH_2CH_3$, -$NH_2$, -NH($CH_3$), -N$(CH_3)_2$, -N($CH_3$)3*A, -NH($CH_2CH_3$), -N$(CH_2CH_3)_2$ et-N$(CH_2CH_3)_3^+A^-$ ; sachant que $A^-$ est un anion anorganique ou organique qui est de préférence sélectionné dans le groupe composé de $Cl^-$, $Br^-$, $I^-$, dihydrogène phosphate, hydrogène phosphate, phosphate, hydrogène carbonate, carbonate, acétate, lactate et citrate ou représente d'autres anions tolérés physiologiquement et les radicaux Y des groupes partants.

8. Copolymère selon une ou plusieurs des revendications 1 à 7, **caractérisé en ce que** les liants sont dérivés d'agents de réticulation bi- et/ou polyfonctionnels.

9. Copolymère selon une ou plusieurs des revendications 1 à 8, destiné à une utilisation dans le traitement de l'insuffisance rénale.

10. Copolymère selon une ou plusieurs des revendications 1 à 9, destiné à une utilisation dans un traitement par dialyse.

11. Solution d'hémodialyse ou de dialyse péritonéale contenant au moins un copolymère selon une ou plusieurs des revendications 1 à 10.

12. Kit qui est conçu pour la préparation de la solution d'hémodialyse ou de dialyse péritonéale selon la revendication 11, contenant

- un premier composant,
- un second composant et
- éventuellement un ou plusieurs autres composants ;

sachant que la solution d'hémodialyse ou de dialyse péritonéale selon la revendication 11 est obtenue par mélange du premier composant avec le second composant et éventuellement avec l'autre ou les autres composants.

13. Composition fixe appropriée pour la préparation de la solution d'hémodialyse ou de dialyse péritonéale selon la revendication 11, **caractérisée en ce que** la solution d'hémodialyse ou de dialyse péritonéale selon la revendication 11 est obtenue par dissolution de la composition fixe dans un solvant.

14. Utilisation d'au moins un copolymère selon une ou plusieurs des revendications 1 à 10 ou d'un kit selon la revendication 12 ou d'une composition fixe selon la revendication 13 pour la préparation de la solution d'hémodialyse ou de dialyse péritonéale selon la revendication 11.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4889634 A
- US 4535152 A
- US 6660804 B
- CN 101322932


### In der Beschreibung aufgeführte Nicht-Patentliteratur


- Allylamine-beta-cyclodextrin copolymer - A novel chiral selector for capillary electrophoresis. **CHIARI et al.** JOURNAL OF CHROMATOGRAPHY. ELSEVIER SCIENCE PUBLISHERS B.V, 2000, vol. 894, 95-103
- **A.D. JENKINS ; P. KRATOCHVÍL ; R.F.T. STEPTO ; U.W. SUTER.** *Glossary of basic terms in polymer science (IUPAC Recommendations,* 1996
- *Pure Appl. Chem.,* 1996, vol. 68 (12), 2287-2311
- **J. BLANCHARD ; S. PRONIUK.** *Some Important Considerations in the Use of Cyclodextrins; Pharm. Res.,* 1999, vol. 16 (12), 1796-1798
- *IUPAC Recommendations,* 1994
- *Pure Appl. Chem.,* 1994, vol. 66 (5), 1077-1184